# EUROPEAN PATENT APPLICATION

(11) **EP 4 365 181 A1**
(43) Date of publication of application: **08.05.2024**
(21) Application number: 22833683.0
(22) Date of filing: 30.06.2022
(51) Int. Cl.: C07H 19/056, A61K 31/7056, A61P 35/00

(54) **GALACTOSIDE DERIVATIVE AS GALECTIN-3 INHIBITOR**

(30) Priority: 30.06.2021 KR 20210085915
(71) Applicant: TiumBio Co., Ltd., Seongnam-si, Gyeonggi-do 13449 (KR)
(72) Inventor: LEE, Minhee, Seongnam-si, Gyeonggi-do 13449 (KR); CHOI, Younglok, Seongnam-si, Gyeonggi-do 13449 (KR); CHUNG, Yun Dong, Seongnam-si, Gyeonggi-do 13449 (KR); JEONG, Eun Il, Seongnam-si, Gyeonggi-do 13449 (KR); KIM, Da Young, Seongnam-si, Gyeonggi-do 13449 (KR); PARK, Jeong Su, Seongnam-si, Gyeonggi-do 13449 (KR); KIM, Seon Mi, Seongnam-si, Gyeonggi-do 13449 (KR); KIM, Hun-Taek, Seongnam-si, Gyeonggi-do 13449 (KR)
(74) Representative: Dehns
(86) International application number: PCT/KR2022/009465
(87) International publication number: WO 2023/277630

(57) **Abstract**

A galactoside derivative according to the present invention has the effect of inhibiting galectin-3 by having a structure in which a spiro ring is fused to C1, and appropriate substituents at other carbon positions. Therefore, the galactoside derivative of the present invention and a pharmaceutical composition comprising same are effective in the prevention or treatment of diseases or disorders such as galectin-3-related fibrosis and cancer.

## Description

### Technical Field

The present invention relates to a novel galactoside derivative, and a pharmaceutical use thereof as a galectin-3 inhibitor.

### Background Art

Galectin is a kind of lectin present in the living body of humans or animals and specifically binds to a glycoconjugate containing beta-galactose. To date, about 15 members of galectin family have been reported in mammals, and the galectin family starting with galectin-1 has a molecular weight of about 14 kDa to 36 kDa, which is present in the cytosol, nucleus, extracellular matrix, and the like, and is secreted from monocytes or macrophages into the bloodstream (JPharmacol Exp Ther 351:336-343). Most galectin consists of one or two distinct carbohydrate recognition domains (CRDs). The former case is classified as a proto type, and galectin-1, -2, -5, -7, -10, and the like fall under this category, and may be present as a monomer or a dimer under the influence of the surrounding environment. The latter case is classified as a tandem type, and galectin-4, -6, -8, -9, -12, and the like fall under this category, and have intrinsically dimeric forms. Galectin-3 is the only galectin classified as a chimera type consisting of a carbohydrate recognition domain and a long non-lectin domain among the galectin family, and may be present as a monomer or various types of oligomers depending on the surrounding conditions. The various glycosylated cell surface receptors, proteins and intercellular constituents act as ligands for galectin. Galectin-3 effectively and stably links these ligands based on structural characteristics, and may mediate enhancement of a cell signaling system, adhesion between cells, adhesion between cells and extracellular matrix, and the like.

In particular, the amount of galectin-3 in the extracellular matrix increases significantly under uncontrolled inflammation and pro-fibrosis conditions and contributes to the overactivation of macrophages, hyperproliferation and transformation of fibroblasts, and collagen production, and contributes to leading to the pathological condition. (Cell 76: 597-598; Immunological Reviews 230: 160-171). For example, galectin-3 binds to CD98 and integrin receptor (integrin αMβ2) on the surface of macrophage to activate the PI3K pathway, leading to macrophage polarization. Sustained activation of macrophage M2 triggers fibrosis by causing excessive release of pro-fibrotic mediators (TNF-α, IL-1, IL-4, IL-10, IL-13, IL-17, IL-33, CXCL9, FGF, fibronectin, and CCL18) as well as growth factors such as CTGF, TGF-β, TGF-α, PDGFα, and M-CSF from fibroblasts (Cell Commun. Signal. 16:89). In addition, since the activation of macrophages promotes the synthesis and secretion of galectin-3, it intensifies polarization due to positive feedback. The secreted galectin-3 plays an important role in converting myofibroblasts into the fibrosis phenotype by inducing cross-linking of modified glycans with TGF-β receptors to sustain receptor activation (Am. J. Pathol. 172:288-298). In addition to the action on macrophages, galectin-3 acts as an additional source of lectin for various types of cells involved in the development of fibrosis, thereby promoting the transformation into myofibroblasts and the activity through cell-cell or cell-extracellular matrix contact. (Tissue Barriers, 3:e1026505; PNAS 103:5060-5065). That is, fibroblasts, epithelial cells, and endothelial cells stimulate the activity and proliferation of myofibroblasts through fibroblast-myofibroblast transition (FMT), epithelial-mesenchymal transition (EMT), and endothelial-mesenchymal transition (EndoMT), respectively, and thus galectin-3 contributes to the production and accumulation of ECM (PLoS One 11:e0146887; Aging Dis. 10: 731-745). Galectin-3 is known to contribute to the development of fibrosis in organs such as the lung, liver, kidney, and heart, while suppressed galectin-3 contributes to the resolution of fibrosis (Allergol. Int. 2007, 56:57-65; J Am Coll Cardiol. 60:1249-56). The galectin-3 knock-out mouse showed resistance to various fibrosis-inducing stimuli to the liver, lung, heart, and kidney (Mol Med. 21:453-465; JACC Heart Fail. 3:59-67). When fibrosis was induced in normal mice, galectin-3 expression was increased temporally and spatially in proportion to the progression of fibrosis, but fibrosis was resolved upon administration of a galectin-3 inhibitor (Hypertension 66:767-75; PLoS One. 6:e18683). In addition, the increase in galectin-3 acts as a biomarker of poor prognosis in the treatment of patients with heart failure and pulmonary fibrosis (Int J Mol Med. 41:599-614; Biomolecules 10:389). Therefore, it can be seen that the regulation of galectin-3 has a potential use for the treatment of fibrosis.

Galectin-3, expressed in various cell types in tumor microenvironments, contributes to cancer development and deterioration (Biochim Biophys Acta 1863:427-437). Galectin-3 expression is high in related cancers such as stomach, liver, lung, bladder, head and neck, thyroid, colon, endometrium, thyroid, and breast, and in chronic lymphocytic leukemia and prostate cancer, galectin-3 expression in cancer cells is limited to the nucleus. Galectin-3 increases cell growth through anti-apoptotic function, is involved in angiogenesis as an important component of the tumor microenvironment acting on endothelial cells, and directly acts on tumor cell transformation, migration, invasion, and metastasis through cell adhesion control (J. Biol. Chem. 286:29913-29921). In fact, tumor size and metastasis increased in mice by induction of galectin-3, while they were reduced by galectin-3 knockout mice or treatment with galectin-3 inhibitors (Clin. Exp. Metastasis 28:451-462). Suppression of galectin-3 significantly enhanced tumor cell sensitivity to radiotherapy and pro-apoptotic drugs in vitro and in cell analysis (Mol Cancer Res. 7:1655-62). In addition, repression of galectin-3, which regulates the interaction between tumor cells and immune cells, T-lymphocytes, restored cytotoxic T-lymphocyte activity (Cancer Immunol. Res. 3:412-423; Nat. Commun. 7:12242). Therefore, a useful anticancer effect may be expected by inhibiting galectin-3.

### [PRIOR ART DOCUMENT]

### [Non-Patent Document]

1) J Pharmacol Exp Ther 351:336-343
2) Cell 76: 597-598
3) Immunological Reviews 230: 160-171
4) Cell Commun. Signal. 16:89
5) Tissue Barriers, 3:e1026505
6) PNAS 103:5060-5065
7) PLoS One 11:e0146887
8) Aging Dis. 10: 731-745
9) Allergol. Int. 2007, 56:57-65
10) Am. J. Pathol. 172:288-298
11) J Am Coll Cardiol. 60:1249-56
12) Mol Med. 21:453-465
13) JACC Heart Fail. 3:59-67
14) Hypertension 66:767-75
15) PLoS One. 6:e18683
16) Int J Mol Med. 41:599-614
17) Biomolecules 10:389
18) Biochim Biophys Acta 1863:427-437
19) Clin. Exp. Metastasis 28:451-462
20) J. Biol. Chem. 286:29913-29921
21) Mol Cancer Res. 7:1655-62
22) Cancer Immunol. Res. 3:412-423
23) Nat. Commun. 7:12242

### Disclosure of Invention

### Technical Problem

The present inventors have continued the research to develop a novel compound having galectin-3 inhibitory activity. As a result, the present inventors have confirmed that a galactoside derivative, specifically, the derivative having a structure in which a spiro ring is bonded to carbon 1 of the galactoside ring, has the effect of inhibiting the activity of galectin-3, thereby completing the present invention.

Accordingly, the object of the present invention is to provide galactoside derivatives having an inhibitory effect on galectin-3, and a pharmaceutical composition containing the same which is useful for preventing or treating diseases or disorders such as fibrosis and cancer associated with galectin-3.

### Solution to Problem

According to the above object, the present invention provides a compound represented by Formula (I) below, a stereoisomer, or a pharmaceutically acceptable salt thereof 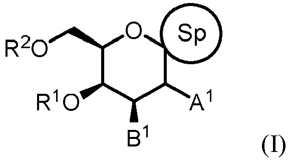 wherein Sp, A¹, B¹, R¹, and R² are the same as defined herein.

The present invention also provides a use of the compound represented by Formula (I) below, the stereoisomer, or the pharmaceutically acceptable salt thereof as a galectin-3 inhibitor.

### Advantageous Effects of Invention

According to the present invention, the compound represented by Formula (I), or the stereoisomer or the pharmaceutically acceptable salt thereof has a structure in which a spiro ring is bonded to carbon 1 and has an appropriate substituent at the carbon at another position, and thus has the effect of inhibiting galectin-3. Accordingly, the galactoside derivatives and the pharmaceutical composition containing the same according to the present invention are useful for preventing or treating diseases or disorders such as fibrosis and cancer associated with galectin-3.

### Best Mode for Carrying out the Invention

Hereinafter, the present invention will be described in more detail.

As used herein, the term "halogen" refers to F, Cl, Br, or I unless otherwise stated.

The term "alkyl," unless otherwise specified, refers to a linear or branched saturated hydrocarbon radical. For example, "C₁₋₁₀ alkyl" refers to alkyl having 1 to 10 carbon atoms. Specifically, C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, and the like.

The term "carbocycle" refers to an aromatic or non-aromatic hydrocarbon ring, which may be saturated or unsaturated, which may include a monocyclic ring or polycyclic ring. For example, "C₃₋₁₀ carbocycle" refers to a carbocycle having a skeleton formed of 3 to 10 carbon atoms. The carbocycle may include, but is not limited to, a C₃₋₁₅ carbocycle, a C₃₋₁₀ carbocycle, a C₃₋₇ carbocycle, and the like depending on the number of carbon atoms constituting the ring.

The term "heterocycle" refers to an aromatic or non-aromatic ring having one or more heteroatoms, which may be saturated or unsaturated, and may include a monocyclic ring or polycyclic ring. For example, "3- to 10-membered heterocycle" means a heterocycle including a total of 3 to 10 atoms constituting the skeleton, including heteroatoms and carbon atoms. The heterocycle may include, but is not limited to, a 3-15 membered heterocycle, a 3-10 membered heterocycle, a 3-7 membered heterocycle, and the like depending on the number of atoms constituting the ring.

The term "cycloalkyl" refers to a non-aromatic hydrocarbon ring radical, which may include a monocyclic ring or polycyclic ring. The cycloalkyl may include a saturated ring, and specific examples thereof may include cyclopropyl, cyclobutyl, cyclopentyl, cyclohexyl, adamantyl, and the like. Further, the cycloalkyl may include an unsaturated ring having one or more carbon-carbon double bonds in the ring as long as the ring is not rendered aromatic by the presence of the double bonds, and for example, cyclopentene or the like may also be included in the category. The cycloalkyl may include, but is not limited to, C₃₋₁₄ cycloalkyl, C₃₋₁₀ cycloalkyl, C₃₋₆ cycloalkyl, C₆₋₁₂ cycloalkyl, or the like depending on the number of carbon atoms constituting the ring.

The term "aryl" refers to an aromatic hydrocarbon ring radical, and specific examples may include phenyl, naphthyl, or the like. Further, the aryl may include a hydrocarbon ring radical in the category if it has at least one ring in which electrons are delocalized by a single bond and a double bond arranged alternately, that is, a conjugated π bond, and may also include, for example, a ring radical fused with benzene, such as, 1,2,3,4-tetrahydronaphthalenyl, or 1,3-dihydroindenyl. The aryl may include, but is not limited to, C₆₋₁₂ aryl, C₆₋₁₀ aryl, or the like depending on the number of carbon atoms constituting the ring.

The term "heteroaryl" refers to an aromatic heterocyclyl having one or more heteroatoms in the ring. Non-limiting examples of heteroaryl include pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinoyl, isoquinolyl, pyridazyl, pyrimidyl, pyrazil (each of which may have one or more substituents on the ring), and the like. Further, the heteroaryl may include a hydrocarbon ring radical in the category if it has at least one ring in which electrons are delocalized by a single bond and a double bond arranged alternately, that is, a conjugated π bond, and may also include, for example, a heterocyclic radical fused with benzene, such as, benzo[d][1,3]dioxolyl. The heteroaryl may include, but is not limited to, 5-to 14-membered heteroaryl, 5- to 10-membered heteroaryl, 5- to 6-membered heteroaryl, 6- to 12-membered heteroaryl, 6- to 10-membered heteroaryl, or the like depending on the number of atoms constituting the ring.

The term "heterocycloalkyl" refers to a non-aromatic heterocyclyl having one or more heteroatoms in the ring. The heterocycloalkyl may include a saturated ring. Further, the heterocycloalkyl may have one or more carbon-carbon double bonds or carbon-heteroatom double bonds in the ring as long as the ring is not rendered aromatic by the presence of the double bonds. Non-limiting examples of heterocycloalkyl include azetidinyl, aziridinyl, pyrrolidinyl, piperidinyl, piperazinyl, homopiperazinyl, morpholino, thiomorpholino, tetrahydrofuranyl, tetrahydrothiofuranyl, tetrahydropyranyl, pyranyl (each of which may have one or more substituents on the ring), and the like. The heterocycloalkyl may include, but is not limited to, 4- to 14-membered heterocycloalkyl, 4- to 10-membered heterocycloalkyl, 4- to 8-membered heterocycloalkyl, 5- to 14-membered heterocycloalkyl, 5- to 12-membered heterocycloalkyl, or the like depending on the number of atoms constituting the ring.

The term "heteroatom" refers to an atom other than carbon (C), specifically nitrogen (N), oxygen (O), or sulfur (S) atom. The above-mentioned heterocyclic ring, heteroaryl, and heterocycloalkyl contain one or more heteroatoms and may, for example, contain one heteroatom, 1 to 2, 1 to 3, or 1 to 4 heteroatoms.

The term "amido" refers to a -NHC(O)- or -C(O)NH- group, wherein the hydrogen atom may be substituted with another group such as alkyl.

The term "substitution" refers to replacing a hydrogen atom in a molecular structure with a substituent, such that the valence on the designated atom is not exceeded, and such that a chemically stable compound results from the substitution. For example, "group A is substituted with substituent B" or "group A has substituent B" means that a hydrogen atom bonded to an atom, such as carbon, which constitutes a skeleton of group A, is replaced with substituent B so that the group A and the substituent B form a covalent bond. Thus, it is substantially difficult or impossible for a group having no removable hydrogen atom to have a substituent. From this viewpoint, in a case where a range of combinations of various groups, which include groups that hardly have a substituent, with substituents are exemplified in the present specification, it should be interpreted that combinations of the groups, for which it is obvious that no substitution is possible, with the substituents are excluded from the range.

The term "moiety" refers to a part of the chemical structure, and for example, since a benzyl group is a group in which a phenyl group is substituted at a methyl group, it may be considered that a phenyl moiety and a methyl moiety are included in the benzyl group. Also, for example, it may be seen that the C₃₋₁₄ cycloalkyl-C₁₋₆ alkyl group includes a C₃₋₁₄ cycloalkyl moiety and a C₁₋₆ alkyl moiety.

In an aspect of the present invention, there is provided a compound represented by Formula (I) below, a stereoisomer, or a pharmaceutically acceptable salt thereof: 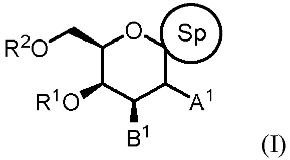
wherein Sp is an unsaturated or saturated C₃₋₁₅ monocyclic or polycyclic carbocycle or 3- to 15-membered heterocycle, and Sp has or does not have 1 to 3 substituents selected from among halogen, CN, NO₂, OH, oxo, Rx and Ry;
A¹ is H, halogen, CN, NO₂, OH, Rx, or Ry;
B¹ is halogen, CN, NO₂, Rx, or Ry;
R¹ and R² are each independently H or a metabolizable group in the body;
Rx is H, C₁₋₆ alkyl, C₂₋₆ alkenyl, C₂₋₆ alkynyl, C₃₋₁₄ cycloalkyl, 4- to 14-membered heterocycloalkyl, C₆₋₁₀ aryl, 5- to 14-membered heteroaryl, C₃₋₁₄ cycloalkyl-C₁₋₆ alkyl-, (4- to 14-membered heterocycloalkyl)-C₁₋₆ alkyl-, C₆₋₁₀ aryl-C₁₋₆ alkyl-, C₆₋₁₀ aryl-C₃₋₆ cycloalkyl-, (5- to 14-membered heteroaryl)-C₁₋₆ alkyl-, C₆₋₁₀ aryl-C₆₋₁₀ aryl-, C₆₋₁₀ aryl-C₆₋₁₀ aryl-C₁₋₆ alkyl, C₆₋₁₀ aryloxy-C₆₋₁₀ aryl-, C₆₋₁₀ aryl-(5- to 14-membered heteroaryl)-, or (5- to 14-membered heteroaryl)-C₆₋₁₀ aryl-C₁₋₆ alkyl-;
Ry's are each independently -ORx, -SRx, -NHORx, -N(OH)Rx, -C(O)Rx, -C(O)NRxRx, -C(O)ORx, -OC(O)Rx, -OC(RxRx)-C(O)Rx, -OC(RxRx)-C(O)ORx, -OC(O)NRxRx, - OC(RxRx)-C(O)NRxRx, -NRxRx, -NRxC(O)Rx, -NRxC(O)ORx, -NRxC(O)NRxRx, - C(=NRx)Rx, -C(=NRx)NRxRx, -NRxC(=NRx)NRxRx, -N=CRxRx, -NRxS(O)Rx, - NRxS(O)₂Rx, -NRxS(O)₂NRxRx, -S(O)Rx, -S(O)NRxRx, -S(O)₂Rx, -S(O)₂NRxRx, -OS(O)₂Rx, -PRxRx, -P(O)RxRx, -OP(O)RxRx, -OP(O)(ORx)₂, or -P(O)₂RxRx;
the C₁₋₆ alkyl moiety has or does not have 1 to 3 substituents selected from halogen; the C₃₋₁₄ cycloalkyl moiety, the 4- to 14-membered heterocycloalkyl moiety, the C₆₋₁₀ aryl moiety and the 5- to 14-membered heteroaryl moiety each independently include a monocyclic or polycyclic ring, and have or do not have 1 to 3 substituents selected from the group consisting of halogen, OH, CN, NO₂, oxo, C₁₋₆ alkylamido, C₁₋₆ alkoxycarbonyl, C₁₋₆ alkyl, haloC₁₋₆ alkyl, C₁₋₆ alkoxy, and haloC₁₋₆ alkoxy; and
the C₃₋₁₄ cycloalkyl moiety and the 4- to 14-membered heterocycloalkyl moiety are each independently saturated or unsaturated within a range having no aromaticity.

In Formula (I) above, Sp has, as a common atom, carbon 1 of a galactoside ring (*i.e*., a 6-membered ring containing an oxygen atom) and forms a spiro structure.

It should be understood that the substituents exemplified in the definition of Ry in Formula (I) above have a single bond or a double bond that meets the electrovalence of each atom even when a single bond or a double bond is not specifically indicated between the atoms constituting the substituents. For example, in the definition of the substituent Ry of Formula (I) above, Rx described in -ORx, -SRx, -NRxRx, or the like refers to Rx linked to a carbon atom, a sulfur atom, a nitrogen atom, or the like by a single bond, that is, may be represented by, for example, -O-Rx, -S-Rx, -N(-Rx)(-Rx), or the like. In addition, in the definition of the substituent Ry of Formula (I) above, (O) described in -C(O)Rx, -S(O)Rx, -P(O)RxRx, or the like refers to an oxygen atom linked to a carbon atom, a sulfur atom, a phosphorus atom, or the like by a double bond, that is, may be represented by, for example, -C(=O)Rx, -S(=O)Rx, - P(=O)RxRx, or the like.

In the definition of the substituent Ry of Formula (I) above, two or more Rx's may be the same or different from each other, and as an example, any one of Rx's may be H, and as another example, all of Rx's may be H, or as another example, none of Rx's may be H.

In an embodiment, Sp is 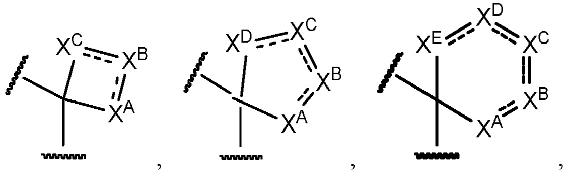 or 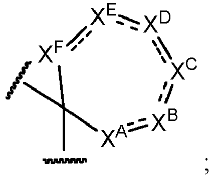
X^{A} to X^{F} are each independently an atom selected from the group consisting of C, O, N, and S, wherein the C atom has or does not have a substituent selected from halogen, CN, NO₂, oxo, Rx, Ry, and Y¹, the N atom has or does not have a substituent selected from Rx, Ry, and Y¹, and the S atom has or does not have an oxo substituent;
Y¹'s are each independently H or OH, or two adjacent Y¹'s may be linked to form an unsaturated or saturated C₃₋₇ carbocycle or a 3- to 7-membered heterocycle, wherein the C₃₋₇ carbocycle and the 3- to 7-membered heterocycle each have or do not have 1 to 3 substituents selected from among halogen, CN, NO₂, OH, oxo, Rx and Ry;
-̅ -̅ -̅ -̅ -̅ is a single bond or a double bond which meets an electrovalence of atoms each independently linked thereto; and
Rx and Ry are each the same as defined in Formula (I) above.

In the embodiment, the Sp ring may have at least one double bond at the position represented by -̅ -̅ -̅ -̅ -̅ (single bond or double bond), but in order to constitute a ring while meeting the electrovalence of an atom linked to -̅ -̅ -̅ -̅ -̅, double bonds cannot exist at the adjacent positions in the ring.

In other embodiments, Sp is selected from the group consisting of 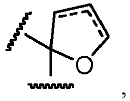 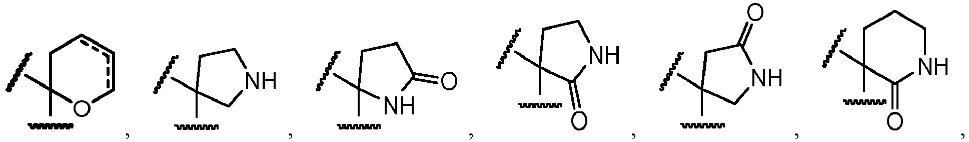 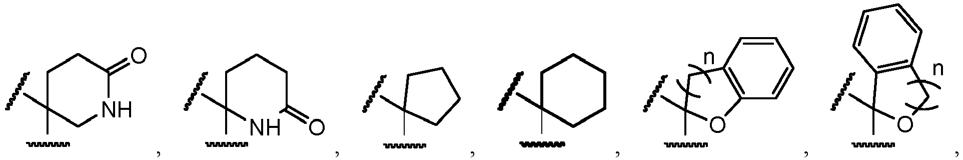 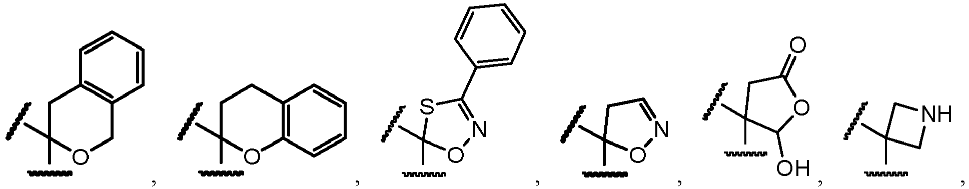 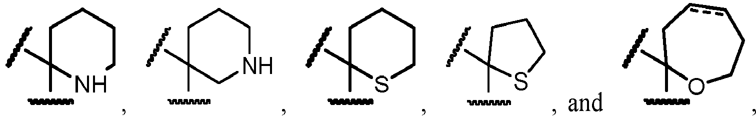 Sp has or does not have one to three substituents selected from among halogen, CN, NO₂, OH, Rx, and Ry;
-̅ -̅ -̅ -̅ -̅ is a single bond or a double bond which meets an electrovalence of atoms each independently linked thereto;
n is 1 or 2; and
Rx and Ry are each the same as defined in Formula (I) above.

In a specific embodiment, Rx's substituted at Sp are each independently C₁₋₆ alkyl, C₆₋₁₀ aryl, 5- to 14-membered heteroaryl, (4- to 14-membered heterocycloalkyl)-C₁₋₆ alkyl-, C₆₋₁₀ aryl-C₁₋₆ alkyl-, C₆₋₁₀ aryl-(5- to 14-membered heteroaryl)-, or (5- to 14-membered heteroaryl)-C₁₋₆ alkyl-;
Ry's substituted at Sp are each independently -ORx, -C(O)Rx, -NRxRx, -N=CRxRx, - NRxC(O)NRxRx, or -NRxC(O)Rx, wherein Rx's contained in Ry's are each independently H, C₁₋₆ alkyl, C₃₋₁₄ cycloalkyl, 4- to 14-membered heterocycloalkyl, C₆₋₁₀ aryl, 5- to 14-membered heteroaryl, C₃₋₁₄ cycloalkyl-C₁₋₆ alkyl-, (4- to 14-membered heterocycloalkyl)-C₁₋₆ alkyl-, C₆₋₁₀ aryl-C₁₋₆ alkyl-, C₆₋₁₀ aryl-C₃₋₆ cycloalkyl-, (5- to 14-membered heteroaryl)-C₁₋₆ alkyl-, C₆₋₁₀ aryl-C₆₋₁₀ aryl-, C₆₋₁₀ aryl-C₆₋₁₀ aryl-C₁₋₆ alkyl-, or C₆₋₁₀ aryloxy-C₆₋₁₀ aryl-; and
the C₁₋₆ alkyl moiety has or does not have 1 to 3 substituents selected from halogen; the C₃₋₁₄ cycloalkyl moiety, the 4- to 14-membered heterocycloalkyl moiety, the C₆₋₁₀ aryl moiety, and the 5- to 14-membered heteroaryl moiety each independently include a monocyclic or polycyclic ring, and have or do not have 1 to 3 substituents selected from the group consisting of halogen, OH, oxo, C₁₋₆ alkyl, haloC₁₋₆ alkyl, C₁₋₆ alkoxy, and haloC₁₋₆ alkoxy; and
the C₃₋₁₄ cycloalkyl moiety and the 4- to 14-membered heterocycloalkyl moiety are each independently saturated or unsaturated within a range having no aromaticity.

In another embodiment, A¹ is -ORx, -OC(O)Rx, -OC(RxRx)-C(O)Rx, -OC(RxRx)-C(O)ORx, -OC(O)NRxRx, -OC(RxRx)-C(O)NRxRx, -OS(O)₂Rx, or -OP(O)(ORx)₂; and Rx's are each independently the same as defined in Formula (I) above.

In a specific embodiment, Rx's included in A¹ are each independently H, C₁₋₆ alkyl, halo C₁₋₆ alkyl, 4- to 14-membered heterocycloalkyl, C₆₋₁₀ aryl, 5- to 14-membered heteroaryl, (4- to 14-membered heterocycloalkyl)-C₁₋₆ alkyl-, C₆₋₁₀ aryl-C₁₋₆ alkyl-, (5- to 14-membered heteroaryl)-C₁₋₆ alkyl-, or (5- to 14-membered heteroaryl)-C₆₋₁₀ aryl-C₁₋₆ alkyl-;
the C₁₋₆ alkyl moiety has or does not have 1 to 3 substituents selected from halogen; the C₃₋₁₄ cycloalkyl moiety, the 4- to 14-membered heterocycloalkyl moiety, the C₆₋₁₀ aryl moiety, and the 5- to 14-membered heteroaryl moiety each independently include a monocyclic or polycyclic ring, and have or do not have 1 to 3 substituents selected from the group consisting of halogen, OH, oxo, C₁₋₆ alkyl, haloC₁₋₆ alkyl, C₁₋₆ alkoxy, and haloC₁₋₆ alkoxy; and
the C₃₋₁₄ cycloalkyl moiety and the 4- to 14-membered heterocycloalkyl moiety are each independently saturated or unsaturated within a range having no aromaticity.

In another embodiment, B¹ is 5- to 14-membered heteroaryl containing one or more N atoms, 4- to 14-membered heterocycloalkyl containing one or more N atoms, C₆₋₁₀ aryl-(5- to 14-membered heteroaryl)-, C₆₋₁₀ aryl, (5- to 14-membered heteroaryl)-C₁₋₆ alkyloxy-, (4- to 14-membered heterocycloalkyl)-C₁₋₆ alkyloxy-, C₆₋₁₀ aryl-C₁₋₆ alkyloxy-, (5- to 14-membered heteroaryl)-amido-, (4- to 14-membered heterocycloalkyl)-amido-, C₆₋₁₀ aryl-C₁₋₆ amido-, (5- to 14-membered heteroaryl)-C₁₋₆ alkylamido-, (4- to 14-membered heterocycloalkyl)-C₁₋₆ alkylamido-, or C₆₋₁₀ aryl-C₁₋₆ alkylamido-, wherein with regard to the 5- to 14-membered heteroaryl and the 4- to 14-membered heterocycloalkyl containing one or more N atoms, any one among the N atoms is linked to the galactoside ring;
the 5- to 14-membered heteroaryl moiety, the 4- to 14-membered heterocycloalkyl moiety, and the C₆₋₁₀ aryl moiety in B¹ each independently include a monocyclic or polycyclic ring, and have or do not have 1 to 5 substituents selected from among halogen, OH, CF₃, CN, NO₂, oxo, and C₁₋₆ alkoxy; and
the 4- to 14-membered heterocycloalkyl moieties are each independently saturated or unsaturated within a range having no aromaticity.

In a specific embodiment, the 5- to 14-membered heteroaryl moiety in B¹ is imidazolyl, pyrazolyl, triazolyl, or chromenonyl.

In another embodiment, R¹ and R² are each independently H, carbamate, ether, phosphate, sulfate, oxyalkylphosphate, oxyalkyl sulfate, carbonate, amide, ester, *N-*acylsulfonamide, sulfonamide, imine, acyloxyalkylamine, phosphate, phsphoroimidate, carbonyloxymethyl, acetylthioethanol, dithioethanol, alkoxyalkylmonoester, or acetyl.

The present invention provides pharmaceutically acceptable salts of compounds represented by Formula (I) above.

The pharmaceutically acceptable salts should have low toxicity to humans and should not have any adverse effects on the biological activity and physicochemical properties of the parent compound. For example, the pharmaceutically acceptable salt may be an acid addition salt formed by a pharmaceutically acceptable free acid.

As the free acid, inorganic acids or organic acids may be used. The inorganic acid may be hydrochloric acid, sulfuric acid, nitric acid, phosphoric acid, perchloric acid, bromic acid, and the like. The organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like. The acid addition salt may be obtained by a conventional method, for example, by dissolving the compound of Formula (I) above in an excess amount of an acidic aqueous solution and precipitating the salt using a water-miscible organic solvent such as methanol, ethanol, acetone, or acetonitrile.

In addition, the pharmaceutically acceptable salt may be an alkali metal salt (e.g., sodium salt) or an alkaline earth metal salt (e.g., potassium salt). The alkali metal salt or alkaline earth metal salt may be obtained, for example, by dissolving the compound of Formula (I) above in an excess amount of alkali metal hydroxide or alkaline earth metal hydroxide solution, filtering off the undissolved compound salt, and evaporating and drying the filtrate.

Further, the compounds of the present invention may have a chiral carbon center and thus may exist in the form of R or S isomers, racemic compounds, individual enantiomers or mixtures thereof, individual diastereomers or mixtures thereof. All such stereoisomers and mixtures thereof may fall within the scope of the present invention.

In addition, the compounds of the present invention may include hydrates and solvates of the compounds of Formula (I). The hydrates and solvates may be prepared using known methods and are preferably non-toxic and water-soluble. In particular, the hydrates and the solvates may preferably be those obtained by binding, to the compound, 1 to 5 molecules of water and alcoholic solvent (in particular, ethanol or the like), respectively.

In a specific embodiment, the compounds of Formula (I) are compounds selected from the following group, a stereoisomer thereof, or a pharmaceutically acceptable salt:
1) <A08> 3-((((2*R*,3'*R*,4'*S*,5'*S*,6'*R*)-3',5'-dihydroxy-6'-(hydroxymethyl)-6-methoxy-3',4', 5', 6'-tetrahydrospiro [chromane-2, 2'-pyran]-4'-yl)oxy)methyl)-5, 6-difluoro-2*H*-chromen-2-one;
2) <A09> 3-((((5*S*,7*R*,8*S*,9*S*,10*R*)-8,10-dihydroxy-7-(hydroxymethyl)-1,6-dioxaspiro[4.5]decan-9-yl)oxy)methyl)-5,6-difluoro-2*H*-chromen-2-one;
3) <A10,A11> (3'*R*,4'*S*,5'*R*,6'*R*)-6'-(hydroxymethyl)-6-methoxy-4'-(4-(3,4,5-trifluorophenyl)-1*H*-1,2,3-triazol-1-yl)-3',4',5',6'-tetrahydrospiro[chromane-2,2'-pyran]-3',5'-diol;
4) <A12> (5*S*,7*R*,8*R*,9*S*,10*R*)-7-(hydroxymethyl)-9-(4-(3,4,5-trifluorophenyl)-1*H-*1,2,3-triazol-1-yl)-1,6-dioxaspiro[4.5]decane-8,10-diol;
5)<A13> (5*S*,7*R*,8*R*,9*S*,10*R*)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol;
6)<A16> (5*S*,7*R*,8*R*,9*S*,10*R*)-7-(hydroxymethyl)-10-(pyridin-3-ylmethoxy)-9-(4-(3,4,5-trifluorophenyl)-1*H*-1,2,3-triazol-1-yl)-1,6-dioxaspiro[4.5]decan-8-ol;
7)<A17> (5*S*,7*R*,8*R*,9*S*,10*R*)-7-(hydroxymethyl)-10-(pyridin-2-ylmethoxy)-9-(4-(3,4,5-trifluorophenyl)-1*H*-1,2,3-triazol-1-yl)-1,6-dioxaspiro[4.5]decan-8-ol;
8)<A18> (5*S*,7*R*,8*R*,9*S*,10*R*)-7-(hydroxymethyl)-10-(pyridin-4-ylmethoxy)-9-(4-(3,4,5-trifluorophenyl)-1H-1,2,3-triazol-1-yl)-1,6-dioxaspiro[4.5]decan-8-ol;
9)<A19> (5*S*,7*R*,8*R*,9*S*,10*R*)-7-(hydroxymethyl)-10-((5-(trifluoromethyl)furan-2-yl)methoxy)-9-(4-(3,4,5-trifluorophenyl)-1*H*-1,2,3-triazol-1-yl)-1,6-dioxaspiro[4.5]decane-8-ol;
10) <A20> 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-1-phenylethanone;
11) <A21> 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)acetic acid;
12)<A22> 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-1-(4-hydroxypiperidin 1-yl)ethanone;
13)<A23> 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-*N-*phenylacetamide;
14)<A24> (2*R*,3*R*,4*S*,5*R*,6*S*)-2-(hydroxymethyl)-4-(4-(3,4,5-trifluorophenyl)-1*H*-1,2,3-triazol-1-yl)-1,7-dioxaspiro[5.5]undecane-3,5-diol;
15)<A25> (2*R*,3*R*,4*S*,5*R*,6*S*)-5-(benzyloxy)-2-(hydroxymethyl)-4-(4-(3,4,5-trifluorophenyl)-1*H*-1,2,3-triazol-1-yl)-1,7-dioxaspiro[5.5]undecan-3-ol;
16)<A26> 4-((((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)methyl)tetrahydro-2*H*-thiopyran 1,1-dioxide;
17)<A27> (5*S*,7*R*,8*R*,9*S*,10*R*)-10-(2,2-difluoroethoxy)-7-(hydroxymethyl)-9-(4-(3,4,5-trifluorophenyl)-1*H*-1,2,3-triazol-1-yl)-1,6-dioxaspiro[4.5]decan-8-ol;
18)<A28> (5*S*,7*R*,8*R*,9*S*,10*R*)-7-(hydroxymethyl)-10-((4-(2-methyl-2*H*-tetrazol-5-yl)benzyl)oxy)-9-(4-(3,4,5-trifluorophenyl)-1*H*-1,2,3-triazol-1-yl)-1,6-dioxaspiro[4.5]decan-8-ol;
19)<A29> *N*-(6-chloropyridazin-3-yl)-2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)acetamide;
20)<A30> (4*S*,5*S*,7*R*,8*R*,9*S*,10*R*)-7-(hydroxymethyl)-4-phenyl-9-(4-(3,4,5-trifluorophenyl)-1*H*-1,2,3-triazol-1-yl)-1,6-dioxaspiro[4.5]decane-8,10-diol;
21)<A31> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl benzoate;
22)<A32> methyl-2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)acetate;
23)<A33> 1-(3,3-difluoroazetidin-1-yl)-2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)ethanone;
24)<A34> 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-1-(3-hydroxyazetidin-1-yl)ethanone;
25)<A35> (5*S*,7*R*,8*R*,9*S*,10*R*)-10-(benzo[*d*][1,3]dioxol-5-ylmethoxy)-7-(hydroxymethyl)-9-(4-(3,4,5-trifluorophenyl)-1*H*-1,2,3-triazol-1-yl)-1,6-dioxaspiro[4.5]decan-8-ol;
26)<A36> (5*S*,7*R*,8*R*,9*S*,10*R*)-10-((2*H*-tetrazol-5-yl)methoxy)-7-(hydroxymethyl)-9-(4-(3,4,5-trifluorophenyl)-1*H*-1,2,3-triazol-1-yl)-1,6-dioxaspiro[4.5]decan-8-ol;
27)<A37> (5*S*,7*R*,8*R*,9*S*,10*R*)-10-((2-hydroxybenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
28)<A38> (5*S*,7*R*,8*R*,9*S*,10*R*)-10-((3-hydroxybenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
29)<A39> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3-fluorobenzoate;
30)<A40> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3-methoxybenzoate;
31) <A41> (5*S*,7*R*,8*R*,9*S*,10*S*)-7-(hydroxymethyl)-9-(4-(3,4,5-trifluorophenyl)-1*H-*1,2,3-triazol-1-yl)-1,6-dioxaspiro[4.5]decane-8,10-diol;
32)<A42> (5*S*,7*R*,8*R*,9*S*,10*S*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl benzoate;
33)<A44> (2*R*,3*R*,4*S*,5*R*,6*S*)-5-(benzo[*d*][1,3]dioxol-5-ylmethoxy)-2-(hydroxymethyl)-4-(4-(3,4,5-trifluorophenyl)-1*H*-1,2,3-triazol-1-yl)-1,7-dioxaspiro[5.5]undecan-3-ol;
34)<A45> (2*R*,3*R*,4*S*,5*R*,6*S*)-5-((2*H*-tetrazol-5-yl)methoxy)-2-(hydroxymethyl)-4-(4-(3,4,5-trifluorophenyl)-1*H*-1,2,3-triazol-1-yl)-1,7-dioxaspiro[5.5]undecan-3-ol;
35)<A46> (2*R*,3*R*,4*S*,5*R*,6*S*)-5-(2,2-difluoroethoxy)-2-(hydroxymethyl)-4-(4-(3,4,5-trifluorophenyl)-1*H*-1,2,3-triazol-1-yl)-1,7-dioxaspiro[5.5]undecan-3-ol;
36)<A47> (2*R*,3*R*,4*S*,5*R*,6*S*)-5-((2-hydroxybenzyl)oxy)-2-(hydroxymethyl)-4-(4-(3,4,5-trifluorophenyl)-1*H*-1,2,3-triazol-1-yl)-1,7-dioxaspiro[5.5]undecan-3-ol;
37)<A48> (2*R*,3*R*,4*S*,5*R*,6*S*)-5-((3-fluorobenzyl)oxy)-2-(hydroxymethyl)-4-(4-(3,4,5-trifluorophenyl)-1*H*-1,2,3-triazol-1-yl)-1,7-dioxaspiro[5.5]undecan-3-ol;
38)<A49> (2*R*,3*R*,4*S*,5*R*,6*S*)-2-(hydroxymethyl)-5-((3-(trifluoromethyl)benzyl)oxy)-4-(4-(3,4,5-trifluorophenyl)-1*H*-1,2,3-triazol-1-yl)-1,7-dioxaspiro[5.5]undecan-3-ol;
39)<A50> (2*R*,3*R*,4*S*,5*R*,6*S*)-2-(hydroxymethyl)-5-(pyridin-2-ylmethoxy)-4-(4-(3,4,5-trifluorophenyl)-1*H*-1,2,3-triazol-1-yl)-1,7-dioxaspiro[5.5]undecan-3-ol;
40) <A51> (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl 3-fluorobenzoate;
41)<A52> (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl 3-(trifluoromethyl)benzoate;
42) < A5 3 > (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl 3-methoxybenzoate;
43)<A54> 2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)acetic acid;
44)<A55> 2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)propanoic acid;
45)<A56> (4*R*,5*S*,7*R*,8*R*,9*S*,10*R*)-4-amino-7-(hydroxymethyl)-9-(4-(3,4,5-trifluorophenyl)-1*H*-1,2,3-triazol-1-yl)-1,6-dioxaspiro[4.5]decane-8,10-diol;
46)<A57> (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl picolinate;
47)<A58> (2*R*,3*R*,4*S*,5*R*,6*S*)-2-(hydroxymethyl)-5-(pyridin-3-ylmethoxy)-4-(4-(3,4,5-trifluorophenyl)-1*H*-1,2,3-triazol-1-yl)-1,7-dioxaspiro[5.5]undecan-3-ol;
48)<A59> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 4-methylbenzenesulfonate;
49)<A60> *N*-((4*R*,5*S,*7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-fluorobenzamide;
50)<A61> (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl nicotinate;
51)<A62> (4*R*,5*S*,7*R*,8*R*,9*S*,10*R*)-4-((3-fluorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
52)<A63> (4*R*,5*S*,7*R*,8*R*,9*S*,10*R*)-4-((2,3-difluoro-6-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
53)<A64> (5*S*,7*R*,8*R*,9*S*,10*R*)-10-((3-chlorobenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
54)<A65> (5*S*,7*R*,8*R*,9*S*,10*R*)-10-((2,3-difluorobenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
55)<A66> 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)propanoic acid;
56)<A69> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate;
57)<A70> (5*S*,7R,8*R*,9*S*,10*R*)-S-hydroxy-7-(hydroxymethyl)-9-(4-(3,4,5-trifluorophenyl)-1*H*-1,2,3-triazol-1-yl)-1,6-dioxaspiro[4.5]decan-10-yl 2-chlorobenzoate;
58) <A71> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2,3-difluorobenzoate;
59)<A72> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl picolinate;
60)<A73> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3-(trifluoromethyl)benzoate;
61) <A74> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3-(trifluoromethyl)benzoate;
62)<A75> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl nicotinate;
63)<A76> (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl dihydrogen phosphate;
64)<A77> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3-fluoropicolinate;
65)<A78> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2,4-difluorobenzoate;
66)<A79> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 4-fluorobenzoate;
67)<A80> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl isonicotinate;
68)<A81> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3-fluoropicolinate;
69)<A82> (*R*)-2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)propanoic acid;
70)<A83> (*R*)-2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)propanoic acid;
71)<A84> (5S*,*7*R*,8*R*,9*S*,10*R*)-10-((5-fluoropyridin-3-yl)methoxy)-7-(hydroxymethyl)-9-(4-(3,4,5-trifluorophenyl)-1*H*-1,2,3-triazol-1-yl)-1,6-dioxaspiro[4.5]decan-8-ol;
72)<A85> (4*R*,5*S*,7*R*,8*R*,9*S*,10*R*)-7-(hydroxymethyl)-4-((pyridin-3-ylmethyl)amino)-9-(4-(3,4,5-trifluorophenyl)-1*H*-1,2,3-triazol-1-yl)-1,6-dioxaspiro[4.5]decane-8,10-diol;
73)<A86> (4*R*,5*S*,7*R*,8*R*,9*S*,10*R*)-4-((3-chlorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
74)<A87> (4*R*,5*S*,7*R*,8*R*,9*S*,10*R*)-4-((2-fluorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
75)<A88,A89> (2*R*,3*R*,4*S*,5*R*)-3,5-dihydroxy-2-(hydroxymethyl)-4-(4-(3,4,5-trifluorophenyl)-1*H*-1,2,3-triazol-1-yl)-1-oxa-7-azaspiro[5.5]undecan-8-one;
76)<A90> (5*S*,7*R*,8*R*,9*S*,10*R*)-7-(hydroxymethyl)-10-((3-(trifluoromethyl)benzyl)oxy)-9-(4-(3,4,5-trifluorophenyl)-1*H*-1,2,3-triazol-1-yl)-1,6-dioxaspiro[4.5]decan-8-ol;
77) <A91> (5*S*,7*R*,8*R*,9*S*,10*R*)-10-((3-fluorobenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
78)<A92> (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 3,5-difluorobenzoate;
79)<A93> (2*R*,3*R*,4*S*,5*R*)-5-(benzyloxy)-3-hydroxy-2-(hydroxymethyl)-4-(4-(3,4,5-trifluorophenyl)-1*H*-1,2,3-triazol-1-yl)-1-oxa-7-azaspiro[5.5]undecan-8-one;
80)<A94> (2*R*,3*R*,4*S*,5*R*)-7-(2,2-difluoroethyl)-3,5-dihydroxy-2-(hydroxymethyl)-4-(4-(3,4,5-trifluorophenyl)-1*H*-1,2,3-triazol-1-yl)-1-oxa-7-azaspiro[5.5]undecan-8-one;
81) <A95> (2*R*,3*R*,4*S*,5*R*)-7-((1,1-dioxidotetrahydro-2*H*-thiopyran-4-yl)methyl)-3,5-dihydroxy-2-(hydroxymethyl)-4-(4-(3,4,5-trifluorophenyl)-1*H*-1,2,3-triazol-1-yl)-1-oxa-7-azaspiro[5.5]undecan-8-one;
82)<A96> (4*R*,5*S*,7*R*,8*R*,9*S*,10*R*)-7-(hydroxymethyl)-4-((3-(trifluoromethyl)benzyl)amino)-9-(4-(3,4, 5-trifluorophenyl)-1*H*-1,2,3 -triazol-1-yl)-1, 6-dioxaspiro[4.5]decane-8,10-diol;
83)<A97> (4*R*,5*S*,7*R*,8*R*,9*S*,10*R*)-4-((4-chlorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
84)<A98> (4*R*,5*S*,7*R*,8*R*,9*S*,10*R*)-7-(hydroxymethyl)-4-((pyridin-2-ylmethyl)amino)-9-(4-(3,4,5-trifluorophenyl)-1*H*-1,2,3-triazol-1-yl)-1,6-dioxaspiro[4.5]decane-8,10-diol;
85)<A99> (4*R*,5*S*,7*R*,8*R*,9*S*,10*R*)-7-(hydroxymethyl)-4-((pyridin-4-ylmethyl)amino)-9-(4-(3,4,5-trifluorophenyl)-1*H*-1,2,3-triazol-1-yl)-1,6-dioxaspiro[4.5]decane-8,10-diol;
86)<A100> (5*R*,7*R*,8*R*,9*S*,10*R*)-7-(hydroxymethyl)-9-(4-(3,4,5-trifluorophenyl)-1*H-*1,2,3-triazol-1-yl)-1,6-dioxaspiro[4.5]decane-8,10-diol;
87)<A101> (7*R*,8*R*,9*S*,10*R*)-7-(hydroxymethyl)-9-(4-(3,4,5-trifluorophenyl)-1*H*-1,2,3-triazol-1-yl)-6-oxaspiro[4.5]decane-8,10-diol;
88)<A102> (4*R*,5*S*,7*R*,8*R*,9*S*,10*R*)-4-((3-bromobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
89)<A103> (4*R*,5*S*,7*R*,8*R*,9*S*,10*R*)-4-((3-chlorobenzyl)(methyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
90)<A104> (4*R*,5*S*,7*R*,8*R*,9*S*,10*R*)-4-((cyclopentylmethyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
91)<A105> (4*R*,5*S*,7*R*,8*R*,9*S*,10*R*)-4-(benzylamino)-7-(hydroxymethyl)-9-(4-(3,4,5-trifluorophenyl)-1*H*-1,2,3-triazol-1-yl)-1,6-dioxaspiro[4.5]decane-8,10-diol;
92)<A106> 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
93)<A107> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)cyclopentanecarboxamide;
94)<A108> 2-cyclopentyl-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
95)<A109> (2*R*,3*R*,4*S*,5*R*,6*R*)-2-(hydroxymethyl)-4-(4-(3,4,5-trifluorophenyl)-1*H-*1,2,3-triazol-1-yl)-1-oxa-7-thiaspiro[5.5]undecane-3,5-diol;
96)<A110> (2*R*,3*R*,4*S*,5*R*,6*R*)-2-(hydroxymethyl)-4-(4-(3,4,5-trifluorophenyl)-1*H-*1,2,3-triazol-1-yl)-1-oxa-8-azaspiro[5.5]undecane-3,5-diol;
97)<A111> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(naphthalen-1-yl)acetamide;
98)<A112> 2-(2-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
99)<A113> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-fluorophenyl)acetamide;
100)<A114> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-phenylcyclopropanecarboxamide;
101)<A115> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(naphthalen-2-yl)acetamide;
102)<A116> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-methoxyphenyl)acetamide;
103)<A117> 2-(4-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
104)<A118> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-methoxybenzamide;
105)<A119> 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzamide;
106)<A120> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-phenoxybenzamide;
107)<A121> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-2-carboxamide;
108)<A122> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-(trifluoromethoxy)benzamide;
109)<A123> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-2-carboxamide;
110)<A124> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-2-carboxamide;
111)<A125> (4*R*,5*S*,7*R*,8*R*,9*S*,10*R*)-4-((2-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
112)<A126> (4*R*,5*S*,7*R*,8*R*,9*S*,10*R*)-4-((5-chloro-2-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
113)<A127> (4*R*,5*S*,7*R*,8*R*,9*S*,10*R*)-4-((3-chloro-2-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
114)<A128> (4*R*,5*S*,7*R*,8*R*,9*S*,10*R*)-4-(((5-chlorofuran-2-yl)methyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
115)<A129> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
116)<A130> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-phenoxybenzamide;
117)<A131> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-4-carboxamide;
118)<A132> (4*R*,5*S*,7*R*,8*R*,9*S*,10*R*)-4-((2-hydroxy-3-methoxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
119)<A133> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
120)<A134> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-3-carboxamide;
121)<A135> 2-([1,1'-biphenyl]-4-yl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
122)<A136> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-(trifluoromethyl)phenyl)acetamide;
123)<A137> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-phenylpropanamide;
124) < A13 8> ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(phenyl)methanone;
125)<A139> 2-(3-chlorophenyl)-1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)ethanone;
126)<A140> 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-(3-methoxyphenyl)ethanone;
127)<A141> (2*R*,3*R*,4*S*,5*R*,6*R*)-8-benzyl-2-(hydroxymethyl)-4-(4-(3,4,5-trifluorophenyl)-1*H*-1,2,3-triazol-1-yl)-1-oxa-8-azaspiro[5.5]undecane-3,5-diol;
128)<A142> (2*R*,3*R*,4*S*,5*R*,6*R*)-2-(hydroxymethyl)-8-(pyridin-3-ylmethyl)-4-(4-(3,4,5-trifluorophenyl)-1*H*-1,2,3-triazol-1-yl)-1-oxa-8-azaspiro[5.5]undecane-3,5-diol;
129)<A143> (2*R*,3*R*,4*S*,5*R*,6*R*)-8-(3-chlorobenzyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
130)<A144> ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(pyridin-3-yl)methanone;
131)<A145> (3-chlorophenyl)((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)methanone;
132)<A146> *N*-((4*R*,5*S*,7*R*,8*R*,9*S,*10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-phenylacetamide;
133)<A147> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2,2-difluoro-2-phenylacetamide;
134)<A148> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(4-phenoxyphenyl)acetamide;
135)<A149> 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
136)<A150> 3-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzamide;
137)<A151> 2-(3-bromophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
138)<A152> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-(trifluoromethyl)benzamide;
139)<A153> (4*R*,5*S*,7*R*,8*R*,9*S*,10*R*)-7-(hydroxymethyl)-4-(1*H*-1,2,3-triazol-1-yl)-9-(4-(3,4,5-trifluorophenyl)-1*H*-1,2,3-triazol-1-yl)-1,6-dioxaspiro[4.5]decane-8,10-diol;
140)<A154> (4*R*,5*S*,7*R*,8*R*,9*S*,10*R*)-7-(hydroxymethyl)-4-(4-phenyl-1*H*-1,2,3-triazol-1-yl)-9-(4-(3,4,5-trifluorophenyl)-1*H*-1,2,3-triazol-1-yl)-1,6-dioxaspiro[4.5]decane-8,10-diol;
141)<A155> (4*R*,5*S,*7*R*,8*R*,9*S*,10*R*)-7-(hydroxymethyl)-4-((3-methoxybenzyl)amino)-9-(4-(3,4,5-trifluorophenyl)-1*H*-1,2,3-triazol-1-yl)-1,6-dioxaspiro[4.5]decane-8,10-diol;
142)<A156> 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-2-carboxamide;
143)<A157> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-3-carboxamide;
144)<A158> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-5,6,7,8-tetrahydronaphthalene-1 -carboxamide;
145)<A159> 7-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-2-carboxamide;
146)<A160> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1,2,3,4-tetrahydronaphthalene-1 -carboxamide;
147)<A161> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-3-carboxamide;
148)<A162> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2,3-dihydro-1*H*-indene-4-carboxamide;
149)<A163> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2,3-dihydro-1*H*-indene-1-carboxamide;
150)<A164> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2'-fluoro-[1,1'-biphenyl]-3-carboxamide;
151)<A165> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide;
152)<A166> 3'-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-3-carboxamide;
153)<A167, A168> 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)propanamide;
154)<A169> 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
155)<A170> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1-naphthamide;
156)<A171> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-phenoxybenzamide;
157)<A172> 4-chloro-*N*-((4*R*,5*S*,7*R*,8R,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
158)<A173> 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
159)<A174> (2*R*,3*R*,4*S*,5*R*,6*R*)-8-(3-chlorobenzyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
160)<A175> (2*R*,3*R*,4*S*,5*R*,6*R*)-8-(3-chlorobenzyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
161)<A176> 4-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
162)<A177> 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-3-carboxamide;
163)<A178> 3-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
164)<A179> 2'-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-3-carboxamide;
165)<A180> 5-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-3-carboxamide;
166)<A181> 3-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-2-carboxamide;
167)<A182> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*d*]isothiazole-3-carboxamide;
168)<A183> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-phenoxyphenyl)acetamide;
169)<A184> 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone;
170)<A185> ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(naphthalen-1-yl)methanone;
171)<A186> ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(naphthalen-2-yl)methanone;
172)<A187> benzo[*b*]thiophen-2-yl((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)methanone;
173)<A188> ((2*R*,3*R*,4*S*,5*S*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(pyridin-2-yl)methanone;
174)<A189> ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(pyridin-4-yl)methanone;
175)<A190> ((2*R*,3*R*,4*S*,5*S*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(thiazol-5-yl)methanone;
176)<A191> (2*R*,3*R*,4*S*,5*S*,6*R*)-2-(hydroxymethyl)-8-phenyl-4-(4-(3,4,5-trifluorophenyl)-1*H*-1,2,3-triazol-1-yl)-1-oxa-8-azaspiro[5.5]undecane-3,5-diol;
177)<A192> 5-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
178)<A193> 1-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
179)<A194> *N-*((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*d*]thiazole-4-carboxamide;
180)<A195> 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
181)<A196> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-5-carboxamide;
182)<A197> 6-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*d*]isothiazole-3-carboxamide;
183)<A198> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-4-carboxamide;
184)<A199> 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*d*]isoxazole-3-carboxamide;
185)<A200> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)imidazo[2,1-*b*]thiazole-5-carboxamide;
186)<A201> 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
187)<A202> (4*R*,5*S*,7*R*,8*R*,9*S*,10*R*)-7-(hydroxymethyl)-4-((*Z*)-((2-hydroxynaphthalen-1-yl)methylene)amino)-9-(4-(3,4,5-trifluorophenyl)-1*H*-1,2,3-triazol-1-yl)-1,6-dioxaspiro[4.5]decane-8,10-diol;
188)<A203> (4*R*,5*S*,7*R*,8*R*,9*S*,10*R*)-7-(hydroxymethyl)-4-((*Z*)-((1-hydroxynaphthalen--yl)methylene)amino)-9-(4-(3,4, 5-trifluorophenyl)-1*H*-1,2,3-triazol-1-yl)-1, 6-dioxaspiro[4.5]decane-8,10-diol;
189)<A204> (4*R*,5*S*,7*R*,8*R*,9*S*,10*R*)-4-(4-(2-chlorophenyl)-1*H*-1,2,3-triazol-1-yl)-7-(hydroxymethyl)-9-(4-(3,4,5-trifluorophenyl)-1*H*-1,2,3-triazol-1-yl)-1,6-dioxaspiro[4.5]decane-8,10-diol;
190)<A205> (4*R*,5*S*,7*R*,8*R*,9*S*,10*R*)-4-(4-(3-chlorophenyl)-1*H*-1,2,3-triazol--yl)-7-(hydroxymethyl)-9-(4-(3,4,5-trifluorophenyl)-1*H*-1,2,3-triazol-1-yl)-1,6-dioxaspiro[4.5]decane-8,10-diol;
191)<A206> (4*R*,5*S*,7*R*,8*R*,9*S*,10*R*)-7-(hydroxymethyl)-4-(phenylamino)-9-(4-(3,4,5-trifluorophenyl)-1*H*-1,2,3-triazol-1-yl)-1,6-dioxaspiro[4.5]decane-8,10-diol;
192)<A207> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-4-carboxamide;
193)<A208> 3-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
194)<A209> 6-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-4-carboxamide;
195)<A210> *N-*((4*R*,5*S*,7*R*,8*R*,9*S*,10*R*)-10-((2*H*-tetrazol-5-yl)methoxy)-8-hydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1 -naphthamide;
196)<A211> 7-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
197)<A212> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
198)<A213> 8-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
199)<A214> (5*S*,7*R*,8*R*,9*S*,10*R*)-7-(hydroxymethyl)-3-methyl-9-(4-(3,4,5-trifluorophenyl)-1*H*-1,2,3-triazol-1-yl)-1,6-dioxaspiro[4.5]decane-8,10-diol;
200)<A215> 1-(3-chlorophenyl)-3-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)urea;
201)<A216> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-fluoroquinoline-5-carboxamide;
202)<A217> 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
203)<A218> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylbenzofuran-3-carboxamide;
204)<A219> 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1*H*-indole-3-carboxamide;
205)<A220> (2*R*,3*R*,4*S*,5*S*,6*R*)-2-(hydroxymethyl)-8-(naphthalen-1-yl)-4-(4-(3,4,5-trifluorophenyl)-1*H*-1,2,3-triazol-1-yl)-1-oxa-8-azaspiro[5.5]undecane-3,5-diol;
206)<A221> (2*R*,3*R*,4*S*,5*R*,6*R*)-2-(hydroxymethyl)-8-(naphthalen-2-yl)-4-(4-(3,4,5-trifluorophenyl)-1*H*-1,2,3-triazol-1-yl)-1-oxa-8-azaspiro[5.5]undecane-3,5-diol;
207)<A222> (2*R*,3*R*,4*S*,5*S*,6*R*)-8-(2-chlorophenyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
208)<A223> (2*R*,3*R*,4*S*,5*S*,6*R*)-8-(3-chlorophenyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
209)<A224> (2*R*,3*R*,4*S*,5*S*,6*R*)-8-(4-chlorophenyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
210)<A225> (2*R*,3*R*,4*S*,5*R*,6*R*)-2-(hydroxymethyl)-8-(thiophen-2-yl)-4-(4-(3,4,5-trifluorophenyl)-1*H*-1,2,3-triazol-1-yl)-1-oxa-8-azaspiro[5.5]undecane-3,5-diol;
211) <A226> (2*R*,3*R*,4*S*,5*S*,6*R*)-2-(hydroxymethyl)-8-(pyrimidin-4-yl)-4-(4-(3,4,5-trifluorophenyl)-1*H*-1,2,3-triazol-1-yl)-1-oxa-8-azaspiro[5.5]undecane-3,5-diol;
212)<A227> (4-chlorophenyl)((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)methanone;
213)<A228> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-6-fluoro-2-methylquinoline-4-carboxamide;
214)<A229> 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylquinoline-4-carboxamide;
215)<A230> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-4-carboxamide;
216)<A231> 6-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylquinoline-4-carboxamide;
217)<A232> 4-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1*H*-indole-3-carboxamide;
218)<A233> 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-methyl-1*H-*indole-3-carboxamide;
219)<A234> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylbenzo[*b*]thiophene-3-carboxamide;
220)<A235> (5*S*,7*R*,8*R*,9*S*,10*R*)-8-hydroxy-7-(hydroxymethyl)-3-methyl-9-(4-(3,4,5-trifluorophenyl)-1*H*-1,2,3-triazol-1-yl)-1,6-dioxaspiro[4.5]decan-10-yl picolinate;
221)<A236> 2-(((5*S*,7*R*,8*R*,9*S*,10*R*)-8-hydroxy-7-(hydroxymethyl)-3-methyl-9-(4-(3,4,5-trifluorophenyl)-1*H*-1,2,3-triazol-1-yl)-1,6-dioxaspiro[4.5]decan-10-yl)oxy)acetic acid;
222)<A237> (5*S*,7*R*,8*R*,9*S*,10*R*)-7-(hydroxymethyl)-9-(4-(3,4,5-trifluorophenyl)-1*H-*pyrazol-1-yl)-1,6-dioxaspiro[4.5]decane-8,10-diol;
223)<A238> 7-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-pyrazol-1-yl)-1,6-dioxaspiro[4.5]decan-4-yl)-1-naphthamide;
224)<A239> 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-pyrazol-1-yl)-1,6-dioxaspiro[4.5]decan-4-yl)-2-methylquinoline-4-carboxamide;
225)<A240> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methyl-1-naphthamide;
226)<A241> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-methoxy-1-naphthamide;
227)<A242> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methoxy-1-naphthamide;
228)<A243> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-methyl-1-naphthamide;
229)<A244> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-8-carboxamide;
230)<A245> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-7-methylquinoline-5-carboxamide;
231)<A246> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)isoquinoline-8-carboxamide;
232)<A247> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)isoquinoline-5-carboxamide;
233)<A248> 4-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-3-carboxamide;
234)<A249> *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-5-fluorobenzo[*b*]thiophene-3-carboxamide;
235)<A250> 4-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-3-carboxamide;
236)<A251> 5-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-3-carboxamide;
237)<A252> 6-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-oxo-1,2-dihydroquinoline-4-carboxamide;
238)<A253> 4-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1*H*-indole-3-carboxamide;
239)<A254> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-oxo-1,2-dihydroquinoline-4-carboxamide;
240)<A255> *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1*H*-indole-3-carboxamide;
241)<A256> 7-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide; and
242)<A257> (5*S*,7*R*,8*R*,9*S*,10*R*)-7-(hydroxymethyl)-10-methoxy-9-(4-(3,4,5-trifluorophenyl)-1*H*-1,2,3-triazol-1-yl)-1,6-dioxaspiro[4.5]decan-8-ol.

In another aspect of the present invention, there is provided a pharmaceutical use of the compound represented by Formula (I) above, the stereoisomer, or the pharmaceutically acceptable salt thereof.

The compound of Formula (I) according to the present invention may inhibit the activity of galectin-3.

Specifically, the compound of Formula (I) according to the present invention may inhibit the binding between galectin-3 and a ligand.

Examples of ligands that may bind to galectin-3 include LAG-3, TLR4, CD98, CD29, CD7, synexin, Bcl-2, Ras, Gemin-4, and the like.

Accordingly, the present invention provides a galectin-3 inhibitor including the compound of Formula (I) above, the stereoisomer, or the pharmaceutically acceptable salt thereof as an active ingredient.

Accordingly, the present invention provides a method for inhibiting the activity of galectin-3, the method including a step of treating a specimen or cells with the compound of Formula (I) above, the stereoisomer, or the pharmaceutically acceptable salt thereof.

In addition, the compound of Formula (I) above, the stereoisomer, or the pharmaceutically acceptable salt thereof may be used to prevent or treat galectin-3 related diseases or disorders.

Here, the "prevention" refers to any act of inhibiting or delaying occurrence, spread, and recurrence of the disease; and the "treatment" refers to any act of ameliorating or beneficially altering symptoms of the disease by administration of the compound.

Accordingly, the present invention provides a use of the compound represented by Formula (I) above, the stereoisomer, or the pharmaceutically acceptable salt thereof for preventing or treating galectin-3 related diseases or disorders.

The present invention also provides a use of the compound represented by Formula (I) above, the stereoisomer, or the pharmaceutically acceptable salt thereof for preparing a medicament for preventing or treating galectin-3 related diseases or disorders.

The present invention also provides a method for preventing or treating galectin-3 related diseases or disorders, the method including administering, to a subject in need thereof, the compound represented by Formula (I) above, the stereoisomer, or the pharmaceutically acceptable salt thereof.

Here, the term "subject in need thereof" refers to any animal, specifically a mammal, such as a monkey, a cow, a horse, a sheep, a pig, a chicken, a turkey, a quail, a cat, a dog, a mouse, a rat, a rabbit, and a guinea pig, including a human (patient), who has or is likely to develop the disease, and specifically, may refer to a mammal. In addition, the subject in need thereof may refer to a biological sample.

In addition, the "administration" means providing a predetermined substance to a subject in need thereof by any appropriate method, and administration of the compound of the present invention may be achieved via any common route as long as the route allows the substance to reach a target tissue.

The present invention also provides a pharmaceutical composition for preventing or treating galectin-3 related diseases or disorders, the composition including the compound represented by Formula (I) above, the stereoisomer, or the pharmaceutically acceptable salt thereof as an active ingredient.

The galectin-3 related diseases or disorders may be selected from the group consisting of inflammation; inflammation-induced thrombosis; atopic dermatitis; acute coronary syndrome; fibrosis; topical fibrosis; fibrotic complications; scleroderma; scars; keloid formation; covid-19; acute lung injury; acute respiratory distress syndrome; viral pneumonia; surgical adhesions; septic shock; cancer; transplant rejection; metastatic cancer; aging; bone disease; pulmonary hypertension; autoimmune diseases; viral infections; metabolic disorders; heart disease; heart failure; ocular diseases; atherosclerosis; metabolic diseases; diabetes; insulin resistance; obesity; Marfans syndrome; Loeys-Dietz syndrome; kidney diseases; fibrotic lung complications; interstitial lung diseases; liver diseases; and uterine diseases.

The fibrosis may be pulmonary fibrosis, hepatic fibrosis, renal fibrosis, ophthalmofibrosis, skin fibrosis, cardiac fibrosis, or the like; the topical fibrosis may be Dupuytren disease, Peyronie disease, or the like; the fibrous complications may be fibrous complications of other therapies such as coronary stent, bile duct stent, cerebral artery stent, and ureteral stent; the autoimmune diseases may be psoriasis, rheumatoid arthritis, or the like; the viral infections may be influenza virus, HIV, herpes virus, coronavirus, or hepatitis C; and the liver diseases may be non-alcoholic fatty hepatitis, non-alcoholic fatty liver disease, or the like.

The cancer may be liver cancer, hepatocellular carcinoma, thyroid cancer, colorectal cancer, testicular cancer, bone cancer, oral cancer, basal cell carcinoma, ovarian cancer, gallbladder carcinoma, biliary tract cancer, head and neck cancer, vesical carcinoma, tongue cancer, esophageal cancer, renal cancer, malignant melanoma, gastric cancer, breast cancer, sarcoma, pharynx carcinoma, uterine cancer, cervical cancer, prostate cancer, rectal cancer, pancreatic cancer, lung cancer, skin cancer, colon cancer, etc.

The pharmaceutical composition may contain, as an active ingredient, the compound represented by Formula (I) above, the stereoisomer, or the pharmaceutically acceptable salt thereof in an amount of 0.1% to 90% by weight, specifically 0.1% to 75% by weight, and more specifically 1% to 50% by weight, with respect to a total weight of the composition.

The pharmaceutical composition may contain conventional and non-toxic pharmaceutically acceptable additives which are blended into a preparation according to a conventional method. For example, the pharmaceutical composition may further contain a pharmaceutically acceptable carrier, diluent, or excipient. In an embodiment, the present invention provides a pharmaceutical composition containing the compound of Formula (I), the stereisomer, or the pharmaceutically acceptable salt thereof, and a pharmaceutically acceptable carrier.

Examples of the additives used in the pharmaceutical composition may include sweeteners, binders, solvents, dissolution aids, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, tragacanth gum, alginic acid, sodium alginate, methyl cellulose, sodium carboxymethyl cellulose, agar, water, ethanol, polyethylene glycol, polyvinylpyrrolidone, sodium chloride, calcium chloride, orange essence, strawberry essence, vanilla flavor, and the like.

The pharmaceutical composition may be made into various preparation forms for oral administration (for example, tablets, pills, powders, capsules, syrups, or emulsions) or parenteral administration (for example, intramuscular, intravenous, or subcutaneous injection).

For example, the pharmaceutical composition may be made into a preparation for oral administration, in which additives used may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like. Specifically, solid preparations for oral administration include tablets, pills, powders, granules, capsules and the like. Such solid preparations may be formulated by mixing at least one excipients, for example, starch, calcium carbonate, sucrose, lactose, gelatin, etc., into the composition. In addition to simple excipients, lubricants such as magnesium stearate and talc may also be used. Further, as liquid preparations for oral administration, suspensions, emulsions, syrups, and the like may be exemplified, and the liquid preparations may contain various excipients such as wetting agents, sweeteners, fragrances, and preservatives in addition to water and liquid paraffin which are commonly used as simple diluents.

In addition, examples of preparations for parenteral administration may include sterilized aqueous solutions, non-aqueous solutions, suspensions, emulsions, freeze-dried preparations, and suppositories. For the non-aqueous solutions and the suspensions, propylene glycol, polyethylene glycol, vegetable oil such as olive oil, injectable ester such as ethyl oleate, or the like may be used. As the suppository base, Witepsol^{™}, macrogol, Tween^{™} 61, cacao butter, laurin fat, glycerogelatin, or the like may be used. Injections may contain conventional additives such as solubilizers, isotonic agents, suspending agents, emulsifiers, stabilizers, and preservatives.

The compound or composition of the present invention may be administered to a patient in a therapeutically effective amount or in a pharmaceutically effective amount.

Here, the "therapeutically effective amount" or "pharmaceutically effective amount" refers to an amount of a compound or composition effective to prevent or treat a target disease, the amount being sufficient to treat the disease at a reasonable benefit/risk ratio, which is applicable to medical treatment, without causing adverse effects. The level of the effective amount may be determined according to factors including the patient's health condition, the type and severity of the disease, the activity of the drug, the patient's sensitivity to the drug, the method of administration, the time of administration, the route of administration, the rate of release, the period of treatment, formulated or co-administered drugs, and other factors well known in the medical art.

Specifically, the effective amount of the compound in the composition of the present invention may vary depending on the age, sex, and body weight of the patient, and is generally 0.1 mg to 1000 mg or 5 mg to 200 mg per 1 kg of body weight per day or every other day. However, the effective amount may increase or decrease depending on route of administration, disease severity, the patient's sex, body weight, and age, and the like. Thus, the scope of the present invention is not limited thereto.

The compound or composition of the present invention may be administered as an individual therapeutic agent or in combination with other therapeutic agents, may be administered sequentially or simultaneously with a conventional therapeutic agent, and may be administered in single or multiple doses. It is important to take into account all of the above factors and to administer the amount in which the maximum effect can be obtained in a minimal amount without side effects, and such amount may be easily determined by a person skilled in the art.

Preferably, the compound or composition of the present invention may be administered for tumor therapy in combination with chemotherapy, radiation therapy, immunotherapy, hormone therapy, bone marrow transplantation, stem cell replacement therapy, other biological therapies, surgical intervention, or combinations thereof. For example, the compound or composition of the present invention may be used as adjunctive therapy in conjunction with other long-term treatment strategies, or may be used to maintain the patient's condition after tumor regression or chemoprophylactic therapy in severe patients.

Preferably, the pharmaceutical composition of the present invention may further include one or more active ingredients, and the additional active ingredient may be a known anticancer agent.

### Modes for the Invention

Hereinafter, the examples will be described for reference in order to assist in understanding of the present invention, and the scope of the present invention is not limited to these examples.

The meanings of abbreviations used in the following reaction schemes and examples are as follows.
- AC: Acetyl
- Bn: Benzyl
- Bu: Butyl
- DIBAL-H: Diisobutylaluminum hydride
- DMSO: Dimethylsulfoxide
- dppf: Diphenylphosphino ferrocene
- Et: Ethyl
- HATU: Hexafluorophosphate azabenzotriazol tetramethyl uronium
- mCPBA: m-chloroperoxybenzoic acid
- Me: Methyl
- NBS: N-bromosuccinimide
- rt: Room temperature
- TBAB: Tetrabutylammonium bromide
- TEA: Triethylamine
- Tf: Triflate
- THP: Tetrahydropyran
- TLC: Thin layer chromatography
- TMS: Tetramethylsilane
- Ts: Tosil

### Example 1: Synthesis of Compound A08

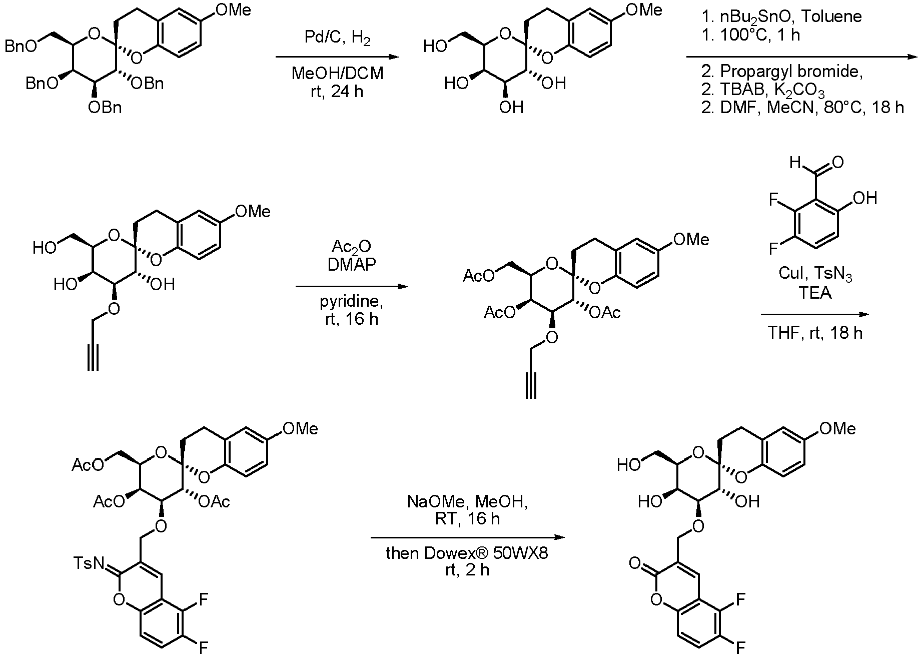

### Synthesis of (2R,3'R,4'S,5'R,6'R)-6'-(hydroxymethyl)-6-methoxy-3',4',5',6'-tetrahydrospiro[chromane-2,2'-pyran]-3',4',5'-triol

In a round-bottomed flask in which 10% Pd/C (10 mg) was dissolved with 1.0 mL of methanol, (2*R*,3'*R*,4*'S*,5'*S*,6'*R*)-3',4',5'-tris(benzyloxy)-6'-((benzyloxy)methyl)-6-methoxy-3',4',5',6'-tetrahydrospiro[chromane-2,2'-pyran] (200.0 mg, 0.297 mmol) (synthesized according to the method described in Tetrahedron 66 (2010) 5229-5234) was dissolved in 2.0 mL of a solution of dichloromethane:methanol (1:2) and the mixture was added dropwise thereto, and degassed with a hydrogen balloon for 10 minutes. The reaction solution was stirred at room temperature in a hydrogen atmosphere for 24 hours. The reaction solution was filtered through Celite and concentrated under reduced pressure to perform silica gel column chromatography with 9% methanol/dichloromethane to obtain the title compound as a white solid (23 mg, yield: 76%).

¹H-NMR (400 MHz, CD₃OD) δ 6.76 (d, J = 8.8 Hz, 1H), 6.70-6.56 (m, 2H), 4.05-3.95 (m, 2H), 3.87-3.78 (m, 1H), 3.71 (s, 3H), 3.70-3.62 (m, 2H), 3.51 (dd, J = 11.2, 6.0 Hz, 1H), 3.11-2.94 (m, 1H), 2.59 (dd, J = 15.7, 5.3 Hz, 1H), 2.23 (td, J = 13.6, 6.1 Hz, 1H), 1.92-1.81 (m, 1H).

### Synthesis of (2R,3'R,4'S,5'S,6'R)-6'-(hydroxymethyl)-6-methoxy-4'-(prop-2-yn-1-yloxy)-3',4',5',6'-tetrahydrospiro[chromane-2,2'-pyran]-3',5'-diol

(2*R*,3'*R*,4'*S*,5'*R*,6'*R*)-6'-(hydroxymethyl)-6-methoxy-3',4',5',6'-tetrahydrospirospiro[chromane-2,2'-pyran]-3',4',5'-triol (37.8 mg, 0.121 mmol) was dissolved in 2.4 mL of toluene, and then nBu₂SnO (3.01 mg, 0.012 mmol) was dispersed therein. This mixture was heated in an argon atmosphere to 100 °C, and stirred at 100 °C for 1 hour. After 1 hour, the reaction mixture was concentrated under reduced pressure as it is. The concentrated mixture was diluted with 2 mL of N,N-dimethylformamide and 0.2 mL of acetonitrile, and propargyl bromide (18 µL, 0.242 mmol), TBAB (39 mg, 0.121 mmol), and potassium carbonate (25 mg, 0.186 mmol) were added thereto. This mixture was heated in an argon atmosphere to 80 °C, and stirred at 80 °C for 18 hours. After checking the progress of the reaction with TLC, the reaction mixture was concentrated under reduced pressure as it is. The concentrate was subjected to silica gel column chromatography with 0% to 5% methanol/dichloromethane to obtain the title compound in white foam (29.7 mg, yield: 70%).

¹H-NMR (400 MHz, CDCl₃) δ = 6.79 (d, J = 9.0 Hz, 1H), 6.68 (dd, J = 9.0, 2.7 Hz, 1H), 6.61 (d, J = 3.1 Hz, 1H), 4.45 (dd, J = 5.1, 2.3 Hz, 2H), 4.31 (dd, J = 3.1, 1.2 Hz, 1H), 4.00 (dd, J = 9.4, 3.1 Hz, 1H), 3.82-3.88 (m, 2H), 3.79-3.80 (m, 1H), 3.70-3.77 (m, 4H), 3.00-3.09 (m, 1H), 2.64 (dd, J = 16.2, 5.3 Hz, 1H), 2.54 (t, J = 2.5 Hz, 1H), 2.33 (td, J = 13.7, 6.3 Hz, 1H), 1.95-2.01 (m, 1H)

### Synthesis of (2R,3'R,4'S,5'S,6'R)-6'-(acetoxymethyl)-6-methoxy-4'-(prop-2-yn-1-yloxy)-3',4',5',6'-tetrahydrospiro[chromane-2,2'-pyran]-3',5'-diyl diacetate

(2*R*,3'*R*,4'*S*,5'*S*,6'*R*)-6'-(hydroxymethyl)-6-methoxy-4'-(prop-2-in-1-yloxy)-3',4',5',6'-tetrahydrospiro[chromane-2,2'-pyran]-3',5'-diol (29.7 mg, 0.085 mmol) was dissolved in 1.5 mL of pyridine, and then Ac₂O (0.08 mL, 0.847 mmol) and 4-dimethylaminopyridine (1.0 mg, 0.008 mmol) were added thereto. This mixture was stirred at room temperature for 16 hours. After checking the progress of the reaction with TLC, the reaction mixture was concentrated under reduced pressure as it is. To remove the residual pyridine, the reaction mixture was azeotroped with n-heptane (10 mL x 3 times). The concentrate was subjected to silica gel column chromatography with 0% to 30% ethyl acetate/n-hexane to obtain the title compound as a white solid (31.3 mg, yield: 78%).

¹H-NMR (400 MHz, CDCl₃) δ = 6.84 (d, J = 8.6 Hz, 1H), 6.71 (dd, J = 8.8, 2.9 Hz, 1H), 6.61 (d, J = 2.7 Hz, 1H), 5.53 (dd, J = 3.6, 1.1 Hz, 1H), 5.24 (d, J = 10.2 Hz, 1H), 4.33 (dd, J = 10.2, 3.5 Hz, 1H), 4.23-4.23 (m, 2H), 4.15-4.18 (m, 1H), 4.08 (dd, J = 11.0, 6.3 Hz, 1H), 3.96 (dd, J = 11.2, 7.2 Hz, 1H), 3.75 (s, 3H), 2.94-3.03 (m, 1H), 2.61 (dd, J = 16.0, 5.5 Hz, 1H), 2.48 (t, J = 2.3 Hz, 1H), 2.16 (s, 3H), 2.12 (s, 3H), 1.97-2.02 (m, 1H), 1.84-1.92 (m, 1H), 1.78 (s, 3H)

### Synthesis of (2R,3'R,4'S,5'S,6'R)-6'-(acetoxymethyl)-4'-((5,6-difluoro-2-(tosilimino)-2H-chromen-3-yl)methoxy)-6-methoxy-3',4',5',6'-tetrahydrospiro[chromane-2,2'-pyran]-3',5'-diyl diacetate

2*R*,3'*R*,4'*S*,5'*S*,6'*R*)-6'-(acetoxymethyl)-6-methoxy-4'-(prop-2-yn-1-yloxy)-3',4',5',6'-tetrahydrospiro[chromane-2,2'-pyran]-3',5'-diyl diacetate (28.0 mg, 0.059 mmol) was dissolved in 1 mL of tetrahydrofuran, and then CuI (2.23 mg, 0.012 mmol), 2,3-difluoro-6-hydroxybenzaldehyde (23.22 mg, 0.146 mmol), and a *p*-toluenesulfonyl azide solution (24 mL, 0.146 mmol in about 0.6 M toluene solution) were added thereto, followed by stirring at room temperature for 1 hour. Then, TEA (33 µL, 0.235 mmol) was added thereto, and the resulting mixture was stirred at room temperature for 18 hours. After checking the progress of the reaction with TLC, a saturated aqueous sodium chloride solution was added to terminate the reaction. The mixture was diluted with ethyl acetate and washed with a saturated aqueous sodium chloride solution, distilled water, and a saturated aqueous sodium chloride solution. The organic layer was recovered, dried over anhydrous sodium sulfate, filtered, and then concentrated under reduced pressure. The concentrate was subjected to silica gel column chromatography with 0% to 40% ethyl acetate/n-hexane to obtain the title compound as a white solid (22.3 mg, yield: 48%).

¹H-NMR (400 MHz, CDCl₃) δ = 7.98 (d, J = 8.2 Hz, 2H), 7.85 (s, 1H), 7.32-7.36 (m, 3H), 7.19-7.21 (m, 1H), 6.82 (d, J = 9.0 Hz, 1H), 6.72 (dd, J = 8.8, 2.9 Hz, 1H), 6.62 (d, J = 2.7 Hz, 1H), 5.61-5.61 (m, 1H), 5.37 (d, J = 10.2 Hz, 1H), 4.62 (d, J = 16.0 Hz, 1H), 4.46 (d, J = 15.7 Hz, 1H), 4.27 (dd, J = 10.4, 3.3 Hz, 1H), 4.06-4.17 (m, 2H), 3.98 (dd, J = 10.8, 6.8 Hz, 1H), 3.76 (s, 3H), 2.95-3.04 (m, 1H), 2.62 (dd, J = 16.4, 5.9 Hz, 1H), 2.44 (s, 3H), 2.17 (s, 4H), 2.05 (s, 3H), 1.86-2.04 (m, 2H), 1.84 (s, 3H)

### Synthesis of 3-((((2R,3'R,4'S,5'S,6'R)-3',5'-dihydroxy-6'-(hydroxymethyl)-6-methoxy-3',4',5',6'-tetrahydrospiro[chromane-2,2'-pyran]-4'-yl)oxy)methyl)-5,6-difluoro-2H-chromen-2-one

(2R,3'R,4'S,5'S,6'R)-6'-(acetoxymethyl)-4'-((5,6-difluoro-2-(tosilimino)-2H-chromen-3-yl)methoxy)-6-methoxy-3',4',5',6'-tetrahydrospiro[chromane-2,2'-pyran]-3',5'-diyl diacetate (20 mg, 0.026 mmol) was dissolved in 0.5 mL of methanol, and then NaOMe (1.38 mg, 0.026 mmol) was added thereto. The resulting mixture was stirred at room temperature for 16 hours. After checking the progress of the reaction with TLC, Dowex^{®}50WX8 resin (30 mg) was added to terminate the reaction. This mixture was filtered through a glass filter, and the filtrate was concentrated under reduced pressure. The concentrate was subjected to silica gel column chromatography with 0% to 5% methanol/dichloromethane to obtain the title compound as a white solid (11.2 mg, yield: 86%).

ESIMS: m/z 507.44 [M+H]+; ¹H-NMR (400 MHz, CD₃OD) δ = 8.39 (s, 1H), 7.51 (q, J = 9.4 Hz, 1H), 7.21-7.23 (m, 1H), 6.78 (d, J = 8.6 Hz, 1H), 6.63-6.67 (m, 2H), 4.77 (dd, J = 15.3, 1.6 Hz, 1H), 4.64 (dd, J = 14.9, 1.6 Hz, 1H), 4.31-4.31 (m, 1H), 3.98 (dd, J = 9.8, 3.1 Hz, 1H), 3.90 (d, J = 10.2 Hz, 1H), 3.83-3.87 (m, 1H), 3.68-3.72 (m, 4H), 3.54 (dd, J = 11.2, 6.1 Hz, 1H), 3.01-3.10 (m, 1H), 2.62 (dd, J = 16.6, 5.3 Hz, 1H), 2.27 (td, J = 13.5, 5.9 Hz, 1H), 1.88-1.92 (m, 1H)

### Example 2: Synthesis of Compound A09

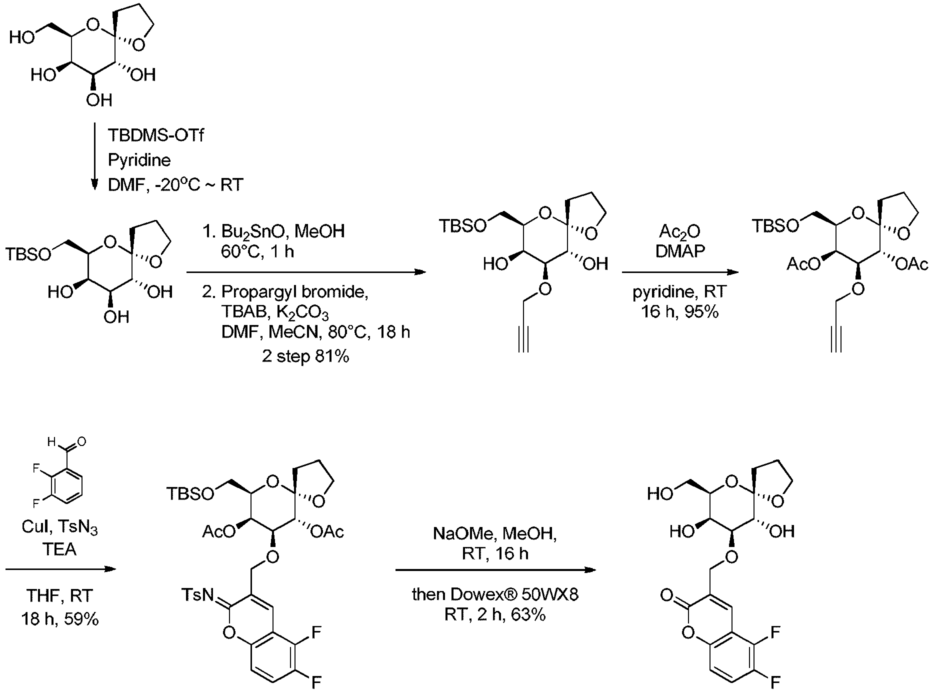

### Synthesis of (5S,7R,8R,9S,10R)-7-(((tert-butyldimethylsilyl)oxy)methyl)-1,6-dioxaspiro[4.5]decane-8,9,10-triol

To a round-bottomed flask, (5*S*,7*R*,8*R*,9*S*,10*R*)-7-(hydroxymethyl)-1,6-dioxaspiro[4.5]decane-8,9,10-triol (49.1 mg, 0.223 mmol) (synthesized according to the method disclosed in Journal of Organic Chemistry (2009), 74(22), 8779-8786) was added, *N*,*N-*dimethylformamide (2 mL) was added in an argon atmosphere to dissolve, and the solution was cooled to -20 °C. Pyridine (36 µL, 0.446 mmol) and tert-butyldimethylsilyl trifluoromethanesulfonate (60 µL, 0.268 mmol) were sequentially added, stirred for 20 minutes, and then stirred at room temperature for 30 minutes. The reaction solution was diluted with ethyl acetate and then washed twice with a saturated aqueous ammonium chloride solution. The organic layer was dried over anhydrous sodium sulfate, filtered, and concentrated, and the concentrated reaction mixture was subjected to silica gel column chromatography with 0% to 5% methanol/dichloromethane to obtain the title compound as a colorless oil (99.2 mg).

¹H-NMR (400 MHz, CDCl₃) δ 4.12-4.07 (m, 1H), 4.01-3.91 (m, 2H), 3.91-3.82 (m, 2H), 3.81-3.77 (m, 2H), 3.72-3.67 (m, 1H), 3.11 (d, J = 2.3 Hz, 1H), 2.58 (d, J = 5.8 Hz, 1H), 2.28-2.19 (m, 1H), 2.11-2.00 (m, 1H), 2.00-1.86 (m, 2H), 1.83 (d, J = 9.6 Hz, 1H), 0.90 (s, 9H), 0.08 (d, J = 1.9 Hz, 6H).

### Synthesis of (5S,7R,8S,9S,10R)-7-(((tert-butyldimethylsilyl)oxy)methyl)-9-(prop-2-yn-1-yloxy)-1,6-dioxaspiro[4.5]decane-8,10-diol

(5*S*,7*R*,8*R*,9*S*,10*R*)-7-(((*tert*-butyldimethylsilyl)oxy)methyl)-1,6-dioxaspiro[4.5]decane-8,9,10-tirol (36.1 mg, 0.1079 mmol) was dissolved in 2.15 mL of methanol, and then nBu₂SnO (5.37 mg, 0.022 mmol) was dispersed therein. This mixture was heated in an argon atmosphere to 60 °C, and stirred at 60 °C for 1 hour. After 1 hour, the reaction mixture was concentrated under reduced pressure. The concentrated mixture was diluted with N,N-dimethylformamide (2 mL) and acetonitrile (0.2 mL), and propargyl bromide (16.3 µL, 0.2158 mmol), TBAB (6.95 mg, 0.0216 mmol), and potassium carbonate (22.4 mg, 0.161 mmol) were added thereto. This mixture was heated in an argon atmosphere to 80 °C, and stirred at 80 °C for 18 hours. After checking the progress of the reaction with TLC, the reaction mixture was concentrated under reduced pressure as it is. The concentrate was subjected to silica gel column chromatography with 0% to 35% ethyl acetate/n-hexane to obtain the title compound in pale yellow oil (32.5 mg, yield: 81%).

¹H-NMR (400 MHz, CDCl₃) δ = 4.40 (dd, J = 3.9, 2.3 Hz, 2H), 4.23-4.23 (m, 1H), 3.92-4.03 (m, 3H), 3.85-3.89 (m, 1H), 3.76-3.83 (m, 2H), 3.70 (dd, J = 9.6, 3.3 Hz, 1H), 2.73 (s, 1H), 2.46 (t, J = 2.3 Hz, 1H), 2.21-2.28 (m, 1H), 2.02-2.08 (m, 1H), 1.87-1.98 (m, 3H), 0.90 (s, 9H), 0.07 (s, 6H)

### Synthesis of (5S,7R,8S,9S,10R)-7-(((tert-butyldimethylsilyl)oxy)methyl)-9-(prop-2-yn-1-yloxy)-1,6-dioxaspiro[4.5]decane-8,10-diyl diacetate

(5*S*,7*R*,8*S*,9*S*,10*R*)-7-(((*tert*-butyldimethylsilyl)oxy)methyl)-9-(prop-2-yn-1-yloxy)-1,6-dioxaspiro[4.5]decane-8,10-diol (32.5 mg, 0.087 mmol) was dissolved in 1.5 mL of pyridine, and then Ac₂O (41.2 µL, 0.4362 mmol) and 4-dimethylaminopyridine (1.8 mg, 0.017 mmol) were added thereto. This reaction solution was stirred at room temperature for 16 hours. After checking the progress of the reaction with TLC, the reaction mixture was concentrated under reduced pressure as it is. To remove the residual pyridine, the reaction mixture was azeotroped with n-heptane (10 mL x 3 times). The concentrate was subjected to silica gel column chromatography with 0 to 20% ethyl acetate/n-hexane to obtain the title compound as a white solid (31.3 mg, yield: 95%).

### Synthesis of (5S,7R,8S,9S,10R)-7-(((tert-butyldimethylsilyl)oxy)methyl)-9-((5,6-difluoro-2-(tosilimino)-2H-chromen-3-yl)methoxy)-1,6-dioxaspiro[4.5]decane-8,10-diyl diacetate

(5*S*,7*R*,8*S*,9*S*,10*R*)-7-(((*tert*-butyldimethylsilyl)oxy)methyl)-9-(prop-2-yn-1-yloxy)-1,6-dioxaspiro[4.5]decane-8,10-diyl diacetate (37.7 mg, 0.083 mmol) was dissolved in 1 mL of tetrahydrofuran, and then CuI (3.14 mg, 0.017 mmol), 2,3-difluoro-6-hydroxybenzaldehyde (32.63 mg, 0.206 mmol), and ap-toluenesulfonyl azide solution (0.344 mL, 0.206 mmol in about 0.6 M toluene solution) were added thereto, followed by stirring at room temperature for 1 hour. Then, TEA (46 µL, 0.330 mmol) was added thereto, and the resulting mixture was stirred at room temperature for 18 hours. After checking the progress of the reaction with TLC, a saturated aqueous sodium chloride solution was added to terminate the reaction. The mixture was diluted with ethyl acetate and washed with a saturated aqueous sodium chloride solution, distilled water, and a saturated aqueous sodium chloride solution. The organic layer was recovered, dried over anhydrous sodium sulfate, filtered, and then concentrated under reduced pressure. The concentrate was subjected to silica gel column chromatography with 0% to 40% ethyl acetate/n-hexane to obtain the title compound as a white solid (37.2 mg, yield: 59%).

¹H-NMR (400 MHz, CDCl₃) δ = 7.96 (s, 1H), 7.94 (s, 1H), 7.84 (s, 1H), 7.30-7.34 (m, 3H), 7.19-7.21 (m, 1H), 5.58-5.59 (m, 1H), 5.42 (d, J = 10.2 Hz, 1H), 4.58 (dd, J = 15.7, 1.6 Hz, 1H), 4.37 (dd, J = 15.7, 1.6 Hz, 1H), 3.91-4.04 (m, 4H), 3.55-3.65 (m, 2H), 2.43 (s, 3H), 2.13 (s, 3H), 2.07 (s, 3H), 1.82-2.05 (m, 4H), 0.87 (s, 9H), 0.04 (s, 3H), 0.03 (s, 3H)

### Synthesis of 3-((((5S,7R,8S,9S,10R)-8,10-dihydroxy-7-(hydroxymethyl)-1,6-dioxaspiro[4.5]decan-9-yl)oxy)methyl)-5,6-difluoro-2H-chromen-2-one

(*5*S,7*R*,8*S*,9*S*,10*R*)-7-(((*tert*-butyldimethylsilyl)oxy)methyl)-9-((5,6-difluoro-2-(tosilimino)-2*H-*chromen-3-yl)methoxy)-1,6-dioxaspiro[4.5]decane-8,10-diyl diacetate (35 mg, 0.046 mmol) was dissolved in 0.9 mL of methanol, and then NaOMe (2.48 mg, 0.046 mmol) was added thereto, followed by stirring at room temperature for 16 hours. After checking the progress of the reaction with TLC, Dowex^{®}50WX8 resin (30 mg) was added to terminate the reaction. This mixture was filtered through a glass filter, and the filtrate was concentrated under reduced pressure. The concentrate was subjected to silica gel column chromatography with 0% to 5% methanol/dichloromethane to obtain the title compound as a white solid (11.9 mg, yield: 63%).

ESIMS: m/z 415.27 [M+H]+; ¹H-NMR (400 MHz, CD₃OD) δ = 8.36 (s, 1H), 7.50 (q, J = 9.4 Hz, 1H), 7.19-7.21 (m, 1H), 4.70 (d, J = 15.3 Hz, 1H), 4.58 (d, J = 15.3 Hz, 1H), 4.19-4.20 (m, 1H), 3.93-3.97 (m, 3H), 3.85 (t, J = 6.1 Hz, 1H), 3.65-3.70 (m, 3H), 2.19-2.27 (m, 1H), 2.00-2.06 (m, 1H), 1.89-1.95 (m, 2H)

### Example 3: Synthesis of Compounds A10 and A11

### Synthesis of (2R,3R,4S,5R,6S)-2-(acetoxymethyl)-6-(phenylthio)-4-(4-(3,4,5-trifluorophenyl)-1H-1,2,3-triazol-1-yl)tetrahydro-2H-pyran-3,5-diyl diacetate

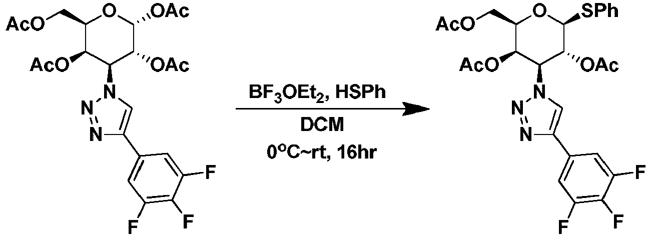

(2*R*,3*R*,4*S*,5*R*,6*R*)-6-(acetoxylmethyl)-4-(4-(3,4,5-trifluorophenyl)-1*H*-1,2,3-triazol-1-yl)tetrahydro-2H-pyran-2,3,5-triyl triacetate (50 mg, 0.094 mmol) (synthesized according to the method disclosed on page 160 of WO 2016/120403 under the name of GalectoBiotech AB) was dissolved in 2 mL of dichloromethane, and then thiophenol (15 µL, 0.141 mmol) and BF₃OEt₂ (24 µL, 0.189 mmol) were sequentially slowly added dropwise thereto at 0 °C, and the mixture was stirred for 16 hours while elevating the temperature from 0 °C to room temperature. A saturated aqueous sodium hydrogen carbonate solution was added to terminate the reaction, and then extracted with dichloromethane, dried over anhydrous sodium sulfate, filtered, and then concentrated. The concentrated reaction mixture was subjected to silica gel column chromatography with 23% ethyl acetate/n-hexane to obtain the title compound as a white solid (30 mg, yield: 55%).

¹H-NMR (400 MHz, CDCl₃) δ 7.76 (s, 1H), 7.58-7.54 (m, 2H), 7.43-7.34 (m, 5H), 5.70 (dd, J = 11.0, 9.6 Hz, 1H), 5.58-5.54 (m, 1H), 5.17 (dd, J = 11.0, 3.2 Hz, 1H), 4.87 (d, J = 9.6 Hz, 1H), 4.19-4.09 (m, 3H), 2.05 (d, J = 5.6 Hz, 6H), 1.97 (s, 3H).

### Synthesis of (2R,3R,4S,5R,6S)-2-(hydroxymethyl)-6-(phenylthio)-4-(4-(3,4,5-trifluorophenyl)-1H-1,2,3-triazol-1-yl)tetrahydro-2H-pyran-3,5-diol

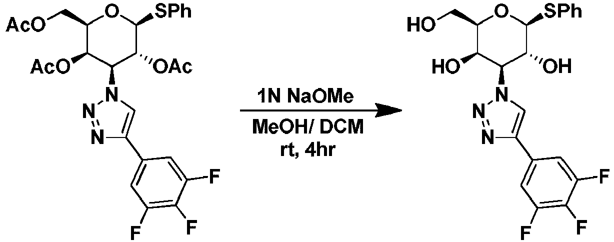

(2*R*,3*R*,4*S*,5*R*,6*S*)-2-(acetoxymethyl)-6-(phenylthio)-4-(4-(3,4,5-trifluorophenyl)-1*H-*1,2,3-triazol-1-yl)tetrahydro-2*H*-pyran-3,5-diyl diacetate (30 mg, 0.052 mmol) was dissolved in 3 mL of methanol: dichloromethane (2:1), and a 1M sodium methoxide methanol solution (78 µL, 0.078 mmol) was dropwise thereto, and the mixture was stirred at room temperature for 4 hours. Dowex (resin) 50W*8 hydrogen form (90 mg) was added dropwise to the reaction solution, and the mixture was stirred at room temperature for 30 minutes. The mixture was then filtered through Celite and then concentrated. The concentrated reaction mixture was subj ected to silica gel column chromatography with 3.2% methanol/dichloromethane to obtain the title compound as a white solid (22 mg, yield: 94%).

¹H-NMR (400 MHz, CD₃OD) δ 8.50 (s, 1H), 7.67-7.57 (m, 4H), 7.36-7.25 (m, 3H), 4.91-4.89 (m, 1H), 4.32-4.24 (m, 1H), 4.14 (d, J = 2.9 Hz, 1H), 3.87-3.67 (m, 4H).

### Synthesis of 1-((2R,3R,4S,5R,6S)-3,5-bis(benzyloxy)-2-((benzyloxy)methyl)-6-(phenylthio)tetrahydro-2H-pyran-4-yl)-4-(3,4,5-trifluorophenyl)-1H-1,2,3-triazole

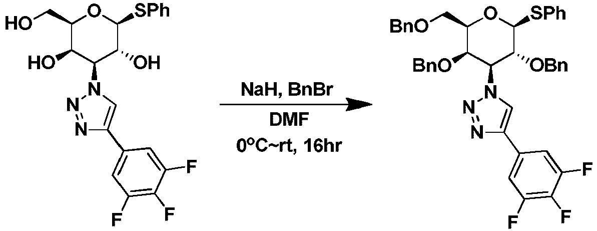

(2*R*,3*R*,4*S*,5*R*,6*S*)-2-(hydroxymethyl)-6-(phenylthio)-4-(4-(3,4,5-trifluorophenyl)-1*H-*1,2,3-triazol-1-yl)tetrahydro-2*H*-pyran-3,5-diol (22 mg, 0.049 mmol) was dissolved in 2 mL of N,N-dimethylformamide, and 60% NaH (6.4 mg, 0.160 mmol) was added dropwise thereto at 0 °C, and the mixture was stirred at 0 °C for 10 minutes. BnBr (21 µL, 0.176 mmol) was added dropwise thereto at 0 °C, and the mixture was stirred for 16 hours from 0 °C to room temperature. A saturated aqueous sodium hydrogen carbonate solution was added at 0 °C to terminate the reaction, and then extracted with ethyl acetate to obtain an organic layer. The organic layer was dried over anhydrous sodium sulfate, filtered, and then concentrated. The concentrated reaction mixture was subjected to silica gel column chromatography with 15% ethyl acetate/n-hexane to obtain the title compound as a white solid (30 mg, yield: 85%).

¹H-NMR (400 MHz, CDCl₃) δ 7.69-7.59 (m, 2H), 7.38-7.28 (m, 12H), 7.15-6.96 (m, 7H), 6.85-6.76 (m, 2H), 4.93 (dd, J= 10.5, 3.1 Hz, 1H), 4.80 (d, J = 9.3 Hz, 1H), 4.71 (d, J= 11.1 Hz, 1H), 4.59-4.47 (m, 3H), 4.24-4.11 (m, 2H), 4.08 (d, J = 2.9 Hz, 1H), 3.93 (t, J = 7.0 Hz, 1H), 3.85 (d, J = 11.0 Hz, 1H), 3.78 (d, J = 7.0 Hz, 2H).

### Synthesis of (3R,4S,5R,6R)-3,5-bis(benzyloxy)-6-((benzyloxy)methyl)-4-(4-(3,4,5-trifluorophenyl)-1H-1,2,3-triazol-1-yl)tetrahydro-2H-pyran-2-ol

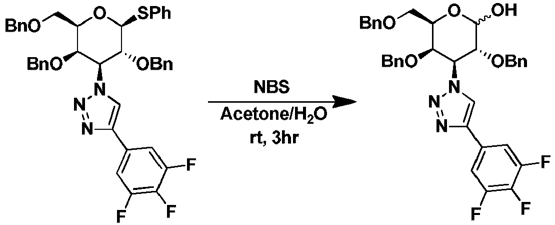

1-((2*R*,3*R*,4*S*,5*R*,6*S*)-3,5-bis(benzyloxy)-2-((benzyloxy)methyl)-6-(phenylthio)tetrahydro-2*H*-pyran-4-yl)-4-(3,4,5-trifluorophenyl)-1*H*-1,2,3-triazole (30 mg, 0.041 mmol) was dissolved in 2 mL of acetone:water (9:1), and NBS (9 mg, 0.050 mmol) was added dropwise thereto, and the mixture was stirred at room temperature for 2 hours. NBS (15 mg, 0.082 mmol) was further added dropwise thereto, and the mixture was stirred at room temperature for 1 hour. The reaction mixture was concentrated, and then ethyl acetate and a saturated aqueous sodium hydrogen carbonate solution were added thereto, and extracted to obtain an organic layer. The organic layer was dried over anhydrous sodium sulfate, filtered, and then concentrated. The concentrated reaction mixture was subjected to silica gel column chromatography with 35% ethyl acetate/n-hexane to obtain the title compound as a white solid (22 mg, yield: 84%).

### Synthesis of (3R,4S,5R,6R)-3,5-bis(benzyloxy)-6-((benzyloxy)methyl)-4-(4-(3,4,5-trifluorophenyl)-1H-1,2,3-triazol-1-yl)tetrahydro-2H-pyran-2-one

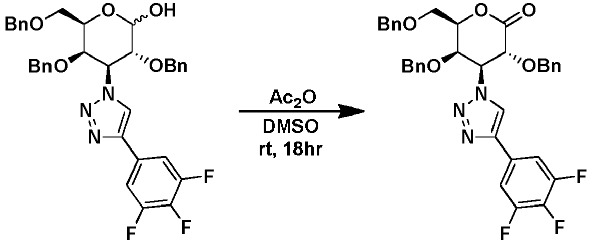

(3*R*,4*S*,5*R*,6*R*)-3,5*-*bis(benzyloxy)-6-((benzyloxy)methyl)-4-(4-(3,4,5-trifluorophenyl)-1*H*-1,2,3-triazol-1-yl)tetrahydro-2*H*-pyran-2-ol (22 mg, 0.035 mmol) was dissolved in 1 mL of dimethylsulfoxide, and then Ac₂O (0.3 mL) was added dropwise thereto, followed by stirring at room temperature for 18 hours. Ethyl acetate and a saturated aqueous sodium hydrogen carbonate solution were added thereto, and extracted to obtain an organic layer. The organic layer was dried over anhydrous sodium sulfate, filtered, and then concentrated. The concentrated reaction mixture was subjected to silica gel column chromatography with 18% ethyl acetate/n-hexane to obtain the title compound as a white solid (14 mg, yield: 64%).

¹H-NMR (400 MHz, CDCl₃) δ 7.38-7.27 (m, 5H), 7.25-7.19 (m, 3H), 7.18-7.04 (m, 7H), 6.99 (d, J = 5.7 Hz, 3H), 5.14 (dd, J = 11.2, 2.3 Hz, 1H), 4.97 (d, J = 11.7 Hz, 1H), 4.81 (d, J = 11.7 Hz, 1H), 4.72-4.63 (m, 2H), 4.58 -4.44 (m, 3H), 4.39 (s, 1H), 4.04 (d, J = 11.2 Hz, 1H), 3.80 (d, J = 7.4 Hz, 2H).

### Synthesis of (E)-methyl 2-((3R,4S,5R,6R)-3,5-bis(benzyloxy)-6-((benzyloxy)methyl)-4-(4-(3,4,5-trifluorophenyl)-1H-1,2,3-triazol-1-yl)tetrahydro-2H-pyran-2-ylidene)acetate

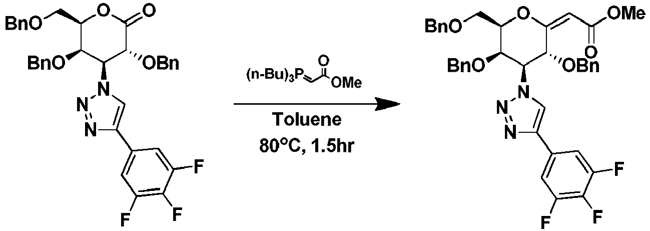

Methyl 2-(tributylphosphoranylidene)acetate (12 mg, 0.044 mmol) was dissolved in 1 mL of toluene, and then (3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-one (14 mg, 0.022 mmol) was dissolved in 1 mL of toluene, and the mixture was added dropwise thereto. The temperature was elevated to 80 °C and the reaction mixture was stirred for 1.5 hours and concentrated. The concentrated reaction mixture was subjected to silica gel column chromatography with 20% ethyl acetate/n-hexane to obtain the title compound as a white solid (10 mg, yield: 66%).

¹H-NMR (400 MHz, CDCl₃) δ 7.35-7.21 (m, 9H), 7.17 (s, 1H), 7.16-7.12 (m, 2H), 7.08-6.97 (m, 6H), 5.72 (d, J = 1.5 Hz, 1H), 5.07 (dd, J = 10.5, 3.3 Hz, 1H), 4.67-4.58 (m, 3H), 4.52 (dd, J = 11.4, 4.5 Hz, 2H), 4.31-4.21 (m, 2H), 3.96-3.86 (m, 1H), 3.83 (d, J = 7.4 Hz, 2H), 3.74 (s, 3H).

### Synthesis of (E)-2-((3R,4S,5R,6R)-3,5-bis(benzyloxy)-6-((benzyloxy)methyl)-4-(4-(3,4,5-trifluorophenyl)-1H-1,2,3-triazol-1-yl)tetrahydro-2H-pyran-2-ylidene)ethanol

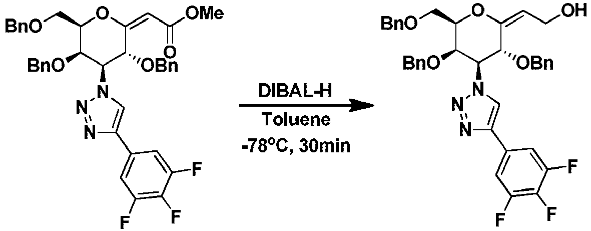

(E)-Methyl 2-((3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ylidene)acetate (10 mg, 0.015 mmol) was dissolved in 1 mL of toluene, and then 1.0M DIBAL-H (32 µL, 0.032 mmol) was added dropwise thereto at -78 °C, followed by stirring at -78 °C for 30 minutes. A solution obtained by dissolving potassium sodium tartrate tetrahydrate (45 mg, 0.160 mmol) in 2 mL of distilled water was slowly added dropwise at -78 °C to terminate the reaction, and then 4 mL of ethyl acetate was added dropwise thereto, followed by stirring at room temperature for 1 hour. The reaction mixture was extracted with ethyl acetate to obtain an organic layer. The organic layer was dried over anhydrous sodium sulfate, filtered, and then concentrated. The concentrated reaction mixture was subjected to silica gel column chromatography with 33% ethyl acetate/n-hexane to obtain the title compound as a white solid (9 mg, yield: 94%).

¹H-NMR (400 MHz, CDCl₃) δ 7.37-7.26 (m, 6H), 7.23 (dd, J = 11.2, 5.2 Hz, 2H), 7.17-7.12 (m, 3H), 7.10 (s, 1H), 7.08-6.98 (m, 6H), 5.55 (td, J = 6.4, 1.5 Hz, 1H), 4.96 (dd, J = 10.9, 3.3 Hz, 1H), 4.67 (d, J = 11.8 Hz, 1H), 4.59-4.50 (m, 3H), 4.39-4.28 (m, 4H), 4.14-4.11 (m, 1H), 4.04 (t, J = 6.8 Hz, 1H), 3.82-3.75 (m, 2H), 3.71-3.66 (m, 1H).

### Synthesis of (E)-2-((3R,4S,5R,6R)-3,5-bis(benzyloxy)-6-((benzyloxy)methyl)-4-(4-(3,4,5-trifluorophenyl)-1H-1,2,3-triazol-1-yl)tetrahydro-2H-pyran-2-ylidene)ethyl acetate

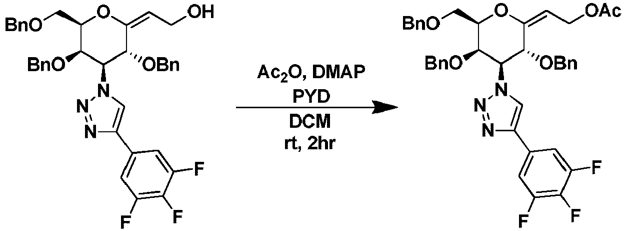

(*E*)-2-((3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ylidene)ethanol (9 mg, 0.014 mmol) was dissolved in 1 mL of dichloromethane, and then pyridine (3 µL, 0.041 mmol), 4-dimethylaminopyridine (0.6 mg, 0.001 mmol), and Ac₂O (3 µL, 0.034 mmol) were sequentially added dropwise thereto, followed by stirring at room temperature for 1 hour. After the reaction was terminated with 2 mL of a solution of distilled water and 1N aqueous hydrochloric acid solution (1:1), the reaction mixture was extracted with dichloromethane to obtain an organic layer. The organic layer was dried over anhydrous sodium sulfate, filtered, and concentrated. The concentrated reaction mixture was concentrated twice under reduced pressure after adding dichloromethane and n-heptane to obtain the title compound as a white solid (10 mg, yield: 100%).

¹H-NMR (400 MHz, CDCl₃) δ 7.37-7.27 (m, 6H), 7.26-7.21 (m, 3H), 7.14-7.12 (m, 3H), 7.10 (s, 1H), 7.09-7.00 (m, 5H), 5.47-5.44 (m, 1H), 4.96 (dd, J = 10.9, 3.2 Hz, 1H), 4.89 (dd, J = 12.0, 8.2 Hz, 1H), 4.72-4.63 (m, 2H), 4.61-4.48 (m, 3H), 4.48-4.42 (m, 1H), 4.29 (d, J = 10.1 Hz, 1H), 4.16 (d, J = 2.2 Hz, 1H), 4.11-4.05 (m, 1H), 3.85-3.76 (m, 3H), 2.20-2.00 (m, 3H).

### Synthesis of 1-((3'R,4'S,5'R,6'R)-3',5'-bis(benzyloxy)-6'-((benzyloxy)methyl)-6-methoxy-3',4',5',6'-tetrahydrospiro[chromane-2,2'-pyran]-4'-yl)-4-(3,4,5-trifluorophenyl)-1H-1,2,3-triazole & 1-((3'R,4'S,5'R,6'R)-3',5'-bis(benzyloxy)-6'-((benzyloxy)methyl)-6-methoxy-3',4',5',6'-tetrahydrospiro[chromane-2,2'-pyran]-4'-yl)-4-(3,4,5-trifluorophenyl)-1H-1,2,3-triazole

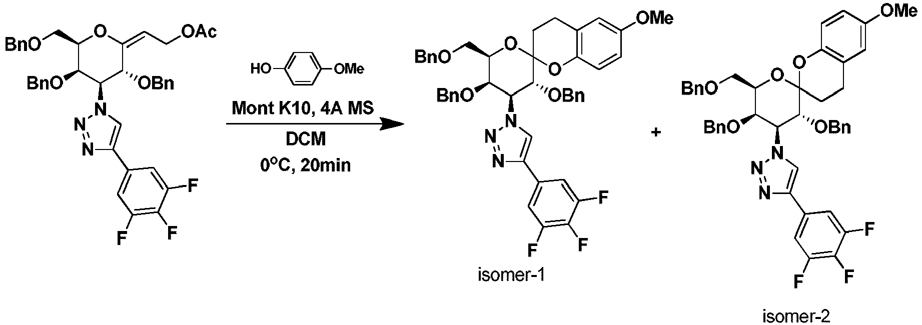

(*E*)-2-((3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2H-pyran-2-ylidene)ethyl acetate (30 mg, 0.043 mmol) was dissolved in 3 mL of dichloromethane, and then 4 Å molecular sieves (60 mg) was added dropwise thereto, followed by stirring at room temperature for 10 minutes. Then, 4-methoxyphenol (6 mg, 0.051 mmol) and montmorillonite K10 (60 mg) were sequentially added dropwise thereto, followed by stirring at 50 °C for 16 hours. The reaction mixture was cooled to room temperature, filtered through Celite, and the filrate was concentrated under reduced pressure. The concentrated filtrate was extracted with ethyl acetate, dried over anhydrous sodium sulfate, filtered, and concentrated. The concentrated reaction mixture was subjected to silica gel column chromatography with 15% ethyl acetate/n-hexane, and finally purified by preparative thin layer chromatography to obtain isomer-1 of the title as a white solid (4 mg, yield 12%) and isomer-2 of the title as a white solid (4 mg, yield 12%).

Isomer-1: ¹H-NMR (400 MHz, CDCl₃) δ 7.67 (s, 1H), 7.31-7.27 (m, 3H), 7.21 -7.16 (m, 4H), 7.14 -7.04 (m, 6H), 7.02-6.97 (m, 2H), 6.94-6.85 (m, 5H), 6.81-6.72 (m, 6H), 6.64 (d, J = 2.8 Hz, 3H), 6.54 (dd, J = 8.8, 2.9 Hz, 1H), 6.36 (d, J = 2.9 Hz, 1H), 5.69 (dd, J = 11.2, 3.1 Hz, 1H), 5.26 (s, 1H), 4.56-4.41 (m, 5H), 4.39-4.31 (m, 3H), 4.30-4.25 (m, 1H), 4.18 (s, 1H), 3.87 (d, J = 10.9 Hz, 1H), 3.81 (s, 2H), 3.77-3.71 (m, 10H), 3.67 (s, 3H), 3.51 (dd, J = 9.1, 5.4 Hz, 1H), 3.13-3.03 (m, 1H), 2.72-2.63 (m, 1H), 2.33-2.24 (m, 1H), 2.10-2.01 (m, 2H).

Isomer-2: ¹H-NMR (400 MHz, CDCl₃) δ 7.32-7.27 (m, 2H), 7.23-7.19 (m, 5H), 7.13-7.07 (m, 4H), 7.07-7.01 (m, 2H), 7.02 - 6.92 (m, 5H), 6.79-6.73 (m, 1H), 6.66 (d, J = 2.9 Hz, 1H), 5.02 (dd, J = 11.6, 3.2 Hz, 1H), 4.64 (d, J = 11.9 Hz, 1H), 4.55 (d, J = 11.8 Hz, 1H), 4.47 (q, J = 11.7 Hz, 2H), 4.36 (dd, J = 25.1, 11.5 Hz, 2H), 4.06 (d, J = 3.0 Hz, 1H), 4.00-3.93 (m, 1H), 3.79 (s, 3H), 3.78 - 3.70 (m, 2H), 3.64-3.61 (m, 1H), 2.86-2.79 (m, 1H), 2.76 -2.68 (m, 1H), 2.44-2.33 (m, 1H), 2.20 (td, J = 14.2, 5.4 Hz, 1H), 2.09-1.96 (m, 4H).

### Synthesis of (3'R,4'S,5'R,6'R)-6'-(hydroxymethyl)-6-methoxy-4'-(4-(3,4,5-trifluorophenyl)-1H-1,2,3-triazol-1-yl)-3',4',5',6'-tetrahydrospiro[chromane-2,2'-pyran]-3',5'-diol, isomer-1

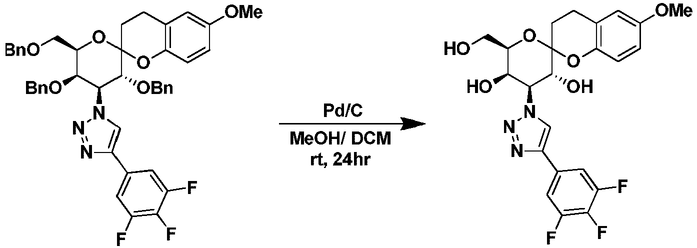

1-((3'*R*,4'*S*,5'*R*,6'*R*)-3',5'-bis(benzyloxy)-6'-((benzyloxy)methyl)-6-methoxy-3',4',5',6'-tetrahydrospiro[chromane-2,2'-pyran]-4'-yl)-4-(3,4,5-trifluorophenyl)-1*H*-1,2,3-triazole and isomer-1 (4 mg, 0.005 mmol) were dissolved in 2 mL of a solution of dichloromethane:methanol (1:2), and the mixture was added dropwise to a round-bottom flask, in which Pd/C (1 mg) was dissolved in 1 mL of a solution of dichloromethane:methanol (1:2), and degassed with a hydrogen balloon for 10 minutes, followed by stirring in a hydrogen atmosphere at room temperature for 24 hours. The reaction solution was filtered through Celite, concentrated, and subjected to silica gel column chromatography with 50% ethyl acetate/n-hexane to obtain the title isomer-1 (A10) as a white solid (23 mg, yield: 76%).

ESIMS: m/z 494.46 [M+H]+; ¹H-NMR (400 MHz, CDCl₃) δ 8.19 (s, 1H), 7.51-7.45 (m, 2H), 6.84 (d, J = 8.8 Hz, 1H), 6.77-6.71 (m, 1H), 6.67 (d, J = 3.0 Hz, 1H), 5.19 (dd, J = 10.8, 2.4 Hz, 1H), 4.58 (s, 1H), 4.43-4.32 (m, 1H), 4.26 (s, 1H), 4.03 (d, J = 4.0 Hz, 1H), 3.92 (s, 3H), 3.10 (s, 1H), 2.72 (dd, J = 16.9, 7.5 Hz, 1H), 2.45 -2.36 (m, 1H), 2.21-2.19 (m, 1H), 2.11-2.07 (m, 3H), 2.05-2.01 (m, 1H).

### Synthesis of (3'R,4'S,5'R,6'R)-6'-(hydroxymethyl)-6-methoxy-4'-(4-(3,4,5-trifluorophenyl)-1H-1,2,3-triazol-1-yl)-3',4',5',6'-tetrahydrospiro[chromane-2,2'-pyran]-3',5'-diol, isomer-2

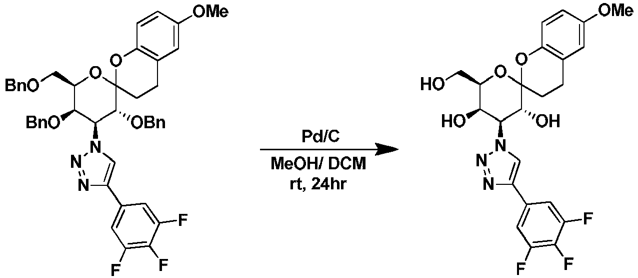

1-((3'*R*,4'*S*,5'*R*,6'*R*)-3',5'-bis(benzyloxy)-6'-((benzyloxy)methyl)-6-methoxy-3',4',5',6'-tetrahydrospiro[chromane-2,2'-pyran]-4'-yl)-4-(3,4,5-trifluorophenyl)-1H-1,2,3-triazole and isomer-2 (4 mg, 0.005 mmol) were dissolved in 2 mL of a solution of dichloromethane:methanol (1:2), and the mixture was added dropwise to a round-bottom flask, in which Pd/C (1 mg) was dissolved in 1 mL of a solution of dichloromethane:methanol (1:2), and degassed with a hydrogen balloon for 10 minutes, followed by stirring in a hydrogen atmosphere at room temperature for 24 hours. The reaction solution was filtered with celite, concentrated, and subjected to silica gel column chromatography with 50% ethyl acetate/n-hexane to obtain the title isomer-2 (A11) as a white solid (23 mg, yield: 76%).

ESIMS: m/z 494.40 [M+H]+; ¹H-NMR (400 MHz, CD₃OD) δ 8.57 (s, 1H), 7.63 (dd, J = 8.7, 6.7 Hz, 2H), 6.73 (d, J = 8.6 Hz, 1H), 6.68-6.61 (m, 2H), 5.08 (dd, J = 11.7, 3.0 Hz, 1H), 4.63 (d, J = 11.7 Hz, 1H), 4.58 (s, 1H), 4.10 (d, J = 2.6 Hz, 1H), 3.94 (t, J = 6.1 Hz, 1H), 3.71(s, 3H), 2.97-2.91 (m, 1H), 2.70 (dd, J = 16.5, 4.3 Hz, 1H), 2.54 (dd, J = 14.4, 3.6 Hz, 1H), 2.13 (td, J = 14.1, 5.2 Hz, 1H), 2.06-1.97 (m, 1H).

### Example 4: Synthesis of Compound A12

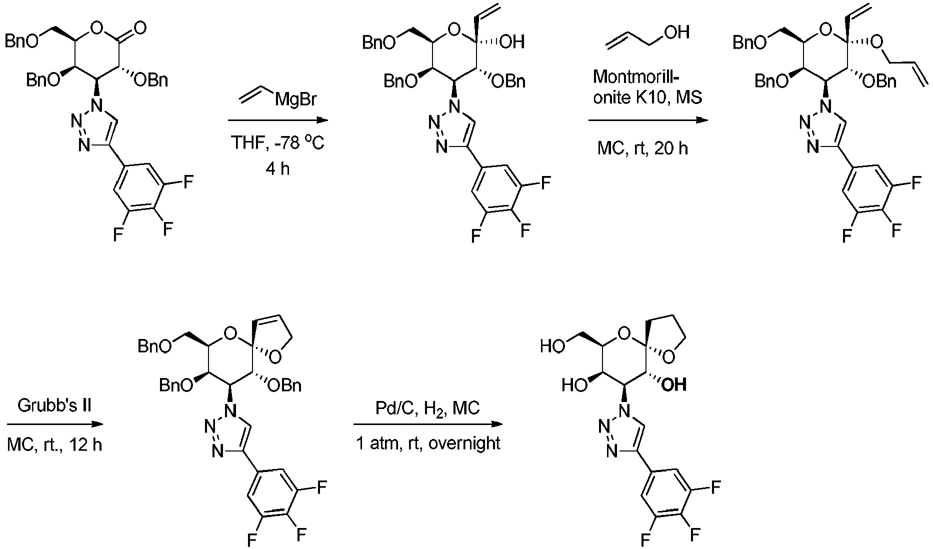

### Synthesis of (2S,3R,4S,5R,6R)-3,5-bis(benzyloxy)-6-((benzyloxy)methyl)-4-(4-(3,4,5-trifluorophenyl)-1H-1,2,3-triazol-1-yl)-2-vinyltetrahydro-2H-pyran-2-ol

(3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-one (50.0 mg, 0.079 mmol) was dissolved in 5.0 mL of tetrahydrofuran, then 1.0 M vinylmagnesium bromide (tetrahydrofuran solution) (111 µL, 0.111 mmol) was added dropwise thereto at -78 °C, and then the mixture was stirred at -78 °C for 4 hours. Then, a saturated aqueous sodium hydrogen carbonate solution was added thereto at 0 °C to terminate the reaction, followed by stirring from 0 °C to room temperature for 30 minutes. The reaction mixture was extracted with ethyl acetate to recover an organic layer. The organic layer was dried over anhydrous sodium sulfate, filtered, and then concentrated. The concentrated reaction mixture was subjected to silica gel column chromatography with 0% to 30% ethyl acetate/n-hexane to obtain the title compound as a colorless oil (33.8 mg, yield: 65.1%).

¹H-NMR (400 MHz, CDCl₃) δ 7.48 (s, 1H), 7.40-7.26 (m, 7H), 7.17-7.03 (m, 8H), 6.85 (d, J = 6.2 Hz, 2H), 6.14 (dd, J = 17.0, 10.3 Hz, 1H), 5.73 (d, J = 17.2 Hz, 1H), 5.46 (d, J = 10.6 Hz, 1H), 5.24 (dd, J = 10.9, 3.2 Hz, 1H), 4.56 (d, J = 11.8 Hz, 1H), 4.53-4.45 (m, 4H), 4.17 (dd, J = 11.1, 6.5 Hz, 2H), 4.11 (d, J = 3.1 Hz, 1H), 3.90 (d, J = 11.0 Hz, 1H), 3.75 (t, J = 9.2 Hz, 1H), 3.68 (dd, J = 9.2, 5.7 Hz, 1H)

### Synthesis of 1-((2S,3R,4S,5R,6R)-3,5-bis(benzyloxy)-6-((benzyloxy)methyl)-2-vinyltetrahydro-2H-pyran-4-yl)-4-(3,4,5-trifluorophenyl)-1H-1,2,3-triazole

(2*S*,3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)-2-vinyltetrahydro-2*H*-pyran-2-ol (30 mg, 0.046 mmol), 4 Å molecular sieves (240 wt%, 72 mg), and montmorillonite K-10 (240 wt%, 72 mg) were dissolved in 3 mL of dichloromethane, and then allyl alcohol (16 µL, 0.274 mmol) was added dropwise thereto, followed by stirring at room temperature for 20 hours. The reaction mixture was filtered through Celite and then concentrated and subjected to silica gel column chromatography with 0% to 30% ethyl acetate/n-hexane to obtain the title compound as a white solid (26.0 mg, yield: 81.2%).

¹H-NMR (400 MHz, CDCl₃) δ 7.48 (s, 1H), 7.36-7.26 (m, 7H), 7.13-7.03 (m, 8H), 6.90-6.87 (m, 2H), 6.11-5.92 (m, 3H), 5.70 (dd, J = 17.5, 1.7 Hz, 1H), 5.52-5.47 (m, 2H), 5.37-5.28 (m, 1H), 5.19 (dd, J = 10.4, 1.6 Hz, 1H), 4.59-4.48 (m, 3H), 4.45 (d, J = 11.1 Hz, 1H), 4.31 (d, J = 11.5 Hz, 1H), 4.18 (dd, J = 13.2, 5.9 Hz, 2H), 4.12-4.03 (m, 1H), 3.95 (dd, J = 12.9, 5.8 Hz, 1H), 3.80 (dd, J = 20.3, 9.8 Hz, 2H), 3.67 (dd, J = 9.2, 5.7 Hz, 1H).

### Synthesis of 1-((5S,7R,8R,9S,10R)-8,10-bis(benzyloxy)-7-((benzyloxy)methyl)-1,6-dioxaspiro[4.5]dec-3-en-9-yl)-4-(3,4,5-trifluorophenyl)-1H-1,2,3-triazole

1-((2*S*,3*R*,4*S*,5*R*,6*R*)-2-(allyloxy)-3,5-bis(benzyloxy)-6-((benzyloxy)methyl)-2-vinyltetrahydro-2*H*-pyran-4-yl)-4-(3,4,5-trifluorophenyl)-1*H*-1,2,3-triazole (25.0 mg, 0.036 mmol) was dissolved in 5.0 mL of dichloromethane, and then Grubbs catalyst II (1.8 mg, 0.002 mmol) was added dropwise thereto, and the mixture was stirred at room temperature for 12 hours. The reaction mixture was concentrated, and then subjected to silica gel column chromatography with 0% to 20% ethyl acetate/n-hexane to obtain the title compound as a colorless oil (25.0 mg).

¹H-NMR (400 MHz, CDCl₃) δ 7.41 (s, 1H), 7.36-7.27 (m, 6H), 7.25-7.19 (m, 3H), 7.17-7.03 (m, 6H), 6.91-6.83 (m, 2H), 6.38 (d, J = 6.0 Hz, 1H), 5.96-5.86 (m, 1H), 5.34 (dd, J = 11.3, 3.1 Hz, 1H), 4.80 (dd, J = 27.3, 13.7 Hz, 2H), 4.56-4.38 (m, 6H), 4.23 (d, J = 11.5 Hz, 1H), 4.14 (d, J = 2.0 Hz, 1H), 3.83 (d, J = 10.9 Hz, 1H), 3.72 (t, J = 9.0 Hz, 1H), 3.67-3.60 (m, 1H).

### Synthesis of (5S,7R,8R,9S,10R)-7-(hydroxymethyl)-9-(4-(3,4,5-trifluorophenyl)-1H-1,2,3-triazol-1-yl)-1,6-dioxaspiro[4.5]decane-8,10-diol

1-((5*S*,7*R*,8*R*,9*S*,10*R*)-8,10-bis(benzyloxy)-7-((benzyloxy)methyl)-1,6-dioxaspiro[4.5]dec-3-en-9-yl)-4-(3,4,5-trifluorophenyl)-1*H*-1,2,3-triazole (25.0 mg, 0.037 mmol) was dissolved in 6.0 mL of a solution of dichloromethane:methanol (1:2), and the mixture was added dropwise to a round-bottom flask, in which 10% Pd/C (7.5 mg, 30 wt%) was dissolved in 6.0 mL of a solution of dichloromethane:methanol (1:2), and degassed with a hydrogen balloon for 10 minutes. The reaction solution was stirred at room temperature in a hydrogen atmosphere for 24 hours. The reaction solution was filtered through Celite and then concentrated and subjected to silica gel column chromatography with 0% to 5% methanol/dichloromethane to obtain the title compound as a white solid (18.9 mg, yield: 99%).

ESIMS: m/z 402.35 [M+H]+; ¹H-NMR (400 MHz, CD₃OD) δ 8.50 (s, 1H), 7.64 (dd, J = 8.6, 6.8 Hz, 2H), 5.03 (dd, J = 11.2, 2.8 Hz, 1H), 4.42 (d, J = 11.1 Hz, 1H), 4.15-3.98 (m, 4H), 3.68 (d, J = 6.1 Hz, 2H), 2.30 (dd, J = 19.7, 9.2 Hz, 1H), 2.11 (dd, J = 18.2, 7.0 Hz, 1H), 2.05-1.84 (m, 2H).

### Example 5: Synthesis of Compound A13

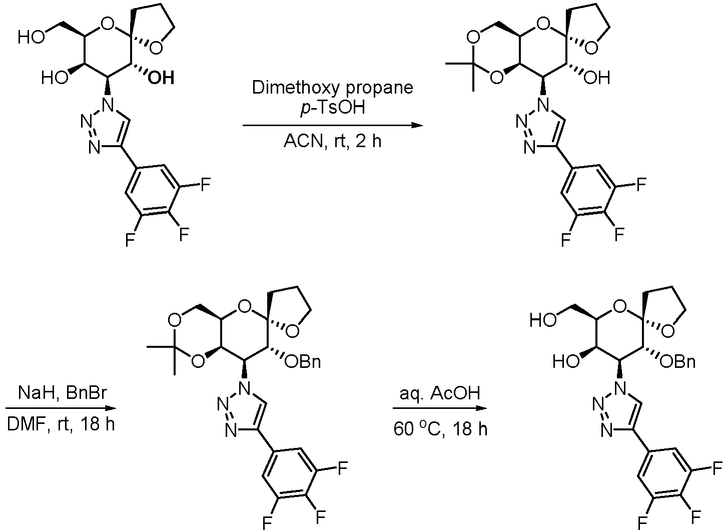

### Synthesis of (2S,4a'R,7'R,8'R,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-ol

(5S,7R,8R,9S,10R)-7-(hydroxymethyl)-9-(4-(3,4,5-trifluorophenyl)-1H-1,2,3-triazol-1-yl)-1,6-dioxaspiro[4.5]decane-8,10-diol (20.6 mg, 0.051 mmol) was dissolved in 5.0 mL of acetonitrile, and (1S)-(+)-10-camphorsulfonic acid (2.4 mg, 0.010 mmol) and dimethylpropane (9.4 µL, 0.077 mmol) were sequentially slowly added dropwise, followed by stirring at room temperature for 2 hours. A saturated aqueous sodium hydrogen carbonate solution was added to terminate the reaction, and then the reaction mixture was extracted with dichloromethane to recover an organic layer. The organic layer was dried over anhydrous magnesium sulfate, filtered, and concentrated under reduced pressure. The concentrate was subjected to silica gel column chromatography with 0% to 30% ethyl acetate/n-hexane to obtain the title compound as a white solid (19.6 mg, yield: 87.1%).

¹H-NMR (400 MHz, CDCl₃) δ 7.97 (s, 1H), 7.50-7.44 (m, 2H), 5.10 (dd, J = 10.9, 3.4 Hz, 1H), 4.39 (d, J = 2.6 Hz, 1H), 4.37-4.29 (m, 1H), 4.10 (s, 1H), 4.04 (m, 2H), 3.91 (d, J = 14.3 Hz, 1H), 3.81 (s, 1 H), 2.37-2.32 (m, 1H), 2.22-2.11 (m, 2H), 1.47 (s, 3H), 1.33 (s, 3H).

### Synthesis of 1-((4a'R,7'R,8'S,8a'R)-7'-(benzyloxy)-2',2'-dimethyltetrahydro-4'H-spiro[cyclopentane-1,6'-pyrano[3,2-d][1,3]dioxin]-8'-yl)-4-(3,4,5-trifluorophenyl)-1H-1,2,3-triazole

(2S,4a'R,7'R,8'R,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-ol (9.0 mg, 0.020 mmol) was dissolved in 1.0 mL of *N*,*N*-dimethylformamide, and then 60% NaH (1.3 mg, 0.033 mmol) and benzyl bromide (3.6 µL, 0.033 mmol) were slowly added thereto at 0 °C, followed by stirring for 18 hours while elevating the temperature from 0 °C to room temperature. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (9.7 mg, yield: 91.5%).

¹H-NMR (400 MHz, CDCl₃) δ 7.90 (s, 1H), 7.42-7.37 (m, 2H), 7.16 (m, 3H), 6.95 (d, J = 6.6 Hz, 2H), 5.47 (dd, J = 11.01, 3.27 Hz, 1H), 4.36 (m, 1H), 4.32 (d, J = 3.3 Hz, 1H), 4.11 (m, 1H), 4.08 (m, 1H), 4.02 (m, 3H), 3.92 (d, J = 1.7 Hz, 1H), 3.89 (d, J = 1.7 Hz, 1H), 3.85 (dd, J = 3.6, 2.1 Hz, 1H), 2.21-2.11 (m, 4H), 1.52 (s, 3H), 1.33 (s, 3H).

### Synthesis of (5S,7R,8R,9S,10R)-10-(benzyloxy)-7-(hydroxymethyl)-9-(4-(3,4,5-trifluorophenyl)-1H-1,2,3-triazol-1-yl)-1,6-dioxaspiro[4.5]decan-8-ol

1-((4a'R,7'R,8'S,8a'R)-7'-(benzyloxy)-2',2'-dimethyltetrahydro-4'*H*-spiro[cyclopentane-1,6'-pyrano[3,2-*d*][1,3]dioxin]-8'-yl)-4-(3,4,5-trifluorophenyl)-1H-1,2,3-triazole (8.0 mg, 0.015 mmol) was dissolved in 1.0 mL of a 50% aqueous acetic acid solution, followed by stirring at 60 °C for 18 hours. The reaction mixture was concentrated, neutralized by adding a saturated aqueous ammonium chloride solution thereto, and then extracted with dichloromethane. An organic layer was recovered, and dried over anhydrous magnesium sulfate, filtered, and concentrated under reduced pressure to perform silica gel column chromatography with 0% to 5% methanol/dichloromethane to obtain the title compound as a white solid (7.6 mg, yield: 95%).

ESIMS: m/z 492.19 [M+H]+; ¹H-NMR (400 MHz, CDCl₃) δ 7.91 (s, 1H), 7.34 (dd, J = 8.4, 6.5 Hz, 2H), 7.21-7.18 (m, 3H), 7.01-6.98 (m, 2H), 4.94 (dd, J = 10.7, 2.3 Hz, 1H), 4.62 (s, 1H), 4.52 (s, 1H), 4.39 (d, J = 10.7 Hz, 1H), 4.25 (d, J = 11.0 Hz, 1H), 4.13-3.87 (m, 6H), 2.25-2.19 (m, 1H), 2.09 (m, 3H), 2.01-1.93 (m, 1H).

### Example 6: Synthesis of Compound A16

### Synthesis of 3-((((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)methyl)pyridine

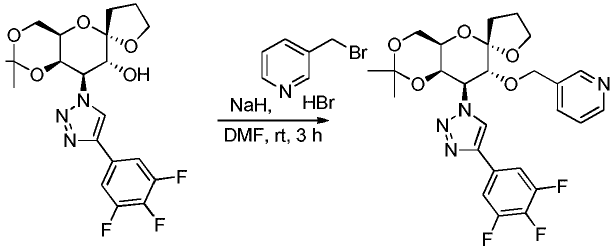

(2S,4a'R,7'R,8'R,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-ol (15.0 mg, 0.034 mmol) was dissolved in 1.0 mL of *N*,*N*-dimethylformamide, and then 60% NaH (2 mg) and 3-(bromomethyl)pyridine hydrobromide (9.5 mg, 0.037 mmol) were sequentially slowly added thereto at 0 °C, followed by stirring for 3 hours while elevating the temperature from 0 °C to room temperature. The reaction solution was diluted with ethyl acetate and then washed twice with a saturated aqueous ammonium chloride solution. The organic layer was recovered, dried over anhydrous sodium sulfate, filtered, and then concentrated under reduced pressure. The concentrate was subjected to silica gel column chromatography with 0% to 2.5% methanol/dichloromethane to obtain the title compound as a white solid (12.1 mg).

¹H-NMR (400 MHz, CDCl₃) δ 8.38 (d, J = 3.8 Hz, 1H), 8.28 (s, 1H), 7.99 (s, 1H), 7.45-7.38 (m, 2H), 7.26-7.21 (m, 1H), 7.10 (dd, J = 7.8, 4.8 Hz, 1H), 5.47 (dd, J = 11.0, 3.3 Hz, 1H), 4.42-4.36 (m, 2H), 4.32 (d, J = 11.0 Hz, 1H), 4.13-3.97 (m, 4H), 3.91 (dd, J = 13.0, 1.6 Hz, 1H), 3.87-3.84 (m, 1H), 2.22-2.06 (m, 3H), 1.99-1.88 (m, 1H), 1.53 (s, 3H), 1.35 (s, 3H).

### Synthesis of (5S,7R,8R,9S,10R)-7-(hydroxymethyl)-10-(pyridin-3-ylmethoxy)-9-(4-(3,4,5-trifluorophenyl)-1H-1,2,3-triazol-1-yl)-1,6-dioxaspiro[4.5]decan-8-ol acetate

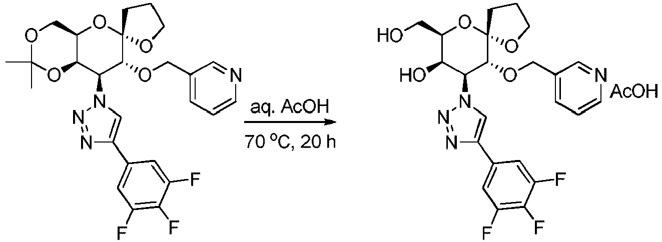

3-((((2*S*,4a'*R*,7'*R*,8'*S*,8a'*R*)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-yl)oxy)methyl)pyridine (12.0 mg) was dissolved in 2.0 mL of a 50% aqueous acetic acid solution, followed by stirring at 70 °C for 20 hours. The reaction mixture was concentrated under reduced pressure to perform silica gel column chromatography with 0% to 5% methanol/dichloromethane to obtain the title compound as a white solid (12 mg).

ESIMS: m/z 493.20 [M+H]+; ¹H-NMR (400 MHz, CDCl₃) δ 8.44 (s, 1H), 8.31 (s, 1H), 7.99 (s, 1H), 7.38 (t, J = 6.3 Hz, 2H), 7.29-7.25 (m, 1H), 7.16-7.09 (m, 1H), 5.03 (dd, J = 10.7, 2.3 Hz, 1H), 4.56 (s, 1H), 4.47 (s, 1H), 4.44 (d, J = 10.7 Hz, 1H), 4.32 (d, J = 11.3 Hz, 1H), 4.12-3.90 (m, 6H), 3.75 (s, 3H), 2.17-2.08 (m, 4H), 2.02-1.91 (m, 2H)

### Example 7: Synthesis of Compound A17

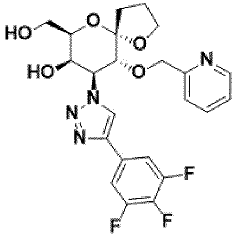

### Synthesis of (5S,7R,8R,9S,10R)-7-(hydroxymethyl)-10-(pyridin-2-ylmethoxy)-9-(4-(3,4,5-trifluorophenyl)-1H-1,2,3-triazol-1-yl)-1,6-dioxaspiro[4.5]decan-8-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5S,7R,8R,9S,10R)-7-(hydroxymethyl)-10-(pyridin-3-ylmethoxy)-9-(4-(3,4,5-trifluorophenyl)-1*H*-1,2,3-triazol-1-yl)-1,6-dioxaspiro[4.5]decan-8-ol acetate.

ESIMS: m/z 493.52 [M+H]+; ¹H-NMR (400 MHz, CDCl₃) δ 8.42 (d, J = 4.0 Hz, 1H), 8.09 (s, 1H), 7.53 (ddd, J = 7.7, 5.7, 1.8 Hz, 1H), 7.38 (dd, J = 8.5, 6.5 Hz, 2H), 7.09 (dd, J = 7.2, 5.4 Hz, 1H), 7.04 (d, J = 7.8 Hz, 1H), 5.06 (dd, J = 10.7, 2.4 Hz, 1H), 4.55 (s, 1H), 4.52-4.48 (m, 2H), 4.47 (d, J = 4.0 Hz, 1H), 4.15-3.99 (m, 4H), 3.98-3.90 (m, 2H), 2.31-2.06 (m, 4H), 2.03-1.90 (m, 1H).

### Example 8: Synthesis of Compound A18

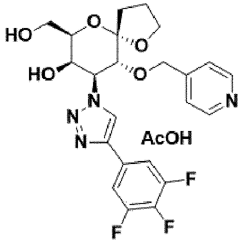

### Synthesis of (5S,7R,8R,9S,10R)-7-(hydroxymethyl)-10-(pyridin-4-ylmethoxy)-9-(4-(3,4,5-trifluorophenyl)-1H-1,2,3-triazol-1-yl)-1,6-dioxaspiro[4.5]decan-8-ol acetate

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5S,7R,8R,9S,10R)-7-(hydroxymethyl)-10-(pyridin-3-ylmethoxy)-9-(4-(3,4,5-trifluorophenyl)-1*H*-1,2,3-triazol-1-yl)-1,6-dioxaspiro[4.5]decan-8-ol acetate.

ESIMS: m/z 493.52 [M+H]+; ¹H-NMR (400 MHz, CDCl₃) δ 8.44 (s, 2H), 7.99 (s, 1H), 7.36 (dd, J = 8.3, 6.4 Hz, 2H), 6.94 (d, J = 5.4 Hz, 2H), 5.08 (dd, J = 10.7, 2.4 Hz, 1H), 4.47 (dd, J = 2.3, 1.2 Hz, 1H), 4.44 (d, J = 10.8 Hz, 1H), 4.34 (d, J = 12.5 Hz, 1H), 4.13-4.02 (m, 1H), 4.01-3.89 (m, 6H), 3.74 (s, 3H), 2.19-2.07 (m, 4H), 2.03-1.89 (m, 2H).

### Example 9: Synthesis of Compound A19

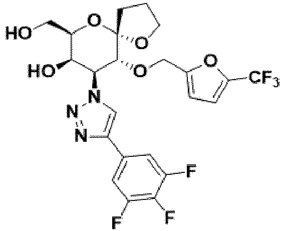

### Synthesis of (55,7R,SR,9S, 105)-7-(hydroxymethyl)-10-((5-(trifluoromethyl)furan-2-yl)methoxy)-9-(4-(3,4,5-trifluorophenyl)-1H-1,2,3-triazol-1-yl)-1,6-dioxaspiro[4.5]decan-8-ol

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 550.22 [M+H]+; ¹H-NMR (400 MHz, CDCl₃) δ 8.07 (s, 1H), 7.46 (dd, J = 8.4, 6.4 Hz, 2H), 6.64-6.60 (m, 1H), 6.17 (d, J = 3.4 Hz, 1H), 4.97 (dd, J = 10.6, 2.3 Hz, 1H), 4.52 (t, J = 1.2 Hz, 1H), 4.50-4.48 (m, 1H), 4.41 (d, J = 10.7 Hz, 1H), 4.11-3.86 (m, 7H), 2.19-1.91 (m, 5H)

### Example 10: Synthesis of Compound A20

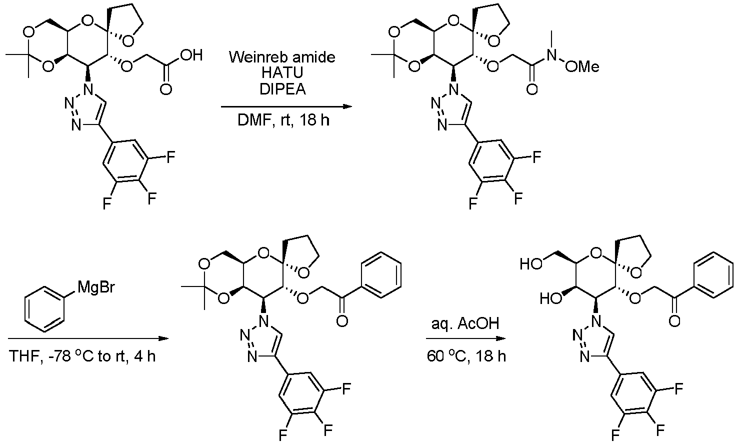

### Synthesis of 2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)-N-methoxy-N-methylacetamide

2-(((2S,4a'R,1'R,8'S,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-1'- yl)oxy)acetic acid (20.0 mg, 0.040 mmol) was dissolved in 2.0 mL of *N*,*N*-dimethylformamide, and then *N*, *O-*dimethylhydroxylamine hydrochloride (4.68.0 mg, 0.048 mmol), HATU (38.0 mg, 0.100 mmol), and *N*,*N*-diisopropylethylamine (34.8 µL, 0.200 mmol) were added dropwise thereto, followed by stirring at room temperature for 18 hours. The reaction solution was then extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 30% ethyl acetate/n-hexane to obtain the title compound as a white solid (15.0 mg, yield: 69%).

¹H-NMR (400 MHz, CDCl₃) δ 8.08 (s, 1H), 7.47 (dd, J = 8.5, 6.5 Hz, 2H), 5.46 (dd, J = 11.0, 3.3 Hz, 1H), 4.47 (d, J = 11.0 Hz, 1H), 4.35 (dd, J = 3.4, 1.2 Hz, 1H), 4.04 (tdd, J = 15.6, 13.3, 5.0 Hz, 4H), 3.91 (dd, J = 13.0, 1.7 Hz, 1H), 3.85 (d, J = 1.4 Hz, 1H), 3.70 (d, J = 14.9 Hz, 1H), 3.40 (s, 3H), 2.98 (s, 3H), 2.52 (d, J = 11.2 Hz, 1H), 2.19-2.07 (m, 2H), 2.05-1.98 (m, 1H), 1.50 (s, 3H), 1.33 (s, 3H).

### Synthesis of 2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)-1-phenylethanone

2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)-*N*-methoxy-*N-*methylacetamide (14.0 mg, 0.026 mmol) was dissolved in 2.0 mL of tetrahydrofuran, and then 1 M phenylmagnesium bromide (62.0 µL, 0.062 mmol) was slowly added dropwise thereto, followed by stirring at room temperature for 2 hours. A saturated aqueous ammonium chloride solution was added to terminate the reaction, and then the reaction mixture was extracted with dichloromethane to recover an organic layer. The organic layer was dried over anhydrous magnesium sulfate, filtered, and concentrated under reduced pressure. The concentrate was subjected to silica gel column chromatography with 0% to 30% ethyl acetate/n-hexane to obtain the title compound as a white solid (10.5 mg, yield: 72.4%).

¹H-NMR (400 MHz, CDCl₃) δ 8.00 (s, 1H), 7.65-7.61 (m, 2H), 7.48 (d, J = 7.5 Hz, 1H), 7.41-7.36 (m, 2H), 7.32 (t, J = 7.9 Hz, 2H), 5.53 (dd, J = 11.2, 3.4 Hz, 1H), 4.64 (d, J = 16.1 Hz, 1H), 4.52 (d, J = 11.1 Hz, 1H), 4.38 (d, J = 2.2 Hz, 1H), 4.20 (d, J = 16.2 Hz, 1H), 4.09 (d, J = 13.0 Hz, 1H), 4.00 (t, J = 6.8 Hz, 2H), 3.91 (d, J = 13.0 Hz, 1H), 3.86 (s, 1H), 2.54-2.44 (m, 1H), 2.20-2.06 (m, 2H), 1.99-1.90 (m, 1H), 1.50 (s, 3H), 1.33 (s, 3H).

### Synthesis of 2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)-1-phenylethanone

2-(((2S,4a'R,1'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-yl)oxy)-1-phenylethanone (8.5 mg, 0.015 mmol) was dissolved in 2.0 mL of a 50% aqueous acetic acid solution, followed by stirring at 60 °C for 18 hours. The reaction mixture was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (4.9 mg, yield: 62%).

ESIMS: m/z 520.29 [M+H]+; ¹H-NMR (400 MHz, CDCl₃) δ 8.13 (s, 1H), 7.63 (d, J = 8.1 Hz, 2H), 7.52-7.49 (m, 1H), 7.40-7.32 (m, 4H), 5.14 (dd, J = 10.7, 2.5 Hz, 1H), 4.49 (d, J = 10.6 Hz, 2H), 4.39 (d, J = 6.9 Hz, 2H), 4.12-3.96 (m, 6H), 2.44-2.40 (m, 1H), 2.17-2.11 (m, 3H), 2.04-1.98 (m, 1H).

### Example 11: Synthesis of Compound A21

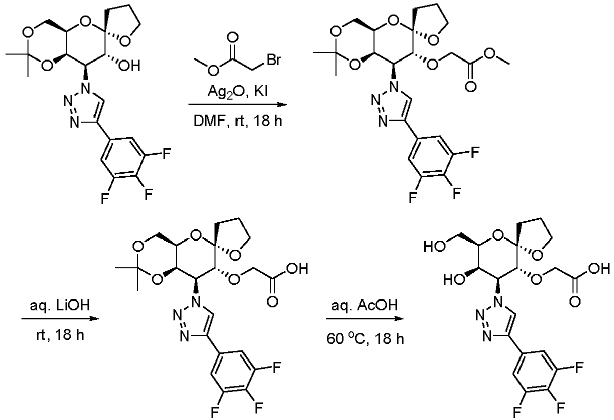

### Synthesis of 2-(((2,S',4aR,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)acetate

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (100.0 mg, 0.227 mmol) was dissolved in 5.0 mL of *N*,*N*-dimethylformamide, and then silver(I) oxide (263.0 mg, 1.133 mmol), potassium iodide (75.0 mg, 0.453 mmol), and methyl bromoacetate (32.5 µL, 0.340 mmol) were sequentially added dropwise thereto, followed by stirring at room temperature for 18 hours. The reaction solution was filtered through Celite and then extracted with ethyl acetate to recover an organic layer. The organic layer was dried over anhydrous magnesium sulfate, filtered, and then concentrated under reduced pressure. The concentrate was subjected to silica gel column chromatography with 0% to 50% ethyl acetate/n-hexane to obtain the title compound as a white solid (91 mg, yield: 78%).

¹H-NMR (400 MHz, CDCl₃) δ 8.04 (s, 1H), 7.54-7.41 (m, 2H), 5.46 (dd, J = 11.1, 3.2 Hz, 1H), 4.39 (d, J = 10.9 Hz, 2H), 4.13-3.97 (m, 3H), 3.96-3.87 (m, 2H), 3.85 (s, 1H), 3.62 (d, J = 16.0 Hz, 1H), 3.58 (s, 3H), 2.47 (dt, J = 15.6, 9.5 Hz, 1H), 2.19-2.09 (m, 2H), 1.99 (d, J = 9.4 Hz, 1H), 1.55 (s, 2H), 1.50 (s, 3H), 1.33 (s, 3H).

### Synthesis of 2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)acetic acid

Methyl 2-(((2*S*,4a'*R*,1'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)acetate (280 mg, 0.545 mmol) was dissolved in 10 mL of distilled water and 10 mL of tetrahydrofuran, and then lithium hydroxide (196 mg, 8.18 mmol) was added dropwise thereto, followed by stirring at room temperature for 18 hours. The reaction solution was diluted with ethyl acetate, the water layer was adjusted to have a pH of 5 to 6 using a 1.0 N aqueous hydrochloric acid solution, and the reaction solution was extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (278.0 mg).

### Synthesis of 2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)acetic acid

2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)acetic acid (20.0 mg, 0.039 mmol) was dissolved in 2.0 mL of a 50% aqueous acetic acid solution, followed by stirring at 60 °C for 18 hours. The reaction mixture was concentrated, then neutralized by a saturated aqueous ammonium chloride solution, and extracted with dichloromethane to recover an organic layer. The organic layer was dried over anhydrous magnesium sulfate, filtered, and then concentrated. The concentrate was subj ected to silica gel column chromatography with 0% to 5% methanol/dichloromethane (containing 1.0% trifluoroacetic acid) to obtain the title compound as a white solid (10.0 mg, yield: 55.9%).

ESIMS: m/z 460.35 [M+H]+; ¹H-NMR (400 MHz, CD₃OD) δ 8.62 (s, 1H), 7.64 (dd, J = 8.9, 6.6 Hz, 2H), 5.22 (dd, J = 11.1, 2.9 Hz, 1H), 4.50 (d, J = 11.2 Hz, 1H), 4.12-4.00 (m, 4H), 3.90 (s, 2H), 3.68 (d, J = 6.2 Hz, 2H), 2.38 (m, 1H), 2.05 (m, 1H), 2.00 (m, 2H).

### Example 12: Synthesis of Compound A22

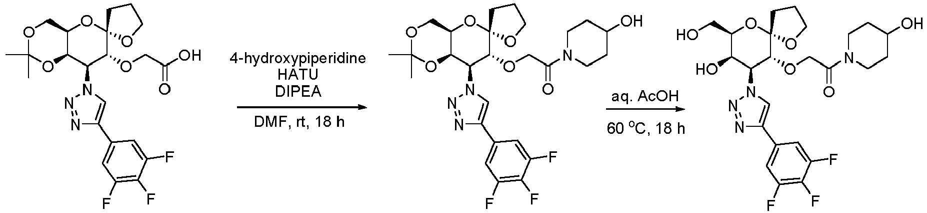

### Synthesis of 2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)-1-(4-hydroxypiperidin 1-yl)ethanone

2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-1'- yl)oxy)acetic acid (20.0 mg, 0.040 mmol) was dissolved in 2.0 mL of N,N-dimethylformamide, and then 4-hydroxypiperidine (4.9 mg, 0.048 mmol), HATU (38.0 mg, 0.100 mmol), and *N*,*N-*diisopropylethylamine (34.8 µL, 0.200 mmol) were added dropwise thereto, followed by stirring at room temperature for 18 hours. The reaction solution was extracted by adding ethyl acetate and distilled water to recover an organic layer. The organic layer was dried over anhydrous magnesium sulfate, filtered, and then concentrated. The concentrated reaction mixture was subjected to silica gel column chromatography with 0% to 30% ethyl acetate/n-hexane to obtain an intermediate as a white solid.

The intermediate was dissolved in 2.0 mL of a 50% aqueous acetic acid solution, followed by stirring at 60 °C for 18 hours. The reaction mixture was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (8.3 mg, yield: 63.8%).

ESIMS: 543.42 m/z [M+H]+; ¹H-NMR (400 MHz, CD₃OD) δ 8.62 (d, J = 4.3 Hz, 1H), 7.66 (s, 2H), 5.22 (d, J = 10.1 Hz, 1H), 4.49 (d, J = 12.8 Hz, 1H), 4.10-4.03 (m, 5H), 3.80 - 3.68 (m, 4H), 3.59 (s, 1H), 2.91-2.81 (m, 3H), 2.27-1.97 (m, 5H), 1.69-1.62 (m, 2H), 1.3 - 1.23 (m, 1H)

### Example 13: Synthesis of Compound A23

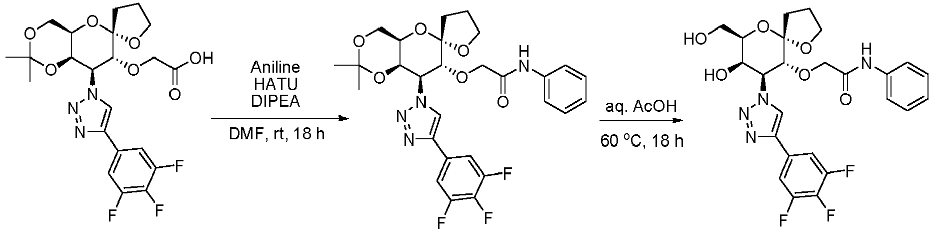

### Synthesis of 2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)-N-phenylacetamide

2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-1'- yl)oxy)acetic acid (20.0 mg, 0.040 mmol) was dissolved in 2.0 mL of N,N-dimethylformamide, and then aniline (5.4 mg, 0.060 mmol), HATU (38.0 mg, 0.100 mmol), and N,N-diisopropylethylamine (34.8 µL, 0.200 mmol) were added dropwise thereto, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrated reaction mixture was subj ected to silica gel column chromatography with 0% to 30% ethyl acetate/n-hexane to obtain the title compound as a white solid (21 mg, yield: 91.7%).

### Synthesis of 2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)-N-phenylacetamide

2-(((2S,4a'R,7'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-yl)oxy)-*N-*phenylacetamide (21.0 mg, 0.037 mmol) was dissolved in 2.0 mL of a 50% aqueous acetic acid solution, followed by stirring at 60 °C for 18 hours. The reaction mixture was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (11.7 mg, yield: 60.0%).

ESIMS: m/z 535.48 [M+H]+; ¹H-NMR (400 MHz, CD₃OD) δ 8.61 (s, 1H), 7.39 (dd, J = 8.9, 6.6 Hz, 2H), 7.32 (dd, J = 8.6, 1.1 Hz, 2H), 7.19-7.12 (m, 2H), 7.01 (t, J = 7.4 Hz, 1H), 5.34 (dd, J = 11.3, 2.9 Hz, 1H), 4.65 (d, J = 11.2 Hz, 1H), 4.28 (d, J = 15.4 Hz, 1H), 4.16 (d, J = 1.7 Hz, 1H), 4.12-4.05 (m, 3H), 3.96 (d, J = 15.4 Hz, 1H), 3.70 (d, J = 6.2 Hz, 2H), 2.27-2.23 (m, 1H), 2.20-2.07 (m, 2H), 2.02-1.94 (m, 1H).

### Example 14: Synthesis of Compound A24

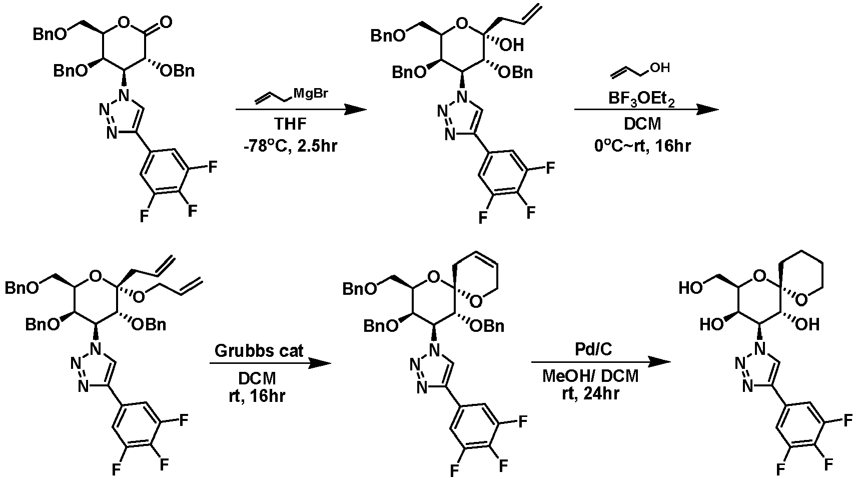

### Synthesis of (2S,3R,4S,5R,6R)-2-allyl-3,5-bis(benzyloxy)-6-((benzyloxy)methyl)-4-(4-(3,4,5-trifluorophenyl)-1H-1,2,3-triazol-1-yl)tetrahydro-2H-pyran-2-ol

(3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-one (200 mg, 0.318 mmol) was dissolved in 6 mL of tetrahydrofuran, then 1.0 M allylmagnesium bromide (ether solution) (445 µL, 0.445 mmol) was added dropwise thereto, and then the mixture was stirred at -78 °C for 1 hour. Then, 1.0 M allylmagnesium bromide (ether solution) (223 µL, 0.223 mmol) was further added dropwise thereto at -78 °C, followed by stirring at -78 °C for 30 minutes and at room temperature for 1 hour. A saturated aqueous sodium hydrogen carbonate solution was added at 0 °C to terminate the reaction, followed by stirring from 0 °C to room temperature for 30 minutes. The reaction mixture was extracted with ethyl acetate to recover an organic layer. The organic layer was dried over anhydrous sodium sulfate, filtered, and then concentrated under reduced pressure. The concentrate was subjected to silica gel column chromatography with 25% ethyl acetate/n-hexane to obtain the title compound as a colorless oil (166 mg, yield: 78%).

¹H-NMR (400 MHz, CDCl₃) δ 7.66 (s, 1H), 7.35-7.27 (m, 8H), 7.18-7.07 (m, 8H), 6.93-6.89 (m, 2H), 6.01-5.84 (m, 1H), 5.31 (dd, J = 11.0, 3.0 Hz, 2H), 5.25 (d, J = 17.3 Hz, 1H), 4.58-4.48 (m, 4H), 4.46-4.38 (m, 2H), 4.23-4.12 (m, 2H), 4.10 (d, J = 3.1 Hz, 1H), 3.93 (d, J = 11.1 Hz, 1H), 3.74 (t, J = 9.0 Hz, 1H), 3.64 (dd, J = 9.1, 5.5 Hz, 1H), 2.95 (s, 1H), 2.63 (d, J = 7.2 Hz, 2H), 2.04 (s, 1H), 1.26 (t, J = 7.1 Hz, 1H).

### Synthesis of 1-((2S,3R,4S,5R,6R)-2-allyl-2-(allyloxy)-3,5-bis(benzyloxy)-6-((benzyloxy)methyl)tetrahydro-2H-pyran-4-yl)-4-(3,4,5-trifluorophenyl)-1H-1,2,3-triazole

(2*S*,3*R*,4*S*,5*R*,6*R*)-2-allyl-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ol (84 mg, 0.125 mmol) was dissolved in 3 mL of dichloromethane, and then allyl alcohol (51 µL, 0.750 mmol) was added dropwise thereto, the temperature was lowered to 0 °C, and BF₃OEt₂ (16 µL, 0.125 mmol) was slowly added dropwise, followed by stirring for 16 hours while elevating the temperature from 0 °C to room temperature. A saturated aqueous sodium hydrogen carbonate solution was added to terminate the reaction, and then extracted with dichloromethane, dried over anhydrous sodium sulfate, filtered, and then concentrated. The concentrated reaction mixture was subjected to silica gel column chromatography with 19% ethyl acetate/n-hexane to obtain the title compound as a white solid (61 mg, yield: 69%).

¹H-NMR (400 MHz, CDCl₃) δ 7.64 (s, 1H), 7.37-7.26 (m, 9H), 7.16-7.02 (m, 7H), 6.88 (d, J = 7.7 Hz, 1H), 6.04-5.91 (m, 2H), 5.54-5.45 (m, 1H), 5.35 (d, J = 17.2 Hz, 1H), 5.23-5.19 (m, 3H), 4.60-4.40 (m, 4H), 4.36 (d, J = 11.0 Hz, 1H), 4.26 (d, J = 11.3 Hz, 1H), 4.18-4.03 (m, 4H), 3.87 (d, J = 11.2 Hz, 1H), 3.73 (t, J = 8.6 Hz, 1H), 3.65-3.62 (m, 1H), 2.79-2.56 (m, 1H), 2.71-2.60 (m, 1H).

### Synthesis of 1-((2R,3R,4S,5R,6S)-3,5-bis(benzyloxy)-2-((benzyloxy)methyl)-1,7-dioxaspiro[5.5]undec-9-en-4-yl)-4-(3,4,5-trifluorophenyl)-1H-1,2,3-triazole

1-((2*S*,3*R*,4*S*,5*R*,6*R*)-2-allyl-2-(allyloxy)-3,5-bis(benzyloxy)-6-((benzyloxy)methyl)tetrahydro-2*H*-pyran-4-yl)-4-(3,4,5-trifluorophenyl)-1*H*-1,2,3-triazole (61 mg, 0.085 mmol) was dissolved in 2 mL of dichloromethane, and then Grubbs catalyst (4 mg, 0.005 mmol) was added dropwise thereto, and the mixture was stirred at room temperature for 16 hours. The reaction mixture was concentrated, and then subjected to silica gel column chromatography with 20% ethyl acetate/n-hexane to obtain the title compound as a colorless oil (48 mg, yield: 83%).

¹H-NMR (400 MHz, CDCl₃) δ 7.74 (s, 1H), 7.35-7.25 (m, 9H), 7.19 (t, J = 4.9 Hz, 2H), 7.15-7.05 (m, 4H), 6.90 (d, J = 7.6 Hz, 2H), 5.82-5.69 (m, 2H), 5.54 (dd, J = 11.2, 2.7 Hz, 1H), 4.61-4.36 (m, 4H), 4.35-4.15 (m, 6H), 3.90 (d, J = 11.0 Hz, 1H), 3.76 (t, J = 8.7 Hz, 1H), 3.71-3.62 (m, 1H), 2.79 (d, J = 17.0 Hz, 1H), 2.11 (d, J = 17.4 Hz, 1H).

### Synthesis of (2R,3R,4S,5R,6S)-2-(hydroxymethyl)-4-(4-(3,4,5-trifluorophenyl)-1H-1,2,3-triazol-1-yl)-1,7-dioxaspiro[5.5]undecane-3,5-diol

1-((2R,3R,4S,5R,6S)-3,5-bis(benzyloxy)-2-((benzyloxy)methy1)-1,7-dioxaspiro[5.5]undec-9-en-4-yl)-4-(3,4,5-trifluorophenyl)-1*H*-1,2,3-triazole (45 mg, 0.066 mmol) was dissolved in 1 mL of a solution of dichloromethane:methanol (1:2), and the mixture was added dropwise to a round-bottom flask, in which Pd/C (5 mg) was dissolved in 1 mL of a solution of dichloromethane:methanol (1:2), and degassed with a hydrogen balloon for 10 minutes, followed by stirring in a hydrogen atmosphere at room temperature for 24 hours. The reaction solution was filtered through Celite and then concentrated and subjected to silica gel column chromatography with 6% methanol/dichloromethane to obtain the title compound as a white solid (23 mg, yield: 76%).

ESIMS: m/z 416.28 [M+H]+; ¹H-NMR (400 MHz, CDCl₃) δ 8.06 (s, 1H), 7.43-7.30 (m, 2H), 4.83 (dd, J = 10.6, 2.6 Hz, 1H), 4.54-4.28 (m, 2H), 4.07 (t, J = 10.9 Hz, 1H), 3.99 (t, J = 5.0 Hz, 2H), 3.92 (t, J = 4.4 Hz, 1H), 3.83-3.70 (m, 2H), 2.48 (t, J = 6.0 Hz, 1H), 2.40 (d, J = 11.3 Hz, 1H), 2.15-2.03 (m, 1H), 1.89-1.85 (m, 1H), 1.71 (d, J = 11.7 Hz, 2H), 1.63 - 1.58 (m, 3H).

### Example 15: Synthesis of Compound A25

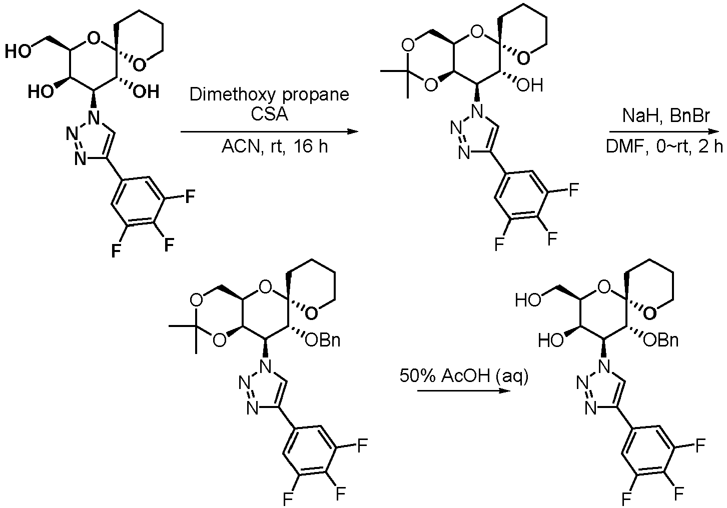

### Synthesis of (2S,4a'R,7'R,8'R,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-ol

(2*R*,3*R*,4*S*,5*R*,6*S*)-2-(hydroxymethyl)-4-(4-(3,4,5-trifluorophenyl)-1*H*-1,2,3-triazol-1-yl)-1,7-dioxaspiro[5.5]undecane-3,5-diol (17 mg, 0.041 mmol) was dissolved in 2 mL of acetonitrile, and then 2,2-dimethoxy propane (8 µL, 0.061 mmol), camphor-10-sulfonic acid (2 mg, 0.008 mmol) were sequentially slowly added dropwise. The reaction mixture was stirred at room temperature for 16 hours, then a saturated aqueous ammonium chloride solution was added thereto to terminate the reaction. The mixture was extracted with ethyl acetate, dried over anhydrous sodium sulfate, filtered, and then concentrated under reduced pressure. The concentrate was subjected to silica gel column chromatography with 3.5% methanol/dichloromethane to obtain the title compound in pale yellow solid (19 mg, yield: 99%).

### Synthesis of 1-((2S,4a'R,7'R,8'S,8a'R)-7'-(benzyloxy)-2',2'-dimethyloctahydro-4'H-spiro[pyran-2,6'-pyrano[3,2-d][1,3]dioxin]-8'-yl)-4-(3,4,5-trifluorophenyl)-1H-1,2,3-triazole

(2S,4a'R,1'R,8'R,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-*d*][1,3]dioxin]-7'-ol (10 mg, 0.020 mmol) was dissolved in 1 mL of *N*,*N*-dimethylformamide, and then 60% NaH (1.3 mg, 0.033 mmol) and BnBr (4 µL, 0.033 mmol) were slowly added thereto at 0 °C, followed by stirring for 2 hours while elevating the temperature from 0 °C to room temperature. The reaction solution was extracted by adding ethyl acetate and distilled water to recover an organic layer. The organic layer was dried over anhydrous sodium sulfate, filtered, and then concentrated under reduced pressure. The concentrate was subjected to silica gel column chromatography with 3.0% methanol/dichloromethane to obtain the title compound as a white solid (8 mg, yield: 67%).

### Synthesis of (2R,3R,4S,5R,6S)-5-(benzyloxy)-2-(hydroxymethyl)-4-(4-(3,4,5-trifluorophenyl)-1H-1,2,3-triazol-1-yl)-1,7-dioxaspiro[5.5]undecan-3-ol

1-((2*S*,4a'*R*,7'*R*,8'*S*,8a'*R*)-7'-(benzyloxy)-2',2'-dimethyloctahydro-4'*H*-spiro[pyran-2,6'-pyrano[3,2-*d*][1,3]dioxin]-8'-yl)-4-(3,4,5-trifluorophenyl)-1*H*-1,2,3-triazole (8 mg, 0.015 mmol) was dissolved in 1 mL of a 50% aqueous acetic acid solution, followed by stirring at 60 °C for 16 hours. The reaction mixture was concentrated, then neutralized by adding a saturated aqueous ammonium chloride solution, and then extracted with dichloromethane and dried over anhydrous sodium sulfate, filtered, and then concentrated under reduced pressure. The concentrate was subjected to silica gel column chromatography with 3.5% methanol/dichloromethane to obtain the title compound as a white solid (6 mg, yield: 81%).

ESIMS: m/z 506.31 [M+H]+; ¹H-NMR (400 MHz, CDCl₃) δ 7.83 (s, 1H), 7.36-7.29 (m, 2H), 7.19 -7.16 (m, 3H), 7.01 -6.97 (m, 2H), 4.95 (dd, J = 10.6, 2.2 Hz, 1H), 4.64 (s, 1H), 4.51 (s, 1H), 4.31 (d, J = 11.0 Hz, 1H), 4.10 (d, J = 10.6 Hz, 1H), 4.06-3.99 (m, 1H), 3.96-3.87 (m, 3H), 3.86-3.80 (m, 1H), 3.76-3.71 (m, 1H), 2.28-2.24 (m, 1H), 1.97-1.94 (m, 2H), 1.60-1.57 (m, 3H).

### Example 16: Synthesis of Compound A26

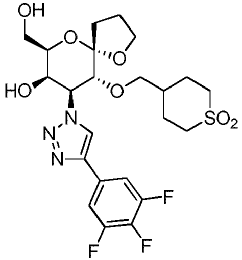

### Synthesis of 4-((((5S,7R,8R,9S, 10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)methyl)tetrahydro-2H-thiopyran 1,1-dioxide

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5S,7R,8R,9S,10R)-10-(benzyloxy)-7-(hydroxymethyl)-9-(4-(3,4,5-trifluorophenyl)-1H-1,2,3-triazol-1-yl)-1,6-dioxaspiro[4.5]decan-8-ol.

ESIMS: m/z 548.46 [M+H]+; ¹H-NMR (400 MHz, CDCl₃) δ 8.15 (s, 1H), 7.45 (t, J = 7.2 Hz, 2H), 5.05 (dd, J = 10.8, 2.2 Hz, 1H), 4.39 (s, 2H), 4.24 (d, J = 11.0 Hz, 1H), 4.00-4.06 (m, 3H), 3.94-3.95 (m, 2H), 3.27 (dd, J = 9.0, 6.3 Hz, 1H), 2.88-2.95 (m, 2H), 2.80-2.84 (m, 2H), 2.73 (dd, J = 9.0, 5.5 Hz, 1H), 1.85-2.16 (m, 5H), 1.55-1.76 (m, 5H)

### Example 17: Synthesis of Compound A27

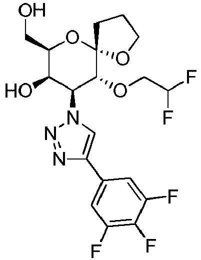

### Synthesis of (5S,7R,8R,9S,10R)-10-(2,2-difluoroethoxy)-7-(hydroxymethyl)-9-(4-(3,4,5-trifluorophenyl)-1H-1,2,3-triazol-1-yl)-1,6-dioxaspiro[4.5]decan-8-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 466.17 [M+H]+; ¹H-NMR (400 MHz, CDCl₃) δ 8.09 (s, 1H), 7.43-7.50 (m, 2H), 5.53 (tt, J = 55.0, 3.8 Hz, 1H), 5.03 (dd, J = 11.0, 2.3 Hz, 1H), 4.48-4.49 (m, 2H), 4.34 (d, J = 10.6 Hz, 1H), 3.90-4.10 (m, 5H), 3.38-3.48 (m, 1H), 3.10-3.21 (m, 1H), 2.08-2.26 (m, 4H), 1.95-2.05 (m, 1H)

### Example 18: Synthesis of Compound A28

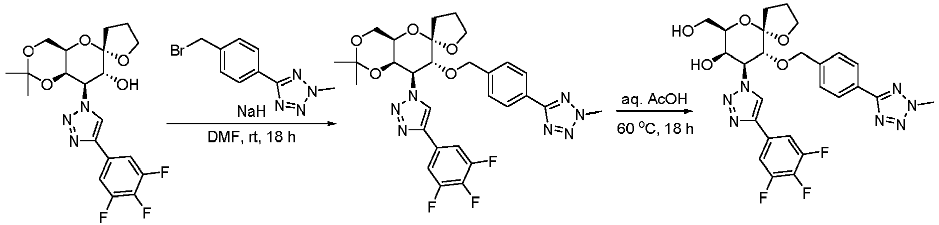

### Synthesis of 5-(4-((((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)methyl)phenyl)-2-methyl-2H-tetrazole

(2*S*,4a'*R*,1'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (12.0 mg, 0.027 mmol) was dissolved in 1.0 mL of N,N-dimethylformamide, and then 60% NaH (1.6 mg, 0.041 mmol) and 5-(4-(bromomethyl)phenyl)-2-methyl-2//-tetrazole (11.6 mg, 0.041 mmol) were slowly added thereto at 0 °C, followed by stirring for 18 hours while elevating the temperature from 0 °C to room temperature. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (15.2 mg, yield: 92.0%).

¹H-NMR (400 MHz, CDCl₃) δ 7.87 (d, J = 8.4 Hz, 2H), 7.67 (s, 1H), 7.05 (d, J = 8.4 Hz, 2H), 5.45 (dd, J = 11.0, 3.3 Hz, 1H), 4.59 (d, J = 12.0 Hz, 1H), 4.41-4.40 (m, 1H), 4.37 (s, 3H), 4.31-4.29 (m, 1H), 4.17 (d, J = 12.1 Hz, 1H), 4.10-4.09 (m, 1H), 4.08-4.06 (m, 1H), 4.05-3.99 (m, 1H), 3.93-3.88 (m, 1H), 3.85-3.84 (m, 1H), 2.27-2.21 (m, 4 H)

### Synthesis of (5S,7R,8R,9S,10R)-7-(hydroxymethyl)-10-((4-(2-methyl-2H-tetrazol-5-yl)benzyl)oxy)-9-(4-(3,4,5-trifluorophenyl)-1H-1,2,3-triazol-1-yl)-1,6-dioxaspiro[4.5]decan-8-ol

5-(4-((((2S,4a'R,7'R,8'S,8a'R)-2',2'-dimethyl-8'-(4-(3,4,5-tiifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)methyl)phenyl)-2-methyl-2*H*-tetrazole (15.2 mg, 0.024 mmol) was dissolved in 1.0 mL of a 50% aqueous acetic acid solution, followed by stirring at 60 °C for 18 hours. The reaction compound was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (13.4 mg, yield: 94.3%).

ESIMS: m/z 574.35 [M+H]+; ¹H-NMR (400 MHz, CD₃OD) δ 8.24 (s, 1H), 7.77 (d, J = 8.4 Hz, 2H), 7.35 (dd, J = 9.1, 6.7 Hz, 2H), 7.06 (d, J = 8.4 Hz, 2H), 5.15 (dd, J = 11.1, 2.9 Hz, 1H), 4.76 (d, J = 12.4 Hz, 1H), 4.46 (d, J = 11.1 Hz, 1H), 4.37 (s, 3H), 4.32 (d, J =12.6 Hz, 1H), 4.08-4.02 (m, 4H), 3.69 (d, J = 6.1 Hz, 2H), 2.31-2.26 (m, 1H), 2.20-1.98 (m, 3H).

### Example 19: Synthesis of Compound A29

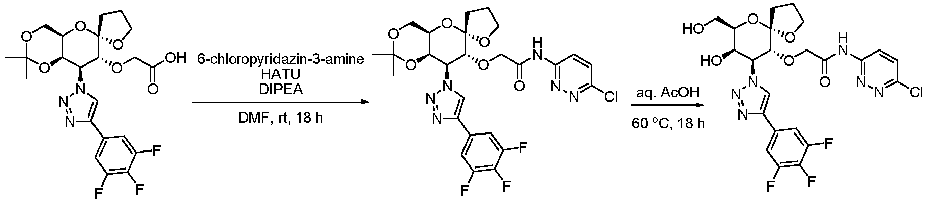

### Synthesis of N-(6-chloropyridazin-3-yl)-2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)acetamide

2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'- yl)oxy)acetic acid (20.0 mg, 0.040 mmol) was dissolved in 3.0 mL of *N*,*N*-dimethylformamide, and then 6-chloropyridazin-3-amine (6.2 mg, 0.048 mmol), HATU (38.0 mg, 0.100 mmol), and *N*,*N-*diisopropylethylamine (34.8 µL, 0.200 mmol) were added dropwise thereto, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 30% ethyl acetate/n-hexane to obtain an intermediate as a white solid (8.1 mg).

The intermediate (8.1 mg, 0.013 mmol) was dissolved in 2.0 mL of a 50% aqueous acetic acid solution, followed by stirring at 60 °C for 18 hours. The reaction mixture was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to amine silica gel column chromatography with 0% to 5% methanol/dichloromethane to obtain the title compound as a white solid (8.3 mg, yield: 85.9%).

ESIMS: m/z 571.45 [M+H]+; ¹H-NMR (400 MHz, DMSO-D₆) δ 10.17 (s, 1H), 8.83 (s, 1H), 8.08 (d, J = 9.4 Hz, 1H), 7.72 (d, J = 9.4 Hz, 1H), 7.58 (dd, J = 8.9, 6.8 Hz, 2H), 5.44 (d, J = 6.2 Hz, 1H), 5.11 (dd, J = 11.2, 2.9 Hz, 1H), 4.64-4.60 (m, 1H), 4.48 (d, J = 11.2 Hz, 1H), 4.18 (d, J = 15.6 Hz, 1H), 4.04 (d, J = 15.6 Hz, 1H), 3.99-3.86 (m, 4H), 3.5-3.43 (m, 2H), 2.32-2.20 (m, 1H), 2.00-1.85 (m, 3H).

### Example 20: Synthesis of Compound A30

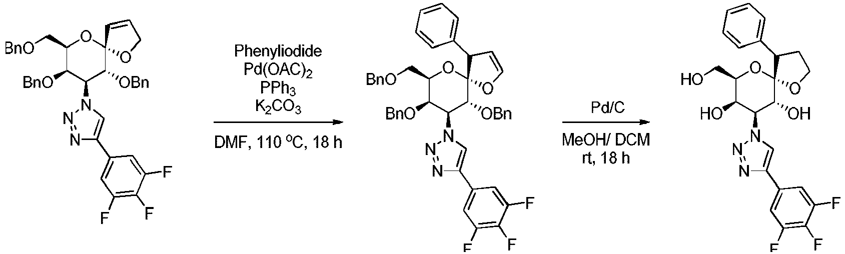

### Synthesis of 1-((4S,5S,7R,8R,9S,10R)-8,10-bis(benzyloxy)-7-((benzyloxy)methyl)-4-phenyl-1,6-dioxaspiro[4.5]dec-2-en-9-yl)-4-(3,4,5-trifluorophenyl)-1H-1,2,3-triazole

1-((5*S*,7*R*,8*R*,9*S*,10*R*)-8,10-bis(benzyloxy)-7-((benzyloxy)methyl)-1,6-dioxaspiro[4.5]dec-3-en-9-yl)-4-(3,4,5-trifluorophenyl)-1*H*-1,2,3-triazole (20.0 mg, 0.030 mmol), iodobenzene (7.3 mg, 0.036 mmol), Pd(OAc)₂ (1.0 mg, 10 mol%), potassium carbonate (8.3 mg, 0.06 mmol), and PPh₃ (1.5 mg, 0.006 mmol) were dried sufficiently, then an argon gas was substituted, and then the mixture was dissolved in 2.0 mL of *N*,*N*-dimethylformamide, followed by stirring at 110 °C for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 50% ethyl acetate/n-hexane to obtain the title compound as a white solid (5.0 mg, yield: 22%).

¹H-NMR (400 MHz, CDCl₃) δ 7.56-7.44 (m, 3H), 7.34-7.05 (m, 18H), 6.98 - 6.94 (m, 2H), 6.28 (d, J = 5.9 Hz, 1H), 5.85-5.80 (m, 1H), 5.02 (d, J = 10.8 Hz, 1H), 4.69-4.61 (m, 2H), 4.55 (t, J = 10.6 Hz, 1H), 4.48 (d, J = 10.5 Hz, 1H), 4.44 (s, 2H), 4.28 (d, J = 10.7 Hz, 1H), 4.11 (d, J = 10.8 Hz, 1H), 4.09-4.04 (m, 1H), 3.76-3.73 (m, 1H), 3.62 (t, J = 9.0 Hz, 1H), 3.50 (dd, J = 8.9, 5.3 Hz, 1H).

### Synthesis of (4S,5S,7R,8R,9S,10R)-7-(hydroxymethyl)-4-phenyl-9-(4-(3,4,5-trifluorophenyl)-1H-1,2,3-triazol-1-yl)-1,6-dioxaspiro[4.5]decane-8,10-diol

1-((4S,5S,7R,8R,9S,10R)-8,10-bis(benzyloxy)-7-((benzyloxy)methyl)-4-phenyl-1,6-dioxaspiro[4.5]dec-2-en-9-yl)-4-(3,4,5-trifluorophenyl)-1*H*-1,2,3-triazole (5.0 mg, 0.005 mmol) was dissolved in 3.0 mL of a solution of dichloromethane:methanol (1:2), and the mixture was added dropwise to a round-bottom flask, in which 10% Pd/C (1.0 mg, 20 wt%) was dissolved in 3.0 mL of a solution of dichloromethane:methanol (1:2), and then degassed with a hydrogen balloon for 10 minutes. The reaction solution was stirred at room temperature in a hydrogen atmosphere for 18 hours. The reaction solution was filtered through Celite and then concentrated and subjected to silica gel column chromatography with 0% to 50% methanol/dichloromethane to obtain the title compound as a white solid (3.1 mg, yield: 95%).

ESIMS: m/z 478.40 [M+H]+; ¹H-NMR (400 MHz, DMSO) δ 8.07 (s, 1H), 7.64-7.61 (m, 2H), 7.42 (s, 2H), 7.19-7.09 (m, 3H), 5.13 (d, J = 8.3 Hz, 1H), 4.93 (d, J = 6.6 Hz, 1H), 4.43 (d, J = 5.3 Hz, 2H), 4.29 (s, 1H), 4.28-4.25 (m, 1H), 3.83-3.80 (m, 1H), 3.76-3.74 (m, 1H), 3.66-3.62 (m, 1H), 1.97-1.94 (m, 2H), 1.93-1.90 (m, 1H).

### Example 21: Synthesis of Compound A31

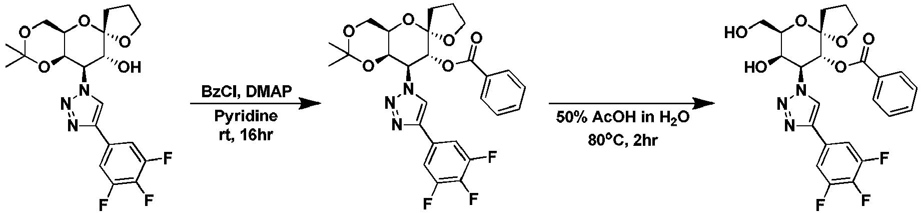

### Synthesis of (2S,4a'R,1'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl benzoate

(2S,4a'R,7'R,8'R,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-ol (15 mg, 0.034 mmol) was dissolved in 2 mL of pyridine, and then benzoyl chloride (6 µL, 0.051 mmol) and 4-dimethylaminopyridine (6 mg, 0.051 mmol) were added dropwise thereto, followed by stirring at room temperature for 16 hours. The reaction solution was extracted by adding ethyl acetate and distilled water to recover an organic layer. The organic layer was dried over anhydrous sodium sulfate, filtered, and then concentrated under reduced pressure. The concentrate was subjected to silica gel column chromatography with 32% ethyl acetate/n-hexane to obtain the title compound as a white solid (15 mg, yield: 81%).

¹H-NMR (400 MHz, CDCl₃) δ 8.02 (s, 1H), 7.87-7.83 (m, 2H), 7.55-7.45 (m, 1H), 7.37-7.35 (m, 4H), 6.09 (d, J = 11.4 Hz, 1H), 5.67-5.64 (m, 1H), 4.42 (d, J = 2.5 Hz, 1H), 4.16 -4.05 (m, 4H), 3.98-3.92 (m, 2H), 2.26-2.17 (m, 1H), 2.15-2.04 (m, 1H), 2.00-1.90 (m, 1H), 1.85 (s, 1H), 1.54 (s, 3H), 1.33 (s, 3H).

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl benzoate

(2S,4aR,7'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-1'-yl)oxy benzoate (14 mg, 0.026 mmol) was dissolved in 1 mL of a 50% aqueous acetic acid solution, followed by stirring at 80 °C for 2 hours. The reaction mixture was concentrated, then neutralized by adding a saturated aqueous ammonium chloride solution, and then extracted with dichloromethane and dried over anhydrous sodium sulfate, filtered, and then concentrated under reduced pressure. The concentrate was subjected to silica gel column chromatography with 2.0% methanol/dichloromethane to obtain the title compound as a white solid (7 mg, yield: 54%).

ESIMS: m/z 506.24 [M+H]+; ¹H-NMR (400 MHz, CDCl₃) δ 8.10 (s, 1H), 7.89-7.83 (m, 2H), 7.56-7.49 (m, 1H), 7.40-7.33 (m, 2H), 7.31-7.27 (m, 3H), 6.16 (d, J = 11.3 Hz, 1H), 5.44 (dd, J = 11.2, 2.6 Hz, 1H), 4.49 (d, J = 13.5 Hz, 2H), 4.20-4.16 (m, 1H), 4.08-4.03 (m, 4H), 2.37-2.26 (m, 1H), 2.18-1.94 (m, 4H), 1.86 -1.82 (m, 1H).

### Example 22: Synthesis of Compound A32

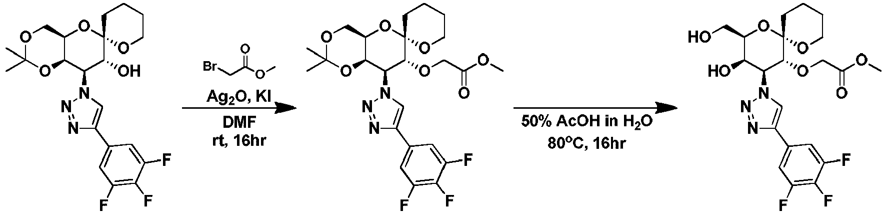

### Synthesis of methyl-2-(((2S,4a'R,1'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)oxy)acetate

(2S,4a'R,1'R,8'R,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-*d*][1,3]dioxin]-1'-ol (7 mg, 0.015 mmol) was dissolved in 1 mL of N,N-dimethylformamide, and then silver(I) oxide (17 mg, 0.075 mmol), potassium iodide (5 mg, 0.030 mmol), and methyl bromoacetate (2 µL, 0.023 mmol) were sequentially added dropwise thereto, followed by stirring at room temperature for 16 hours. The reaction solution was filtered through Celite, then extracted with ethyl acetate, dried over anhydrous sodium sulfate, filtered, and then concentrated under reduced pressure. The concentrate was subjected to silica gel column chromatography with 50% ethyl acetate/n-hexane to obtain the title compound as a white solid (8 mg, yield: 67%).

### Synthesis of methyl-2-(((2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl)oxy)acetate

Methyl-2-(((2S,4a'R,7'R,8'S,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-*d*][1,3]dioxin]-1'-yl)oxy) acetate (8 mg) was dissolved in 2 mL of a 50% aqueous acetic acid solution, followed by stirring at 80 °C for 16 hours. The reaction mixture was concentrated, then neutralized by adding a saturated aqueous ammonium chloride solution, and then extracted with dichloromethane and dried over anhydrous sodium sulfate, filtered, and then concentrated under reduced pressure. The concentrate was subjected to silica gel column chromatography with 4.0% methanol/dichloromethane to obtain the title compound as a white solid (1 mg, yield: 13% (two steps)).

ESIMS: m/z 488.23 [M+H]+; ¹H-NMR (400 MHz, CDCl₃) δ 8.10 (s, 1H), 7.49-7.43 (m, 2H), 5.06 (d, J = 12.8 Hz, 1H), 4.51 (s, 1H), 4.43 (s, 1H), 4.08 (d, J = 10.7 Hz, 1H), 4.04-3.99 (m, 1H), 3.95-3.90 (m, 2H), 3.82-3.78 (m, 1H), 3.74-3.65 (d, J = 7.0 Hz, 5H), 3.58 (s, 3H), 2.17-2.15 (m, 2H), 2.13-2.10 (m, 1H), 2.08-2.04 (m, 1H), 2.02-1.98 (m, 1H), 1.73 (s, 6H).

### Example 23: Synthesis of Compound A33

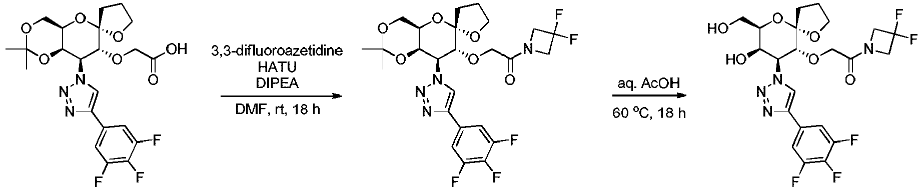

### Synthesis of 1-(3,3-difluoroazetidin-1-yl)-2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)ethanone

2-(((2S,4a'R,1'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'- yl)oxy)acetic acid (20.0 mg, 0.040 mmol) was dissolved in 3.0 mL of N,N-dimethylformamide, and then a aqueous 3,3-difluoroazetidine hydrochloride solution (6.2 mg, 0.048 mmol), HATU (38.0 mg, 0.100 mmol), and *N*,*N*-diisopropylethylamine (34.8 µL, 0.200 mmol) were added dropwise thereto, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain an intermediate as a white solid (18 mg).

The intermediate was dissolved in 2.0 mL of a 50% aqueous acetic acid solution, followed by stirring at 60 °C for 18 hours. The reaction mixture was concentrated, then neutralized by adding ammonium chloride, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (13.4 mg, yield: 80.0%).

ESIMS: m/z 535.35 [M+H]+; ¹H-NMR (400 MHz, CD₃OD) δ 8.66-8.63 (m, 1H), 7.66-7.61 (m, 2H), 5.29-5.18 (m, 1H), 4.46 (d, J = 11.2 Hz, 1H), 4.18 (d, J = 14.9 Hz, 1H), 4.15-4.00 (m, 6H), 3.91-3.85 (m, 1H), 3.69 (dd, J = 6.2, 2.4 Hz, 2H), 3.07 (t, J = 13.5 Hz, 1H), 2.86 (t, J = 13.7 Hz, 1H), 2.25-1.90 (m, 4H).

### Example 24: Synthesis of Compound A34

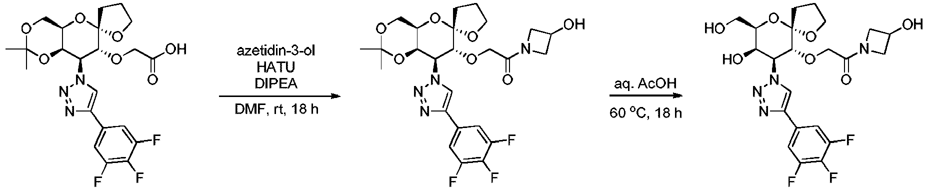

### Synthesis of 2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)-1-(3-hydroxyazetidin-1-yl)ethanone

2-(((2S,4a'R,7'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-1'- yl)oxy)acetic acid (20.0 mg, 0.040 mmol) was dissolved in 3.0 mL of *N*,*N*-dimethylformamide, and then azetidin-3-ol (3.5 mg, 0.048 mmol), HATU (38.0 mg, 0.100 mmol), and *N*,*N*-diisopropylethylamine (34.8 µL, 0.200 mmol) were added dropwise thereto, followed by stirring at room temperature for 18 hours. The reaction solution was extracted by adding ethyl acetate and distilled water to recover an organic layer. The organic layer was dried over anhydrous magnesium sulfate, filtered, and then concentrated. The concentrated reaction mixture was subjected to silica gel column chromatography with 0% to 30% ethyl acetate/n-hexane to obtain an intermediate as a colorless oil (17.4 mg, yield: 78%).

The intermediate (16.0 mg, 0.029 mmol) was dissolved in 2.0 mL of a 50% aqueous acetic acid solution, followed by stirring at 60 °C for 18 hours. The reaction mixture was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to amine silica gel column chromatography with 0% to 5% methanol/dichloromethane to obtain the title compound as a white solid (11.5 mg, yield: 68.7%).

ESIMS: m/z 515.44 [M+H]+; ¹H-NMR (400 MHz, CD₃OD) δ 8.65 (s, 1H), 7.69-7.63 (m, 2H), 5.20 (dd, J = 11.1, 2.9 Hz, 1H), 4.47-4.39 (m, 2H), 4.27-4.17 (m, 1H), 4.10-3.98 (m, 5H), 3.85-3.80 (m, 2H), 3.68 (d, J = 6.1 Hz, 2H), 3.59 (s, 2H), 2.28-2.20 (m, 1H), 2.12-1.98 (m, 3H).

### Example 25: Synthesis of Compound A35

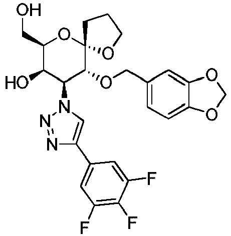

### Synthesis of (5S,7R,8R,9S,10R)-10-(benzo[d][1,3]dioxol-5-ylmethoxy)-7-(hydroxymethyl)-9-(4-(3,4,5-trifluorophenyl)-1H-1,2,3-triazol-1-yl)-1,6-dioxaspiro[4.5]decan-8-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5S,7R,8R,9S,10R)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 536.42 [M+H]+; ¹H-NMR (400 MHz, CDCl₃) δ 7.90 (s, 1H), 7.34-7.43 (m, 2H), 6.58-6.60 (m, 1H), 6.42-6.44 (m, 2H), 5.84 (dd, J = 12.9, 1.2 Hz, 2H), 4.91 (dd, J = 10.6, 2.0 Hz, 1H), 4.65 (s, 1H), 4.49 (s, 1H), 4.36 (d, J = 10.6 Hz, 1H), 4.16 (d, J = 11.0 Hz, 1H), 3.86-4.10 (m, 4H), 3.82 (d, J = 11.0 Hz, 1H), 2.25-2.28 (m, 1H), 1.87-2.17 (m, 5H)

### Example 26: Synthesis of Compound A36

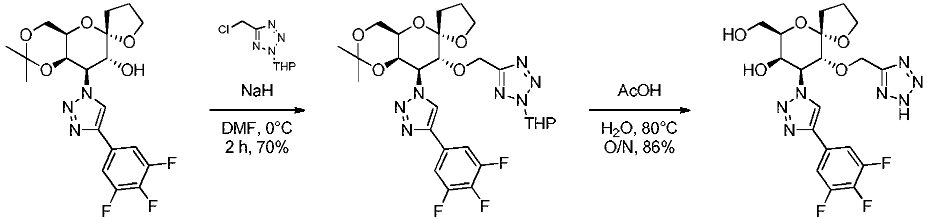

### Synthesis of 5-((((2S,4a'R,1'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)methyl)-2-(tetrahydro-2H-pyran-2-yl)-2H-tetrazole

(2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (15.0 mg, 0.034 mmol) was dissolved in 1.0 mL of *N*,*N*-dimethylformamide, and then 60% NaH (2.0 mg, 0.051 mmol) and 5-(chloromethyl)-2-(tetrahydro-2*H*-pyran-2-yl)-2*H*-tetrazole (13.8 mg, 0.068 mmol) were slowly added thereto at 0 °C, followed by stirring for 18 hours while elevating the temperature from 0 °C to room temperature. The reaction solution was diluted with ethyl acetate and then washed twice with a saturated aqueous ammonium chloride solution. The organic layer was recovered, dried over anhydrous sodium sulfate, filtered, and then concentrated under reduced pressure. The concentrate was subjected to silica gel column chromatography with 0% to 40% ethyl acetate/n-hexane to obtain the title compound as a white solid (a diastereomer mixture of 1:1) (14.5 mg, yield: 70%).

¹H-NMR (400 MHz, CDCl₃) δ 8.02-8.08 (m, 1H), 7.47-7.51 (m, 2H), 5.81-5.91 (m, 1H), 5.44-5.49 (m, 1H), 4.58-4.63 (m, 1H), 4.42-4.46 (m, 1H), 4.27-4.37 (m, 2H), 3.84-4.10 (m, 6H), 3.71-3.75 (m, 1H), 2.23-2.33 (m, 1H), 1.91-2.20 (m, 6H), 1.60-1.69 (m, 4H), 1.51-1.51 (m, 3H), 1.33-1.33 (m, 3H)

### Synthesis of (5S,7R,8R,9S,10R)-10-((2H-tetrazol-5-yl)-7-(hydroxymethyl)-9-(4-(3,4,5-trifluorophenyl)-1H-1,2,3-triazol-1-yl)-1,6-dioxaspiro[4.5]decan-8-ol

5-((((2S,4a'R,7'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-yl)oxy)methyl)-2-(tetrahydro-2*H*-pyran-2-yl)-2*H*-tetrazole (14.5 mg, 0.024 mmol) was dissolved in 1.0 mL of a 50% aqueous acetic acid solution, followed by stirring at 60 °C for 18 hours. The reaction mixture was concentrated, then neutralized by adding a saturated aqueous ammonium chloride solution, and then extracted with dichloromethane and dried over anhydrous magnesium sulfate, filtered, and then concentrated under reduced pressure. The concentrate was subjected to silica gel column chromatography with 0% to 10% methanol/dichloromethane to synthesize the title compound as a white solid (10.0 mg, yield: 86%).

ESIMS: m/z 484.45 [M+H]+; ¹H-NMR (400 MHz, CD₃OD) δ 8.57 (s, 1H), 7.56-7.63 (m, 2H), 5.24 (dd, J = 11.2, 2.9 Hz, 1H), 4.75 (dd, J = 33.6, 13.3 Hz, 2H), 4.62 (d, J = 11.3 Hz, 1H), 4.10-4.11 (m, 1H), 4.05-4.08 (m, 1H), 3.96-4.03 (m, 2H), 3.69 (d, J = 6.3 Hz, 2H), 1.98-2.17 (m, 3H), 1.85-1.93 (m, 1H)

### Example 27: Synthesis of Compound A37

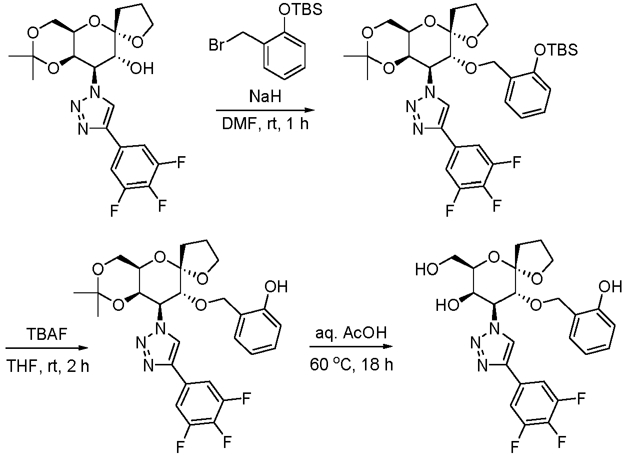

### Synthesis of 1-((2S,4a'R,1'R,8'S,8a'R)-1'-((2-(tert-butyldimethylsilyl)oxy)benzyl)oxy)-2',2'-dimethylhexahydro-3H,4'H-spiro[furan-2,6'-pyrano[3,2-d][1,3]dioxin]-8'-yl)-4-(3,4,5-trifluorophenyl)-1H-1,2,3-triazole

(2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (15.0 mg, 0.034 mmol) was dissolved in 1.0 mL of *N*,*N*-dimethylformamide, and then 60% NaH (2.0 mg, 0.051 mmol) and 2-(bromomethyl)phenoxy)(*tert*-butyl)dimethylsilane (20.5 mg, 0.068 mmol) were slowly added dropwise thereto at 0 °C, followed by stirring for 18 hours while elevating the temperature from 0 °C to room temperature. The reaction solution was diluted with ethyl acetate and then washed twice with a saturated aqueous ammonium chloride solution. The organic layer was recovered, dried over anhydrous sodium sulfate, filtered, and then concentrated under reduced pressure. The concentrate was subjected to silica gel column chromatography with 0% to 25% ethyl acetate/n-hexane to obtain the title compound as a white solid (18.7 mg, yield: 83%).

### Synthesis of 2-((((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3] dioxin]-7'-yl)oxy)methyl)phenol

1-((2S,4a'R,7'R,8'S,8a'R)-7'-((2-((tert-butyldimethylsilyl)oxy)benzyl)oxy)-2',2'-dimethylhexahydro-3H,4'H-spiro[furan-2,6'-pyrano[3,2-d][1,3]dioxin]-8'-yl)-4-(3,4,5-trifluorophenyl)-1*H*-1,2,3-triazole (18.7 mg, 0.028 mmol) was dissolved in 0.7 mL of tetrahydrofuran, then TBAF 1.0M (tetrahydrofuran solution) (0.056 mmol), followed by stirring at room temperature for 2 hours. The reaction mixture was concentrated under reduced pressure, then the concentrate was concentrated and subjected to silica gel column chromatography with 0% to 40% ethyl acetate/n-hexane to obtain the title compound as a white solid (7.9 mg, yield: 51%).

¹H-NMR (400 MHz, CDCl₃) δ 7.97 (s, 1H), 7.39-7.45 (m, 2H), 7.03-7.08 (m, 1H), 6.68-6.79 (m, 3H), 5.43 (dd, J = 10.8, 3.3 Hz, 1H), 4.50-4.54 (m, 1H), 4.36 (d, J = 2.0 Hz, 1H), 4.31-4.33 (m, 1H), 4.21 (d, J = 11.7 Hz, 1H), 4.10 (dd, J = 13.3, 2.0 Hz, 2H), 3.99-4.04 (m, 2H), 3.91 (dd, J = 13.1, 1.4 Hz, 2H), 3.84 (s, 1H), 2.07-2.19 (m, 4H), 1.89-2.00 (m, 2H), 1.53 (s, 3H), 1.35 (s, 3H)

### Synthesis of (5S,7R,8R,9S,10R)-10-((2-hydroxybenzyl)oxy)-7-(hydroxymethyl)-9-(4-(3,4,5-trifluorophenyl)-1H-1,2,3-triazol-1-yl)-1,6-dioxaspiro[4.5]decan-8-ol

2-((((2S,4a'R,7'R,8'S,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-yl)oxy)methyl)phenol (7.9 mg, 0.014 mmol) was dissolved in 1.0 mL of a 50% aqueous acetic acid solution, followed by stirring at 60 °C for 18 hours. The reaction mixture was concentrated, then neutralized by adding a saturated aqueous ammonium chloride solution, and then extracted with dichloromethane and dried over anhydrous magnesium sulfate, filtered, and then concentrated under reduced pressure. The concentrate was subjected to silica gel column chromatography with 0% to 5% methanol/dichloromethane to obtain the title compound as a white solid (3.0 mg, yield: 42%).

ESIMS: m/z 508.39 [M+H]+; ¹H-NMR (400 MHz, CDCl₃) δ 7.93 (s, 1H), 7.31 (dd, J = 8.2, 6.7 Hz, 2H), 7.08-7.13 (m, 1H), 6.77 (d, J = 8.2 Hz, 1H), 6.69 (d, J = 4.3 Hz, 2H), 6.67 (s, 1H), 4.93 (dd, J = 10.6, 2.3 Hz, 1H), 4.59 (d, J = 2.3 Hz, 1H), 4.56 (s, 1H), 4.52 (s, 1H), 4.41 (d, J = 10.6 Hz, 1H), 3.90-4.11 (m, 6H), 3.74 (d, J = 8.6 Hz, 1H), 2.22 (d, J = 6.7 Hz, 1H), 2.10-2.18 (m, 4H)

### Example 28: Synthesis of Compound A38

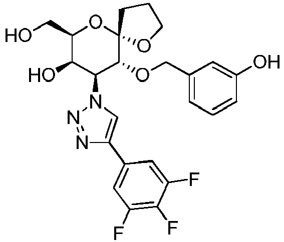

### Synthesis of (5S,7R,8R,9S,10R)-10-((3-hydroxybenzyl)oxy)-7-(hydroxymethyl)-9-(4-(3,4,5-trifluorophenyl)-1H-1,2,3-triazol-1-yl)-1,6-dioxaspiro[4.5]decan-8-ol

The title compound was obtained by the synthesis from (2S,4a'R,1'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-10-((2-hydroxybenzyl)oxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 508.39 [M+H]+; ¹H-NMR (400 MHz, CDCl₃) δ 7.96 (s, 1H), 7.31-7.36 (m, 2H), 7.03 (t, J = 7.8 Hz, 1H), 6.62 (dd, J = 7.8, 2.3 Hz, 1H), 6.54 (d, J = 7.4 Hz, 1H), 6.43 (s, 1H), 5.57 (s, 1H), 4.99 (dd, J = 10.6, 2.3 Hz, 1H), 4.69 (s, 1H), 4.50 (s, 1H), 4.38 (d, J = 10.6 Hz, 1H), 4.26 (d, J = 11.3 Hz, 1H), 3.86-4.11 (m, 6H), 3.74 (d, J = 11.0 Hz, 1H), 2.09-2.14 (m, 2H), 2.02-2.06 (m, 1H), 1.92-2.00 (m, 1H)

### Example 29: Synthesis of Compound A39

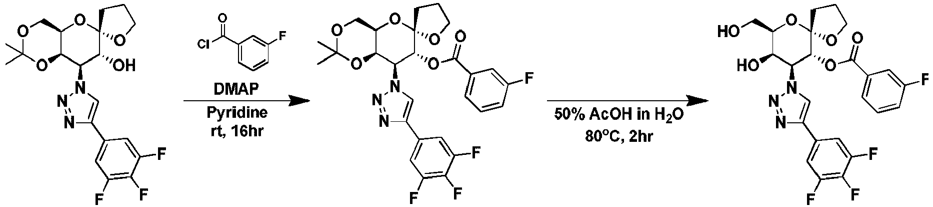

### Synthesis of (2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl3-fluorobenzoate

(2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (15 mg, 0.034 mmol) was dissolved in 2 mL of pyridine, and then 3-fluorobenzoyl chloride (6 µL, 0.051 mmol) and 4-dimethylaminopyridine (6 mg, 0.051 mmol) were added dropwise thereto, followed by stirring at room temperature for 16 hours. The reaction solution was extracted by adding ethyl acetate and distilled water to recover an organic layer. The organic layer was dried over anhydrous sodium sulfate, filtered, and then concentrated under reduced pressure. The concentrate was subjected to silica gel column chromatography with 2.0% methanol/dichloromethane to obtain the title compound as a white solid (15 mg, yield: 100%).

¹H-NMR (400 MHz, CDCl₃) δ 8.02 (s, 1H), 7.94-7.89 (m, 2H), 7.79-7.74 (m, 2H), 7.67-7.62 (m, 1H), 7.53-7.49 (m, 1H), 7.47 -7.44 (m, 2H), 7.40-7.29 (m, 4H), 7.24-7.18 (m, 1H), 6.06 (d, J = 11.4 Hz, 1H), 5.65 (dd, J = 11.3, 3.2 Hz, 1H), 4.45-4.40 (m, 1H), 4.14 (dd, J = 13.2, 2.3 Hz, 1H), 4.10-3.99 (m, 2H), 3.97 -3.94 (m, 2H), 2.22-2.18 (m, 1H), 2.16-2.06 (m, 1H), 1.97-1.88 (m, 1H), 1.84 -1.80 (m, 1H), 1.55-1.51 (m, 2H), 1.34-1.29 (m, 2H).

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3-fluorobenzoate

A mixture (34 mg) of (2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl3-fluorobenzoate and benozylchloride was dissolved in 2 mL of a 50% aqueous acetic acid solution, followed by stirring at 80 °C for 16 hours. The reaction mixture was concentrated, then neutralized by adding a saturated aqueous ammonium chloride solution, and then extracted with dichloromethane and dried over anhydrous sodium sulfate, filtered, and then concentrated under reduced pressure. The concentrate was subjected to silica gel column chromatography with 2.3% methanol/dichloromethane to obtain the title compound as a white solid (12 mg, yield: 86% (two steps)).

ESIMS: m/z 524.43 [M+H]+; ¹H-NMR (400 MHz, CDCl₃) δ 8.13 (s, 1H), 7.68-7.64 (m, 1H), 7.55 -7.51 (m, 1H), 7.39-7.29 (m, 3H), 7.25-7.19 (m, 1H), 6.14 (d, J = 11.2 Hz, 1H), 5.44 (dd, J = 11.2, 2.5 Hz, 1H), 4.54-4.39 (m, 2H), 4.18 (dd, J = 3.5, 2.5 Hz, 1H), 4.08-4.03 (m, 4H), 2.29-2.20 (m, 1H), 2.18-2.05 (m, 2H), 2.00-1.92 (m, 1H), 1.89-1.79 (m, 1H).

### Example 30: Synthesis of Compound A40

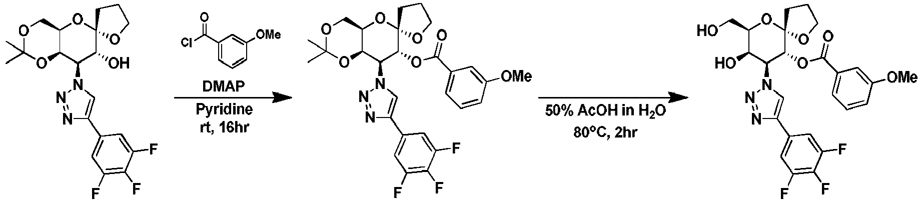

### Synthesis of (2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl3-methoxybenzoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (15 mg, 0.034 mmol) was dissolved in 2 mL of pyridine, and then 3-methoxybenzoyl chloride (7 µL, 0.051 mmol) and 4-dimethylaminopyridine (6 mg, 0.051 mmol) were sequentially added dropwise thereto, followed by stirring at room temperature for 16 hours. The reaction solution was extracted by adding ethyl acetate and distilled water to recover an organic layer. The organic layer was dried over anhydrous sodium sulfate, filtered, and then concentrated under reduced pressure. The concentrate was subjected to silica gel column chromatography with 30% ethyl acetate/n-hexane to obtain the title compound as a colorless oil (20 mg, yield: 100%).

¹H-NMR (400 MHz, CDCl₃) δ 8.01 (s, 1H), 7.46 -7.43 (m, 1H), 7.40-7.28 (m, 3H), 7.08-7.02 (m, 1H), 6.06 (d, J = 11.4 Hz, 1H), 5.66 (dd, J = 11.4, 3.3 Hz, 1H), 4.44-4.40 (m, 1H), 4.17-4.10 (m, 1H), 4.10-3.99 (m, 2H), 3.96 (dd, J = 10.8, 2.2 Hz, 2H), 3.87 (s, 1H), 3.79 (s, 3H), 2.21-2.18 (m, 1H), 2.14-2.03 (m, 1H), 2.00-1.91 (m, 1H), 1.87-1.80 (m, 1H), 1.54 (s, 3H), 1.33 (s, 3H).

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3-methoxybenzoate

(2S,4a'R,7'R,8'R,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-yl 3-methoxybenzoate (20 mg) was dissolved in 2 mL of a 50% aqueous acetic acid solution, followed by stirring at 80 °C for 2 hours. The reaction mixture was concentrated, then neutralized by adding a saturated aqueous ammonium chloride solution, and then extracted with dichloromethane and dried over anhydrous sodium sulfate, filtered, and then concentrated under reduced pressure. The concentrate was subjected to silica gel column chromatography with 2.5% methanol/dichloromethane to obtain the title compound as a white solid (17 mg, yield: 93% (two steps)).

ESIMS: m/z 536.34 [M+H]+; ¹H-NMR (400 MHz, CDCl₃) δ 8.08 (s, 1H), 7.48-7.44 (m, 1H), 7.38 -7.33 (m, 1H), 7.31-7.27 (m, 2H), 7.08 -7.04 (m, 1H), 6.14 (d, J = 11.3 Hz, 4H), 5.48-5.35 (m, 1H), 4.48 (d, J = 24.5 Hz, 2H), 4.20-4.17 (m, 5H), 4.10-3.96 (m, 4H), 2.31 (t, J = 6.1 Hz, 1H), 2.19-1.93 (m, 4H), 1.88-1.75 (m, 1H).

### Example 31: Synthesis of Compound A41

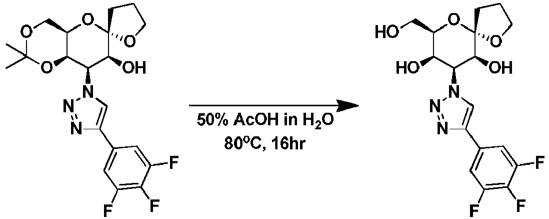

### Synthesis of (5S,7R,8R,9S,10R)-7-(hydroxymethyl)-9-(4-(3,4,5-trifluorophenyl)-1H-1,2,3-triazol-1-yl)-1,6-dioxaspiro[4.5]decane-8,10-diol

(2*S*,4a'*R*,7'*S*,8'*R*,8a'*R*)-2',2'-dimethyl-8'-(4-(3,4,5-trifluorophenyl)-1*H*-1,2,3-triazol-1-yl)hexahydro-3*H*,4*H*-spiro[furan-2,6'-pyrano[3,2-*d*][1,3]dioxin]-7'-ol (12 mg, 0.027 mmol) was dissolved in 2 mL of a 50% aqueous acetic acid solution, followed by stirring at 80 °C for 16 hours. The reaction mixture was concentrated, then neutralized by adding a saturated aqueous sodium hydrogen carbonate solution, and then extracted with dichloromethane and dried over anhydrous sodium sulfate, filtered, and then concentrated under reduced pressure. The concentrate was subjected to silica gel column chromatography with 5.0% methanol/dichloromethane to obtain the title compound as a white solid (6 mg, yield: 50%).

ESIMS: m/z 402.29 [M+H]+; ¹H-NMR (400 MHz, dimethylsulfoxide-d₆) δ 8.94-8.71 (m, 1H), 7.79-7.54 (m, 2H), 5.76 (s, 1H), 5.45-5.40 (m, 2H), 4.84-4.60 (m, 1H), 4.41-4.38 (m, 1H), 4.34-4.21 (m, 1H), 4.18-4.07 (m, 1H), 3.68-3.48 (m, 3H), 1.95-1.68 (m, 5H), 1.65-1.53 (m, 1H).

### Example 32: Synthesis of Compound A42

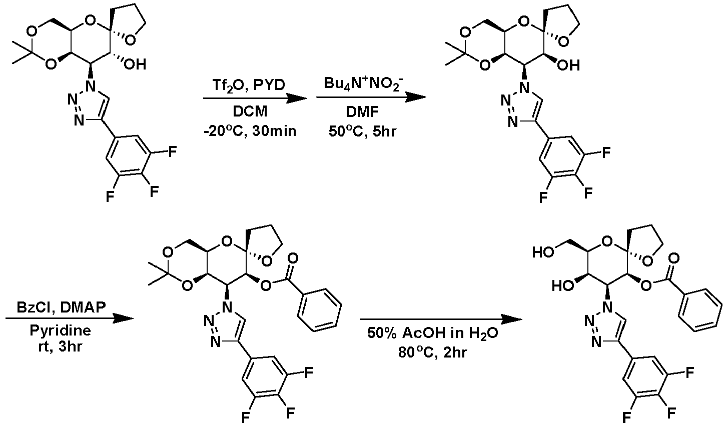

### Synthesis of (2S,4a'R,7'S,8'R,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3] dioxin] -7'-ol

(2S,4a'R,7'R,8'R,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-ol (52 mg, 0.118 mmol) was dissolved in 2 mL of dichloromethane, and then pyridine (19 µL, 0.236 mmol) was added dropwise thereto. Then, the temperature was lowered to -20 °C, and then Tf₂O (24 µL, 0.142 mmol) was slowly added dropwise. The reaction mixture was stirred at -20 °C for 30 minutes, then dichloromethane and a 5% aqueous hydrochloric acid solution were added to terminate the reaction, and then the mixture was extracted with dichloromethane to obtain an organic layer. The extracted organic layer was washed with a saturated aqueous sodium hydrogen carbonate solution. The organic layer was dried over anhydrous sodium sulfate, filtered, then concentrated, and dried in vacuum for 10 minutes. The reaction mixture was dissolved in 5 mL of *N*,*N*-dimethylformamide, tetrabutylammonium azide (102 mg, 0.354 mmol) was added dropwise thereto, followed by stirring at 50 °C for 5 hours. The temperature was lowered to 0 °C, ethyl acetate and a 5% aqueous hydrochloric acid solution were added thereto to terminate the reaction, and then the reaction mixture was extracted three times with ethyl acetate. The extracted organic layer was washed sequentially with a saturated aqueous sodium hydrogen carbonate solution and a saturated aqueous sodium chloride solution. The organic layer was dried over anhydrous sodium sulfate, filtered, and then concentrated. The concentrated reaction mixture was subjected to silica gel column chromatography with 60% ethyl acetate/n-hexane to obtain the title compound as a white solid (32 mg, yield: 62% (two steps)).

¹H-NMR (400 MHz, CDCl₃) δ 8.05 (d, J = 7.1 Hz, 1H), 7.45 (td, J = 7.6, 1.0 Hz, 2H), 5.76-5.59 (m, 2H), 4.60 (s, 1H), 4.53 (dd, J = 12.7, 8.7 Hz, 1H), 4.20-4.03 (m, 4H), 3.97 (dd, J = 14.6, 7.6 Hz, 1H), 3.85 (dd, J = 15.4, 7.4 Hz, 1H), 2.14-2.02 (m, 1H), 1.97-1.74 (m, 2H), 1.52 (d, J = 4.5 Hz, 3H), 1.43 (d, J = 4.5 Hz, 3H), 1.34-1.19 (m, 1H).

### Synthesis of (2S,4a'R,7'S,8'S,8a'R)-2',2'-dimethyl-8'-(4-(3,4,5-trifluorophenyl)-1H-1,2,3 -triazol-1-yl)hexahydro-3H,4'H-spiro[furan-2,6'-pyrano[3,2-d][1,3]dioxin]-7'-yl benzoate

*(2S,4a'R,7'S, 8'R, 8a'R)-2',2'-dimethyl-8'-(4-(3,4, 5-trifluorophenyl)-1H-1,2,3-triazol-1-*yl)hexahydro-3*H*,4'*H*-spiro[furan-2,6'-pyrano[3,2-*d*][1,3]dioxin]-7'-ol (17 mg, 0.039 mmol) was dissolved in 2 mL of pyridine, and then benzoyl chloride (7 µL, 0.058 mmol) and 4-dimethylaminopyridine (7 mg, 0.058 mmol) were added dropwise thereto, followed by stirring at room temperature for 3 hours. The reaction solution was extracted by adding ethyl acetate and distilled water to recover an organic layer. The organic layer was dried over anhydrous sodium sulfate, filtered, and then concentrated under reduced pressure. The concentrate was subjected to silica gel column chromatography with 50% ethyl acetate/n-hexane to obtain the title compound as a white solid (15 mg, yield: 72%).

¹H-NMR (400 MHz, CDCl₃) δ 8.04-7.94 (m, 3H), 7.55 (t, J = 7.4 Hz, 1H), 7.48-7.36 (m, 4H), 5.54 (dd, J = 9.4, 4.0 Hz, 1H), 4.95 (d, J = 9.4 Hz, 1H), 4.70-4.61 (m, 1H), 4.41-4.26 (m, 2H), 4.19-4.00 (m, 3H), 2.77 (m, 2H), 2.13-1.98 (m, 2H), 1.46 (s, 3H), 1.43 (s, 3H).

### Synthesis of (5S,7R,8R,9S,10S)-8-hydroxy-7-(hydroxymethyl)-9-(4-(3,4,5-trifluorophenyl)-1H-1,2,3-triazol-1-yl)-1,6-dioxaspiro[4.5]decan-10-yl benzoate

(2*S*,4a'*R*,7'*S*,8'*S*,8a'*R*)-2',2'-dimethyl-8'-(4-(3,4,5-trifluorophenyl)-1*H*-1,2,3-triazol-1-yl)hexahydro-3H,4'H-spiro[furan-2,6'-pyrano[3,2-*d*][1,3]dioxin]-7'-yl benzoate (15 mg) was dissolved in 2 mL of a 50% aqueous acetic acid solution, followed by stirring at 80 °C for 16 hours. The reaction mixture was concentrated, then neutralized by adding a saturated aqueous sodium hydrogen carbonate solution, and then extracted with dichloromethane and dried over anhydrous sodium sulfate, filtered, and then concentrated under reduced pressure. The concentrate was subjected to silica gel column chromatography with 5.0% methanol/dichloromethane to obtain the title compound as a white solid (7 mg, yield: 50%).

ESIMS: m/z 506.35 [M+H]+; ¹H-NMR (400 MHz, CD₃OD) δ 8.56 (s, 1H), 7.99-7.90 (m, 2H), 7.65-7.51 (m, 3H), 7.48-7.38 (m, 2H), 5.54 (dd, J = 9.1, 4.6 Hz, 1H), 5.06 (d, J = 9.1 Hz, 1H), 4.50 (dt, J = 23.3, 11.6 Hz, 1H), 4.37-4.25 (m, 3H), 3.95-3.78 (m, 2H), 2.96-2.74 (m, 2H), 2.10-1.98 (m, 2H).

### Example 33: Synthesis of Compound A44

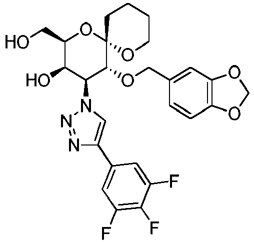

### Synthesis of (2R,3R,4S,5R,6S)-5-(benzo[d][1,3]dioxol-5-ylmethoxy)-2-(hydroxymethyl)-4-(4-(3,4,5-trifluorophenyl)-1H-1,2,3-triazol-1-yl)-1,7-dioxaspiro[5.5]undecan-3-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (5*S*,7*R*,8*R*,9*S*,10*R*)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 550.41 [M+H]+; ¹H-NMR (400 MHz, CDCl₃) δ 7.81 (s, 1H), 7.32-7.39 (m, 2H), 6.55-6.57 (m, 1H), 6.42-6.43 (m, 2H), 5.82 (dd, J = 16.2, 1.4 Hz, 2H), 4.92 (dd, J = 10.6, 2.3 Hz, 1H), 4.50 (s, 1H), 4.25 (d, J = 11.0 Hz, 1H), 4.08 (d, J = 10.6 Hz, 1H), 4.00-4.03 (m, 1H), 3.91-3.94 (m, 2H), 3.80-3.83 (m, 2H), 3.70-3.77 (m, 1H), 1.88-2.01 (m, 2H), 1.58-1.72 (m, 6H)

### Example 34: Synthesis of Compound A45

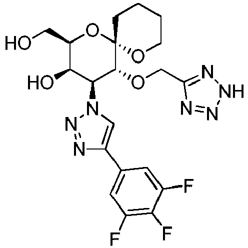

### Synthesis of (2R,3R,4S,5R,6S)-5-((2H-tetrazol-5-yl)methoxy)-2-(hydroxymethyl)-4-(4-(3,4,5-trifluorophenyl)-1H-1,2,3-triazol-1-yl)-1,7-dioxaspiro[5.5]undecan-3-ol

The title compound was obtained by the synthesis from (2S,4a'R,1'R,8'R,8a'R)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (5*S*,7*R*,8*R*,9*S*,10*R*)-10-((2*H*-tetrazol-5-yl)methoxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 498.31 [M+H]+; ¹H-NMR (400 MHz, CD₃OD) δ 8.51 (s, 1H), 7.53-7.60 (m, 2H), 5.27 (dd, J = 11.2, 2.9 Hz, 1H), 4.80 (dd, J = 43.4, 13.7 Hz, 2H), 4.34 (d, J = 11.3 Hz, 1H), 4.12 (dd, J = 2.7, 1.2 Hz, 1H), 3.91-3.94 (m, 1H), 3.71-3.88 (m, 4H), 1.89-2.07 (m, 2H), 1.54-1.71 (m, 4H)

### Example 35: Synthesis of Compound A46

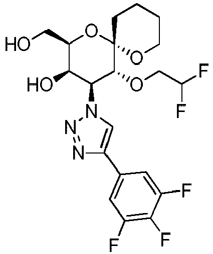

### Synthesis of (2R,3R,4S,5R,6S)-5-(2,2-difluoroethoxy)-2-(hydroxymethyl)-4-(4-(3,4,5-trifluorophenyl)-1H-1,2,3-triazol-1-yl)-1,7-dioxaspiro[5.5]undecan-3-ol

The title compound was obtained by the synthesis from (2S,4a'R,7'R,8'R,8a'R)-2'2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (5S,7R,8R,9S,10R)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 480.22 [M+H]+; ¹H-NMR (400 MHz, CD₃OD) δ 8.63 (s, 1H), 7.60-7.68 (m, 2H), 5.54 (tt, J = 55.4, 3.9 Hz, 1H), 5.22 (dd, J = 11.2, 2.9 Hz, 1H), 4.18 (d, J = 11.3 Hz, 1H), 4.12 (dd, J = 2.9, 1.0 Hz, 1H), 3.89-3.93 (m, 1H), 3.71-3.86 (m, 4H), 3.58-3.69 (m, 1H), 3.41-3.52 (m, 1H), 1.95-2.04 (m, 2H), 1.55-1.74 (m, 4H)

### Example 36: Synthesis of Compound A47

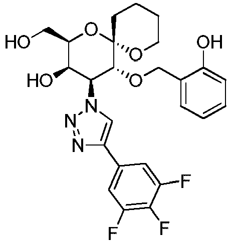

### Synthesis of (2R,3R,4S,5R,6S)-5-((2-hydroxybenzyl)oxy)-2-(hydroxymethyl)-4-(4-(3,4,5-trifluorophenyl)-1H-1,2,3-triazol-1-yl)-1,7-dioxaspiro[5.5]undecan-3-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5S,7R,8R,9S,10R)-10-((2-hydroxybenzyl)oxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 522.44 [M+H]+; ¹H-NMR (400 MHz, CD₃OD) δ 8.50 (s, 1H), 7.53-7.57 (m, 2H), 6.93 (t, J = 8.0 Hz, 1H), 6.80 (d, J = 7.0 Hz, 1H), 6.60 (t, J = 7.0 Hz, 2H), 5.17 (dd, J = 11.0, 2.7 Hz, 1H), 4.52 (d, J = 11.3 Hz, 1H), 4.23 (t, J = 11.2 Hz, 2H), 4.10 (s, 1H), 3.90 (t, J = 5.7 Hz, 1H), 3.73-3.85 (m, 4H), 1.99-2.14 (m, 2H), 1.53-1.71 (m, 4H)

### Example 37: Synthesis of Compound A48

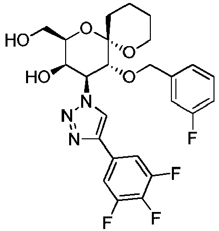

### Synthesis of (2R,3R,4S,5R,6S)-5-((3-fluorobenzyl)oxy)-2-(hydroxymethyl)-4-(4-(3,4,5-trifluorophenyl)-1H-1,2,3-triazol-1-yl)-1,7-dioxaspiro[5.5]undecan-3-ol

The title compound was obtained by the synthesis from (2*S*,4a'*R*,7'*R*,8'*R*,8a'*R*)-2'2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (2*R*,3*R*,4*S*,5*R*,6*S*)-5-(benzyloxy)-2-(hydroxymethyl)-4-(4-(3,4,5-trifluorophenyl)-1*H*-1,2,3-triazol-1-yl)-1,7-dioxaspiro[5.5]undecan-3-ol.

ESIMS: m/z 524.39 [M+H]+; ¹H-NMR (400 MHz, CDCl₃) δ 7.85 (s, 1H), 7.39-7.30 (m, 2H), 7.16-7.09 (m, 1H), 6.89-6.82 (m, 1H), 6.76-6.70 (m, 2H), 4.98 (dd, J = 10.7, 2.3 Hz, 1H), 4.57 (t, J = 1.2 Hz, 1H), 4.50 (s, 1H), 4.33 (d, J = 11.2 Hz, 1H), 4.11 f(d, J = 10.7 Hz, 1H), 4.06-4.00 (m, 1H), 3.98-3.91 (m, 2H), 3.88 (d, J= 11.3 Hz, 1H), 3.87-3.81 (m, 1H), 3.78-3.70 (m, 1H), 2.23 (dd, J = 7.2, 4.7 Hz, 1H), 1.99-1.88 (m, 2H), 1.78-1.66 (m, 3H), 1.64-1.57 (m, 1H)

### Example 38: Synthesis of Compound A49

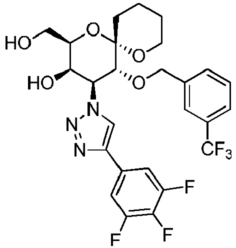

### Synthesis of (2R,3R,4S,5R,6S)-2-(hydroxymethyl)-5-((3-(trifluoromethyl)benzyl)oxy)-4-(4-(3,4,5-trifluorophenyl)-1H-1,2,3-triazol-1-yl)-1,7-dioxaspiro[5.5]undecan-3-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (2*R*,3*R*,4*S*,5*R*,6*S*)-5-(benzyloxy)-2-(hydroxymethyl)-4-(4-(3,4,5-trifluorophenyl)-1*H*-1,2,3-triazol-1-yl)-1,7-dioxaspiro[5.5]undecan-3-ol.

ESIMS: m/z 574.29 [M+H]+; ¹H-NMR (400 MHz, CDCl₃) δ 7.87 (s, 1H), 7.43 (d, J = 7.7 Hz, 1H), 7.37-7.26 (m, 4H), 7.15 (d, J = 7.2 Hz, 1H), 5.02 (d, J = 10.5 Hz, 1H), 4.50 (s, 2H), 4.41 (d, J = 11.6 Hz, 1H), 4.15 (d, J = 10.6 Hz, 1H), 4.09-4.00 (m, 1H), 3.99-3.92 (m, 3H), 3.88-3.82 (m, 1H), 3.78-3.71 (m, 1H), 2.22-2.17 (m, 1H), 1.99-1.91 (m, 2H), 1.78-1.66 (m, 3H), 1.64-1.57 (m, 1H)

### Example 39: Synthesis of Compound A50

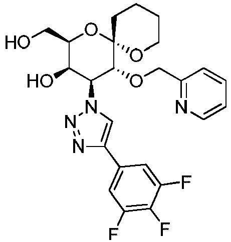

### Synthesis of (2R,3R,4S,5R,6S)-2-(hydroxymethyl)-5-(pyridin-2-ylmethoxy)-4-(4-(3,4,5-trifluorophenyl)-1H-1,2,3-triazol-1-yl)-1,7-dioxaspiro[5.5]undecan-3-ol

The title compound was obtained by the synthesis from (2S,4a'R,7'R,8'R,8a'R)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (2*R*,3*R*,4*S*,5*R*,6*S*)-5-(benzyloxy)-2-(hydroxymethyl)-4-(4-(3,4,5-trifluorophenyl)-1*H*-1,2,3-triazol-1-yl)-1,7-dioxaspiro[5.5]undecan-3-ol.

ESIMS: m/z 507.50 [M+H]+; ¹H-NMR (400 MHz, CDCl₃) δ 8.40 (d, J = 4.1 Hz, 1H), 8.03 (s, 1H), 7.50 (t, J = 7.6 Hz, 1H), 7.40-7.32 (m, 2H), 7.09-7.01 (m, 2H), 5.07 (dd, J = 10.7, 2.2 Hz, 1H), 4.61 (s, 1H), 4.54 (d, J = 12.0 Hz, 1H), 4.51 (s, 1H), 4.19 (d, J = 10.8 Hz, 1H), 4.14 (d, J = 12.1 Hz, 1H), 4.08-3.90 (m, 3H), 3.87-3.80 (m, 1H), 3.73 (t, J = 11.3 Hz, 1H), 2.47 (s, 1H), 2.03-1.87 (m, 2H), 1.78-1.53 (m, 4H)

### Example 40: Synthesis of Compound A51

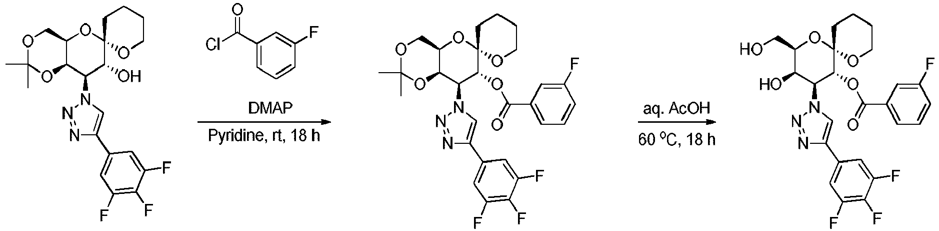

### Synthesis of (2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl 3-fluorobenzoate

(2*S*,4a'*R*,1'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol (20.0 mg, 0.044 mmol) was dissolved in 2.0 mL of pyridine, and then 4-dimethylaminopyridine (8.1 mg, 0.066 mmol) and 3-fluorobenzoyl chloride (8.4 mg, 0.053 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with in 0% to 5% methanol/dichloromethane to obtain an intermediate as a white solid (25.0 mg).

The intermediate was dissolved in 5.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (21.6 mg, yield: 91.0%).

ESIMS: m/z 538.38 [M+H]+; ¹H-NMR (400 MHz, CD₃OD)) δ 8.52 (s, 1H), 7.86-7.62 (m, 2H), 7.56-7.47 (m, 2H), 7.46-7.38 (m, 2H), 7.35-7.26 (m, 2H), 5.85 (d, J = 11.2 Hz, 1H), 4.24 (s, 1H), 4.05 (t, J = 6.1 Hz, 1H), 3.94-3.74 (m, 4H), 3.32 (d, J = 14.1 Hz, 1H), 2.03-1.98 (m, 1H), 1.73 (d, J = 13.8 Hz, 1H), 1.69-1.50 (m, 2H).

### Example 41: Synthesis of Compound A52

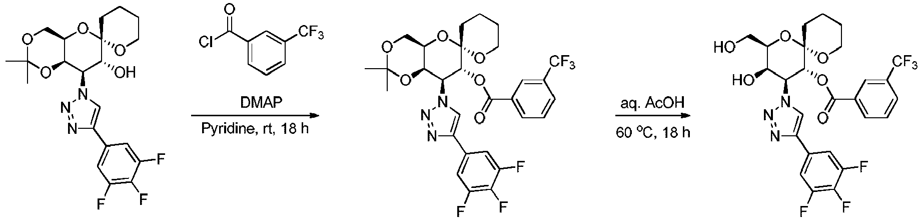

### Synthesis of (2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl 3-(trifluoromethyl)benzoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-ol (20.0 mg, 0.044 mmol) was dissolved in 2.0 mL of pyridine, and then 4-dimethylaminopyridine (8.1 mg, 0.066 mmol) and 3-(trifluoromethyl)benzoyl chloride (8.4 mg, 0.053 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain an intermediate as a white solid (27.0 mg).

The intermediate was dissolved in 5.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (24.0 mg, yield: 93.0%).

ESIMS: m/z 588.27 [M+H]+; ¹H-NMR (400 MHz, CD₃OD) δ 8.55 (s, 1H), 8.08 (d, J = 8.7 Hz, 2H), 7.87 (d, J = 7.7 Hz, 1H), 7.63 (t, J = 7.7 Hz, 1H), 7.56-7.42 (m, 2H), 5.88 (d, J = 11.2 Hz, 1H), 5.50 (dd, J = 11.3, 2.7 Hz, 1H), 4.26 (s, 1H), 4.07 (t, J = 6.3 Hz, 1H), 3.95-3.73 (m, 4H), 2.12-1.92 (m, 1H), 1.75 (d, J = 13.6 Hz, 1H), 1.68-1.51 (m, 4H).

### Example 42: Synthesis of Compound A53

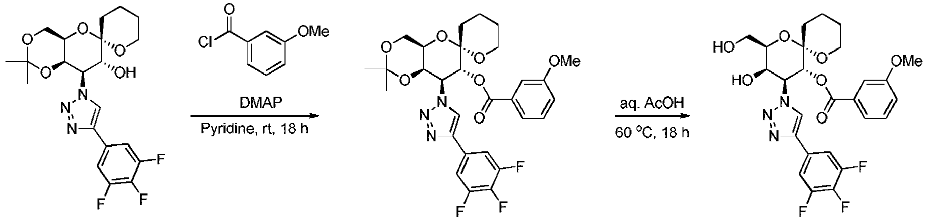

### Synthesis of (2R,3,R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl 3-methoxybenzoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-ol (20.0 mg, 0.044 mmol) was dissolved in 2.0 mL of pyridine, and then 4-dimethylaminopyridine (8.1 mg, 0.066 mmol) and 3-methoxybenzoyl chloride (9.0 mg, 0.053 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5.0% methanol/dichloromethane to obtain an intermediate as a white solid (22 mg).

The intermediate was dissolved in 5.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (17.5 mg, yield: 72.6%).

ESIMS: m/z 550.54 [M+H]+; ¹H-NMR (400 MHz, CD₃OD) δ 8.50 (s, 1H), 7.53-7.45 (m, 2H), 7.41 (dd, J = 7.7, 1.0 Hz, 1H), 7.33-7.24 (m, 2H), 7.10 (dd, J = 8.1, 2.7 Hz, 1H), 5.84 (d, J = 11.4 Hz, 1H), 5.47 (dd, J = 11.4, 2.8 Hz, 1H), 4.23 (d, J = 2.6 Hz, 1H), 4.05 (t, J = 6.1 Hz, 1H), 3.87-3.81 (m, 4H), 3.77 (s, 3H), 2.08-1.93 (m, 1H), 1.77-1.69 (m, 1H), 1.65-1.53 (m, 4H).

### Example 43: Synthesis of Compound A54

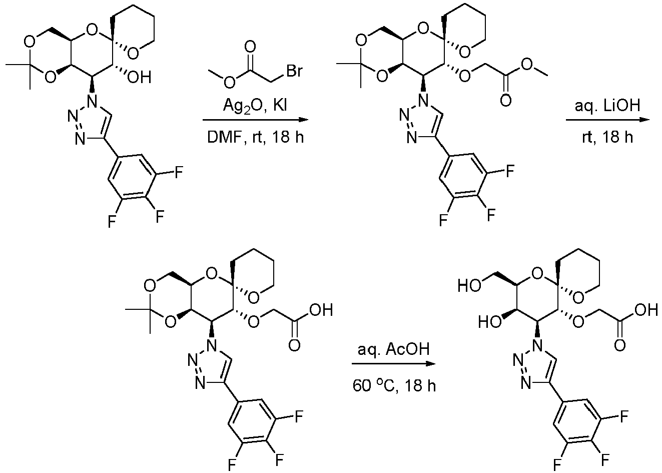

### Synthesis of methyl-2-(((2S,4a'R,7'R,8'S,8a'R)-2',2'-dimethyl-8'-(4-(3,4,5-trifluorophenyl)- 1H-1,2,3-triazol-1-yl)octahydro-47/-spiro[pyran-2,6'-pyrano[3,2-d][1,3]dioxin]-7'-yl)oxy)acetate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-ol (20.0 mg, 0.044 mmol) was dissolved in 1.0 mL of *N*,*N*-dimethylformamide, and then silver(I) oxide (102.0 mg, 0.44 mmol), potassium iodide (29.0 mg, 0.176 mmol), and methyl bromoacetate (20.2 mg, 0.132 mmol) were sequentially added dropwise thereto, followed by stirring at room temperature for 18 hours. The reaction solution was filtered through Celite, then extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (23.2 mg).

### Synthesis of 2-(((2S,4a'R,1'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)oxy)acetic acid

Methyl 2-(((2S,4a'R,7'R,8'S,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-*d*][1,3]dioxin]-1'-yl)oxy)acetate (23.2 mg, 0.044 mmol) was dissolved in 1.0 mL of distilled water and 1.0 mL of tetrahydrofuran, and then lithium hydroxide (27.7 mg, 0.666 mmol) was added dropwise thereto, followed by stirring at room temperature for 18 hours. The reaction solution was washed with ethyl acetate, the water layer was adjusted to have a pH of 5 to 6 using a 1.0 N aqueous hydrochloric acid solution, and the reaction solution was extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (22.1 mg).

### Synthesis of 2-(((2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl)oxy)acetic acid

2-(((2S,4a'R,7'R,8'S,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-d][1,3]dioxin]-1'-yl)oxy)acetic acid (22.1 mg, 0.043 mmol) was dissolved in 2.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane (containing 1.0% trifluoroacetic acid) to obtain the title compound as a white solid (19 mg, yield: 97.0%).

ESIMS: m/z 474.46 [M+H]+; ¹H-NMR (400 MHz, CD₃OD) δ 8.59 (s, 1H), 7.66-7.60 (m, 2H), 5.28-5.21 (m, 1H), 4.22 (dd, J = 11.1, 2.4 Hz, 1H), 4.15-4.10 (m, 1H), 4.04 (d, J = 3.3 Hz, 1H), 3.98 (t, J = 3.5 Hz, 1H), 3.93-3.89 (m, 1H), 3.84-3.70 (m, 4H), 2.16-1.93 (m, 4H), 1.70-1.65 (m, 2H).

### Example 44: Synthesis of Compound A55

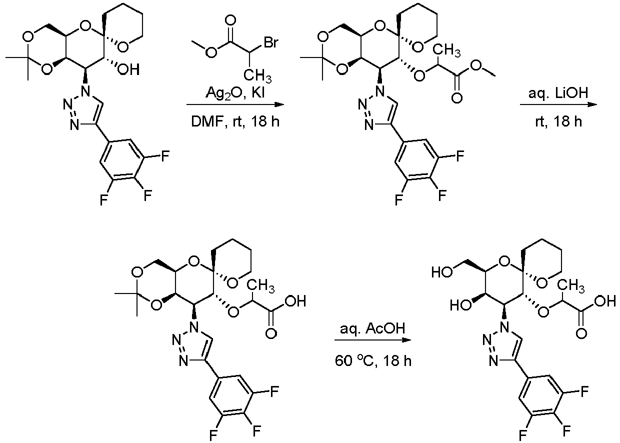

### Synthesis of methyl-2-(((2S,4a'R,1'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)oxy)propanoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol (20.0 mg, 0.044 mmol) was dissolved in 1.0 mL of N,N-dimethylformamide, and then silver(I) oxide (102 mg, 0.44 mmol), potassium iodide (29 mg, 0.176 mmol), and methyl 2-bromopropanoate (22 mg, 0.132 mmol) were sequentially added dropwise thereto, followed by stirring at room temperature for 18 hours. The reaction solution was filtered through Celite, then extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (23.8 mg).

### Synthesis of 2-(((2S,4a'R,7'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)oxy)propanoic acid

Methyl 2-(((2*S*,4a'*R*,1'*R*,8'*S*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-yl)oxy)propanoate (23.8 mg, 0.044 mmol) was dissolved in 1.0 mL of distilled water and 1.0 mL of tetrahydrofuran, and then lithium hydroxide (27.7 mg, 0.666 mmol) was added dropwise thereto, followed by stirring at room temperature for 18 hours. The reaction solution was washed with ethyl acetate, the water layer was adjusted to have a pH of 5 to 6 using a 1.0 N aqueous hydrochloric acid solution, and the reaction solution was extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (22.0 mg).

### Synthesis of 2-(((2,R,3,R,4S,5R,6,S)-3-hydroxy-2-(hydroxymethyl)-4-(4-(3,4,5-trifluorophenyl)-1H-1,2,3-triazol-1-yl)-1,7-dioxaspiro[5.5]undecan-5-yl)oxy)propanoic acid

2-(((2S,4a'R,1'R,8'S,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-*d*][1,3]dioxin]-1'-yl)oxy)propanoic acid (22.0 mg, 0.042 mmol) was dissolved in 2.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane (containing 1.0% trifluoroacetic acid) to obtain the title compound as a white solid (19.6 mg, yield: 96.0%).

ESIMS: m/z 488.35 [M+H]+; ¹H-NMR (400 MHz, CD₃OD) δ 8.67 (s, 1H), 7.69-7.61 (m, 2H), 5.22-5.16 (m, 1H), 4.20-4.16 (m, 1H), 4.10-4.06 (m, 1H), 3.93-3.87 (m, 1H), 3.80-3.72 (m, 3H), 3.65-3.55 (m, 2H), 2.25-2.15 (m, 1H), 2.07-1.93 (m, 2H), 1.67-1.59 (m, 3H), 0.92-0.76 (m, 3H).

### Example 45: Synthesis of Compound A56

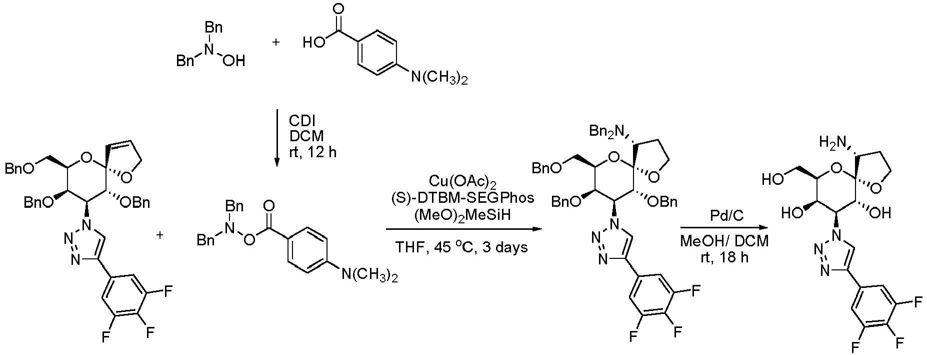

### Synthesis of (4R,5S,7R,8R,9S,10R)-N,N-dibenzyl-8,10- bis(benzyloxy)-7-((benzyloxy)methyl)-9-(4-(3,4,5-trifluorophenyl)-1H-1,2,3-triazol-1-yl)-1,6-dioxaspiro[4.5]decan-4-amine

Cu(OAc)₂ (8.1 mg, 0.045 mmol) and (S)-DTBM-SEGPHOS^{®} (58.1 mg, 0.049 mmol) were dissolved in 10 mL of tetrahydrofuran, stirred for 5 minutes, and then dimethoxymethylsilane (3.6 mg, 0.027 mmol) was added thereto. After reaction at room temperature for 15 minutes, 4-(((dibenzylamino)oxy)carbonyl)-*N*,*N*-dimethylaniline (348.1 mg, 0.896 mmol) was added thereto. 1-((5S,7R,8R,9S,10R)-8,10-bis(benzyloxy)-7-((benzyloxy)methyl)-1,6-dioxaspiro[4.5]dec-3-en-9-yl)-4-(3,4,5-trifluorophenyl)-1*H*-1,2,3-triazole (600 mg, 0.896 mmol) was dissolved in 10 mL of tetrahydrofuran, and slowly added dropwise to the reaction mixture, followed by stirring at 45 °C for 3 days. The reaction solution was filtered through Celite and then concentrated and subjected to silica gel column chromatography with 0% to 50% ethyl acetate/n-hexane to obtain the title compound as a white solid (204 mg, yield: 38%).

¹H-NMR (400 MHz, CDCl₃) δ 7.70 (s, 1H), 7.46-7.42 (m, 4H), 7.41-7.38 (m, 3H), 7.33-7.27 (m, 5H), 7.24-7.17 (m, 8H), 7.14-7.09 (m, 2H), 7.05-7.02 (m, 3H), 6.64-6.58 (m, 2H), 5.28 (dd, J = 11.2, 3.0 Hz, 1H), 4.58 (d, J = 11.1 Hz, 2H), 4.43 (d, J = 1.8 Hz, 1H), 4.37-4.30 (m, 2H), 4.16 (s, 1H), 4.13-4.05 (m, 3H), 3.96-3.88 (m, 2H), 3.76 (t, J = 9.0 Hz, 1H), 3.66-3.55 (m, 2H), 3.43-3.38 (m, 3H), 3.30 (d, J = 11.4 Hz, 1H), 2.26-2.13 (m, 1H), 2.06-1.96 (m, 1H).

### Synthesis of (4R,5S,7R,8R,9S,10R)-4-amino-7-(hydroxymethyl)-9-(4-(3,4,5-trifluorophenyl)-1H-1,2,3-triazol-1-yl)-1,6-dioxaspiro[4.5]decane-8,10-diol

1-((5*S*,7*R*,8*R*,9*S*,10*R*)-*N*,*N*-dibenzyl-8,10-bis(benzyloxy)-7-((benzyloxy)methyl)-9-(4-(3,4,5-trifluorophenyl)-1*H*-1,2,3-triazol-1-yl)-1,6-dioxaspiro[4.5]decan-4-amine (204 mg, 0.235 mmol) was dissolved in 20 mL of a solution of dichloromethane:methanol (1:2), and the mixture was added dropwise to a round-bottom flask, in which 10% Pd/C (204 mg, 0.024 mmol) was dissolved in 10 mL of a solution of dichloromethane:methanol (1:2), and then degassed with a hydrogen balloon for 10 minutes. The reaction solution was stirred at room temperature in a hydrogen atmosphere for 18 hours. The reaction solution was filtered through Celite and then concentrated and subjected to silica gel column chromatography with 0% to 50% ethyl acetate/n-hexane to obtain the title compound as a white solid (59.8 mg, yield: 60.2%).

ESIMS: m/z 417.16 [M+H]+; ¹H-NMR (400 MHz, CD₃OD) δ 8.52 (s, 1H), 7.69-7.56 (m, 2H), 5.02 (dd, J = 11.2, 2.8 Hz, 1H), 4.43 (d, J = 11.2 Hz, 1H), 4.15-4.01 (m, 2H), 3.92 (dd, J = 16.5, 8.3 Hz, 1H), 3.81-3.68 (m, 2H), 3.60 (dd, J = 10.4, 8.2 Hz, 1H), 3.34 (s, 1H), 2.40 (s, 1H), 2.13-1.86 (m, 3H).

### Example 46: Synthesis of Compound A57

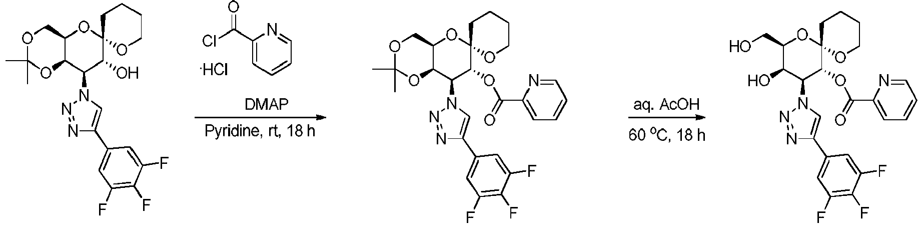

### Synthesis of (2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl 3-picolinate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-ol (20.0 mg, 0.044 mmol) was dissolved in 2.0 mL of pyridine, and then 4-dimethylaminopyridine (8.1 mg, 0.066 mmol) and picolinoyl chloride hydrochloride (20.0 mg, 0.053 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain an intermediate as a white solid (27.0 mg).

The intermediate was dissolved in 5.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (5.0 mg, yield: 20.0%).

ESIMS: m/z 521.4 [M+H]+; ¹H-NMR (400 MHz, CD₃OD) δ 8.59 (d, J = 4.1 Hz, 1H), 8.54 (s, 1H), 8.01-7.90 (m, 2H), 7.60-7.48 (m, 3H), 5.93 (d, J = 11.4 Hz, 1H), 5.51 (dd, J = 11.4, 2.9 Hz, 1H), 4.25 (s, 1H), 4.06 (t, J = 6.1 Hz, 1H), 3.88-3.69 (m, 4H), 2.08-1.96 (m, 2H), 1.78-1.59 (m, 4H).

### Example 47: Synthesis of Compound A58

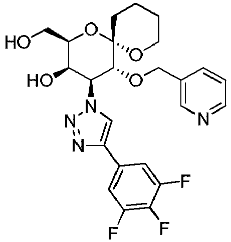

### Synthesis of (2R,3R,4S,5R,6S)-2-(hydroxymethyl)-5-(pyridin-3-ylmethoxy)-4-(4-(3,4,5-trifluorophenyl)-1H-1,2,3-triazol-1-yl)-1,7-dioxaspiro[5.5]undecan-3-ol

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-1'-ol in a similar manner to the preparation method of (5*S*,7*R*,8*R*,9*S*,10*R*)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 507.44 [M+H]+; ¹H-NMR (400 MHz, CD₃OD) δ 8.58 (s, 1H), 8.25 (d, J = 4.3 Hz, 1H), 8.13 (s, 1H), 7.55-7.59 (m, 2H), 7.39 (d, J = 7.8 Hz, 1H), 7.18-7.22 (m, 1H), 5.24 (dd, J = 11.0, 2.7 Hz, 1H), 4.69 (d, J = 11.7 Hz, 1H), 4.29 (dd, J = 23.1, 11.7 Hz, 2H), 4.13 (d, J = 1.6 Hz, 1H), 3.72-3.94 (m, 5H), 1.96-2.10 (m, 2H), 1.56-1.81 (m, 4H)

### Example 48: Synthesis of Compound A59

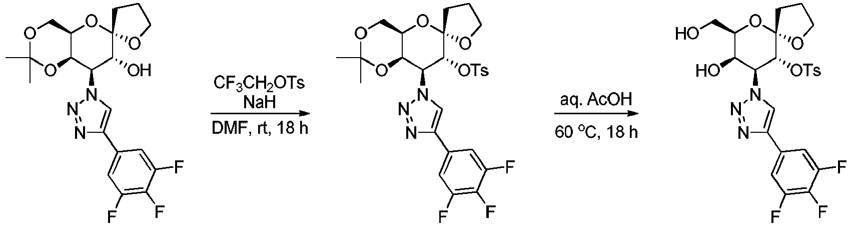

### Synthesis of (2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl 4-methylbenzenesulfonate

(2S,4a'R,7'R,8'R,8a'R)-2',2'-dimethyl-8'-(4-(3,4,5-trifluorophenyl)-1H-1,2,3-triazol-1-yl)hexahydro-3*H*,4'*H*-spiro[furan-2,6'-pyrano[3,2-*d*][1,3]dioxin]-7'-ol (20.0 mg, 0.044 mmol) was dissolved in 1.0 mL of *N*,*N*-dimethylformamide, and then 60% NaH (1.3 mg, 0.033 mmol) and 2,2,2-trifluoroethyl 4-methylbenzenesulfonate (33 mg, 0.132 mmol) were slowly added dropwise thereto at 0 °C, followed by stirring for 18 hours while elevating the temperature from 0 °C to room temperature. The reaction solution was diluted with ethyl acetate and then washed twice with a saturated aqueous ammonium chloride solution. The organic layer was recovered, dried over anhydrous sodium sulfate, filtered, and then concentrated under reduced pressure. The concentrate was subjected to silica gel column chromatography with 0 to 35% ethyl acetate/n-hexane to obtain the title compound as a white solid (8.3 mg, yield: 30%).

¹H-NMR (400 MHz, CDCl₃) δ 7.89 (s, 1H), 7.28-7.39 (m, 4H), 7.00 (d, J = 8.2 Hz, 2H), 5.55 (dd, J = 11.0, 3.1 Hz, 1H), 5.22 (d, J = 11.0 Hz, 1H), 4.31 (d, J = 2.3 Hz, 1H), 3.99-4.15 (m, 2H), 3.84 (d, J = 8.2 Hz, 1H), 3.63-3.67 (m, 2H), 2.22 (s, 3H), 1.86-2.17 (m, 4H), 1.65-1.75 (m, 2H), 1.53 (s, 3H), 1.29 (s, 3H)

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 4-methylbenzenesulfonate

(2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl 4-methylbenzenesulfonate (14.7 mg, 0.024 mmol) was dissolved in 1.0 mL of a 50% aqueous acetic acid solution, followed by stirring at 60 °C for 18 hours. The reaction mixture was concentrated, then neutralized by adding a saturated aqueous ammonium chloride solution, and then extracted with dichloromethane and dried over anhydrous magnesium sulfate, filtered, and then concentrated under reduced pressure. The concentrate was subjected to silica gel column chromatography with 0% to 4% methanol/dichloromethane to obtain the title compound as a white solid (11.5 mg, yield: 84%).

ESIMS: m/z 570.38 [M+H]+; ¹H-NMR (400 MHz, CDCl₃) δ 7.87 (s, 1H), 7.28-7.35 (m, 4H), 7.00 (s, 1H), 6.98 (s, 1H), 5.29-5.31 (m, 1H), 5.20-5.23 (m, 1H), 4.37 (s, 1H), 4.01 (d, J = 3.5 Hz, 2H), 3.91-3.92 (m, 1H), 3.82-3.84 (m, 1H), 3.69 (t, J = 10.6 Hz, 1H), 2.16 (s, 3H), 2.00-2.09 (m, 1H), 1.84-1.94 (m, 1H), 1.55-1.78 (m, 5H), 1.06-0.93 (1H)

### Example 49: Synthesis of Compound A60

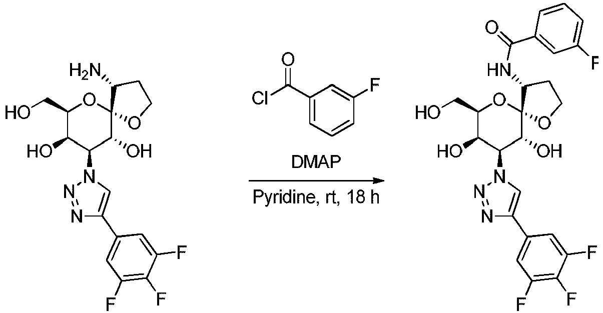

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3-fluorobenzamide

(4R,5S,7R,8R,9S,10R)-*N*,*N*-dibenzyl-8,10-bis(benzyloxy)-7-((benzyloxy)methyl)-9-(4-(3,4,5-trifluorophenyl)-1*H*-1,2,3-triazol-1-yl)-1,6-dioxaspiro[4.5]decan-4-amine (10 mg, 0.024 mmol) was dissolved in 1.0 mL of pyridine, and then 4-dimethylaminopyridine (4.4 mg, 0.036 mmol) and 3-fluorobenzoyl chloride (4.6 mg, 0.029 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (4.0 mg, yield: 31%).

ESIMS: m/z 539.32 [M+H]+; ¹H-NMR (400 MHz, CD₃OD) δ 8.52 (s, 1H), 7.73 (ddd, J = 7.8, 1.6, 1.0 Hz, 1H), 7.68-7.58 (m, 3H), 7.52 (td, J = 8.0, 5.7 Hz, 1H), 7.35-7.28 (m, 1H), 5.07 (dd, J = 11.2, 2.9 Hz, 1H), 5.00 (dd, J = 10.1, 8.7 Hz, 1H), 4.41 (d, J = 11.2 Hz, 1H), 4.23-4.14 (m, 2H), 4.11 (dd, J = 3.0, 1.1 Hz, 1H), 4.07 (d, J = 7.4 Hz, 1H), 3.82 (dd, J = 11.5, 7.6 Hz, 1H), 3.74 (dd, J = 11.5, 4.8 Hz, 1H), 2.48-2.39 (m, 1H), 2.18 (ddd, J = 19.2, 11.8, 9.1 Hz, 1H).

### Example 50: Synthesis of Compound A61

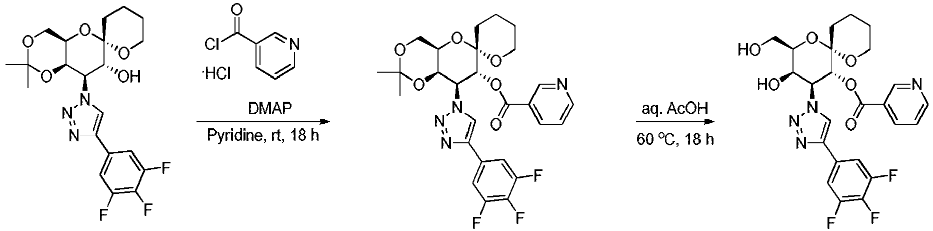

### Synthesis of (2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl nicotinate

(2S,4a'R,7'R,8'R,8a'R)-2',2'-dimethyl-8'-(4-(3,4,5-trifluorophenyl)-1H-1,2,3-triazol-1-yl)octahydro-4'*H*-spiro[pyran-2,6'-pyrano[3,2-*d*][1,3]dioxin]-7'-ol (20.0 mg, 0.044 mmol) was dissolved in 2.0 mL of pyridine, and then 4-dimethylaminopyridine (8.1 mg, 0.066 mmol) and nicotinoyl chloride hydrochloride (20.0 mg, 0.053 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain an intermediate as a white solid (8.0 mg).

The intermediate was dissolved in 5.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (5.0 mg, yield: 20.0%).

ESIMS: m/z 521.43 [M+H]+; ¹H-NMR (400 MHz, CD₃OD) δ 8.93 (s, 1H), 8.69 (s, 1H), 8.57 (s, 1H), 8.21 (d, J = 8.2 Hz, 1H), 7.58 -7.43 (m, 3H), 5.88 (d, J = 11.3 Hz, 1H), 5.49 (dd, J = 11.3, 2.7 Hz, 1H), 4.26 (s, 1H), 4.06 (t, J = 6.1 Hz, 1H), 3.88-3.69 (m, 4H), 2.08-1.96 (m, 2H), 1.78-1.59 (m, 4H).

### Example 51: Synthesis of Compound A62

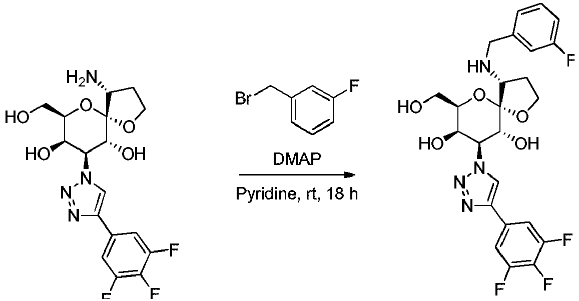

### Synthesis of (4R,5S,7R,8R,9S,10R)-4-((3-fluorobenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4R,5S,7R,8R,9S,10R)-4-amino-7-(hydroxymethyl)-9-(4-(3,4,5-trifluorophenyl)-1H-1,2,3-triazol-1-yl)-1,6-dioxaspiro[4.5]decane-8,10-diol (10 mg, 0.024 mmol) was dissolved in 1.0 mL of pyridine, and then 4-dimethylaminopyridine (8.8 mg, 0.072 mmol) and 3-fluorobenzoyl bromide (3.53 µL, 0.029 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to silica amine gel column chromatography with 0% to 5% methanol/dichloromethane to obtain the title compound as a white solid (1.2 mg, yield: 10%).

ESIMS: m/z 525.27 [M+H]+; ¹H-NMR (400 MHz, CD₃OD) δ 8.53 (s, 1H), 7.69-7.59 (m, 2H), 7.36 (dd, J= 13.7, 7.8 Hz, 1H), 7.26-7.17 (m, 2H), 7.04-6.96 (m, 1H), 5.03 (dd, J= 11.1, 2.8 Hz, 1H), 4.48 (d, J = 11.2 Hz, 1H), 4.16-4.03 (m, 3H), 3.95-3.86 (m, 2H), 3.79-3.74 (m, 1H), 3.72-3.68 (dd, J = 11.4, 5.0 Hz, 1H), 3.62-3.51 (m, 2H), 2.38-2.26 (m, 1H), 2.06-1.90 (m, 2H).

### Example 52: Synthesis of Compound A63

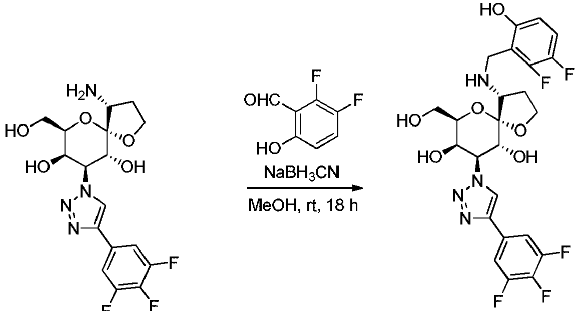

### Synthesis of (4R,5S,7R,8R,9S,10R)-4-((2,3-difluoro-6-hydroxybenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (10 mg, 0.024 mmol) was dissolved in 2.0 mL of methanol, and then 2,3-difluoro-6-hydroxybenzaldehyde (4.6 mg, 0.036 mmol) and sodium cyanoborohydride (3.0 mg, 0.036 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to amine silica gel column chromatography with 0% to 5% methanol/dichloromethane to obtain the title compound as a white solid (9.4 mg, yield: 70.1%).

ESIMS: m/z 559.42 [M+H]+; ¹H-NMR (400 MHz, CD₃OD) δ 8.52 (s, 1H), 7.64 (dd, J = 8.6, 6.8 Hz, 2H), 7.01 (dd, J = 19.1, 9.2 Hz, 1H), 6.60-6.50 (m, 1H), 5.03 (dd, J = 11.2, 2.7 Hz, 1H), 4.43 (d, J = 11.2 Hz, 1H), 4.18-3.98 (m, 4H), 3.95 (dd, J = 16.2, 8.2 Hz, 1H), 3.72 (qd, J = 11.5, 6.2 Hz, 2H), 3.65-3.57 (m, 1H), 2.44-2.28 (m, 1H), 2.06-1.87 (m, 2H).

### Example 53: Synthesis of Compound A64

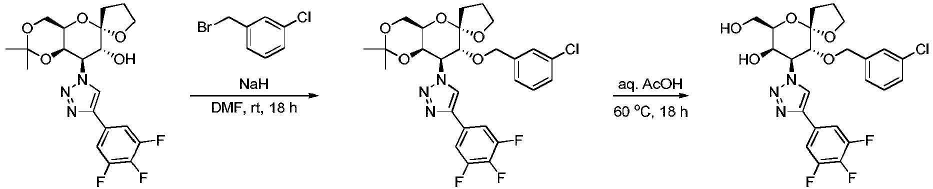

### Synthesis of 1-((2S,4a'R,7'R,8'S,8a'R)-7'-((3-chlorobenzyl)oxy)-2',2'-dimethylhexahydro-3H,4'H-spiro[furan-2,6'-pyrano[3,2-d][1,3]dioxin]-8'-yl)-4-(3,4,5-trifluorophenyl)-1H-1,2,3-triazole

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.045 mmol) was dissolved in 1.0 mL of *N*,*N*-dimethylformamide, and then 60% NaH (2.7 mg, 0.068 mmol) and 1-(bromomethyl)-3-chlorobenzene (14 mg, 0.068 mmol) were slowly added thereto at 0 °C, followed by stirring for 18 hours while elevating the temperature from 0 °C to room temperature. The reaction solution was extracted by adding ethyl acetate and distilled water to recover an organic layer. The organic layer was dried over anhydrous magnesium sulfate, filtered, and then concentrated under reduced pressure. The concentrated reaction mixture was subj ected to silica gel column chromatography with 0% to 5% methanol/dichloromethane to obtain the title compound as a white solid (25.0 mg).

### Synthesis of (5S,7R,8R,9S,10R)-10-((3-chlorobenzyl)oxy)-7-(hydroxymethyl)-9-(4-(3,4,5-trifluorophenyl)-1H-1,2,3-triazol-1-yl)-1,6-dioxaspiro[4.5]decan-8-ol

1-((2*S*,4a'*R*,7'*R*,8'*S*,8a'*R*)-7'-((3-chlorobenzyl)oxy)-2',2'-dimethylhexahydro-3*H*,4'*H-*spiro[furan-2,6'-pyrano[3,2-*d*][1,3]dioxin]-8'-yl)-4-(3,4,5-trifluorophenyl)-1*H*-1,2,3-triazole (25.0 mg, 0.044 mmol) was dissolved in 1.0 mL of a 50% aqueous acetic acid solution, followed by stirring at 60 °C for 18 hours. The reaction mixture was concentrated, then neutralized by adding a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (22.9 mg, yield: 96.8%).

ESIMS: m/z 526.47 [M+H]+; ¹H-NMR (400 MHz, CDCl₃) δ 7.91 (s, 1H), 7.40-7.33 (m, 2H), 7.16-7.08 (m, 2H), 6.97 (s, 1H), 6.85 (d, J = 7.1 Hz, 1H), 4.96 (dd, J = 2.07, 10.74 Hz, 1H), 4.57 (s, 1H), 4.50 (s, 1H), 4.40 (d, J = 10.7 Hz, 1H), 4.29 (d, J = 11.4 Hz, 1H), 4.13-3.86 (m, 6H), 3.84 (d, J = 11.4 Hz, 1H), 2.21 (s, 1H), 2.14-2.09 (m, 2H), 1.99-1.97 (m, 1H).

### Example 54: Synthesis of Compound A65

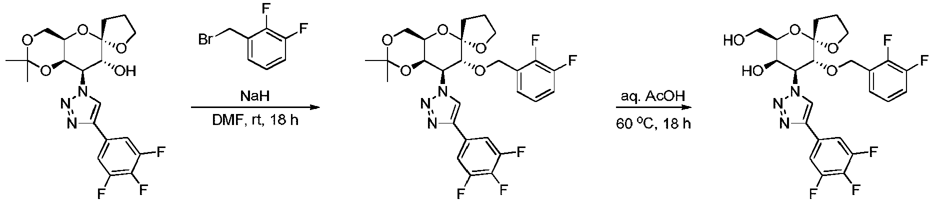

### Synthesis of 1-((2S,4a'R,7'R,8'S,8a'R)-7'-((2,3-difluorobenzyl)oxy)-2',2'-dimethylhexahydro-3H,4'H-spiro[furan-2,6'-pyrano[3,2-d][1,3]dioxin]-8'-yl)-4-(3,4,5-trifluorophenyl)-1H-1,2,3-triazole

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.045 mmol) was dissolved in 1.0 mL of *N*,*N*-dimethylformamide, and then 60% NaH (2.7 mg, 0.068 mmol) and 1-(bromomethyl)-2,3-difluorobenzene (14 mg, 0.068 mmol) were slowly added thereto at 0 °C, followed by stirring for 18 hours while elevating the temperature from 0 °C to room temperature. The reaction solution was extracted by adding ethyl acetate and distilled water to recover an organic layer. The organic layer was dried over anhydrous magnesium sulfate, filtered, and then concentrated under reduced pressure. The concentrated reaction mixture was subjected to silica gel column chromatography with 0% to 5% methanol/dichloromethane to obtain the title compound as a white solid (26 mg).

### Synthesis of (5S,7R,8R,9S, 10R)-10-((2,3-difluorobenzyl)oxy)-7-(hydroxymethyl)-9-(4-(3,4,5-trifluorophenyl)-1H-1,2,3-triazol-1-yl)-1,6-dioxaspiro[4.5]decan-8-ol

1-((2*S*,4a'*R*,7'*R*,8'*S*,8a'*R*)-7'-((2,3-difluorobenzyl)oxy)-2',2'-dimethylhexahydro-3*H*,4'*H-*spiro[furan-2,6'-pyrano[3,2-*d*][1,3]dioxin]-8'-yl)-4-(3,4,5-trifluorophenyl)-1*H*-1,2,3-triazole(26 mg, 0.046 mmol) was dissolved in 1.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (19.2 mg, yield: 81.0%).

ESIMS: m/z 528.23 [M+H]+; ¹H-NMR (400 MHz, CDCl₃) δ 7.98 (s, 1H), 7.44-7.37 (m, 2H), 7.02 (dd, J = 17.2, 8.7 Hz, 1H), 6.90 (dd, J = 12.8, 7.3 Hz, 1H), 6.70 (t, J = 7.0 Hz, 1H), 4.96 (dd, J = 10.8, 2.1 Hz, 1H), 4.59 (s, 1H), 4.50 (s, 1H), 4.40 (dd, J = 14.4, 10.9 Hz, 2H), 4.09-3.90 (m, 6H), 2.22-2.10 (m, 4H), 2.01-1.96 (m, 1H).

### Example 55: Synthesis of Compound A66

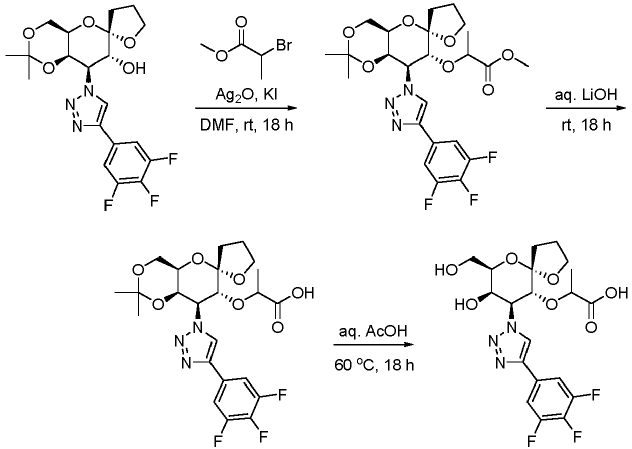

### Synthesis of 2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3] dioxin]-7'-yl)oxy)propanoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.045 mmol) was dissolved in 1.0 mL of *N*,*N*-dimethylformamide, and then silver(I) oxide (104.3 mg, 0.45 mmol), potassium iodide (30.0 mg, 0.180 mmol), and methyl 2-bromopropanoate (22.6 mg, 0.135 mmol) were sequentially added dropwise thereto, followed by stirring at room temperature for 18 hours. The reaction solution was filtered through Celite, then washed with ethyl acetate, and the filtrate was washed with a saturated aqueous ammonium chloride solution. The organic layer was recovered, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (17.0 mg, yield: 72%).

### Synthesis of 2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3] dioxin] -7'-yl)oxy)propanoic acid

Methyl 2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)propanoate (17.0 mg, 0.545 mmol) was dissolved in 10 mL of distilled water and 10 mL of tetrahydrofuran, and then lithium hydroxide (11.6 mg, 0.483 mmol) was added dropwise thereto, followed by stirring at room temperature for 18 hours. The reaction solution was diluted with ethyl acetate, the water layer was adjusted to have a pH of 5 to 6 using a 1.0 N aqueous hydrochloric acid solution, and the reaction solution was extracted with ethyl acetate, dried over anhydrous magnesium sulfate, filtered, and then concentrated under reduced pressure. The concentrate was subjected to silica gel column chromatography with 50% ethyl acetate/n-hexane to obtain the title compound as a white solid (13.5 mg).

### Synthesis of 2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)propanoic acid

2-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy) propanoic acid (20.0 mg, 0.039 mmol) was dissolved in 2.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (10.9 mg, yield: 94%).

ESIMS: m/z 474.24 [M+H]+; ¹H-NMR (400 MHz, CD₃OD) δ 8.70-8.68 (m, 1H), 7.68-7.64 (m, 2H), 5.15 (dd, J = 10.53, 2.10 Hz, 1H), 4.85-4.76 (m, 1H), 4.45 (d, J = 10.7 Hz, 1H), 4.14-4.02 (m, 3H), 3.68 (d, J = 6.0 Hz, 1H), 3.66-3.42 (m, 2H), 2.52-2.44 (m, 1H), 2.19-1.87 (m, 3H), 1.21-0.74 (m, 3H)

### Example 56: Synthesis of Compound A69

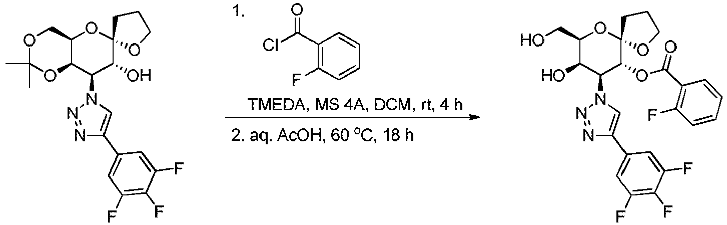

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 2-fluorobenzoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.044 mmol) was dissolved in 1.0 mL of dichloromethane, and then 4 Å molecular sieves (40 mg) and TMEDA (6.7 µL, 0.045 mmol) were added dropwise thereto, and then the mixture was stirred at room temperature for 10 minutes, and 2-fluorobenzoyl chloride was added thereto, followed by stirring at room temperature for 4 hours. The reaction solution was diluted with ethyl acetate, and then washwed twice with distilled water. The organic layer was recovered, dried over anhydrous sodium sulfate, filtered, and concentrated under reduced pressure to obtain a compound of (2*S*,4a'*R*,7'*R*, 8'*S*, 8a'*R*)-2',2'-dimethyl-8'-(4-(3,4, 5-trifluorophenyl)-1H-1,2,3 -triazol-1-yl)hexahydro-3*H*,4'*H*-spiro[furan-2,6'-pyrano[3,2-*d*][1,3]dioxin]-7'-yl 2-fluorobenzoate, and the next reaction was directly performed without an additional purification process.

(2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl 2-fluorobenzoate (0.045 mmol) was dissolved in 1.0 mL of a 50% aqueous acetic acid solution, followed by stirring at 60 °C for 18 hours. The reaction mixture was concentrated, then neutralized by adding a saturated aqueous ammonium chloride solution, and then extracted with dichloromethane and dried over anhydrous magnesium sulfate, filtered, and then concentrated under reduced pressure. The concentrate was subjected to silica gel column chromatography with 0% to 3% methanol/dichloromethane to obtain the title compound as a white solid (17.5 mg, yield: 74%).

ESIMS: m/z 524.39 [M+H]+; ¹H-NMR (400 MHz, CD₃OD) δ 8.55 (s, 1H), 7.53-7.62 (m, 4H), 7.17 (t, J = 7.6 Hz, 1H), 7.09-7.14 (m, 1H), 6.16 (d, J = 11.3 Hz, 1H), 5.42 (dd, J = 11.3, 2.7 Hz, 1H), 4.24 (s, 1H), 4.19 (t, J = 6.1 Hz, 1H), 4.00-4.12 (m, 2H), 3.75 (d, J = 5.9 Hz, 2H), 2.07-2.22 (m, 2H), 1.99-2.03 (m, 1H), 1.83-1.92 (m, 1H)

### Example 57: Synthesis of Compound A70

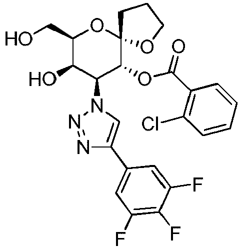

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 2-chlorobenzoate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 540.46 [M+H]+; ¹H-NMR (400 MHz, CD₃OD) δ 8.57 (s, 1H), 7.72-7.76 (m, 2H), 7.49-7.56 (m, 3H), 7.38 (t, J = 7.8 Hz, 1H), 6.13 (d, J = 11.3 Hz, 1H), 5.46 (dd, J = 11.3, 2.7 Hz, 1H), 4.25 (s, 1H), 4.21 (t, J = 6.1 Hz, 1H), 4.02-4.13 (m, 2H), 3.76 (d, J = 6.3 Hz, 2H), 2.06-2.20 (m, 2H), 1.93-2.01 (m, 1H), 1.79-1.88 (m, 1H)

### Example 58: Synthesis of Compound A71

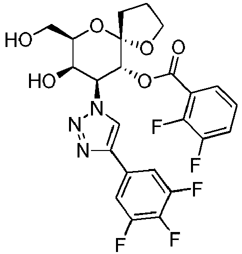

### Synthesis of (5S,7R,8R,9S, 10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 2,3-difluorobenzoate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 542.41 [M+H]+; ¹H-NMR (400 MHz, CD₃OD) δ 8.58 (s, 1H), 7.53-7.57 (m, 2H), 7.38-7.48 (m, 2H), 7.13-7.18 (m, 1H), 6.17 (d, J = 11.3 Hz, 1H), 5.42 (dd, J = 11.3, 2.7 Hz, 1H), 4.25 (s, 1H), 4.19 (t, J = 6.1 Hz, 1H), 4.01-4.12 (m, 2H), 3.75 (d, J = 5.9 Hz, 2H), 2.07-2.18 (m, 2H), 1.99-2.04 (m, 1H), 1.84-1.92 (m, 1H)

### Example 59: Synthesis of Compound A72

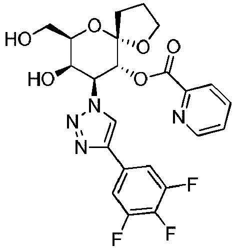

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl picolinate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl 3-fluorobenzoate.

ESIMS: m/z 507.5 [M+H]+; ¹H-NMR (400 MHz, CD₃OD) δ 8.57-8.59 (m, 2H), 7.91-7.97 (m, 2H), 7.50-7.59 (m, 3H), 6.22 (d, J = 11.3 Hz, 1H), 5.49 (dd, J = 11.3, 2.7 Hz, 1H), 4.26 (s, 1H), 4.22 (t, J = 6.1 Hz, 1H), 4.03-4.12 (m, 2H), 3.76 (d, J = 6.3 Hz, 2H), 2.06-2.15 (m, 2H), 1.96-2.05 (m, 1H), 1.81-1.89 (m, 1H)

### Example 60: Synthesis of Compound A73

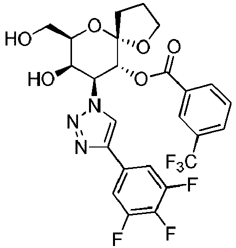

### Synthesis of (5S,7R,8R,9S, 10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3-(trifluoromethyl)benzoate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 574.41 [M+H]+; ¹H-NMR (400 MHz, CD₃OD) δ 8.59 (s, 1H), 8.04-8.07 (m, 2H), 7.87 (d, J = 7.8 Hz, 1H), 7.62 (t, J = 7.8 Hz, 1H), 7.49-7.53 (m, 2H), 6.16 (d, J = 11.3 Hz, 1H), 5.48 (dd, J = 11.3, 2.7 Hz, 1H), 4.26 (s, 1H), 4.22 (t, J = 6.1 Hz, 1H), 4.02-4.14 (m, 2H), 3.76 (d, J = 5.9 Hz, 2H), 2.08-2.17 (m, 2H), 1.95-2.03 (m, 1H), 1.80-1.85 (m, 1H)

### Example 61: Synthesis of Compound A74

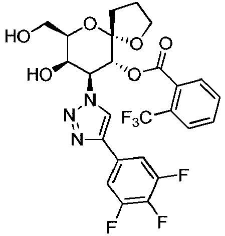

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3-(trifluoromethyl)benzoate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 574.35 [M+H]+; ¹H-NMR (400 MHz, CD₃OD) δ 8.58 (s, 1H), 7.72 (d, J = 7.8 Hz, 1H), 7.56-7.66 (m, 4H), 7.26 (d, J = 7.4 Hz, 1H), 6.16 (d, J = 11.3 Hz, 1H), 5.43 (dd, J = 11.3, 2.7 Hz, 1H), 4.23 (d, J = 2.0 Hz, 1H), 4.18 (t, J = 6.1 Hz, 1H), 3.98-4.09 (m, 2H), 3.75 (d, J = 6.3 Hz, 2H), 2.09-2.12 (m, 2H), 2.01-2.06 (m, 1H), 1.84-1.93 (m, 1H)

### Example 62: Synthesis of Compound A75

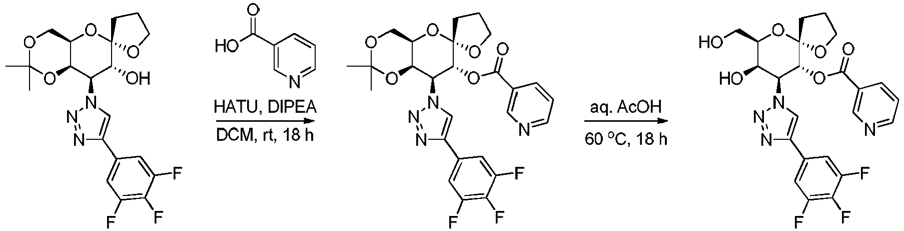

### Synthesis of (2S,4a'R,7'R,8'S,8a'R)-2',2'-dimethyl-8'-(4-(3,4,5-trifluorophenyl)-1H-1,2,3 -triazol-1 -yl)hexahydro-3H,4'H-spiro[furan-2,6'-pyrano[3,2-d][1,3]dioxin]-7'-yl nicotinate

(2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (51.2 mg, 0.116 mmol) was dissolved in 1.0 mL of dichloromethane, and then nicotinic acid (28.6 mg, 0.232 mmol), HATU (88.1 mg, 0.232 mmol), and *N,N*-diisopropylethylamine (0.1 mL, 0.578 mmol) were sequentially added dropwise thereto at room temperature, followed by stirring at room temperature for 18 hours. The reaction solution was diluted with ethyl acetate and then washed twice with a saturated aqueous sodium hydrogen carbonate solution. The organic layer was recovered, dried over anhydrous sodium sulfate, filtered, and then concentrated under reduced pressure. The concentrate was subjected to silica gel column chromatography with 0% to 3% methanol/dichloromethane to obtain the title compound as a white solid (17.0 mg, yield: 27%).

¹H-NMR (400 MHz, CDCl₃) δ 9.05 (s, 1H), 8.73 (s, 1H), 8.09 (d, J = 8.2 Hz, 1H), 8.04 (s, 1H), 7.30-7.39 (m, 3H), 6.06 (d, J = 11.3 Hz, 1H), 5.65 (dd, J = 11.3, 3.1 Hz, 1H), 4.44 (d, J = 2.3 Hz, 1H), 3.95-4.16 (m, 5H), 2.20-2.26 (m, 1H), 2.06-2.16 (m, 1H), 1.79-1.97 (m, 2H), 1.53 (s, 3H), 1.35 (s, 3H)

### Synthesis of (5S,7R,8R,9S10S)-8-hydroxy-7-(hydroxymethyl)-9-(4-(3,4,5-trifluorophenyl)-1H-1,2,3-triazol-1-yl)-1,6-dioxaspiro[4.5]decan-10-yl nicotinate

(2*S*,4a'*R*,7'*R*,8'*S*,8a',*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl nicotinate (16.0 mg, 0.029 mmol) was dissolved in 1.0 mL of a 50% aqueous acetic acid solution, followed by stirring at 60 °C for 18 hours. The reaction mixture was concentrated, neutralized by adding a saturated aqueous ammonium chloride solution thereto, and then extracted with dichloromethane. An organic layer was recovered, and dried over anhydrous magnesium sulfate, filtered, and concentrated under reduced pressure to perform silica gel column chromatography with 0% to 5% methanol/dichloromethane to obtain the title compound as a white solid (13.4 mg, yield: 91%).

ESIMS: m/z 507.38 [M+H]+; ¹H-NMR (400 MHz, CD₃OD) δ 8.91 (s, 1H), 8.69 (dd, J = 4.9, 1.4 Hz, 1H), 8.60 (s, 1H), 8.18-8.20 (m, 1H), 7.46-7.54 (m, 3H), 6.16 (d, J = 11.3 Hz, 1H), 5.46 (dd, J = 11.3, 3.1 Hz, 1H), 4.26 (d, J = 1.6 Hz, 1H), 4.21 (t, J = 6.3 Hz, 1H), 4.03-4.13 (m, 2H), 3.76 (d, J = 6.3 Hz, 2H), 2.08-2.16 (m, 2H), 1.96-2.04 (m, 1H), 1.80-1.89 (m, 1H)

### Example 63: Synthesis of Compound A76

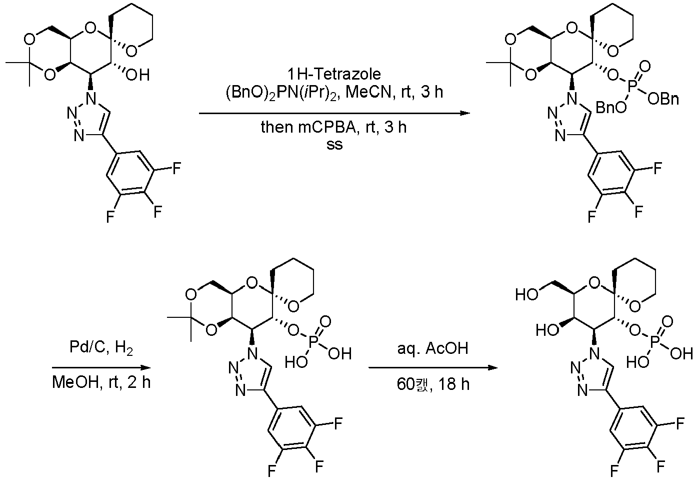

### Synthesis of dibenzyl(2S,4a'R,7'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)phosphate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-ol (78.1 mg, 0.171 mmol) was dissolved in 1.9 mL of a 0.45 M 1H-tetrazole acetonitrile solution, and then *N,N-*diisopropylphosphoramidite (0.23 mL, 0.685 mmol) was slowly added dropwise thereto, followed by stirring at room temperature for 3 hours. It was confirmed with TLC that the starting material disappeared, and then 70% mCPBA (168.8 mg, 0.685 mmol) was added thereto, followed by stirring at room temperature for 2 hours. After confirming the completion of the reaction with TLC, a saturated aqueous sodium hydrogen carbonate solution was added to terminate the reaction. The reaction solution was diluted with ethyl acetate and then washed twice with a saturated aqueous sodium hydrogen carbonate solution. The organic layer was recovered, dried over anhydrous sodium sulfate, filtered, and then concentrated under reduced pressure. The concentrate was subjected to silica gel column chromatography with 0% to 45% ethyl acetate/n-hexane to obtain the title compound in white foam (94.1 mg, yield: 77%).

¹H-NMR (400 MHz, CDCl₃) δ 8.11 (s, 1H), 7.21-7.45 (m, 10H), 7.00 (d, J = 6.3 Hz, 2H), 5.59 (dd, J = 11.0, 3.1 Hz, 1H), 5.03 (t, J = 10.6 Hz, 1H), 4.87-4.96 (m, 2H), 4.32-4.37 (m, 2H), 4.25 (dd, J = 11.7, 6.3 Hz, 1H), 4.11 (d, J = 12.9 Hz, 1H), 3.99 (d, J = 12.9 Hz, 1H), 3.82 (d, J = 10.2 Hz, 1H), 3.62-3.68 (m, 2H), 1.89-2.02 (m, 2H), 1.76 (d, J = 12.5 Hz, 1H), 1.49-1.58 (m, 6H), 1.29 (s, 3H)

### Synthesis of (2S,4a'R,7'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)dihydrogen phosphate

Dibenzyl((2*S*,4a'*R*,7'*R*,8'*S*,8a'*R*)-2',2'-dimethyl-8'-(4-(3,4,5-trifluorophenyl)-1*H*-1,2,3-triazol-1-yl)octahydro-4'H-spiro[pyran-2,6'-pyrano[3,2-*d*][1,3]dioxin]-7'-yl) phosphate (91.0 mg, 0.127 mmol) was dissolved in 3.0 mL of a solution of ethyl acetate:methanol (1:1 v/v), and the mixture was added thereto to a round-bottom flask, in which 10% Pd/C (165 mg, 20 wt%) was dissolved in 3.0 mL of a solution of ethyl acetate:methanol (1:1 v/v), and the reaction solution was degassed with a hydrogen balloon for 10 minutes. The reaction solution was stirred at room temperature in a hydrogen atmosphere for 24 hours. When the reaction was terminated, the resulting solution was filtered through a pad of Celite 545, and the filtrate was concentrated to obtain the title compound (67.3 mg, yield: 99%).

### Synthesis of (2R,3R,4S,5R,6S)-3-hydroxy-2-(hydroxymethyl)-4-(4-(3,4,5-trifluorophenyl)-1H-1,2,3-triazol-1-yl)-1,7-dioxaspiro[5.5]undecan-5-yl dihydrogen phosphate

(2*S*,4a'*R*,7'*R*,8'*S*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-yl dihydrogen phosphate (67.3 mg, 0.127 mmol) was dissolved in a 50% aqueous acetic acid solution, followed by stirring at 60 °C for 18 hours. The reaction mixture was concentrated, then neutralized by adding a saturated aqueous ammonium chloride solution, and then extracted with dichloromethane and dried over anhydrous magnesium sulfate, filtered, and then concentrated under reduced pressure. The concentrate was subjected to silica gel column chromatography with 0% to 5% methanol/dichloromethane (containing about 1% trifluoroacetic acid) to synthesize the title compound as a white solid (55 mg, yield: 87%).

ESIMS: m/z 496.35 [M+H]+; ¹H-NMR (400 MHz, CD₃OD) δ 8.49 (s, 1H), 7.60 (t, J = 6.1 Hz, 2H), 5.23 (d, J = 10.2 Hz, 1H), 4.07 (s, 1H), 3.75-3.92 (m, 5H), 3.33 (s, 1H), 1.98-2.21 (m, 2H), 1.56-1.67 (m, 4H)

### Example 64: Synthesis of Compound A77

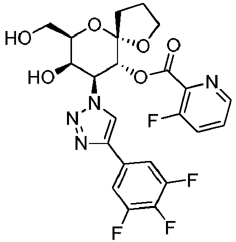

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3-fluoropicolinate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl nicotinate.

ESIMS: m/z 525.27 [M+H]+; ¹H-NMR (400 MHz, CD₃OD) δ 8.57 (s, 1H), 8.04 (q, J = 7.8 Hz, 1H), 7.85 (d, J = 7.0 Hz, 1H), 7.52-7.56 (m, 2H), 7.28 (dd, J = 8.2, 2.0 Hz, 1H), 6.18 (d, J = 11.3 Hz, 1H), 5.47 (dd, J = 11.3, 2.7 Hz, 1H), 4.25 (d, J = 2.0 Hz, 1H), 4.21 (t, J = 6.1 Hz, 1H), 4.08 (td, J = 13.5, 7.6 Hz, 2H), 3.76 (d, J = 6.3 Hz, 2H), 2.06-2.12 (m, 2H), 1.97-2.04 (m, 1H), 1.84-1.90 (m, 1H)

### Example 65: Synthesis of Compound A78

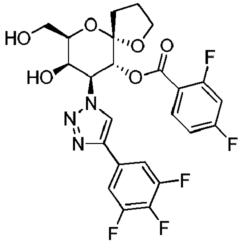

### Synthesis of (5S,7R,8R,9S, 10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 2,4-difluorobenzoate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 542.41 [M+H]+; ¹H-NMR (400 MHz, CD₃OD) δ 8.56 (s, 1H), 7.69-7.75 (m, 1H), 7.52-7.56 (m, 2H), 6.96-7.07 (m, 2H), 6.15 (d, J = 11.3 Hz, 1H), 5.41 (dd, J = 11.3, 3.1 Hz, 1H), 4.23 (d, J = 1.6 Hz, 1H), 4.19 (t, J = 6.1 Hz, 1H), 4.00-4.11 (m, 2H), 3.75 (d, J = 5.9 Hz, 2H), 2.07-2.17 (m, 2H), 1.97-2.05 (m, 1H), 1.83-1.91 (m, 1H)

### Example 66: Synthesis of Compound A79

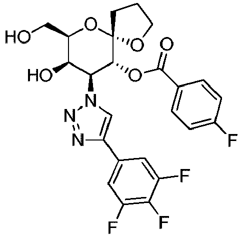

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 4-fluorobenzoate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 524.39 [M+H]+; ¹H-NMR (400 MHz, CD₃OD) δ 8.57 (s, 1H), 7.88 (dd, J = 8.6, 5.5 Hz, 2H), 7.51 (dd, J = 8.4, 6.8 Hz, 2H), 7.12 (t, J = 8.6 Hz, 2H), 6.12 (d, J = 11.3 Hz, 1H), 5.44 (dd, J = 11.3, 2.7 Hz, 1H), 4.24 (s, 1H), 4.20 (t, J = 6.1 Hz, 1H), 4.01-4.12 (m, 2H), 3.76 (d, J = 6.3 Hz, 2H), 2.06-2.15 (m, 2H), 1.92-2.00 (m, 1H), 1.77-1.87 (m, 1H)

### Example 67: Synthesis of Compound A80

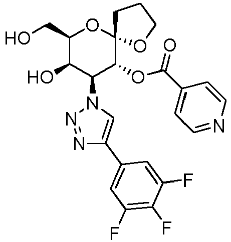

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl isonicotinate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl 3-fluorobenzoate.

ESIMS: m/z 507.25 [M+H]+; ¹H-NMR (400 MHz, CD₃OD) δ 8.91 (s, 1H), 8.69 (dd, J = 4.9, 1.4 Hz, 1H), 8.60 (s, 1H), 8.18-8.20 (m, 1H), 7.46-7.54 (m, 3H), 6.16 (d, J = 11.3 Hz, 1H), 5.46 (dd, J = 11.3, 3.1 Hz, 1H), 4.26 (s, 1H), 4.21 (t, J = 6.3 Hz, 1H), 4.03-4.13 (m, 2H), 3.76 (d, J = 6.3 Hz, 2H), 2.08-2.21 (m, 2H), 1.96-2.04 (m, 1H), 1.80-1.89 (m, 1H)

### Example 68: Synthesis of Compound A81

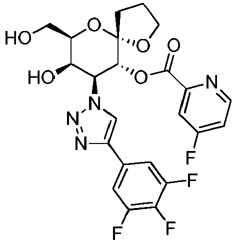

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3-fluoropicolinate

The title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl nicotinate.

ESIMS: m/z 525.33 [M+H]+; ¹H-NMR (400 MHz, CD₃OD) δ 8.57 (s, 1H), 8.49 (d, J = 2.3 Hz, 1H), 8.03 (q, J = 4.4 Hz, 1H), 7.70 (td, J = 8.4, 2.7 Hz, 1H), 7.51-7.55 (m, 2H), 6.19 (d, J = 11.3 Hz, 1H), 5.47 (dd, J = 11.3, 2.7 Hz, 1H), 4.25 (d, J = 1.6 Hz, 1H), 4.21 (t, J = 6.1 Hz, 1H), 4.03-4.12 (m, 2H), 3.76 (d, J = 6.3 Hz, 2H), 2.06-2.16 (m, 2H), 1.92-2.04 (m, 1H), 1.81-1.89 (m, 1H)

### Example 69: Synthesis of Compound A82

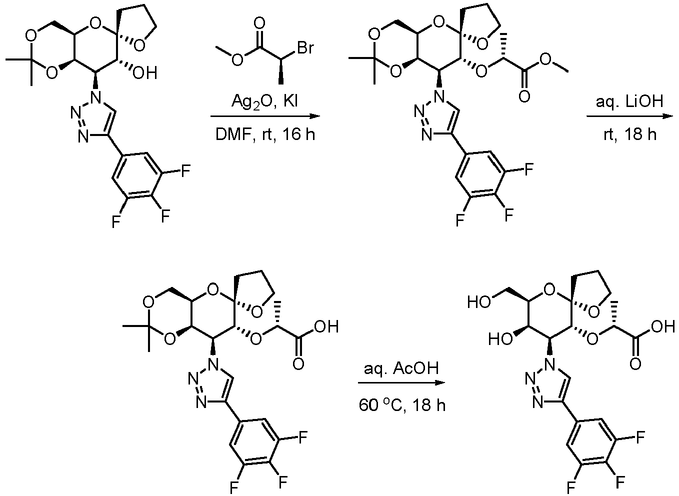

### Synthesis of (R)-methyl 2-(((2S,4a'R,7'R,8'S,8aR)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,]dioxin]-7'-yl)oxy)propanoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.045 mmol) was dissolved in 1.0 mL of *N,N-*dimethylformamide, and then silver(I) oxide (104.3 mg, 0.45 mmol), potassium iodide (30.0 mg, 0.180 mmol), and (,Sη-methyl 2-bromopropanoate (22.6 mg, 0.135 mmol) were sequentially added dropwise thereto, followed by stirring at room temperature for 18 hours. The reaction solution was filtered through Celite, then washed with ethyl acetate, dried over anhydrous magnesium sulfate, filtered, and then concentrated under reduced pressure. The concentrate was subjected to silica gel column chromatography with 50% ethyl acetate/n-hexane to obtain the title compound as a white solid (21.6 mg, yield: 91%).

### Synthesis of (R)-2-(((2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)propanoic acid

(*R*)-Methyl 2-(((2*S*,4a'*R*,7'*R*,8'*S*,8a'*R*)-2',2'-dimethyl-8'-(4-(3,4,5-trifluorophenyl)-1*H-*1,2,3 -triazol-1 -yl)hexahydro-3*H*,4'*H*-spiro[furan-2,6'-pyrano[3,2-*d*][1,3] dioxin]-7'-yl)oxy)propanoate (17.0 mg, 0.545 mmol) was dissolved in 10 mL of distilled water and 10 mL of tetrahydrofuran, and then lithium hydroxide (11.6 mg, 0.483 mmol) was added dropwise thereto, followed by stirring at room temperature for 18 hours. The reaction solution was diluted with ethyl acetate, the water layer was then adjusted to have a pH of 5 to 6 using a 1.0 N aqueous hydrochloric acid solution, and the reaction solution was extracted with ethyl acetate to recover an organic layer, the organic layer was dried over anhydrous magnesium sulfate, filtered, and then concentrated under reduced pressure. The concentrate was subjected to silica gel column chromatography with 0% to 50% ethyl acetate/n-hexane to obtain the title compound as a white solid (21.1 mg).

### Synthesis of (R)-2-(((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)propanoic acid

(R)-2-(((2*S*,4a'*R*,7'*R*,8'*S*,8a'-*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy) propanoic acid (20.0 mg, 0.039 mmol) was dissolved in 2.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and concentrated under reduced pressure. The concentrate was subjected to silica gel column chromatography with 0% to 5% methanol/dichloromethane (containing 1.0% trifluoroacetic acid) to obtain the title compound as a white solid (22.0 mg, yield: 99%).

ESIMS: m/z 474.43 [M+H]+; ¹H-NMR (400 MHz, CD₃OD) δ 8.71 (s, 1H), 7.68-7.64 (m, 2H), 5.15 (dd, J = 10.53, 2.10 Hz, 1H), 4.85-4.76 (m, 1H), 4.45 (d, J = 10.7 Hz, 1H), 4.14-3.98 (m, 3H), 3.68 (d, J = 6.0 Hz, 1H), 3.68-3.43 (m, 2H), 2.52-2.44 (m, 1H), 2.15-1.91 (m, 3H), 0.81 (d, J = 6.7 Hz, 3H)

### Example 70: Synthesis of Compound A83

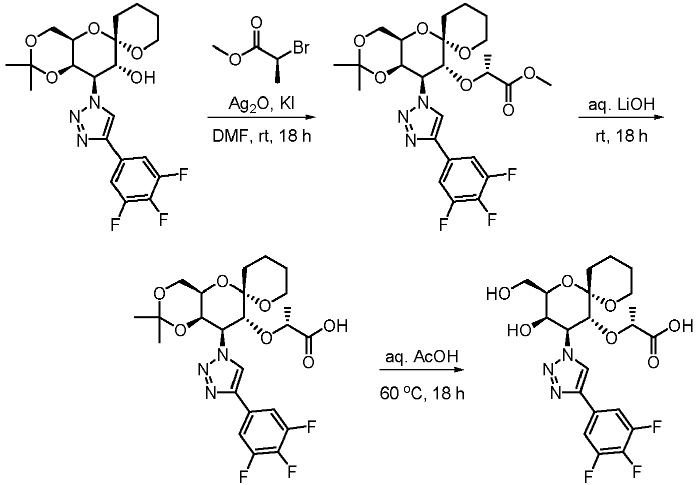

### Synthesis of (R)-methyl 2-(((2S,4a'R,7'R,8'S,8aR)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)oxy)propanoate

(2*S*,4a'*R*,7'*R*,8'*R*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-ol (20.0 mg, 0.044 mmol) was dissolved in 1.0 mL of *N*,*N*-dimethylformamide, and then silver(I) oxide (102 mg, 0.44 mmol), potassium iodide (29 mg, 0.176 mmol), and (*S*)-methyl 2-bromopropanoate (22 mg, 0.132 mmol) were sequentially added dropwise thereto, followed by stirring at room temperature for 18 hours. The reaction solution was filtered through Celite, then extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (23.8 mg).

### Synthesis of (R)-2-(((2S,4a'R,7'R,8'S,8a'R)-2',2'-dimethyl-8'-(4-(3,4,5-trifluorophenyl)-1H-1,2,3-triazol-1-yl)octahydro-4'H-spiro[pyran-2,6'-pyrano[3,2-d][1,3]dioxin]-7'-yl)oxy)propanoic acid

(*R*)-Methyl 2-(((2*S*,4a'*R*,7'*R*,8'*S*,8a'*R*)-2',2'-dimethyl-8'-(4-(3,4,5-trifluorophenyl)-1*H-*1,2,3-triazol-1-yl)octahydro-4'*H*-spiro[pyran-2,6'-pyrano[3,2-*d*][1,3]dioxin]-7'-yl)oxy)propanoate (23.8 mg, 0.044 mmol) was dissolved in 1.0 mL of distilled water and 1.0 mL of tetrahydrofuran, and then lithium hydroxide (27.7 mg, 0.666 mmol) was added dropwise thereto, followed by stirring at room temperature for 18 hours. The reaction solution was washed with ethyl acetate, the water layer was adjusted to have a pH of 5 to 6 using a 1.0 N aqueous hydrochloric acid solution, and the reaction solution was extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a white solid (22.0 mg).

Synthesis of (*R*)-2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)propanoic acid (*R*)-2-(((2S,4a'*R*,7'*R*,8'*S*,8a'*R*)-2',2'-dimethyl-8'-(4-(3,4,5-trifluorophenyl)-1*H*-1,2,3-triazol-1-yl)octahydro-4'*H*-spiro[pyran-2,6'-pyrano[3,2-*d*][1,3]dioxin]-7'-yl)oxy)propanoic acid (22.0 mg, 0.042 mmol) was dissolved in 2.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane (containing 1.0% trifluoroacetic acid) to obtain the title compound as a white solid (2.1 mg). ESIMS: m/z 488.54 [M+H]+

### Example 71: Synthesis of Compound A84

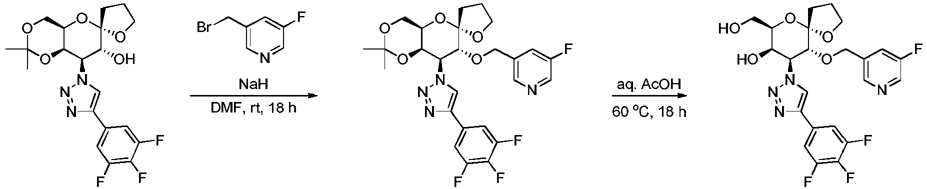

### Synthesis of 3-((((2S,4a'R,7'R,8'S,8a'R)-2',2'-dimethyl-8'-(4-(3,4,5-trifluorophenyl)-1H-1,2,3 -triazol-1 -yl)hexahydro-3H,4'H-spiro[furan-2,6'-pyrano[3,2-d][1,3] dioxin]-7'-yl)oxy)methyl)-5-fluoropyridine

(2*S*,4a'*R*,7'*R*,8'*R*,8a',*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.045 mmol) was dissolved in 1.0 mL of *N,N*-dimethylformamide, and then 60% NaH (2.7 mg, 0.068 mmol) and 3-(bromomethyl)-5-fluoropyridine (13 mg, 0.068 mmol) were slowly added thereto at 0 °C, followed by stirring for 18 hours while elevating the temperature from 0 °C to room temperature. The reaction solution was extracted by adding ethyl acetate and distilled water to recover an organic layer. The organic layer was dried over anhydrous magnesium sulfate, filtered, and then concentrated under reduced pressure. The concentrated reaction mixture was subj ected to silica gel column chromatography with 0% to 5% methanol/dichloromethane to obtain the title compound as a white solid (16 mg).

### Synthesis of (5S,7R,8R,9S,10R)-10-((5-fluoropyridin-3-yl)methoxy)-7-(hydroxymethyl)-9-(4-(3,4,5-trifluorophenyl)-1H-1,2,3-triazol-1-yl)-1,6-dioxaspiro[4.5]decan-8-ol

3-((((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)methyl)-5-fluoropyridine (16 mg, 0.029 mmol) was dissolved in 1.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (11.0 mg, yield: 74.0%).

ESIMS: m/z 511.41 [M+H]+; ¹H-NMR (400 MHz, CDCl₃) δ 8.29 (d, J = 2.4 Hz, 1H), 8.10 (s, 1H), 8.00 (s, 1H), 7.40 (dd, J = 8.0, 6.8 Hz, 2H), 7.02 (d, J = 9.0 Hz, 1H), 5.05 (dd, J = 10.7, 1.9 Hz, 1H), 4.49-4.42 (m, 3H), 4.38 (d, J = 11.6 Hz, 1H), 4.10 (t, J = 4.5 Hz, 1H), 4.07-3.91 (m, 5H), 2.19-2.08 (m, 4H), 1.99-1.94 (m, 1H).

### Example 72: Synthesis of Compound A85

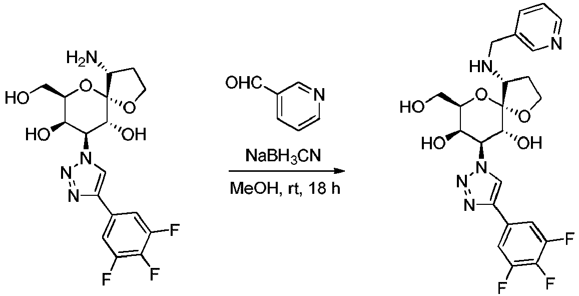

### Synthesis of (4R,5S,7R,8R,9S,10R)-7-(hydroxymethyl)-4-((pyridin-3-ylmethoxy)amino)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (7.0 mg, 0.017 mmol) was dissolved in 1.0 mL of methanol, and then nicotinaldehyde (2.7 mg, 0.025 mmol) and sodium cyanoborohydride (2.0 mg, 0.025 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to amine silica gel column chromatography with 0% to 5% methanol/dichloromethane to obtain the title compound as a white solid (7.5 mg, yield: 87%).

ESIMS: m/z 508.45 [M+H]+; ¹H-NMR (400 MHz, CD₃OD) δ 8.61 (d, J = 1.4 Hz, 1H), 8.54 (s, 1H), 8.46 (dd, J = 4.9, 1.4 Hz, 1H), 7.94 (d, J = 7.7 Hz, 1H), 7.65 (dd, J = 8.7, 6.6 Hz, 2H), 7.44 (dd, J = 7.9, 4.6 Hz, 1H), 5.04 (dd, J = 11.2, 2.7 Hz, 1H), 4.50 (d, J = 11.1 Hz, 1H), 4.15-4.05 (m, 3H), 3.98-3.90 (m, 3H), 3.78-3.73 (m, 1H), 3.73-3.67 (m, 1H), 3.60 (dd, J = 9.4, 8.4 Hz, 1H), 2.38-2.30 (m, 1H), 2.03-1.88 (m, 2H).

### Example 73: Synthesis of Compound A86

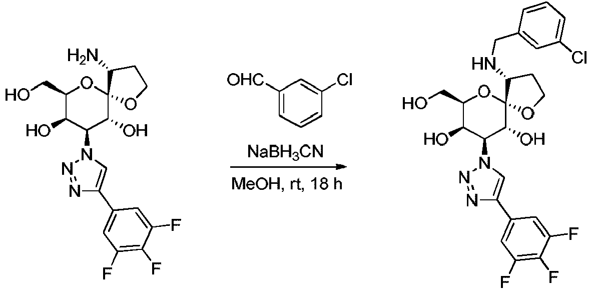

### Synthesis of (4R,5S,7R,8R,9S,10R)-4-((3-chlorobenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (7.0 mg, 0.017 mmol) was dissolved in 1.0 mL of methanol, and then 3-chlorobenzaldehyde (3.5 mg, 0.025 mmol) and sodium cyanoborohydride (2.0 mg, 0.025 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to amine silica gel column chromatography with 0% to 5% methanol/dichloromethane to obtain the title compound as a white solid (4.7 mg, yield: 51%).

ESIMS: m/z 541.4 [M+H]+; ¹H-NMR (400 MHz, CD₃OD) δ 8.53 (s, 1H), 7.64 (dd, J = 8.7, 6.8 Hz, 2H), 7.48 (s, 1H), 7.38-7.24 (m, 3H), 5.03 (dd, J = 11.1, 2.8 Hz, 1H), 4.58 (s, 1H), 4.48 (d, J = 11.1 Hz, 1H), 4.15-4.06 (m, 2H), 4.05 (d, J = 2.0 Hz, 1H), 3.96-3.87 (m, 2H), 3.77 (td, J = 9.6, 2.3 Hz, 1H), 3.72-3.67 (m, 1H), 3.58 (dd, J = 9.3, 8.4 Hz, 1H), 2.37-2.28 (m, 1H), 2.04-1.87 (m, 2H).

### Example 74: Synthesis of Compound A87

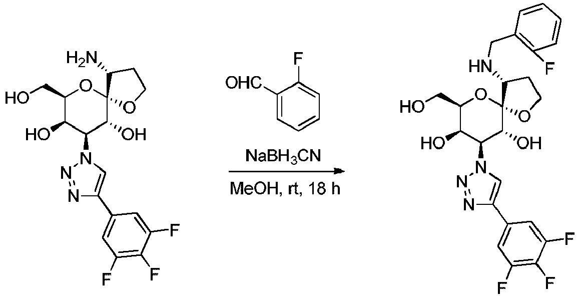

### Synthesis of (4R,5S,7R,8R,9S,10R)-4-((2-fluorobenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (7.0 mg, 0.017 mmol) was dissolved in 1.0 mL of methanol, and then 2-fluorobenzaldehyde (3.1 mg, 0.025 mmol) and sodium cyanoborohydride (2.0 mg, 0.025 mmol) were slowly added thereto at room temperature, followed by stirring at room temperature for 18 hours. The reaction solution was extracted by adding ethyl acetate and distilled water to recover an organic layer. The organic layer was dried over anhydrous magnesium sulfate, filtered, and then concentrated under reduced pressure. The concentrate was subjected to amine silica gel column chromatography with 0% to 5% methanol/dichloromethane to obtain the title compound as a white solid (5.5 mg, yield: 62%).

ESIMS: m/z 525.46 [M+H]+; ¹H-NMR (400 MHz, CD₃OD) δ 8.51 (s, 1H), 7.63 (dd, J = 8.8, 6.7 Hz, 2H), 7.48 (td, J = 7.7, 1.5 Hz, 1H), 7.36-7.28 (m, 1H), 7.18 (td, J = 7.5, 0.9 Hz, 1H), 7.14-7.07 (m, 1H), 5.02 (dd, J = 11.2, 2.9 Hz, 1H), 4.46 (d, J = 11.2 Hz, 1H), 4.15-4.06 (m, 2H), 4.03 (d, J = 1.8 Hz, 1H), 3.99-3.89 (m, 3H), 3.79-3.72 (m, 1H), 3.71-3.65 (m, 1H), 3.65-3.57 (m, 1H), 2.40-2.29 (m, 1H), 2.05-1.84 (m, 2H).

### Example 75: Synthesis of Compounds A88 and A89

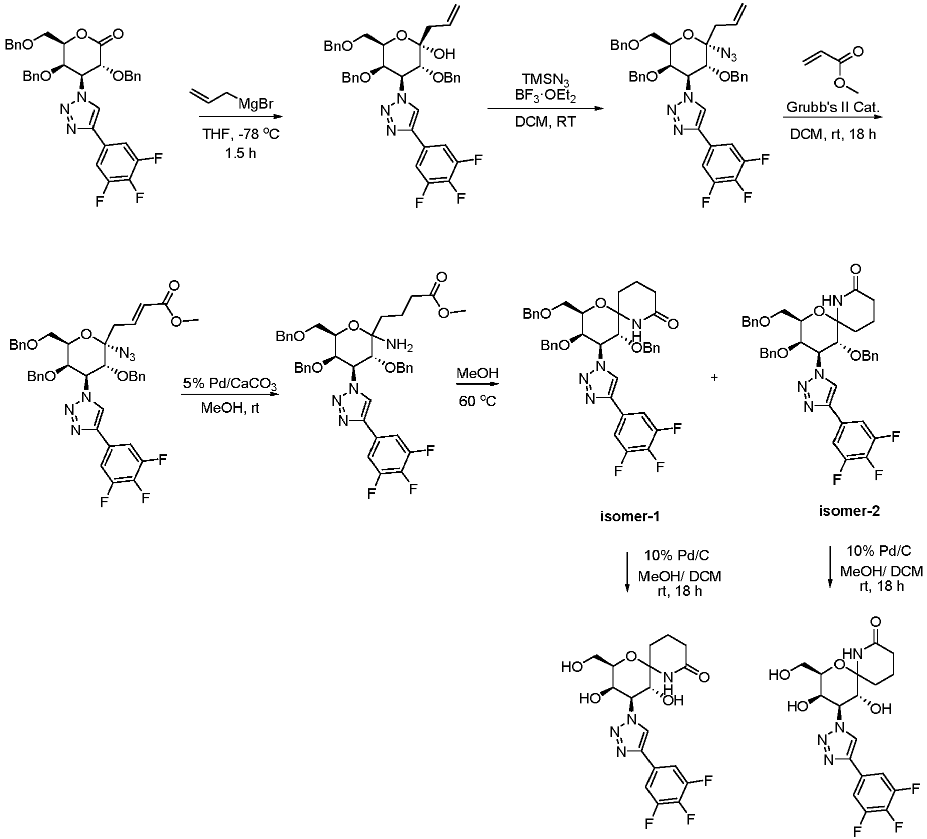

### Synthesis of (2S,3R,4S,5R,6R)-2-allyl-3,5-bis(benzyloxy)-6-((benzyloxy)methyl)-4-(4-(3,4,5-trifluorophenyl)-1H-1,2,3-triazol-1-yl)tetrahydro-2H-pyran-2-ol

(3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-one (1.98 g, 3.15 mmol) was dissolved in 31 mLof tetrahydrofuran, and then 1.0 M allylmagnesium bromide (1.0 M ether solution) (6.29 mL, 6.290 mmol) was added dropwise thereto at -78 °C, followed by stirring at -78 °C for 1.5 hours. Next, a saturated aqueous sodium hydrogen carbonate solution (10 mL) was added to terminate the reaction, followed by stirring at room temperature for 30 minutes. The reaction mixture was diluted with 150 mL of ethyl acetate, and then washed three times with 20 mL of a saturated aqueous sodium hydrogen carbonate solution. The organic layer was recovered, dried over anhydrous sodium sulfate, filtered, and then concentrated under reduced pressure. The concentrate was subjected to silica gel column chromatography with 25% ethyl acetate/n-hexane to obtain the title compound as a colorless oil (2.02 g, yield: 96%).

¹H-NMR (400 MHz, CDCl₃) δ 7.66 (s, 1H), 7.35-7.27 (m, 8H), 7.18-7.07 (m, 8H), 6.93-6.89 (m, 2H), 6.01-5.84 (m, 1H), 5.31 (dd, J = 11.0, 3.0 Hz, 2H), 5.25 (d, J = 17.3 Hz, 1H), 4.58-4.48 (m, 4H), 4.46-4.38 (m, 2H), 4.23-4.12 (m, 2H), 4.10 (d, J = 3.1 Hz, 1H), 3.93 (d, J = 11.1 Hz, 1H), 3.74 (t, J = 9.0 Hz, 1H), 3.64 (dd, J = 9.1, 5.5 Hz, 1H), 2.95 (s, 1H), 2.63 (d, J = 7.2 Hz, 2H), 2.04 (s, 1H), 1.26 (t, J = 7.1 Hz, 1H).

### Synthesis of 1-((2S,3R,4S,5R,6R)-2-allyl-2-azido-3,5-bis(benzyloxy)-6-((benzyloxy)methyl)tetrahydro-2H-pyran-4-yl)-4-(3,4,5-trifluorophenyl)-1H-1,2,3-triazole

(2*S*,3*R*,4*S*,5*R*,6*R*)-2-allyl-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ol (1.68 g, 2.50 mmol) was dissolved in 50 mL of dichloromethane, and then azidotrimethylsilane (1.64 mL, 12.5 mmol) was added dropwise thereto, BF₃·OEt₂ (0.3 mL, 2.50 mmol) was slowly added dropwise thereto at room temperature, followed by stirring at room temperature for 19 hours. A saturated aqueous sodium hydrogen carbonate solution (10 mL) was added to terminate the reaction, and then extracted with dichloromethane, dried over anhydrous sodium sulfate, filtered, and then concentrated. The concentrated reaction mixture was subjected to silica gel column chromatography with 15% ethyl acetate/n-hexane to obtain the title compound as a colorless oil (1.25 g, yield: 72%).

¹H-NMR (400 MHz, CDCl₃) δ 7.63 (s, 1H), 7.26-7.35 (m, 8H), 7.07-7.17 (m, 7H), 6.93 (d, J = 7.0 Hz, 2H), 5.96-6.06 (m, 1H), 5.27-5.34 (m, 2H), 5.20 (dd, J = 11.0, 2.7 Hz, 1H), 4.45-4.60 (m, 4H), 4.31 (d, J = 11.0 Hz, 2H), 4.08-4.10 (m, 2H), 3.96 (d, J = 11.3 Hz, 1H), 3.65-3.76 (m, 2H), 2.78-2.94 (m, 2H)

### Synthesis of (E)-methyl 4-((2S,3R,4S,5R,6R)-2-azido-3,5-bis(benzyloxy)-6-((benzyloxy)methyl)-4-(4-(3,4,5-trifluorophenyl)-1H-1,2,3-triazol-1-yl)tetrahydro-2H-pyran-2-yl)but-2-enoate

1-((2*S*,3*R*,4*S*,5*R*,6*R*)-2-allyl-2-azido-3,5-bis(benzyloxy)-6-((benzyloxy)methyl)tetrahydro-2*H*-pyran-4-yl)-4-(3,4,5-trifluorophenyl)-1*H*-1,2,3-triazole (120.0 mg, 0.172 mmol) was dissolved in 50.0 mL of dichloromethane, and then Grubbs catalyst II (8.8 mg, 0.010 mmol) and methyl acrylate (44.5 mg, 0.517 mmol) were sequentially added dropwise thereto, and the mixture was stirred at room temperature for 18 hours while light is blocked. The reaction solution was filtered through Celite, then extracted with ethyl acetate, dried over anhydrous magnesium sulfate, filtered, and then concentrated under reduced pressure. The concentrate was subjected to silica gel column chromatography with 0% to 50% ethyl acetate/n-hexane to obtain the title compound as a colorless oil (55 mg, yield: 43%).

¹H-NMR (400 MHz, CDCl₃) δ 7.68 (s, 1H), 7.41-7.27 (m, 8H), 7.23-7.01 (m, 8H), 6.93 (d, J = 6.6 Hz, 2H), 6.01 (d, J = 15.6 Hz, 1H), 5.20 (dd, J = 10.8, 2.7 Hz, 1H), 4.62-4.46 (m, 3H), 4.30 (dd, J = 14.1, 11.0 Hz, 3H), 4.14-4.06 (m, 2H), 4.00 (d, J = 11.3 Hz, 1H), 3.78 (s, 3H), 3.77-3.71 (m, 1H), 3.70-3.64 (m, 1H), 3.04-2.94 (m, 1H), 2.94-2.83 (m, 1H).

### Synthesis of methyl 4-((3R,4S,5R,6R)-2-amino-3,5-bis(benzyloxy)-6-((benzyloxy)methyl)-4-(4-(3,4,5-trifluorophenyl)-1H-1,2,3-triazol-1-yl)tetrahydro-2H-pyran-2-yl)butanoate

(*E*)-methyl 4-((2*S*,3*R*,4*S*,5*R*,6*R*)-2-azido-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-yl)but-2-enoate (50 mg, 0.066 mmol) was dissolved in 5.0 mL of methanol, and the mixture was added dropwise to a round-bottom flask, in which 5% Pd/CaCO₃ (25.0 mg, 0.024 2.5 wt%) was dissolved in 10 mL of a solution of dichloromethane:methanol (1:2), and then degassed with a hydrogen balloon for 10 minutes. The reaction solution was stirred at room temperature in a hydrogen atmosphere for 18 hours. The reaction solution was filtered through Celite and then concentrated and subjected to silica gel column chromatography with 0% to 50% ethyl acetate/n-hexane to obtain the title compound as a white solid (42.0 mg, yield: 88.0%).

¹H-NMR (400 MHz, CDCl₃) δ 7.74 (s, 1H), 7.36-7.26 (m, 7H), 7.24-7.21 (m, 1H), 7.20-7.00 (m, 7H), 6.93-6.85 (m, 2H), 5.29 (dd, J = 10.7, 3.1 Hz, 1H), 4.65-4.58 (m, 1H), 4.57 (s, 1H), 4.53 (d, J = 7.1 Hz, 2H), 4.39 (d, J = 10.9 Hz,1H), 4.10 (d, J = 10.7 Hz, 1H), 4.04 (d, J = 2.1 Hz, 1H), 4.00 (d, J = 10.9 Hz, 1H), 3.95 (d, J = 11.2 Hz, 1H), 3.86-3.76 (m, 1H), 3.75-3.69 (m, 1H), 3.67 (s, 3H), 3.59 (dd, J = 9.0, 5.5 Hz, 1H), 2.45-2.33 (m, 2H), 1.91-1.80 (m, 3H), 1.75-1.67 (m, 1H).

### Synthesis of (2R,3R,4S,5R)-3,5-bis(benzyloxy)-2-((benzyloxy)methyl)-4-(4-(3,4,5-trifluorophenyl)-1H-1,2,3-triazol-1-yl)-1-oxa-7-azaspiro[5.5]undecan-8-one

Methyl 4-((3*R*,4*S*,5*R*,6*R*)-2-amino-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-yl)butanoate (42.0 mg, 0.057 mmol) was dissolved in 50.0 mL of dichloromethane, followed by stirring at 60 °C for 5 days. The reaction mixture was concentrated, and then subjected to silica gel column chromatography with 0% to 5% methanol/dichloromethane to obtain the title compound as a white solid (isomer-1: 5.5 mg, yield: 13.8%; isomer-2: 4.6 mg, yield: 11.6%).

Isomer-1: ¹H-NMR (400 MHz, CDCl₃) δ 7.72 (s, 1H), 7.38-7.24 (m, 9H), 7.24-7.16 (m, 2H), 7.16-7.02 (m, 4H), 6.92-6.79 (m, 2H), 5.21 (dd, J = 11.3, 2.9 Hz, 1H), 4.58-4.44 (m, 3H), 4.40 (d, J = 11.6 Hz, 1H), 4.23 (d, J = 11.4 Hz, 1H), 4.17-4.08 (m, 3H), 3.93 (d, J = 10.9 Hz, 1H), 3.71 (t, J = 8.8 Hz, 1H), 3.59 (dd, J = 8.9, 5.3 Hz, 1H), 2.57-2.46 (m, 1H), 2.39-2.28 (m, 1H), 2.22-2.08 (m, 1H), 2.02-1.93 (m, 2H), 1.83-1.70 (m, 1H).

Isomer-2: ¹H-NMR (400 MHz, CDCl₃) δ 7.49 (s, 1H), 7.37-7.24 (m, 9H), 7.18-7.04 (m, 6H), 6.85-6.79 (m, 1H), 6.17 (s, 1H), 5.00 (dd, J = 11.3, 3.0 Hz, 1H), 4.59 (d, J = 11.4 Hz, 1H), 4.51 (t, J = 11.0 Hz, 3H), 4.26 (d, J = 11.3 Hz, 1H), 4.05 (d, J = 2.5 Hz, 1H), 3.98-3.90 (m, 2H), 3.88 (d, J = 11.0 Hz, 1H), 3.73 (t, J = 8.7 Hz, 1H), 3.67-3.61 (m, 1H), 2.56-2.46 (m, 1H), 2.38-2.26 (m, 1H), 2.21-2.06 (m, 2H), 2.00-1.91 (m, 1H), 1.90-1.80 (m, 1H).

### Synthesis of (2R,3R,4S,5R)-3,5-dihydroxy-2-(hydroxymethyl)-4-(4-(3,4,5-trifluorophenyl)-1H-1,2,3-triazol-1-yl)-1-oxa-7-azaspiro[5.5]undecan-8-one (isomer-1)

(2*R*,3*R*,4*S*,5*R*)-3, 5-bis(benzyloxy)-2-((benzyloxy)methyl)-4-(4-(3,4, 5-trifluorophenyl)-1*H*-1,2,3-triazol-1-yl)-1-oxa-7-azaspiro[5.5]undecan-8-one (isomer-1, 5.5 mg, 0.008 mmol) was dissolved in 2.0 mL of a solution of dichloromethane:methanol (1:1), and the mixture was added dropwise to a round-bottom flask, in which 10% Pd/C (25.0 mg, 4.5 wt%) was dissolved in 2.0 mL of a solution of dichloromethane:methanol (1:1), and then degassed with a hydrogen balloon for 10 minutes. The reaction solution was stirred at room temperature in a hydrogen atmosphere for 18 hours. The reaction solution was filtered through Celite, concentrated, and subjected to silica gel column chromatography with 50% ethyl acetate/n-hexane to obtain the title isomer-1 as a white solid (3.4 mg, yield: 99.0%).

ESIMS: m/z 429.44 [M+H]+; ¹H-NMR (400 MHz, CD₃OD) δ 8.55-8.47 (m, 1H), 7.67-7.58 (m, 2H), 5.00 (dd, J = 11.4, 2.6 Hz, 1H), 4.36 (d, J = 11.5 Hz, 1H), 4.06 (d, J = 2.6 Hz, 1H), 3.87 (t, J = 5.6 Hz, 1H), 3.78-3.71 (m, 1H), 3.71-3.65 (m, 1H), 2.44-2.24 (m, 3H), 2.09-1.97 (m, 2H), 1.87-1.81 (m, 1H).

### Synthesis of (2R,3R,4S,5R)-3,5-dihydroxy-2-(hydroxymethyl)-4-(4-(3,4,5-trifluorophenyl)-1H-1,2,3-triazol-1-yl)-1-oxa-7-azaspiro[5.5]undecan-8-one (isomer-2)

(2*R*,3*R*,4*S*,5*R*)-3,5-bis(benzyloxy)-2-((benzyloxy)methyl)-4-(4-(3,4,5-trifluorophenyl)-1*H*-1,2,3-triazol-1-yl)-1-oxa-7-azaspiro[5.5]undecan-8-one (isomer-2, 4.6 mg, 0.007 mmol) was dissolved in 2.0 mL of a solution of dichloromethane:methanol (1:1), and the mixture was added dropwise to a round-bottom flask, in which 10% Pd/C (25.0 mg, 4.5 wt%) was dissolved in 2.0 mL of a solution of dichloromethane:methanol (1:1), and then degassed with a hydrogen balloon for 10 minutes. The reaction solution was stirred at room temperature in a hydrogen atmosphere for 18 hours. The reaction solution was filtered through Celite, concentrated, and subjected to silica gel column chromatography with 50% ethyl acetate/n-hexane to obtain the title isomer-2 as a white solid (3.6 mg, yield: 99.0%).

ESIMS: m/z 429.41 [M+H]+; ¹H-NMR (400 MHz, CD₃OD) δ 8.50 (s, 1H), 7.64 (dd, J = 8.6, 6.8 Hz, 2H), 5.30 (dd, J = 11.7, 2.8 Hz, 1H), 4.37 (d, J = 11.4 Hz, 1H), 4.11 (d, J = 2.8 Hz, 1H), 3.93 (t, J = 6.0 Hz, 1H), 3.69 (d, J = 6.0 Hz, 2H), 2.48-2.39 (m, 1H), 2.38-2.30 (m, 1H), 2.21-2.11 (m, 2H), 2.00-1.92 (m, 1H), 1.83-1.74 (m, 1H).

### Example 76: Synthesis of Compound A90

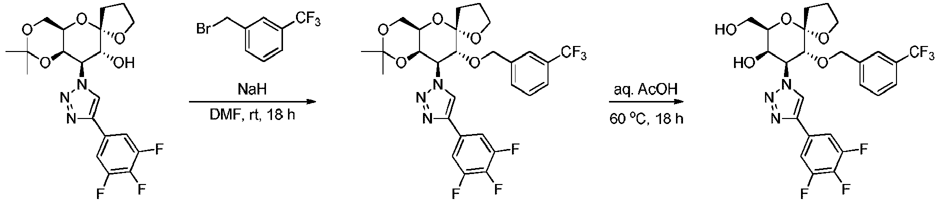

### Synthesis of 1-((2S,4a'R,7'R,8'S,8a'R)-2',2'-dimethyl-7'-((3-trifluoromethyl)benzyl)oxy)hexahydro-3H,4'H-spiro[furan-2,6'-pyrano[3,2-d][1,3]dioxin]-8'-yl)-4-(3,4,5-trifluorophenyl)-1H-1,2,3-triazole

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20.0 mg, 0.045 mmol) was dissolved in 1.0 mL of *N*,*N*-dimethylformamide, and then 60% NaH (2.7 mg, 0.068 mmol) and 1-(bromomethyl)-3-(trifluoromethyl)benzene (16.3 mg, 0.068 mmol) were slowly added thereto at 0 °C, followed by stirring for 18 hours while elevating the temperature from 0 °C to room temperature. The reaction solution was extracted by adding ethyl acetate and distilled water to recover an organic layer. The organic layer was dried over anhydrous magnesium sulfate, filtered, and then concentrated under reduced pressure. The concentrate was subjected to silica gel column chromatography with 3.0% methanol/dichloromethane to obtain the title compound as a white solid (21.5 mg).

### Synthesis of (5S,7R,8R,9S10R)-7-(hydroxymethyl)-10-((3-(trifluoromethyl)benzyl)oxy)-9-(4-(3,4,5-trifluorophenyl)-1H-1,2,3-triazol-1-yl)-1,6-dioxaspiro[4.5]decan-8-ol

1-((2*S*,4a'*R*,7'*R*,8'*S*,8a'*R*)-2',2'-dimethyl-7'-((3-trifluoromethyl)b enzyl)oxy)hexahydro-3*H*,4'*H*-spiro[furan-2,6'-pyrano[3,2-*d*][1,3]dioxin]-8'-yl)-4-(3,4,5-trifluorophenyl)-1*H*-1,2,3-triazole (16 mg, 0.029 mmol) was dissolved in 1.0 mL of a 50% aqueous acetic acid solution, followed by stirring at 60 °C for 18 hours. The reaction mixture was concentrated, then neutralized with a saturated aqueous ammonium chloride solution, and extracted with dichloromethane to recover an organic layer. The organic layer was dried over anhydrous magnesium sulfate, filtered, and then concentrated under reduced pressure. The concentrate was subjected to silica gel column chromatography with 0% to 5% methanol/dichloromethane to obtain the title compound as a white solid (17.0 mg, yield: 76.0%).

ESIMS: m/z 560.36 [M+H]+; ¹H-NMR (400 MHz,CDCl₃) δ 7.94 (s, 1H), 7.44 (d, J = 8.0 Hz, 1H), 7.39-7.26 (m, 4H), 7.14 (d, J = 7.4 Hz, 1H), 5.01 (dd, J = 10.7, 2.3 Hz, 1H), 4.52 (t, J = 1.2 Hz, 1H), 4.50 (dt, J = 2.3, 1.3 Hz, 1H), 4.44 (d, J = 10.7 Hz, 1H), 4.37 (d, J = 11.4 Hz, 1H), 4.14-3.85 (m, 6H), 2.21-2.09 (m, 4H), 1.97 (d, J = 7.9 Hz, 1H).

### Example 77: Synthesis of Compound A91

Synthesis of 1-((2S,4a'*R*,7'*R*,8'*S*,8a'*R*)-7'-((3-fluorobenzyl)oxy)-2',2'-dimethylhexahydro-3*H*,4'*H*-spiro[furan-2,6'-pyrano[3,2-*d*][1,3]dioxin]-8'-yl)-4-(3,4,5-trifluorophenyl)-1*H*-1,2,3-triazole

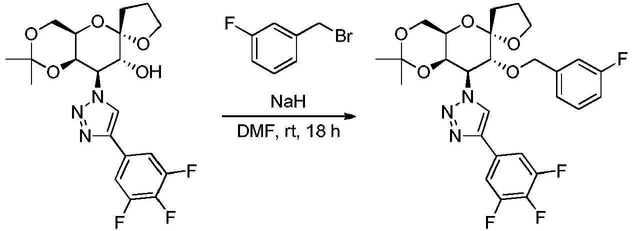

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (20 mg, 0.045 mmol) was dissolved in 1.0 mL of *N*,*N*-dimethylformamide, and then 60% sodium hydride (60% dispersion in mineral oil) (2.7 mg, 0.068 mmol) and benzyl bromide (12.9 mg, 0.068 mmol) were slowly added thereto at 0 °C, followed by stirring for 18 hours while elevating the temperature from 0 °C to room temperature. The reaction solution was extracted with ethyl acetate, dried over anhydrous magnesium sulfate, filtered, and then concentrated. The concentrated reaction mixture was subjected to silica gel column chromatography with 0% to 5% methanol/dichloromethane to obtain the title compound (24.0 mg, yield: 97.0%).

¹H-NMR (400 MHz, CDCl₃) δ 7.94 (s, 1H), 7.46-7.35 (m, 2H), 7.14-7.09 (m, 1H), 6.83-6.77 (m, 1H), 6.71 (d, J = 7.1 Hz, 1H), 6.67 (d, J = 9.5 Hz, 1H), 5.51-5.43 (m, 1H), 4.39-4.33 (m, 2H), 4.31 (d, J = 10.9 Hz, 1H), 4.13-3.97 (m, 4H), 3.91 (d, J = 13.1 Hz, 1H), 3.86 (s, 1H), 2.22-2.12 (m, 2H), 2.00-1.87 (m, 2H), 1.53 (s, 3H), 1.34 (s, 3H).

### Synthesis of (5S,7R,8R,9S,10R)-10-((3-fluorobenzyl)oxy)-7-(hydroxymethyl)-9-(4-(3,4,5-trifluorophenyl)-1H-1,2,3-triazol-1-yl)-1,6-dioxaspiro[4.5]decan-8-ol

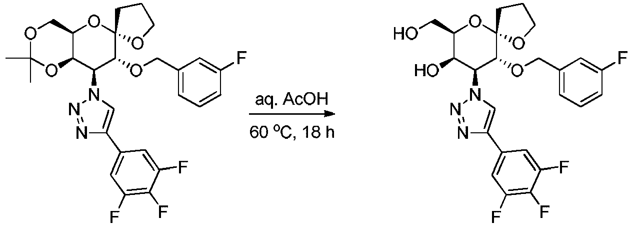

1-((2*S*,4a'*R*,7'*R*,8'*S*,8a'*R*)-7'-((3-fluorobenzyl)oxy)-2',2'-dimethylhexahydro-3*H*,4'*H-*spiro[furan-2,6'-pyrano[3,2-*d*][1,3]dioxin]-8'-yl)-4-(3,4,5-trifluorophenyl)-1*H*-1,2,3-triazole was dissolved in 1.0 mL of 50% AcOH in purified water, followed by stirring at 60 °C for 18 hours. The reaction solution was extracted with sat. NH₄Cl, then extracted with dichloromethane, dried over anhydrous magnesium sulfate, filtered, and then concentrated. The concentrated reaction mixture was subjected to silica gel column chromatography with 0% to 5% methanol/dichloromethane to obtain the title compound (13 mg, yield: 57%).

ESIMS: m/z 510.47 [M+H]+; ¹H-NMR (400 MHz, CDCl₃) δ 7.93-7.91 (m, 1H), 7.38-7.33 (m, 2H), 7.19-7.12 (m, 1H), 6.91-6.84 (m, 1H), 6.77-6.69 (m, 2H), 4.99-4.94 (m, 1H), 4.59-4.56 (m, 1H), 4.51-4.48 (m, 1H), 4.42-4.38 (m, 1H), 4.30-4.25 (m, 1H), 4.12-3.85 (m, 5H), 2.23-2.18 (m, 1H), 2.11-2.07 (m, 2H), 2.00-1.94 (m, 1H).

### Example 78: Synthesis of Compound A92

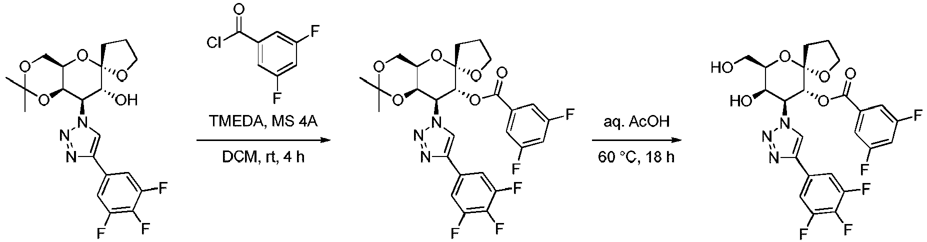

### Synthesis of (2S,4a'R,7'R,8'S,8a'R)-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl 3,5-difluorobenzoate

The crude title compound was obtained by the synthesis from (2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H-*spiro[furan-2,6'-pyrano[3,2-*d*][1,3]dioxin]-7'-ol in a similar manner to the preparation method of synthesis of (2S,4a'R,7'R,8'S,8a'R)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl 2-fluorobenzoate.

### Synthesis of (5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl 3,5-difluorobenzoate

The title compound as a white solid (16.4 mg, yield: 87%) was synthesized from crude (2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl 3,5-difluorobenzoate (about 0.035 mmol) in a similar manner to the preparation method of (5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl 2-fluorobenzoate.

ESIMS: m/z 542.39 [M+H]+; ¹H-NMR (400 MHz, CD₃OD) δ = 8.58 (S, 1H), 7.55-7.51 (m, 2H), 7.37 (d, J = 5.5 Hz, 2H), 7.20 (t, J = 8.8 Hz, 1H), 6.12 (d, J = 11.3 Hz, 1H), 5.45 (dd, J = 11.3, 2.7 Hz, 1H), 4.25 (d, J = 2.0 Hz, 1H), 4.20 (t, J = 6.3 Hz, 1H), 4.11-4.02 (m, 2H), 3.76 (d, J = 6.3 Hz, 2H), 2.17-2.07 (m, 2H), 1.97-1.92 (m, 1H), 1.88-1.80 (m, 1H)

### Example 79: Synthesis of Compound A93

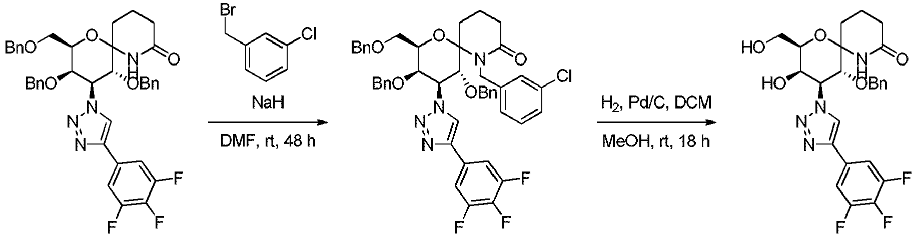

### Synthesis of (2R,3R,4S,5R)-3,5-bis(benzyloxy)-2-((benzyloxy)methyl)-7-(3-chlorobenzyl)-4-(4-(3,4,5-trifluorophenyl)-1H-1,2,3-triazol-1-yl)-1-oxa-7-azaspiro[5.5]undecan-8-one

(2*R*,3*R*,4*S*,5*R*)-3,5-bis(benzyloxy)-2-((benzyloxy)methyl)-4-(4-(3,4,5-trifluorophenyl)-1*H*-1,2,3-triazol-1-yl)-1-oxa-7-azaspiro[5.5]undecan-8-one (isomer-2) (18 mg, 0.026 mmol) was dissolved in 1 mL of *N*,*N*-dimethylformamide, and then cooled to 0 °C. Next, sodium hydride (60% dispersion in mineral oil, 2.06 mg, 0.051 mmol) was added thereto, followed by stirring at 0 °C for 1 hour. Then, 1-(bromomethyl)-3-chlorobenzene (7.94 mg, 0.039 mmol) was added thereto, followed by stirring at room temperature for 48 hours. The reaction solution was diluted with 10 mL of ethyl acetate. This was washed with a saturated aqueous ammonium chloride solution (3 mL × 1 time), water (3 mL × 1 time), and a saturated aqueous sodium chloride solution (3 mL x 1 time). The organic layer was dried over anhydrous sodium sulfate, filtered, and concentrated under reduced pressure. The remaining concentrate was subjected to silica gel column chromatography with 0% to 33% ethyl acetate/n-hexane to obtain the title compound as a colorless oil (17.0 mg, yield: 80%).

¹H-NMR (400 MHz, CDCl₃) δ = 7.59 (S, 1H), 7.36-7.07 (m, 15H), 6.66 (S, 2H), 4.96-4.92 (m, 2H), 4.68-4.44 (m, 5H), 4.11-3.99 (m, 3H), 3.83-3.68 (m, 3H), 3.31-3.28 (m, 1H), 2.55-2.51 (m, 1H), 2.42-2.35 (m, 2H), 2.09-2.06 (m, 1H), 1.84-1.84 (m, 2H)

### Synthesis of (2R,3R,4S,5R)-5-(benzyloxy)-3-hydroxy-2-(hydroxymethyl)-4-(4-(3,4,5-trifluorophenyl)-1H-1,2,3-triazol-1-yl)-1-oxa-7-azaspiro[5.5]undecan-8-one

(2*R*,3*R*,4*S*,5*R*)-3,5-bis(benzyloxy)-2-((benzyloxy)methyl)-7-(3-chlorobenzyl)-4-(4-(3,4,5-trifluorophenyl)-1*H*-1,2,3-triazol-1-yl)-1-oxa-7-azaspiro[5.5]undecan-8-one (17.0 mg, 0.021 mmol) was dissolved in 1 mL of a solution of methanol: dichloromethane (2:1 v/v), and then 10% palladium/carbon (12 mg, 0.011 mmol) was added thereto. A hydrogen balloon was added thereto, and the mixture was degassed for 30 minutes and then stirred in a hydrogen atmosphere for 18 hours. After the completion of the reaction was confirmed by thin layer chromatography, the reaction mixture was filtered through a pad of Celite 545. The filtrate was concentrated under reduced pressure and the remaining concentrate was subjected to column chromatography with 0% to 10% methanol/dichloromethane to synthesize the title compound as a white solid (3.8 mg, yield: 42%).

ESIMS: m/z 519.39 [M+H]+; ¹H-NMR (400 MHz, CD₃OD) δ = 8.36 (S, 1H), 7.61-7.57 (m, 2H), 7.42-7.40 (m, 2H), 7.29-7.25 (m, 2H), 7.20-7.17 (m, 1H), 4.99 (dd, J = 11.5, 2.5 Hz, 1H), 4.74 (d, J = 3.5 Hz, 1H), 4.12-4.06 (m, 2H), 3.99-3.95 (m, 1H), 3.74-3.73 (m, 2H), 3.64-3.62 (m, 1H), 2.03-1.98 (m, 2H), 1.62-1.49 (m, 4H)

### Example 80: Synthesis of Compound A94

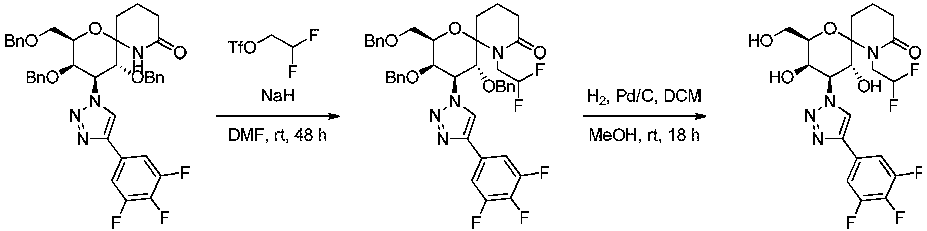

### Synthesis of (2R,3R,4S,5R)-3,5-bis(benzyloxy)-2-((benzyloxy)methyl)-7-(2,2-difluoroethyl)-4-(4-(3,4,5-trifluorophenyl)-1H-1,2,3-triazol-1-yl)-1-oxa-7-azaspiro[5.5]undecan-8-one

(2*R*,3*R*,4*S*,5*R*)-3,5-bis(benzyloxy)-2-((benzyloxy)methyl)-4-(4-(3,4,5-trifluorophenyl)-1*H*-1,2,3-triazol-1-yl)-1-oxa-7-azaspiro[5.5]undecan-8-one (isomer-2) (15.0 mg, 0.022 mmol) was dissolved in 1 mL of *N*,*N*-dimethylformamide, and then cooled to 0 °C. In addition, sodium hydride (60% dispersion in mineral oil, 1.29 mg, 0.032 mmol) was added thereto, followed by stirring at 0 °C for 30 minutes. Next, 2,2-difluoroethyl trifluoromethanesulfonate (5.6 µL, 0.043 mmol) was added thereto, and the reaction mixture was stirred at 0 °C for 48 hours. The reaction mixture was diluted with 10 mL of ethyl acetate. This was washed with a saturated aqueous ammonium chloride solution (3 mL x 1 time), water (3 mL x 1 time), and a saturated aqueous sodium chloride solution (3 mL x 1 time). The organic layer was dried over anhydrous sodium sulfate, filtered, and concentrated under reduced pressure. The remaining concentrate was subjected to silica gel column chromatography with 0% to 35% ethyl acetate/n-hexane to obtain the title compound as a clear liquid (11.0 mg, yield: 67%).

¹H-NMR (400 MHz, CDCl₃) δ = 7.67 (S, 1H), 7.35-7.27 (m, 7H), 7.19-7.11 (m, 7H), 6.80-6.78 (m, 2H), 6.30 (d, J = 57.1 Hz, 1H), 4.97 (dd, J = 11.0, 2.3 Hz, 1H), 4.65 (d, J = 11.3 Hz, 1H), 4.52 (dd, J = 14.3, 11.9 Hz, 2H), 4.39 (d, J = 11.0 Hz, 1H), 4.28 (d, J = 11.0 Hz, 1H), 4.15-4.06 (m, 3H), 4.04-4.01 (m, 1H), 3.87 (d, J = 11.0 Hz, 1H), 3.77-3.64 (m, 3H), 2.55-2.49 (m, 2H), 2.38-2.31 (m, 1H), 2.09-2.00 (m, 1H), 1.90-1.89 (m, 1H), 1.82-1.77 (m, 1H)

### Synthesis of (2R,3R,4S,5R)-7-(2,2-difluoroethyl)-3.5-dihydroxy-2-(hydroxymethyl)-4-(4-(3,4,5-trifluorophenyl)-1H-1,2,3-triazol-1-yl)-1-oxa-7-azaspiro[5.5]undecan-8-one

(2*R*,3*R*,4*S*,5*R*)-3,5-bis(benzyloxy)-2-((benzyloxy)methyl)-7-(2,2-difluoroethyl)-4-(4-(3,4,5-trifluorophenyl)-1*H*-1,2,3-triazol-1-yl)-1-oxa-7-azaspiro[5.5]undecan-8-one (11.0 mg, 0.014 mmol) was dissolved in 1 mL of a solution of methanol: dichloromethane (2:1 v/v), and then 10% palladium/carbon (15 mg, 0.014 mmol) was added thereto. A hydrogen balloon was added thereto, and the mixture was degassed for 30 minutes and then stirred in a hydrogen atmosphere for 18 hours. After the completion of the reaction was confirmed by thin layer chromatography, the reaction mixture was filtered through a pad of Celite 545. The filtrate was concentrated under reduced pressure and the remaining concentrate was subjected to column chromatography with 0% to 10% methanol/dichloromethane to synthesize the title compound as a white solid (3.4 mg, yield: 49%).

ESIMS: m/z 493.37 [M+H]+; ¹H-NMR (400 MHz, CD₃OD) δ = 8.55 (S, 1H), 7.67-7.63 (m, 2H), 6.29-5.99 (m, 1H), 5.03 (dd, J = 11.3, 2.3 Hz, 1H), 4.72 (d, J = 11.3 Hz, 1H), 4.20-4.12 (m, 1H), 4.09 (d, J = 1.6 Hz, 1H), 3.93 (t, J = 5.7 Hz, 1H), 3.84-3.68 (m, 3H), 2.51-2.29 (m, 4H), 1.92-1.90 (m, 2H)

### Example 81: Synthesis of Compound A95

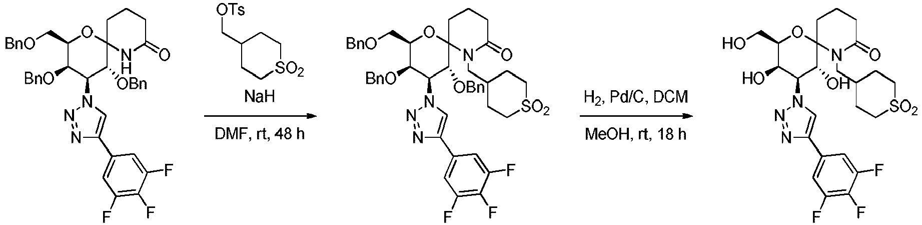

### Synthesis of (2R,3R,4S,5R)-3,5-bis(benzyloxy)-2-((benzyloxy)methyl)-7-((1,1-dioxidotetrahydro-2H-thiopyran-4-yl)-4-(4-(3,4,5-trifluorophenyl)-1H-1,2,3-triazol-1-yl)-1-oxa-7-azaspiro[5.5]undecan-8-one

(2*R*,3*R*,4*S*,5*R*)-3,5-bis(benzyloxy)-2-((benzyloxy)methyl)-4-(4-(3,4,5-trifluorophenyl)-1*H*-1,2,3-triazol-1-yl)-1-oxa-7-azaspiro[5.5]undecan-8-one (isomer-2) (16.0 mg, 0.023 mmol) was dissolved in 1 mL of *N,N*-dimethylformamide, and then cooled to 0 °C. In addition, sodium hydride (60% dispersion in mineral oil, 1.38 mg, 0.035 mmol) was added thereto, and then the reaction mixture was stirred at 0 °C for 30 minutes. Next, (1,1-dioxidotetrahydro-2*H-*thiopyran-4-yl)methyl 4-methylbenzenesulfonate (21.9 mg, 0.069 mmol) was added thereto, and the reaction mixture was stirred at room temperature for 48 hours.

The reaction mixture was diluted with 10 mL of ethyl acetate. This was washed with a saturated aqueous ammonium chloride solution (3 mL × 1 time), water (3 mL x 1 time), and a saturated aqueous sodium chloride solution (3 mL × 1 time). The organic layer was dried over anhydrous sodium sulfate, filtered, and concentrated under reduced pressure. The remaining concentrate was subjected to silica gel column chromatography with 0% to 80% ethyl acetate/n-hexane to obtain the title compound as a clear liquid (7.8 mg, yield: 40%).

¹H-NMR (400 MHz, CDCl₃) δ = 7.67 (S, 1H), 7.38-7.28 (m, 8H), 7.23-7.09 (m, 7H), 6.78-6.76 (m, 2H), 4.90 (dd, J = 11.3, 2.0 Hz, 1H), 4.57-4.54 (m, 3H), 4.33 (d, J = 11.3 Hz, 1H), 4.26 (d, J = 10.2 Hz, 1H), 4.11-4.05 (m, 2H), 3.95 (d, J = 10.6 Hz, 1H), 3.79 (t, J = 8.6 Hz, 1H), 3.70-3.64 (m, 2H), 3.15-2.99 (m, 5H), 2.87-2.80 (m, 1H), 2.52-2.47 (m, 1H), 2.42-2.35 (m, 1H), 2.29-2.26 (m, 2H), 2.16-2.04 (m, 3H), 1.94-1.68 (m, 4H)

### Synthesis of (2R,3R,4S,5R)-7-((1,1-dioxidotetrahydro-2H-thiopyran-4-yl)methyl)-3,5-dihydroxy-2-(hydroxymethyl)-4-(4-(3,4,5-trifluorophenyl)-1H-1,2,3-triazol-1-yl)-1-oxa-7-azaspiro[5.5]undecan-8-one

(2*R*,3*R*,4*R*,5*S*)-3,5-bis(benzyloxy)-2-((benzyloxy)methyl)-7-((1,1-dioxidotetrahydro-2*H*-thiopyran-4-yl)methyl)-4-(4-(3,4,5-trifluorophenyl)-1*H*-1,2,3-triazol-1-yl)-1-oxa-7-azaspiro[5.5]undecan-8-one (7.8 mg, 0.009 mmol) was dissolved in 1 mL of a solution of methanol: dichloromethane (2:1 v/v), and then 10% palladium/carbon (5.40 mg, 0.005 mmol) was added thereto. A hydrogen balloon was added thereto, and the mixture was degassed for 30 minutes and then stirred in a hydrogen atmosphere for 18 hours. After the completion of the reaction was confirmed by thin layer chromatography, the reaction mixture was filtered through a pad of Celite 545. The filtrate was concentrated under reduced pressure and the remaining concentrate was subjected to column chromatography with 0% to 10% methanol/dichloromethane to synthesize the title compound as a white solid (4.3 mg, yield: 83%).

ESIMS: m/z 575.40 [M+H]+; ¹H-NMR (400 MHz, CD₃OD) δ = 8.60 (S, 1H), 7.68-7.64 (m, 2H), 5.02 (dd, J = 11.3, 2.3 Hz, 1H), 4.73 (d, J = 11.3 Hz, 1H), 4.10 (d, J = 1.6 Hz, 1H), 3.92 (t, J = 5.7 Hz, 1H), 3.81 (dd, J = 13.9, 6.5 Hz, 1H), 3.73 (d, J = 5.5 Hz, 2H), 3.37-3.34 (m, 1H), 3.30-3.30 (m, 3H), 2.89 (S, 1H), 2.51-2.46 (m, 1H), 2.41-2.33 (m, 2H), 2.30-2.26 (m, 1H), 2.22-2.18 (m, 2H), 1.88-1.78 (m, 5H)

### Example 82: Synthesis of Compound A96

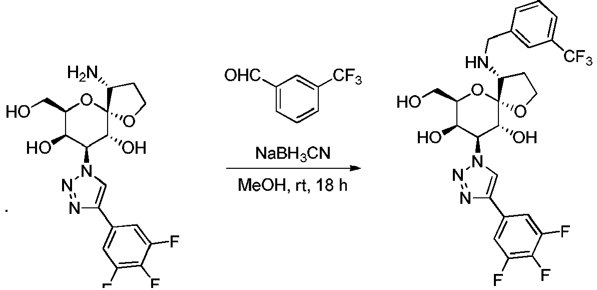

### Synthesis of (4R,5S,7R,8R,9S,10R)-7-(hydroxymethyl)-4-((3-(trifluoromethyl)benzyl)amino)-9-(4-(3,4, 5-trifluorophenyl)-1H-1,2,3 -triazol-1-yl)-1, 6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 3-(trifluoromethyl)benzaldehyde (9.4 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (7.5 mg, yield: 37%).

ESIMS: m/z 575.46 [M+H]+; ¹H-NMR (400 MHz, CD₃OD) δ 8.52 (s, 1H), 7.76 (s, 1H), 7.73-7.53 (m, 5H), 5.04 (dd, J = 11.1, 2.8 Hz, 1H), 4.51 (d, J = 11.2 Hz, 1H), 4.15-4.05 (m, 4H), 3.98 (s, 1H), 3.95-3.90 (m, 1H), 3.81-3.74 (m, 3H), 3.70 (dd, J = 11.5, 4.8 Hz, 1H), 3.64-3.58 (m, 1H), 3.32 (m, 1H), 2.40-2.29 (m, 1H), 2.07-1.92 (m, 2H).

### Example 83: Synthesis of Compound A97

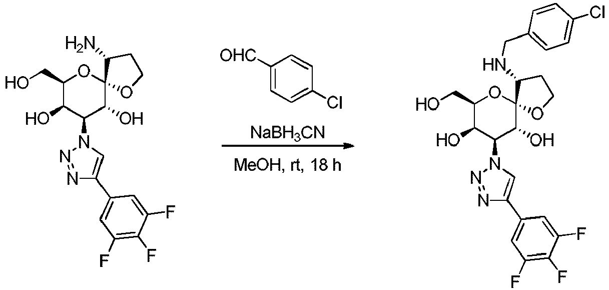

### Synthesis of (4R,5S,7R,8R,9S10R)-4-((4-chlorobenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 4-fluorobenzaldehyde (7.6 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (7.3 mg, yield: 38%).

ESIMS: m/z 541.38 [M+H]+; ¹H-NMR (400 MHz, CD₃OD) δ 8.52 (s, 1H), 7.67-7.61 (m, 2H), 7.38 (dd, J = 24.7, 8.3 Hz, 3H), 5.03 (dd, J = 11.1, 2.7 Hz, 1H), 4.48 (d, J = 11.2 Hz, 1H), 4.16-4.07 (m, 2H), 4.05 (d, J = 2.2 Hz, 1H), 3.96-3.83 (m, 3H), 3.79-3.67 (m, 2H), 3.61-3.54 (m, 1H), 2.37-2.28 (m, 1H), 2.06-1.87 (m, 2H).

### Example 84: Synthesis of Compound A98

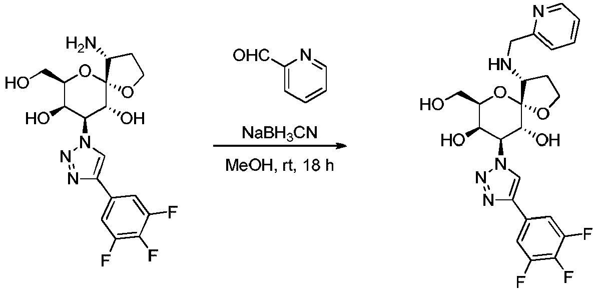

### Synthesis of (4R,5S,7R,8R,9S,10R)-7-(hydroxymethyl)-4-((pyridin-2-ylmethyl)amino)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then picolinaldehyde (5.8 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (7.3 mg, yield: 38%).

ESIMS: m/z 508.36 [M+H]+; ¹H-NMR (400 MHz, CD₃OD) δ 8.54 (s, 1H), 7.85 (t, J = 7.7 Hz, 1H), 7.68-7.60 (m, 2H), 7.55 (d, J = 7.8 Hz, 1H), 7.38-7.32 (m, 1H), 5.04 (dd, J = 11.2, 2.8 Hz, 1H), 4.55 (d, J = 11.2 Hz, 1H), 4.19-4.04 (m, 5H), 3.95 (dd, J = 16.0, 8.2 Hz, 1H), 3.82-3.64 (m, 3H), 2.39-2.29 (m, 1H), 2.05-1.91 (m, 2H).

### Example 85: Synthesis of Compound A99

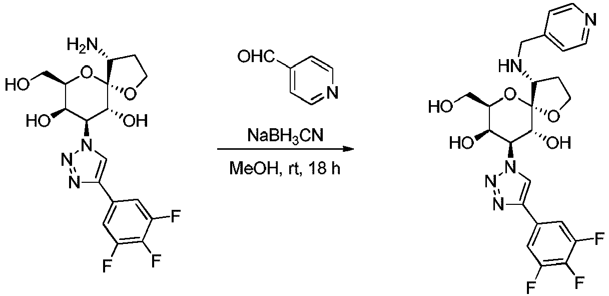

### Synthesis of (4R,5S,7R,8R,9S,10R)-7-(hydroxymethyl)-4-((pyridin-4-ylmethoxy)amino)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then isonicotinaldehyde (5.8 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (5.8 mg, yield: 32%).

ESIMS: m/z 508.42 [M+H]+; ¹H-NMR (400 MHz, CD₃OD) δ 8.55 (s, 1H), 8.50 (d, J = 5.9 Hz, 2H), 7.69-7.61 (m, 2H), 7.53 (d, J = 5.9 Hz, 2H), 5.05 (dd, J = 11.2, 2.8 Hz, 1H), 4.54 (d, J = 11.3 Hz, 1H), 4.16-4.08 (m, 3H), 4.01 (d, J = 6.5 Hz, 1H), 3.94 (dd, J = 15.8, 8.0 Hz, 1H), 3.75 (ddd, J = 16.6, 11.5, 6.2 Hz, 2H), 3.60 (dd, J = 15.5, 6.8 Hz, 1H), 2.40-2.30 (m, 1H), 2.05-1.98 (m, 2H).

### Example 86: Synthesis of Compound A100

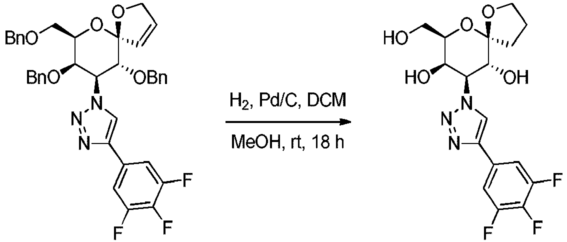

### Synthesis of (5R,7R,8R,9S,10R)-7-(hydroxymethyl)-9-(4-(3,4,5-trifluorophenyl)-1H-1,2,3-triazol-1-yl)-1,6-dioxaspiro[4.5]decane-8,10-diol

1-((5*R*,7*R*,8*R*,9*S*,10*R*)-8,10-bis(benzyloxy)-7-((benzyloxy)methyl)-1.6-dioxaspiro[4.5]dec-3-en-9-yl)-4-(3,4,5-trifluorophenyl)-1*H*-1,2,3-triazole (5.7 mg, 0.009 mmol) was dissolved in 1 mL of a solution of methanol: dichloromethane (2:1 v/v), and then 10% palladium/carbon (9 mg, 0.009 mmol) was added thereto. A hydrogen balloon was added thereto, and the mixture was degassed for 30 minutes and then stirred in a hydrogen atmosphere for 18 hours. After the completion of the reaction was confirmed by thin layer chromatography, the reaction mixture was filtered through a pad of Celite 545. The filtrate was concentrated under reduced pressure and the remaining concentrate was subjected to column chromatography with 0% to 10% methanol/dichloromethane to synthesize the title compound as a white solid (2.6 mg, yield: 76%).

ESIMS: m/z 402.39 [M+H]+; ¹H-NMR (400 MHz, CD₃OD) δ = 8.51 (S, 1H), 7.66-7.62 (m, 2H), 4.82 (dd, J = 11.9, 2.9 Hz, 1H), 4.45 (d, J = 11.7 Hz, 1H), 4.07 (q, J = 6.9 Hz, 2H), 3.97 (q, J = 6.9 Hz, 1H), 3.80-3.75 (m, 1H), 3.72-3.67 (m, 2H), 2.32-2.24 (m, 1H), 2.18-2.07 (m, 2H), 2.05-1.97 (m, 1H)

### Example 87: Synthesis of Compound A101

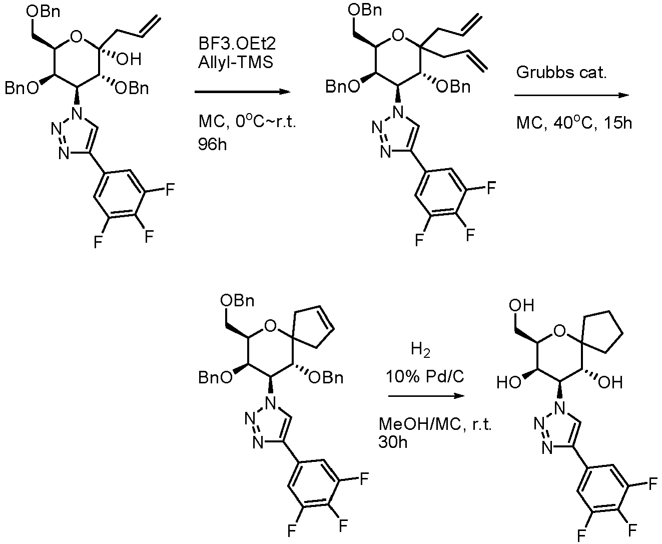

### Synthesis of 1-((7R,8R,9S,10R)-8,10-bis(benzyloxy)-7-((benzyloxy)methyl)-6-oxaspiro[4.5]dec-2-en-9-yl)-4-(3,4,5-trifluorophenyl)-1H-1,2,3-triazole

(2*S*,3*R*,4*S*,5*R*,6*R*)-2-allyl-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ol (50 mg, 0.074 mmol) was dissolved in 2 mL of dichloromethane, and the temperature was lowered to 0 °C, and then allytrimethylsilane (235 µL, 1.48 mmol), and BF₃OEt₂ (11 µL, 0.089 mmol) were sequentially slowly added dropwise thereto, followed by stirring for 24 hours while elevating the temperature from 0 °C to room temperature. The temperature of the reaction solution was lowered again to 0 °C, and then allytrimethylsilane (240 µL, 1.48 mmol), and BF₃OEt₂ (11 µL, 0.089 mmol) were sequentially slowly added dropwise thereto, followed by stirring for 72 hours while elevating the temperature from 0 °C to room temperature. The reaction solution was cooled to 0 °C, then a saturated aqueous sodium hydrogen carbonate solution was added to terminate the reaction. The mixture was extracted with dichloromethane, dried over anhydrous sodium sulfate filtered, and then concentrated. The concentrate was subjected to silica gel column chromatography with 5% to 10% ethyl acetate/n-hexane to obtain the compound as a colorless oil (1-((3*R*,4*S*,5*R*,6*R*)-2,2-diallyl-3,5-bis(benzyloxy)-6-((benzyloxy)methyl)tetrahydro-2*H*-pyran-4-yl)-4-(3,4,5-trifluorophenyl)-1*H*-1,2,3-triazole) This compound was dissolved in 2 mL of dichloromethane, then Grubbs catalyst (2 mg) was added, and then stirred at room temperature for 15 hours. The reaction mixture was concentrated, and then subjected to silica gel column chromatography with 5% to 15% ethyl acetate/n-hexane to obtain the title compound as a colorless oil (3.6 mg).

¹H-NMR (400 MHz, CDCl₃) δ 7.75 (s, 1H), 7.35 - 7.00 (m, 15H), 6.83 (m, 2H), 5.69 (s, 2H), 4.92 (dd, J = 11.1, 2.9 Hz, 1H), 4.59-4.46 (m, 3H), 4.41 (d, J = 11.2 Hz, 1H), 4.23 (d, J = 10.9 Hz, 1H), 4.10-3.91 (m, 4H), 3.74 (t, J = 8.9 Hz, 1H), 3.63 (dd, J = 9.0, 5.2 Hz, 1H), 2.91-2.51 (m, 4H).

### Synthesis of (7R,8R,9S,10R)-7-(hydroxymethyl)-9-(4-(3,4,5-trifluorophenyl)-1H-1,2,3-triazol-1-yl)-6-oxaspiro[4.5]decane-8,10-diol

1-((7*R*,8*R*,9*S*,10*R*)-8,10-bis(benzyloxy)-7-((benzyloxy)methyl)-6-oxaspiro[4.5]dec-2-en-9-yl)-4-(3,4,5-trifluorophenyl)-1*H*-1,2,3-triazole (3.6 mg) was dissolved in 3 mL of methanol, and then 10% palladium/carbon (3.6 mg) was added thereto. A hydrogen balloon was added thereto, and the mixture was degassed for 30 minutes and then stirred in a hydrogen atmosphere for 15 hours. Dichloromethane (1 mL) and 10% palladium/carbon (3 mg) were added to the reaction solution, and then the mixture was stirred in a hydrogen atmosphere for 15 hours. The reaction mixture was fitered through a pad of Celite 545. The filtrate was concentrated under reduced pressure and the remaining concentrate was subjected to silica gel column chromatography with 0% to 5% methanol/dichloromethane to obtain the title compound as a white solid (1.5 mg).

ESIMS: m/z 400.37 [M+H]+; ¹H-NMR (400 MHz, CDCl₃) δ 8.13 (s, 1H), 7.48-7.39 (m, 2H), 4.61-4.56 (m, 1H), 4.49-4.43 (m, 1H), 4.40-4.36 (m, 1H), 4.05-4.01 (m, 1H), 3.94 (br-s, 2H), 3.78-3.72 (m, 1H), 2.31-1.55 (m, 10H).

### Example 88: Synthesis of Compound A102

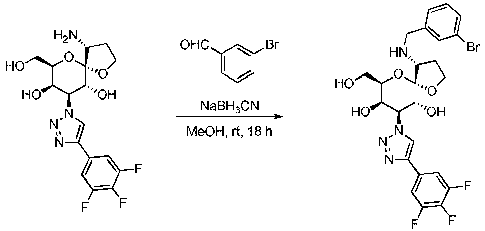

### Synthesis of (4R,5S,7R,8R,9S10R)-4-((3-bromobenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 3-bromobenzaldehyde (9.99 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (7.5 mg, yield: 36%).

ESIMS: m/z 585.44 [M+H]+; ¹H-NMR (400 MHz, CD₃OD) δ 8.53 (s, 1H), 7.70-7.55 (m, 3H), 7.42 (dd, J = 16.8, 8.2 Hz, 2H), 7.27 (t, J = 7.7 Hz, 1H), 5.03 (dd, J = 11.2, 2.8 Hz, 1H), 4.48 (d, J = 11.4 Hz, 1H), 4.14-4.03 (m, 3H), 3.97-3.90 (m, 1H), 3.87 (d, J = 5.9 Hz, 1H), 3.73 (ddd, J = 16.4, 11.7, 6.3 Hz, 2H), 3.61-3.55 (m, 1H), 2.37-2.28 (m, 1H), 2.02 (d, J = 10.8 Hz, 1H), 1.95 (dd, J = 11.1, 9.4 Hz, 1H).

### Example 89: Synthesis of Compound A103

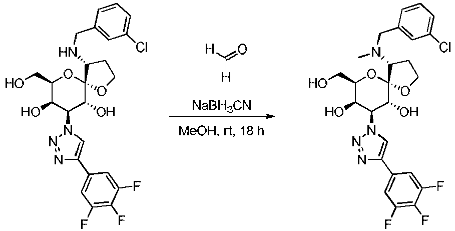

### Synthesis of (4R,5S,7R,8R,9S,10R)-4-((3-chlorobenzyl)(methyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (10.0 mg, 0.019 mmol) was dissolved in 1.0 mL of methanol, and then 37 wt% formaldehyde (2.3 mg, 0.028 mmol) and sodium cyanoborohydride (2.3 mg, 0.028 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (10 mg, yield: 97.8%).

ESIMS: m/z 555.45 [M+H]+; ¹H-NMR (400 MHz, CD₃OD) δ 8.51 (s, 1H), 7.60 (dd, J = 8.5, 6.7 Hz, 2H), 7.54 (s, 1H), 7.37-7.23 (m, 3H), 5.05 (dd, J = 11.1, 2.5 Hz, 1H), 4.60 (d, J = 11.1 Hz, 1H), 4.15-4.05 (m, 3H), 3.96 (dd, J = 16.7, 10.0 Hz, 2H), 3.86-3.77 (m, 1H), 3.77-3.64 (m, 2H), 3.53 (t, J = 9.1 Hz, 1H), 2.38 (s, 3H), 2.27 (dd, J = 20.6, 9.6 Hz, 1H), 2.22-2.09 (m, 1H).

### Example 90: Synthesis of Compound A104

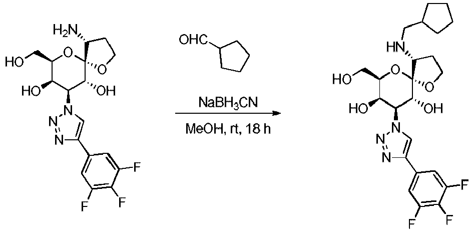

### Synthesis of (4R,5S,7R,8R,9S,10R)-4-((cyclopentylmethyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then cyclopentancarbaldehyde (9.99 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (4.0 mg, yield: 22.2%).

¹H-NMR (400 MHz, CD₃OD) δ 8.51 (d, J = 5.8 Hz, 1H), 7.65 (ddd, J = 5.8, 5.4, 2.0 Hz, 2H), 5.04 (dd, J = 11.3, 2.8 Hz, 1H), 4.59 (s, 1H), 4.48 (dd, J = 22.6, 11.1 Hz, 2H), 4.11-4.06 (m, 3H), 4.00-3.93 (m, 1H), 3.77 (ddd, J = 12.7, 5.6, 2.7 Hz, 2H), 3.73-3.61 (m, 3H), 2.28 (dd, J = 14.7, 8.0 Hz, 1H), 2.06-2.00 (m, 1H), 1.97-1.89 (m, 1H), 1.76-1.69 (m, 2H), 1.67-1.58 (m, 2H), 1.57-1.48 (m, 2H).

### Example 91: Synthesis of Compound A105

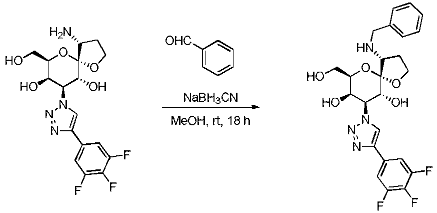

### Synthesis of (4R,5S,7R,8R,9S,10R)-4-(benzylamino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then benzaldehyde (5.7 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (9.3 mg, yield: 51%).

ESIMS: m/z 507.50 [M+H]+; ¹H-NMR (400 MHz, CD₃OD) δ 8.53 (s, 1H), 7.64 (dd, J = 8.4, 7.0 Hz, 2H), 7.42 (d, J = 7.2 Hz, 2H), 7.36 (t, J = 7.4 Hz, 2H), 7.28 (t, J = 7.2 Hz, 1H), 5.03 (dd, J = 11.2, 2.7 Hz, 1H), 4.49 (d, J = 11.3 Hz, 1H), 4.15-4.07 (m, 2H), 4.04 (d, J = 1.8 Hz, 1H), 3.97-3.86 (m, 3H), 3.73 (ddd, J = 16.4, 11.5, 6.2 Hz, 2H), 3.63 (t, J = 8.7 Hz, 1H), 2.39-2.30 (m, 1H), 2.04-1.89 (m, 1H).

### Example 92: Synthesis of Compound A106

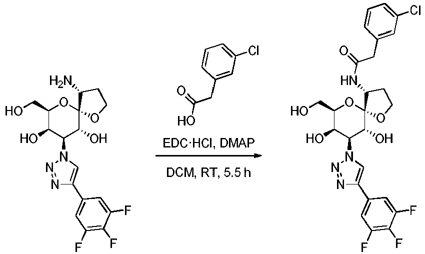

### Synthesis of 2-(3-chlorophenyl)-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)acetamide

(4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (15.6 mg, 0.038 mmol) was dissolved in 1.0 mL of dichloromethane, and then 2-(3-chlorophenyl)acetic acid (12.3 mg, 0.072 mmol), *N-*(3-dimethylaminopropyl)-*N'*-ethylcarbodiimide hydrochloride (13.8 mg, 0.072 mmol), triethylamine (20 µL, 0.144 mmol), 4-dimethylaminopyridine (0.2 mg, 0.018 mmol) were sequentially added dropwise thereto at room temperature, followed by stirring for 5.5 hours. The reaction solution was diluted with dichloromethane (5 mL), and then washed with a saturated aqueous sodium hydrogen carbonate solution (3 mL × 2). The organic layer was dried over anhydrous sodium sulfate, filtered, and then concentrated under reduced pressure. The concentrate was subjected to silica gel column chromatography with 0% to 10% methanol/dichloromethane to synthesize the title compound as a white solid (9.6 mg, yield: 70%).

ESIMS: m/z 569.50 [M+H]+; ¹H-NMR (400 MHz, CD₃OD) δ = 8.43 (S, 1H), 7.62-7.58 (m, 2H), 7.39 (S, 1H), 7.34-7.22 (m, 3H), 5.01 (dd, J = 11.3, 2.7 Hz, 1H), 4.75 (t, J = 9.4 Hz, 1H), 4.31 (d, J = 11.3 Hz, 1H), 4.13-4.10 (m, 3H), 4.01 (q, J = 8.2 Hz, 1H), 3.82-3.74 (m, 2H), 3.65 (d, J = 14.5 Hz, 1H), 3.58 (d, J = 14.5 Hz, 1H), 2.37-2.30 (m, 1H), 2.11-1.99 (m, 1H)

### Example 93: Synthesis of Compound A107

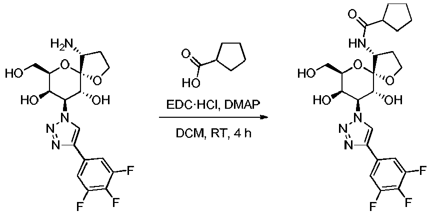

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)cyclopentancarboxamide

The title compound (13.1, yield: 71%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (15.6 mg, 0.038 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 513.55 [M+H]+; ¹H-NMR (400 MHz, CDCl₃) δ = 7.62 (S, 1H), 7.36-7.29 (m, 8H), 7.22-7.18 (m, 2H), 7.17-7.10 (m, 5H), 6.97 (d, J = 7.0 Hz, 2H), 5.09 (dd, J = 11.0, 2.7 Hz, 1H), 4.58-4.49 (m, 3H), 4.43 (d, J = 10.6 Hz, 1H), 4.31-4.23 (m, 3H), 4.13 (d, J = 2.7 Hz, 1H), 3.93 (d, J = 11.0 Hz, 1H), 3.71-3.66 (m, 4H), 2.28-2.21 (m, 1H), 2.04-1.82 (m, 3H)

### Example 94: Synthesis of Compound A108

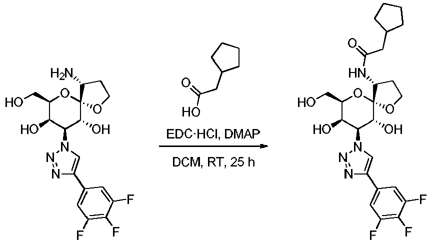

### Synthesis of 2-cyclopentyl-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)acetamide

The title compound (9.5 mg, yield: 75%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (15.1 mg, 0.036 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 527.48 [M+H]+; ¹H-NMR (400 MHz, CD₃OD) δ = 8.48 (S, 1H), 7.64-7.60 (m, 2H), 5.02 (dd, J = 11.3, 2.7 Hz, 1H), 4.74 (t, J = 9.2 Hz, 1H), 4.35 (d, J = 11.0 Hz, 1H), 4.13-4.10 (m, 3H), 4.00 (q, J = 8.1 Hz, 1H), 3.79 (dd, J = 11.3, 6.7 Hz, 1H), 3.74 (dd, J = 11.3, 5.1 Hz, 1H), 2.37-2.22 (m, 4H), 2.06-1.96 (m, 1H), 1.91-1.81 (m, 2H), 1.69-1.58 (m, 4H), 1.31-1.20 (m, 2H)

### Example 95: Synthesis of Compound A109

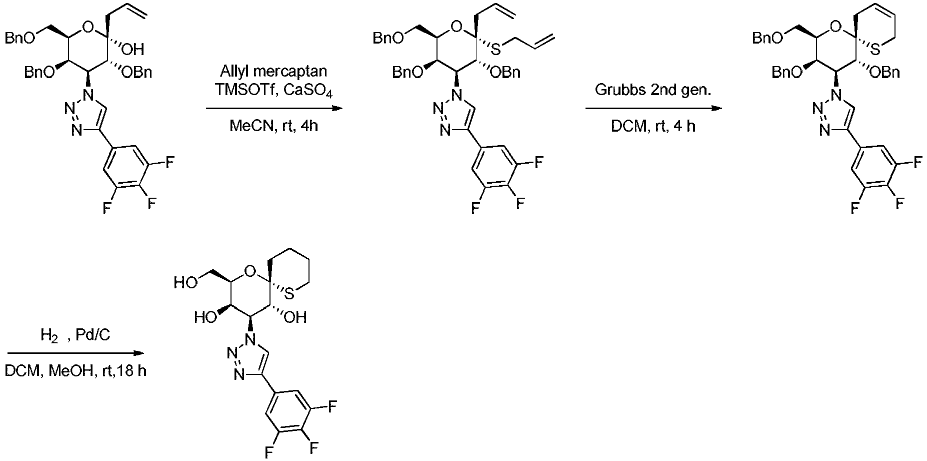

### Synthesis of 1-((2R,3R,4S,5R,6R)-2-allyl-2-(allylthio)-3,5-bis(benzyloxy)-6-((benzyloxy)methyl)tetrahydro-2H-pyran-4-yl)-4-(3,4,5-trifluorophenyl)-1H-1,2,3-triazole

(2*S*,3*R*,4*S*,5*R*,6*R*)-2-allyl-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ol (55.1 mg, 0.082 mmol) was dissolved in 1.6 mL of acetonitrile, and then anhydrous calcium sulfate (110.2 mg, 200 wt%) and 70% allylmercaptan (28 µL, 0.246 mmol) were added thereto. The reaction mixture was stirred in an argon atmosphere at room temperature for 30 minutes. Trimethylsilyl trifluoromethanesulfonate (22 µL, 0.123 mmol) was then added drop by drop for 5 minutes. The reaction mixture was stirred in an argon atmosphere at room temperature for 4 hours. It was confirmed by thin layer chromatography that all of the starting materials were consumed, and then 2 mL of a saturated aqueous sodium hydrogen carbonate solution was added to terminate the reaction. The reaction mixture was extracted with ethyl acetate (5 mL × 3), and the organic layer was mixed, dried over anhydrous sodium sulfate, and then filtered. The filtrate was concentrated, and the remaining concentrate was subjected to column chromatography with 0% to 15% ethyl acetate/n-hexane to obtain the title compound as a clear oil (34.2 mg, yield: 57%).

¹H-NMR (400 MHz, CDCl₃) δ = 7.57 (S, 1H), 7.32 (t, J = 5.3 Hz, 7H), 7.15-7.00 (m, 8H), 6.93 (d, J = 7.0 Hz, 2H), 6.12-6.01 (m, 1H), 5.94-5.84 (m, 1H), 5.35 (dd, J = 10.8, 2.2 Hz, 1H), 5.28 (d, J = 10.2 Hz, 1H), 5.24 (d, J = 3.1 Hz, 1H), 5.20 (d, J = 3.9 Hz, 1H), 5.09 (d, J = 9.8 Hz, 1H), 4.60-4.37 (m, 7H), 4.04 (d, J = 11.0 Hz, 1H), 3.97 (d, J = 11.3 Hz, 1H), 3.74 (t, J = 8.6 Hz, 1H), 3.66 (dd, J = 9.2, 5.7 Hz, 1H), 3.25 (dd, J = 13.3, 7.4 Hz, 1H), 3.14 (dd, J = 12.7, 6.8 Hz, 1H), 3.03 (dd, J = 15.3, 4.3 Hz, 1H), 2.87 (dd, J = 15.3, 9.4 Hz, 1H)

### Synthesis of 1-((2R,3R,4S,5R,6R)-3,5-bis(benzyloxy)-2-((benzyloxy)methyl)-1-oxa-7-thiaspiro[5.5]undec-9-en-4-yl)-4-(3,4,5-trifluorophenyl)-1H-1,2,3-triazole

(1-((2*R*,3*R*,4*S*,5*R*,6*R*)-2-allyl-2-(allylthio)-3,5-bis(benzyloxy)-6-((benzyloxy)methyl)tetrahydro-2*H*-pyran-4-yl)-4-(3,4,5-trifluorophenyl)-1*H*-1,2,3-triazole (34.1 mg, 0.047 mmol) was dissolved in 2.3 mL of dichloromethane, and then Grubbs generation 2 catalyst (2 mg, 0.002 mmol) was added thereto. The reaction mixture was stirred in an argon atmosphere at room temperature for 4 hours. The reaction mixture was concentrated under reduced pressure, and the remaining concentrate was subjected to column chromatography with 0% to 15% ethyl acetate/n-hexane to obtain the title compound as a clear oil (24.7 mg, yield: 75%).

¹H-NMR (400 MHz, CDCl₃) δ = 7.69 (S, 1H), 7.34-7.28 (m, 9H), 7.21-7.17 (m, 2H), 7.14-7.06 (m, 5H), 6.94 (d, J = 7.0 Hz, 2H), 5.90-5.86 (m, 1H), 5.75-5.71 (m, 1H), 5.30 (dd, J = 11.0, 3.1 Hz, 2H), 4.59 (d, J = 5.9 Hz, 1H), 4.56 (S, 1H), 4.51 (d, J = 2.3 Hz, 1H), 4.48 (d, J = 2.3 Hz, 1H), 4.43 (d, J = 11.0 Hz, 1H), 4.25 (d, J = 10.6 Hz, 1H), 4.15 (d, J = 11.3 Hz, 1H), 4.08 (d, J = 2.7 Hz, 1H), 3.93 (d, J = 11.0 Hz, 1H), 3.79 (t, J = 8.4 Hz, 1H), 3.67 (dd, J = 9.4, 6.3 Hz, 1H), 3.28 (d, J = 17.6 Hz, 1H), 3.04 (d, J = 17.6 Hz, 1H), 2.93 (dd, J = 16.8, 5.5 Hz, 1H), 2.37 (d, J = 14.9 Hz, 1H)

### Synthesis of (2R,3R,4S,5R,6R)-2-(hydroxymethyl)-4-(4-(3,4,5-trifluorophenyl)-1H-1,2,3-triazol-1-yl)-1-oxa-7-thiaspiro[5.5]undecane-3,5-diol

1-((2*R*,3*R*,4*S*,5*R*,6*R*)-3,5-bis(benzyloxy)-2-((benzyloxy)methyl)-1-oxa-7-thiaspiro[5.5]undec-9-en-4-yl)-4-(3,4,5-trifluorophenyl)-1*H*-1,2,3-triazole (15 mg, 0.021 mmol) was dissolved in 1 mL of a solution of methanol: dichloromethane (2:1 v/v), and then 10% palladium/carbon (22.3 mg, 0.021 mmol) was added thereto. A hydrogen balloon was added thereto, bubbling was performed for 30 minutes, and the mixture was then stirred in a hydrogen atmosphere for 18 hours. The reaction mixture was filtered through a pad of Celite 545 and concentrated under reduced pressure. The remaining filtrate was subjected to column chromatography with 0% to 10% methanol/dichloromethane to synthesize the title compound as a white solid (1.6 mg, yield: 18%).

ESIMS: m/z 432.47 [M+H]+; ¹H-NMR (400 MHz, CD₃OD) δ = 8.45 (S, 1H), 7.64-7.60 (m, 2H), 4.96 (dd, J = 11.3, 2.7 Hz, 1H), 4.40 (t, J = 6.1 Hz, 1H), 4.22 (d, J = 11.3 Hz, 1H), 4.11 (d, J = 2.0 Hz, 1H), 3.83-3.74 (m, 2H), 2.88 (t, J = 12.7 Hz, 1H), 2.41 (t, J = 17.0 Hz, 1H), 2.09-1.88 (m, 3H), 1.69 (t, J = 15.5 Hz, 1H)

### Example 96: Synthesis of Compound A110

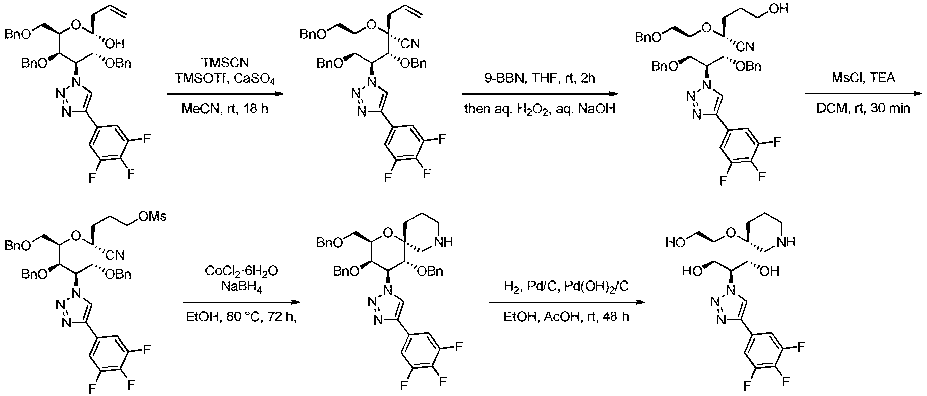

### Synthesis of (2R,3R,4S,5R,6R)-2-allyl-3,5-bis(benzyloxy)-6-((benzyloxy)methyl)-4-(4-(3,4,5-trifluorophenyl)-1H-1,2,3-triazol-1-yl)tetrahydro-2H-pyran-2-carbonitrile

(2*S*,3*R*,4*S*,5*R*,6*R*)-2-allyl-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-ol (30.0 mg, 0.045 mmol) was dissolved in 0.9 mL of acetonitrile, and then anhydrous calcium sulfate (60.0 mg, 200 wt%) and trimethylsilylcyanide (18 µL, 0.134 mmol) were added thereto. The reaction mixture was stirred in an argon atmosphere at room temperature for 30 minutes. Trimethylsilyl trifluoromethanesulfonate (12 µL, 0.068 mmol) was then added drop by drop for 5 minutes. The reaction mixture was stirred in an argon atmosphere at room temperature for 18 hours. It was confirmed by thin layer chromatography that all of the starting materials were consumed, and then 2 mL of a saturated aqueous sodium hydrogen carbonate solution was added to terminate the reaction. The reaction mixture was extracted with ethyl acetate (5 mL x 3), and the organic layer was mixed, dried over anhydrous sodium sulfate, and then filtered. The filtrate was concentrated, and the remaining concentrate was subjected to column chromatography with 0% to 20% ethyl acetate/n-hexane to obtain the title compound as a clear oil (18.3 mg, yield: 59%).

¹H-NMR (400 MHz, CDCl₃) δ = 7.62 (S, 1H), 7.34-6.96 (m, 17H), 5.99-5.90 (m, 1H), 5.33-5.25 (m, 2H), 5.10 (dd, J = 11.0, 2.7 Hz, 1H), 4.60-4.42 (m, 4H), 4.32-4.27 (m, 2H), 4.21 (d, J = 11.3 Hz, 1H), 4.14 (d, J = 2.0 Hz, 1H), 3.95 (d, J = 11.0 Hz, 1H), 3.85-3.67 (m, 2H), 2.88 (dd, J = 14.5, 6.3 Hz, 1H), 2.70 (dd, J = 14.5, 7.4 Hz, 1H)3.66 (dd, J = 9.2, 5.7 Hz, 1H), 3.25 (dd, J = 13.3, 7.4 Hz, 1H), 3.14 (dd, J = 12.7, 6.8 Hz, 1H), 3.03 (dd, J = 15.3, 4.3 Hz, 1H), 2.87 (dd, J = 15.3, 9.4 Hz, 1H)

### Synthesis of (2R,3R,4S,5R,6R)-3,5-bis(benzyloxy)-6-((benzyloxy)methyl)-2-(3-hydroxypropyl)-4-(4-(3,4,5-trifluorophenyl)-1H-1,2,3-triazol-1-yl)tetrahydro-2H-pyran-2-carbonitrile

(2*R*,3*R*,4*S*,5*R*,6*R*)-2-allyl-3,5-bis(benzyloxy)-6-((benzyloxy)methyl)-4-(4-(3,4,5-trifluorophenyl)-1*H*-1,2,3-triazol-1-yl)tetrahydro-2*H*-pyran-2-carbonitrile (52.4 mg, 0.077 mmol) was dissolved in 9-borabicyclo[3.3.1]nonane (1.5 mL in a 0.5 M tetrahydrofuran solution), followed by stirring in an argon atmosphere at room temperature for 2 hours. Next, the reaction mixture was cooled to 0 °C, and 1 mL of a 5N aqueous NaOH solution and 1 mL of a 35% hydrogen peroxide solution were slowly added dropwise thereto. The reaction mixture was stirred at 0 °C for 16 hours. The reaction mixture was diluted with 10 mL of ethyl acetate and 5 mL of distilled water, and then washed with a saturated aqueous sodium chloride solution (3 mL x 2 times). The organic layer was dried over anhydrous sodium sulfate, filtered, and concentrated under reduced pressure. The remaining concentrate was subjected to silica gel column chromatography with 0% to 35% ethyl acetate/n-hexane to obtain the title compound as a white solid (16.3 mg, yield: 30%).

¹H-NMR (400 MHz, CDCl₃) δ = 7.62 (S, 1H), 7.36-7.29 (m, 8H), 7.22-7.18 (m, 2H), 7.17-7.10 (m, 5H), 6.97 (d, J = 7.0 Hz, 2H), 5.09 (dd, J = 11.0, 2.7 Hz, 1H), 4.58-4.49 (m, 3H), 4.43 (d, J = 10.6 Hz, 1H), 4.31-4.23 (m, 3H), 4.13 (d, J = 2.7 Hz, 1H), 3.93 (d, J = 11.0 Hz, 1H), 3.71-3.66 (m, 4H), 2.28-2.21 (m, 1H), 2.04-1.82 (m, 3H)

### Synthesis of 3-((2R,3R,4S,5R,6R)-3,5-bis(benzyloxy)-6-((benzyloxy)methyl)-2-cyano-4-(4-(3,4,5-trifluorophenyl)-1H-1,2,3-triazol-1-yl)tetrahydro-2H-pyran-2-yl)propyl methansulfonate

(2*R*,3*R*,4*S*,5*R*,6*R*)-3,5-bis(benzyloxy)-6-((benzyloxy)methyl)-2-(3-hydroxypropyl)-4-(4-(3,4,5-trifluorophenyl)-1*H*-1,2,3-triazol-1-yl)tetrahydro-2H-pyran-2-carbonitrile (35.2 mg, 0.050 mmol) was dissolved in 1 mL of dichloromethane, and then methansulfonylchloride (6 µL, 0.076 mmol) and triethylamine (21 µL, 0.151 mmol) were added thereto. The reaction mixture was stirred at room temperature for 0.5 hours. The reaction mixture was concentrated under reduced pressure, and the remaining concentrate was subjected to silica gel column chromatography with 0% to 40% ethyl acetate/n-hexane to obtain the title compound as a colorless oil (38.1 mg, yield: 99%).

### Synthesis of (2R,3R,4S,5R,6R)-3,5-bis(benzyloxy)-2-((benzyloxy)methyl)-4-(4-(3,4,5-trifluorophenyl)-1H-1,2,3-triazol-1-yl)-1-oxa-8-azaspiro[5.5]undecane

3-((2*R*,3*R*,4*S*,5*R*,6*R*)-3,5-bis(benzyloxy)-6-((benzyloxy)methyl)-2-cyano-4-(4-(3,4,5-trifluorophenyl)-1*H*-1,2,3-triazol-1-yl)tetrahydro-2*H*-pyran-2-yl)propyl methansulfonate (38.1 mg, 0.049 mmol) was dissolved in 2.45 mL of ethanol in a sealed tube and then cooled to 0 °C. Next, cobalt(II) chloride hexahydrate (11.6 mg, 0.049 mmol) was added and stirred for 10 minutes. Sodium borohydride (18.5 mg, 0.490 mmol) was then added thereto and a lid of the sealed tube was closed. The reaction mixture was stirred at 80 °C for 3 days. The reaction mixture was cooled to 0 °C, and 5 mL of a saturated sodium hydrogen carbonate solution was added thereto. This was extracted with dichloromethane (5 mL × 3 times) to obtain an organic layer. The obtained organic layer was dried over anhydrous sodium sulfate, filtered, and then concentrated under reduced pressure. The remaining concentrate was subjected to silica gel column chromatography with 5% methanol/dichloromethane to obtain the title compound as a white solid (14.9 mg, yield: 44%).

¹H-NMR (400 MHz, CDCl₃) δ = 7.90 (S, 1H), 7.31-7.28 (m, 6H), 7.24-7.12 (m, 9H), 6.88-6.86 (m, 2H), 5.20 (dd, J = 11.3, 2.0 Hz, 1H), 4.64 (d, J = 11.3 Hz, 1H), 4.50 (d, J = 11.7 Hz, 1H), 4.46 (d, J = 11.0 Hz, 1H), 4.33 (d, J = 11.0 Hz, 2H), 4.17 (d, J = 11.0 Hz, 1H), 4.13 (S, 1H), 4.06-3.95 (m, 4H), 3.75 (t, J = 8.6 Hz, 1H), 3.26 (d, J = 11.3 Hz, 1H), 3.10 (d, J = 13.7 Hz, 1H), 2.57 (t, J = 12.3 Hz, 1H), 2.20-2.14 (m, 1H), 1.84 (d, J = 5.5 Hz, 2H), 1.55 (d, J = 13.7 Hz, 1H)

### Synthesis of (2R,3R,4S,5R,6R)-2-(hydroxymethyl)-4-(4-(3,4,5-trifluorophenyl)-1H-1,2,3-triazol-1-yl)-1-oxa-8-azaspiro[5.5]undecane-3,5-diol

(2*R*,3*R*,4*S*,5*R*,6*R*)-3,5-bis(benzyloxy)-2-((benzyloxy)methyl)-4-(4-(3,4,5-trifluorophenyl)-1*H*-1,2,3-triazol-1-yl)-1-oxa-8-azaspiro[5.5]undecane (14.9 mg) was dissolved in 4 mL of a solution of ethanol:acetic acid (3:1 v/v), and then 10% palladium/carbon (29.8 mg, 20 wt%) and 20% palldiumhydroxide/carbon (14.9 mg, 10 wt%) were added thereto. A hydrogen balloon was added thereto, bubbling was performed for 30 minutes, and the mixture was then stirred in a hydrogen atmosphere for 48 hours. The reaction mixture was filtered through a pad of Celite 545 and concentrated under reduced pressure. The remaining filtrate was subjected to column chromatography with 0% to 10% methanol/dichloromethane to synthesize the title compound as a white solid (7.2 mg, yield: 80%).

ESIMS: m/z 415.46 [M+H]+; ¹H-NMR (400 MHz, CD₃OD) δ = 8.46 (S, 1H), 7.64-7.60 (m, 2H), 4.92 (dd, J = 11.3, 2.7 Hz, 1H), 4.13 (d, J = 11.7 Hz, 1H), 4.06 (d, J = 2.0 Hz, 1H), 3.89-3.80 (m, 2H), 3.69 (dd, J= 11.3, 4.3 Hz, 1H), 3.42 (d, J = 14.5 Hz, 1H), 2.99 (d, J = 12.9 Hz, 1H), 2.85 (d, J = 14.5 Hz, 1H), 2.55-2.49 (m, 1H), 2.10-1.96 (m, 2H), 1.74-1.72 (m, 1H), 1.53-1.50 (m, 1H)

### Example 97: Synthesis of Compound A111

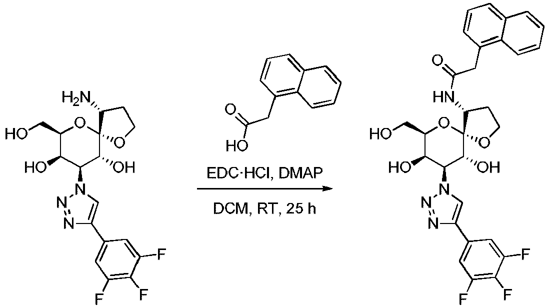

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(naphthalen-1-yl)acetamide

The title compound (9.7 mg, yield: 69%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 585.29 [M+H]+; ¹H-NMR (400 MHz, CD₃OD) δ = 8.26 (S, 1H), 8.09 (d, J = 8.6 Hz, 1H), 7.89 (d, J = 7.8 Hz, 1H), 7.81 (d, J = 7.8 Hz, 1H), 7.60-7.43 (m, 6H), 4.97 (dd, J = 11.2, 2.5 Hz, 1H), 4.75 (t, J = 9.4 Hz, 1H), 4.25 (d, J = 11.3 Hz, 1H), 4.15-4.03 (m, 5H), 3.98 (q, J = 8.1 Hz, 1H), 3.75-3.68 (m, 2H), 2.34-2.26 (m, 1H), 2.07-1.97 (m, 1H)

### Example 98: Synthesis of Compound A112

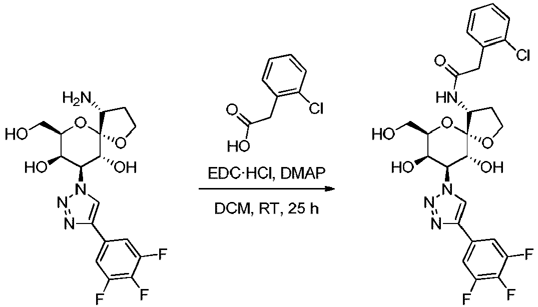

### Synthesis of 2-(2-chlorophenyl)-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)acetamide

The title compound (12.5 mg, yield: 92%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 569.35 [M+H]+; ¹H-NMR (400 MHz, CD₃OD) δ = 8.45 (S, 1H), 7.61-7.57 (m, 2H), 7.42-7.39 (m, 2H), 7.30-7.23 (m, 2H), 5.00 (dd, J = 11.3, 2.0 Hz, 1H), 4.75 (t, J = 9.2 Hz, 1H), 4.38 (d, J = 11.0 Hz, 1H), 4.12-4.08 (m, 3H), 3.99 (q, J = 8.0 Hz, 1H), 3.81-3.69 (m, 4H), 2.35-2.31 (m, 1H), 2.08-1.96 (m, 1H)

### Example 99: Synthesis of Compound A113

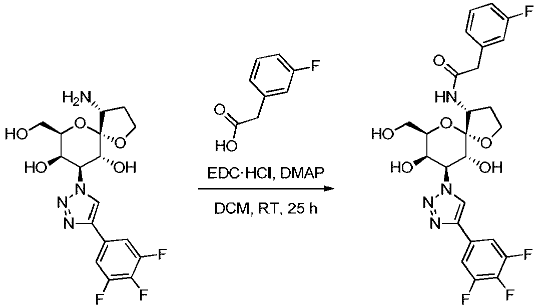

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(3-fluorophenyl)acetamide

The title compound (6.4 mg, yield: 48%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 553.35 [M+H]+; ¹H-NMR (400 MHz, CD₃OD) δ = 8.41 (S, 1H), 7.61-7.57 (m, 2H), 7.35-7.30 (m, 1H), 7.17 (d, J = 7.4 Hz, 1H), 7.12 (d, J = 9.8 Hz, 1H), 6.98 (t, J = 8.4 Hz, 1H), 4.99 (dd, J = 11.2, 2.5 Hz, 1H), 4.76-4.71 (m, 1H), 4.30 (d, J = 11.0 Hz, 1H), 4.11-4.09 (m, 3H), 3.99 (q, J = 8.1 Hz, 1H), 3.80-3.72 (m, 2H), 3.61 (dt, J = 26.2 Hz, 2H), 2.36-2.29 (m, 1H), 2.07-1.97 (m, 1H)

### Example 100: Synthesis of Compound A114

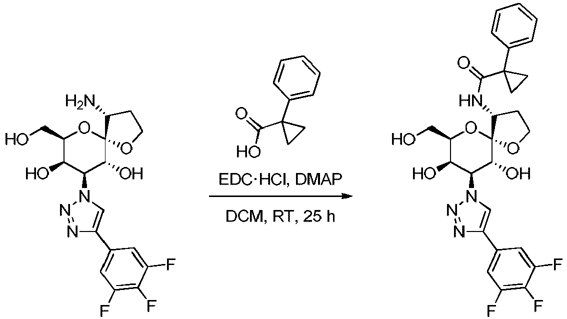

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-phenylcyclopropanecarboxamide

The title compound (4.4 mg, yield: 33%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 561.48 [M+H]+; ¹H-NMR (400 MHz, CD₃OD) δ = 8.54 (S, 1H), 7.66-7.62 (m, 2H), 7.48-7.46 (m, 2H), 7.39 (t, J = 7.2 Hz, 2H), 7.34-7.30 (m, 1H), 4.95 (dd, J = 11.3, 2.7 Hz, 1H), 4.63 (t, J = 9.2 Hz, 1H), 4.25 (d, J = 11.3 Hz, 1H), 4.04 (d, J = 2.0 Hz, 1H), 3.99 (dd, J = 8.8, 3.3 Hz, 1H), 3.95-3.89 (m, 2H), 3.47 (dd, J = 11.0, 5.9 Hz, 1H), 3.34 (dd, J = 11.0, 6.7 Hz, 1H), 2.30-2.23 (m, 1H), 1.82-1.72 (m, 1H), 1.59-1.55 (m, 1H), 1.52-1.47 (m, 1H), 1.23-1.19 (m, 1H), 1.07-1.02 (m, 1H)

### Example 101: Synthesis of Compound A115

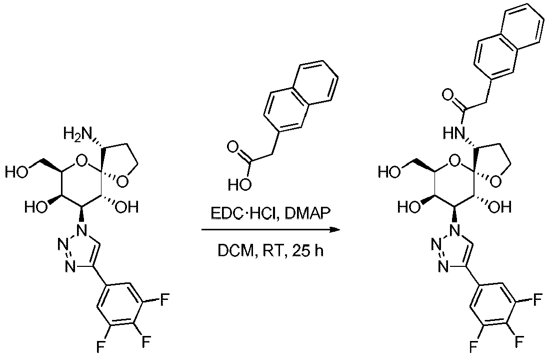

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(naphthalen-2-yl)acetamide

The title compound (7.7 mg, yield: 55%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 585.42 [M+H]+; ¹H-NMR (400 MHz, CD₃OD) δ = 8.27 (S, 1H), 7.83 (d, J = 8.6 Hz, 4H), 7.52-7.42 (m, 5H), 4.99 (dd, J = 11.0, 2.7 Hz, 1H), 4.76 (t, J = 9.2 Hz, 1H), 4.28 (d, J = 11.3 Hz, 1H), 4.12-4.09 (m, 3H), 3.99 (q, J = 8.1 Hz, 1H), 3.83-3.69 (m, 4H), 2.32 (t, J = 3.9 Hz, 1H), 2.14-1.99 (m, 1H)

### Example 102: Synthesis of Compound A116

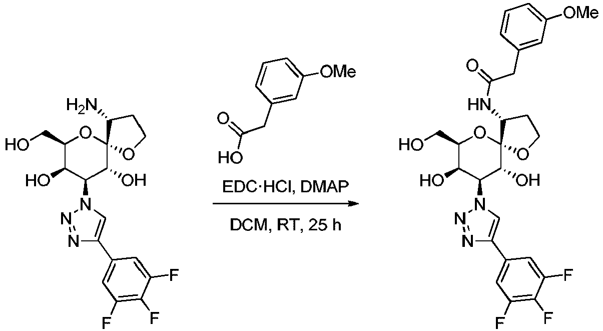

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(3-methoxyphenyl)acetamide

The title compound (7.0 mg, yield: 51%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 565.45 [M+H]+; ¹H-NMR (400 MHz, CD₃OD) δ = 8.40 (S, 1H), 7.61-7.57 (m, 2H), 7.25-7.21 (m, 1H), 6.94-6.91 (m, 2H), 6.82 (d, J = 7.8 Hz, 1H), 4.98 (dd, J = 11.0, 2.3 Hz, 1H), 4.72 (t, J = 9.2 Hz, 1H), 4.28 (d, J = 11.0 Hz, 1H), 4.10-4.07 (m, 3H), 3.98 (q, J = 7.8 Hz, 1H), 3.78-3.70 (m, 5H), 3.61-3.52 (m, 2H), 2.32-2.30 (m, 1H), 2.02-1.95 (m, 1H)

### Example 103: Synthesis of Compound A117

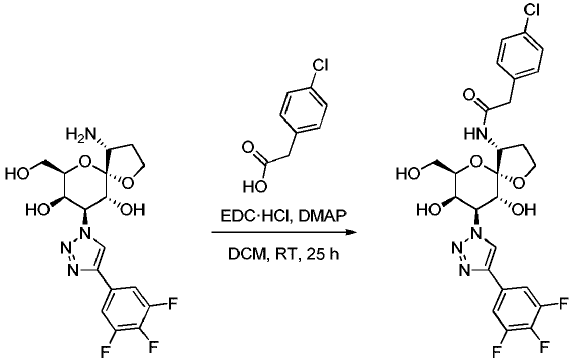

### Synthesis of 2-(4-chlorophenyl)-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)acetamide

The title compound (7.0 mg, yield: 51%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 569.41 [M+H]+; ¹H-NMR (400 MHz, CD₃OD) δ = 8.38 (S, 1H), 7.60-7.57 (m, 2H), 7.37-7.30 (m, 4H), 4.99 (dd, J = 11.3, 2.7 Hz, 1H), 4.74 (t, J = 9.2 Hz, 1H), 4.28 (d, J = 11.3 Hz, 1H), 4.12-4.09 (m, 3H), 3.99 (q, J = 8.1 Hz, 1H), 3.80-3.72 (m, 2H), 3.58 (dt, J = 37.8 Hz, 2H), 2.35-2.28 (m, 1H), 2.07-1.97 (m, 1H)

### Example 104: Synthesis of Compound A118

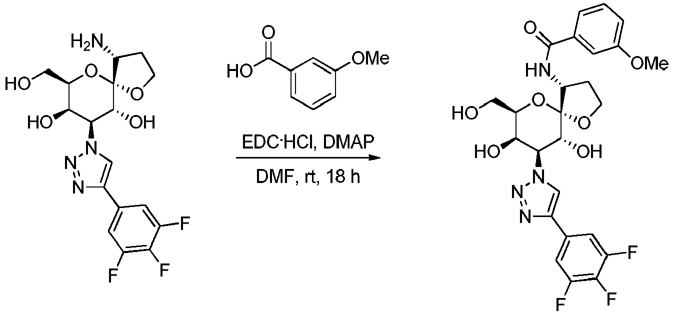

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3-methoxybenzamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then 3-methoxy benzoic acid (6 mg, 0.0396 mmol), *N-*(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (18.6 mg, yield: 94%).

ESIMS: m/z 551.33 [M+H]+; ¹H-NMR (400 MHz, CD₃OD) δ 8.50 (s, 1H), 7.63-7.55 (m, 2H), 7.40 (dt, J = 22.5, 7.8 Hz, 3H), 7.10 (d, J = 8.0 Hz, 1H), 5.05 (d, J = 11.0 Hz, 1H), 5.00-4.95 (m, 1H), 4.40 (d, J = 11.2 Hz, 1H), 4.21-4.01 (m, 4H), 3.84 (s, 2H), 3.82-3.78 (m, 1H), 3.73 (dd, J = 11.5, 4.7 Hz, 1H), 2.69-2.60 (m, 1H), 2.47-2.36 (m, 1H), 2.18 (dd, J = 19.9, 9.8 Hz, 1H)

### Example 105: Synthesis of Compound A119

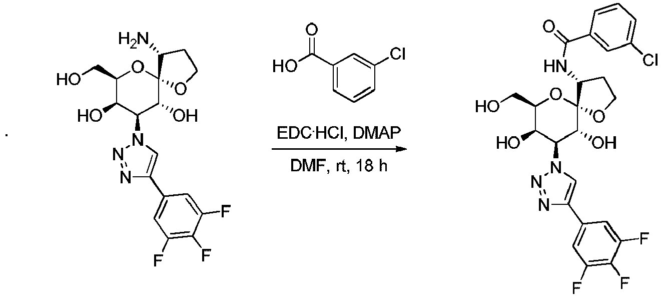

### Synthesis of 3-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then 3-methoxy benzoic acid (6 mg, 0.0396 mmol), *N-*(3-dimethylaminopropyl)-*N'*-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (16.6 mg, yield: 83%).

ESIMS: m/z 555.36 [M+H]+; ¹H-NMR (400 MHz, CD₃OD) δ 8.50 (s, 1H), 7.90 (s, 1H), 7.81 (d, J = 7.8 Hz, 1H), 7.64-7.51 (m, 3H), 7.50-7.44 (m, 1H), 5.05 (d, J = 11.2 Hz, 1H), 5.01-4.95 (m, 1H), 4.40 (d, J = 11.3 Hz, 1H), 4.18-4.14 (m, 1H), 4.10-4.03 (m, 2H), 3.80 (d, J = 7.7 Hz, 1H), 3.73 (dd, J = 11.3, 4.4 Hz, 1H), 2.64 (s, 1H), 2.41 (s, 1H), 2.23-2.09 (m, 1H).

### Example 106: Synthesis of Compound A120

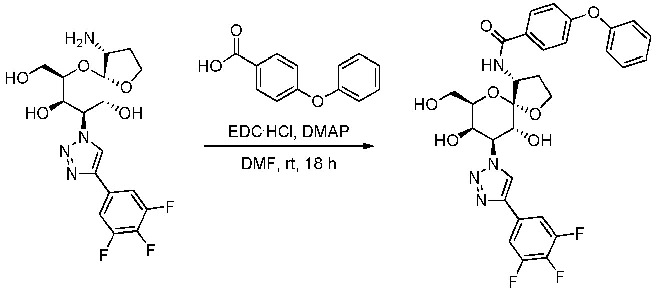

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-4-phenoxybenzamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then 4-phenoxy benzoic acid (8.5 mg, 0.0396 mmol), *N-*(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (20.0 mg, yield: 91%).

ESIMS: m/z 613.46 [M+H]+; ¹H-NMR (400 MHz, CD₃OD) δ 8.50 (s, 1H), 7.88 (d, J = 8.8 Hz, 2H), 7.60 (dd, J = 8.6, 6.8 Hz, 2H), 7.40 (t, J = 8.0 Hz, 2H), 7.18 (t, J = 7.5 Hz, 1H), 7.05 (d, J = 7.8 Hz, 2H), 7.01 (d, J = 8.8 Hz, 2H), 5.05 (dd, J = 11.2, 2.8 Hz, 1H), 4.98 (t, J = 9.3 Hz, 1H), 4.39 (d, J = 11.2 Hz, 1H), 4.15 (d, J = 6.0 Hz, 2H), 4.11-4.00 (m, 2H), 3.80 (dd, J = 11.4, 7.4 Hz, 1H), 3.72 (dd, J = 11.5, 4.8 Hz, 1H), 2.50-2.37 (m, 1H), 2.22-2.08 (m, 1H).

### Example 107: Synthesis of Compound A121

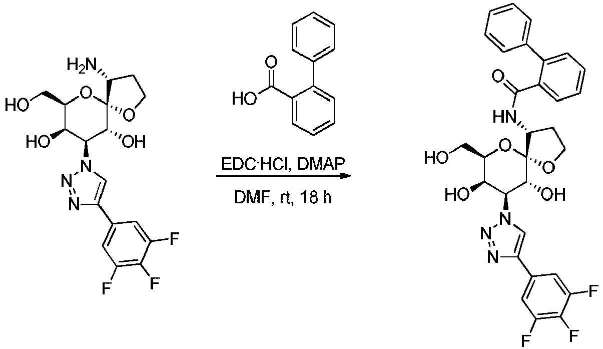

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-[1.1' biphenyl]-2-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then [1,1'-biphenyl]-2-carboxylic acid (7.9 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (11.2 mg, yield: 52%).

ESIMS: m/z 597.47 [M+H]+; ¹H-NMR (400 MHz, CD₃OD) δ 8.41 (s, 1H), 7.64-7.57 (m, 3H), 7.55-7.50 (m, 1H), 7.43 (dd, J = 14.3, 5.3 Hz, 6H), 7.37-7.32 (m, 1H), 4.97 (dd, J= 11.2, 2.7 Hz, 1H), 4.75-4.67 (m, 1H), 4.28 (d, J = 11.2 Hz, 1H), 4.10 (d, J = 1.6 Hz, 2H), 4.05-3.89 (m, 3H), 3.66 (d, J = 6.3 Hz, 1H), 2.27-2.15 (m, 1H), 1.77-1.66 (m, 1H).

### Example 108: Synthesis of Compound A122

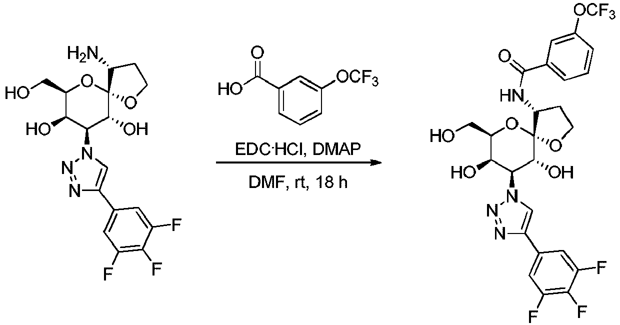

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3-(trifluoromethoxy)benzamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 3-(trifluoromethoxy) benzoic acid (8.2 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (16.9 mg, yield: 78%).

ESIMS: m/z 605.35 [M+H]+; ¹H-NMR (400 MHz, CD₃OD) δ 8.50 (s, 1H), 7.89 (d, J = 7.5 Hz, 1H), 7.80 (s, 1H), 7.65-7.56 (m, 3H), 7.47 (d, J = 8.3 Hz, 1H), 5.09-4.96 (m, 2H), 4.40 (d, J = 11.1 Hz, 1H), 4.21-4.13 (m, 2H), 4.13-4.01 (m, 2H), 3.86-3.68 (m, 2H), 2.50-2.37 (m, 1H), 2.25-2.12 (m, 1H).

### Example 109: Synthesis of Compound A123

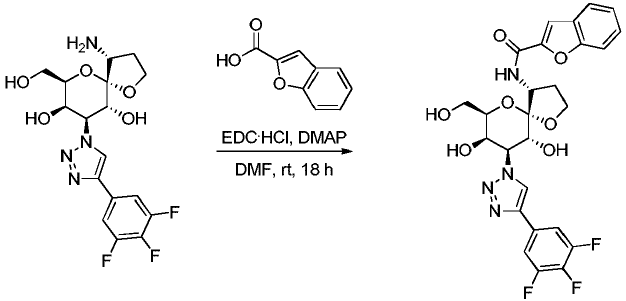

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzofuran-2-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then benzofuran-2-carboxylic acid (6.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (18.1 mg, yield: 90%).

ESIMS: m/z 561.29 [M+H]+; ¹H-NMR (400 MHz, CD₃OD) δ 8.54 (s, 1H), 7.72 (d, J = 7.8 Hz, 1H), 7.63-7.50 (m, 4H), 7.44 (t, J = 7.9 Hz, 1H), 7.31 (t, J = 7.5 Hz, 1H), 5.10-5.00 (m, 2H), 4.45 (d, J = 11.2 Hz, 1H), 4.24-4.16 (m, 2H), 4.13-4.02 (m, 2H), 3.86 (dd, J = 11.2, 7.6 Hz, 1H), 3.76 (dd, J = 11.5, 4.6 Hz, 1H), 2.50-2.38 (m, 1H), 2.30-2.17 (m, 1H).

### Example 110: Synthesis of Compound A124

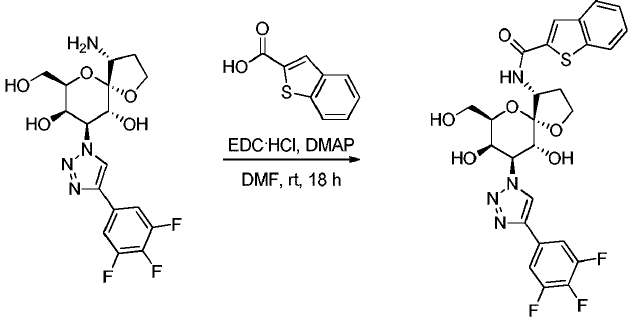

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-2-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then benzo[*b*]thiophene-2-carboxylic acid (7.1 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (20.7 mg, yield: 95%).

ESIMS: m/z 577.37 [M+H]+; ¹H-NMR (400 MHz, CD₃OD) δ 8.53 (s, 1H), 7.95 (s, 1H), 7.94-7.87 (m, 2H), 7.64-7.53 (m, 2H), 7.47-7.36 (m, 2H), 5.07 (dd, J = 11.2, 2.6 Hz, 1H), 5.02-4.96 (m, 1H), 4.44 (d, J = 11.2 Hz, 1H), 4.21-4.14 (m, 2H), 4.12-4.01 (m, 2H), 3.84 (dd, J = 11.3, 7.5 Hz, 1H), 3.76 (dd, J = 11.2, 4.8 Hz, 1H), 2.50-2.39 (m, 1H), 2.21 (dt, J = 18.9, 9.5 Hz, 1H).

### Example 111: Synthesis of Compound A125

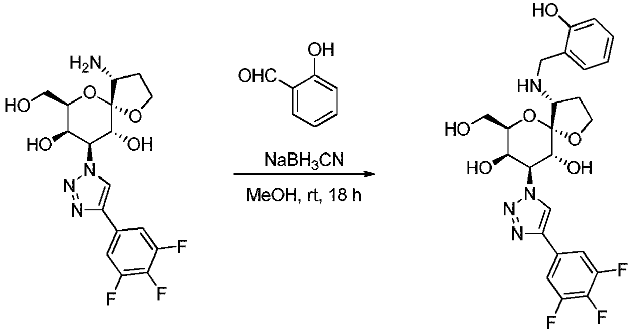

### Synthesis of (4R,5S,7R,8R,9S,10R)-4-((2-hydroxybenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 2-hydroxybenzaldehyde (8.5 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (12 mg, yield: 64%).

ESIMS: m/z 523.38 [M+H]+; ¹H-NMR (400 MHz, CD₃OD) δ 8.52 (s, 1H), 7.67-7.58 (m, 2H), 7.14 (dd, J = 17.0, 7.6 Hz, 2H), 6.79 (d, J = 7.0 Hz, 2H), 5.02 (dd, J = 11.1, 2.6 Hz, 1H), 4.46 (d, J = 11.2 Hz, 1H), 4.13-4.01 (m, 3H), 4.01-3.90 (m, 2H), 3.79-3.61 (m, 3H), 2.40-2.30 (m, 1H), 1.98 (dt, J = 18.2, 8.5 Hz, 2H).

### Example 112: Synthesis of Compound A126

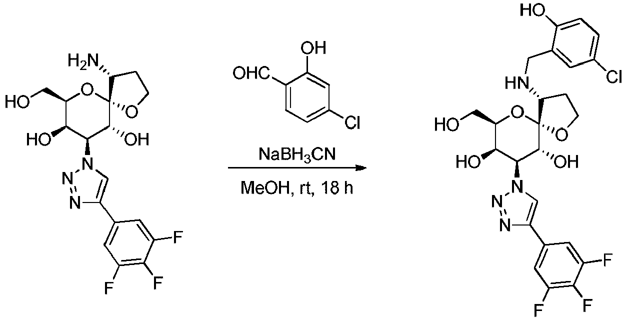

### Synthesis of (4R,5S,7R,8R,9S,10R)-4-((5-chloro-2-hydroxybenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 4-chloro-2-hydroxybenzaldehyde (8.5 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (13.7 mg, yield: 69%).

ESIMS: m/z 557.28 [M+H]+; ¹H-NMR (400 MHz, CD₃OD) δ 8.52 (s, 1H), 7.62 (dd, J = 8.6, 6.7 Hz, 2H), 7.20 (d, J = 2.3 Hz, 1H), 7.10 (dd, J = 8.6, 2.5 Hz, 1H), 6.76 (d, J = 8.6 Hz, 1H), 5.02 (dd, J = 11.2, 2.7 Hz, 1H), 4.46 (d, J = 11.2 Hz, 1H), 4.14-4.01 (m, 3H), 3.97-3.89 (m, 2H), 3.72 (ddd, J = 16.7, 11.5, 6.2 Hz, 2H), 3.63 (t, J = 8.7 Hz, 1H), 2.41-2.31 (m, 1H), 2.04-1.93 (m, 2H).

### Example 113: Synthesis of Compound A127

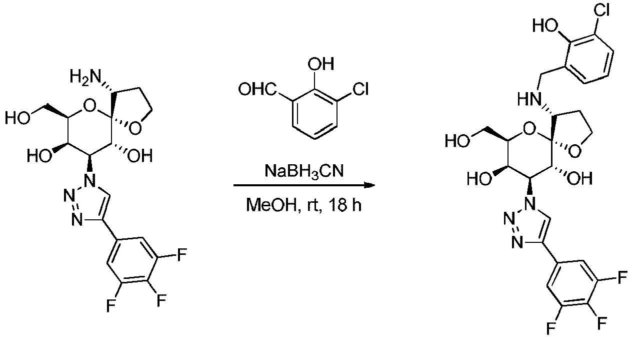

### Synthesis of (4R,5S,7R,8R,9S,10R)-4-((3-chloro-2-hydroxybenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 3-chloro-2-hydroxybenzaldehyde (8.5 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (15 mg, yield: 75%).

ESIMS: m/z 557.28 [M+H]+; ¹H-NMR (400 MHz, CD₃OD) δ 8.56 (s, 1H), 7.63 (dd, J = 8.6, 6.7 Hz, 2H), 7.26 (d, J = 8.0 Hz, 1H), 7.11 (d, J = 7.4 Hz, 1H), 6.80-6.74 (m, 1H), 5.04 (dd, J = 11.2, 2.8 Hz, 1H), 4.49 (d, J = 11.1 Hz, 1H), 4.18 (d, J = 13.5 Hz, 1H), 4.14-4.07 (m, 3H), 3.97 (dd, J = 15.7, 7.6 Hz, 1H), 3.80-3.68 (m, 3H), 2.44-2.35 (m, 1H), 2.13-2.03 (m, 2H).

### Example 114: Synthesis of Compound A128

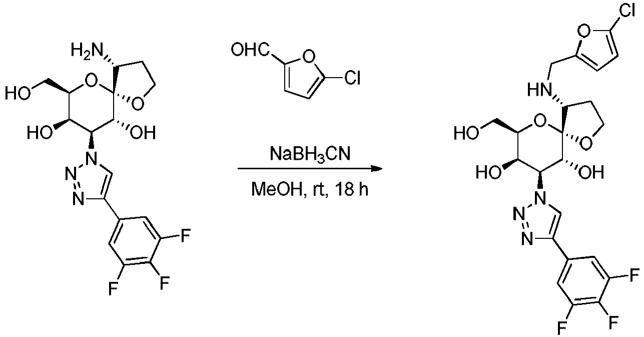

### Synthesis of (4R,5S,7R,8R,9S,10R)-4-(((5-chlorofuran-2-yl) methyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 5-chlorofuran-2-carbaldehyde (7.0 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (13.2 mg, yield: 69%).

ESIMS: m/z 531.32 [M+H]+; ¹H-NMR (400 MHz, CD₃OD) δ 8.51 (s, 1H), 7.68-7.57 (m, 2H), 6.37 (d, J = 2.8 Hz, 1H), 6.21 (d, J = 3.0 Hz, 1H), 5.48 (s, 1H), 5.02 (dd, J = 11.2, 2.8 Hz, 1H), 4.44 (d, J = 11.1 Hz, 1H), 4.14-4.02 (m, 3H), 3.93 (dd, J = 16.7, 8.5 Hz, 1H), 3.85 (d, J = 3.0 Hz, 1H), 3.79-3.66 (m, 2H), 3.60-3.53 (m, 1H), 2.73-2.59 (m, 1H), 2.35-2.20 (m, 1H), 1.98-1.84 (m, 1H).

### Example 115: Synthesis of Compound A129

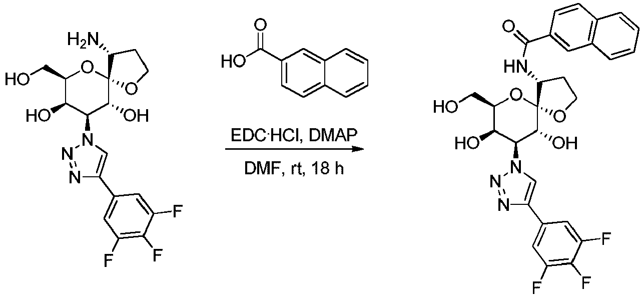

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 2-naphtho acid (7.9 mg, 0.0396 mmol), *N-*(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (17.8 mg, yield: 87%).

ESIMS: m/z 571.33 [M+H]+; ¹H-NMR (400 MHz, CD₃OD) δ 8.53 (s, 1H), 8.45 (s, 1H), 8.02-7.90 (m, 4H), 7.65-7.54 (m, 4H), 5.12-5.04 (m, 2H), 4.47 (d, J = 11.2 Hz, 1H), 4.25-4.17 (m, 2H), 4.15-4.05 (m, 2H), 3.87 (dd, J = 11.4, 7.5 Hz, 1H), 3.77 (dd, J = 11.4, 4.8 Hz, 1H), 2.53-2.44 (m, 1H), 2.30-2.19 (m, 1H).

### Example 116: Synthesis of Compound A130

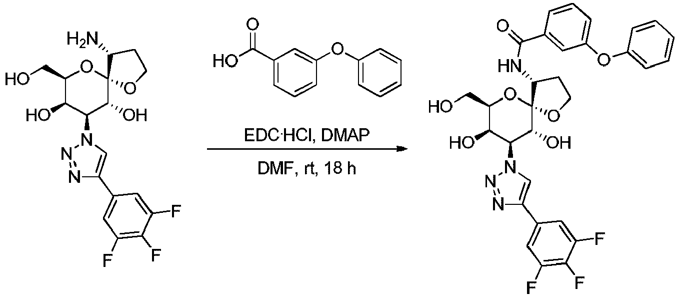

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3-phenoxybenzamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 3-phenoxy benzoic acid (8.5 mg, 0.0396 mmol), *N-*(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (17.3 mg, yield: 79%).

ESIMS: m/z 613.41 [M+H]+; ¹H-NMR (400 MHz, CD₃OD) δ 8.51 (s, 1H), 7.62 (dd, J = 15.2, 8.1 Hz, 3H), 7.53 (s, 1H), 7.47 (t, J = 7.9 Hz, 1H), 7.38 (t, J = 7.7 Hz, 2H), 7.19-7.12 (m, 2H), 7.03 (d, J = 8.5 Hz, 2H), 5.06 (dd, J = 11.2, 2.5 Hz, 1H), 4.98 (t, J = 9.4 Hz, 1H), 4.40 (d, J = 11.2 Hz, 1H), 4.20-4.13 (m, 2H), 4.13-4.01 (m, 2H), 3.82-3.76 (m, 1H), 3.72 (dd, J = 11.4, 4.7 Hz, 1H), 2.47-2.37 (m, 1H), 2.22-2.11 (m, 1H).

### Example 117: Synthesis of Compound A131

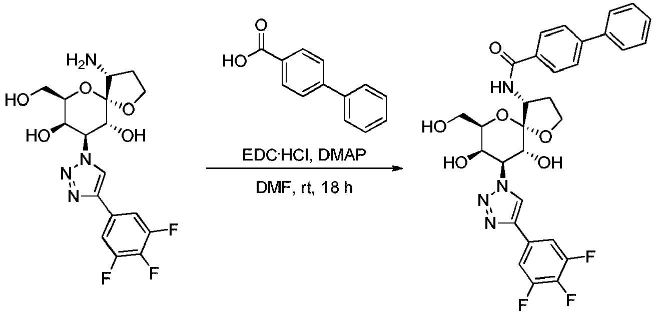

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-[1.1' biphenyl]-4-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then [1,1'-biphenyl]-4-carboxylic acid (7.8 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (18.5 mg, yield: 86%).

ESIMS: m/z 597.41 [M+H]+; ¹H-NMR (400 MHz, CD₃OD) δ 8.53 (s, 1H), 7.98 (d, J = 8.2 Hz, 2H), 7.74 (d, J = 8.3 Hz, 2H), 7.67 (d, J = 7.5 Hz, 2H), 7.61 (dd, J = 8.2, 6.9 Hz, 2H), 7.46 (t, J = 7.6 Hz, 2H), 7.41-7.35 (m, 1H), 5.10-5.00 (m, 2H), 4.45 (d, J = 11.2 Hz, 1H), 4.22-4.16 (m, 2H), 4.14-4.05 (m, 2H), 3.85 (dd, J = 11.4, 7.5 Hz, 1H), 3.76 (dd, J = 11.4, 4.7 Hz, 1H), 2.52-2.40 (m, 1H), 2.27-2.14 (m, 1H).

### Example 118: Synthesis of Compound A132

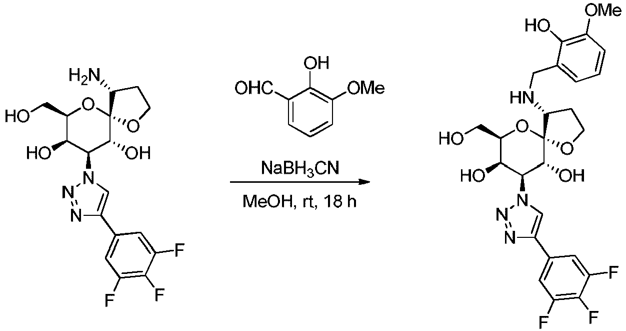

### Synthesis of (4R,5S,7R,8R,9S,10R)-4-((2,3-hydroxy-3-methoxybenzyl)amino)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 2-hydroxy-3-methoxybenzaldehyde (6.6 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (8.3 mg, yield: 42%).

ESIMS: m/z 553.43 [M+H]+; ¹H-NMR (400 MHz, CD₃OD) δ 8.53 (s, 1H), 7.64 (dd, J = 8.4, 6.7 Hz, 2H), 6.93 (dd, J = 7.7, 1.2 Hz, 1H), 6.87-6.79 (m, 2H), 5.03 (dd, J = 11.2, 2.7 Hz, 1H), 4.49 (d, J = 11.3 Hz, 1H), 4.18-3.93 (m, 6H), 3.85 (s, 3H), 3.77-3.67 (m, 3H), 2.38 (s, 1H), 2.02 (d, J = 6.5 Hz, 2H).

### Example 119: Synthesis of Compound A133

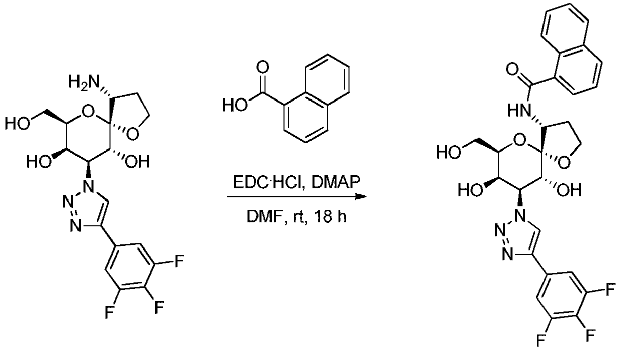

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 1-naphtho acid (7.9 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (7.5 mg, yield: 37%).

ESIMS: m/z 571.39 [M+H]+; ¹H-NMR (400 MHz, CD₃OD) δ 8.57 (s, 1H), 8.39 (d, J = 8.2 Hz, 1H), 8.00 (d, J = 8.3 Hz, 1H), 7.93 (d, J = 8.0 Hz, 1H), 7.73 (d, J = 6.6 Hz, 1H), 7.67-7.52 (m, 5H), 5.15-5.07 (m, 2H), 4.64 (d, J = 11.2 Hz, 1H), 4.20-4.05 (m, 4H), 3.82 (dd, J = 11.5, 7.1 Hz, 1H), 3.73 (dd, J = 11.4, 4.8 Hz, 1H), 2.54-2.46 (m, 1H), 2.24-2.15 (m, 1H).

### Example 120: Synthesis of Compound A134

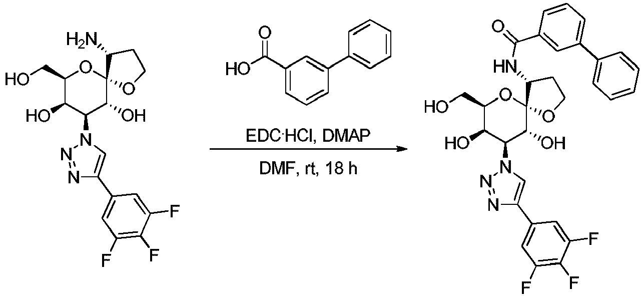

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-[1.1' biphenyl]-3-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then [1,1'-biphenyl]-3-carboxylic acid (7.8 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (17.3 mg, yield: 84%).

ESIMS: m/z 597.41 [M+H]+; ¹H-NMR (400 MHz, CD₃OD) δ 8.52 (s, 1H), 8.14 (s, 1H), 7.85 (dd, J = 15.2, 7.6 Hz, 2H), 7.70 (d, J = 7.3 Hz, 2H), 7.64-7.56 (m, 2H), 7.47 (t, J = 7.6 Hz, 2H), 7.37 (t, J = 7.3 Hz, 1H), 5.10-5.02 (m, 2H), 4.44 (d, J = 11.4 Hz, 1H), 4.23-4.16 (m, 2H), 4.13-4.04 (m, 2H), 3.84 (dd, J = 11.4, 7.6 Hz, 1H), 3.75 (dd, J = 11.4, 4.8 Hz, 1H), 2.51-2.41 (m, 1H), 2.29-2.16 (m, 2H).

### Example 121: Synthesis of Compound A135

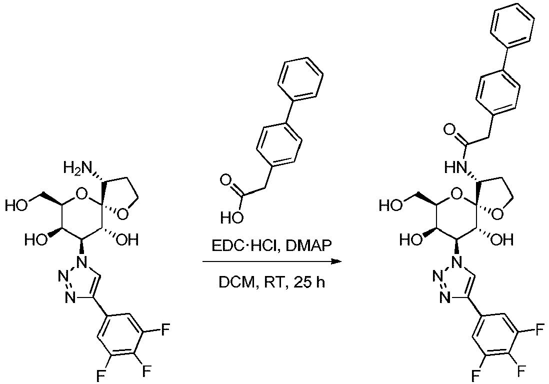

### Synthesis of 2-([1,1'-biphenyl]-4-yl)-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)acetamide

The title compound (13.1 mg, yield: 89%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 611.46 [M+H]+; ¹H-NMR (400 MHz, CD₃OD) δ = 8.34 (S, 1H), 7.59 (t, J = 7.2 Hz, 4H), 7.54-7.50 (m, 2H), 7.47 (d, J = 7.8 Hz, 2H), 7.37 (t, J = 7.4 Hz, 2H), 7.28 (t, J = 7.2 Hz, 1H), 5.01 (dd, J = 11.2, 2.5 Hz, 1H), 4.78 (t, J = 9.2 Hz, 1H), 4.29 (d, J = 11.0 Hz, 1H), 4.12 (t, J = 6.1 Hz, 3H), 4.01 (q, J = 8.1 Hz, 1H), 3.83-3.75 (m, 2H), 3.70 (d, J = 14.1 Hz, 1H), 3.61 (d, J = 14.1 Hz, 1H), 2.37-2.31 (m, 1H), 2.11-2.01 (m, 1H)

### Example 122: Synthesis of Compound A136

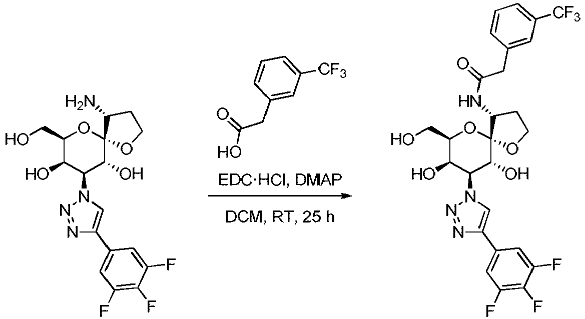

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(3-(trifluoromethyl)phenyl)acetamide

The title compound (8.3 mg, yield: 57%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 603.40 [M+H]+; ¹H-NMR (400 MHz, CD₃OD) δ = 8.42 (S, 1H), 7.67-7.51 (m, 6H), 5.02 (dd, J = 11.2, 2.2 Hz, 1H), 4.75 (t, J = 9.2 Hz, 1H), 4.32 (d, J = 11.0 Hz, 1H), 4.14-4.11 (m, 3H), 4.01 (q, J = 8.2 Hz, 1H), 3.82-3.67 (m, 4H), 2.37-2.33 (m, 1H), 2.09-1.99 (m, 1H)

### Example 123: Synthesis of Compound A137

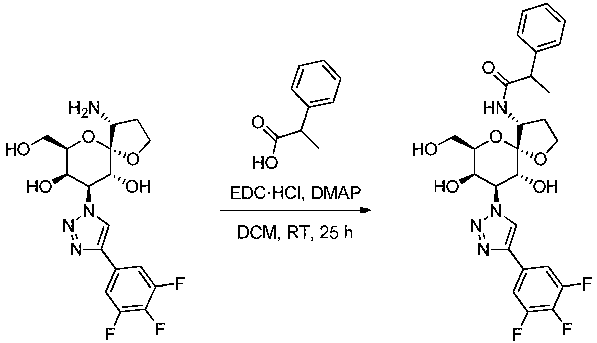

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-phenylpropanamide

The title compound (1:1 stereoisomer mixture, 13.0 mg, yield: 99%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 549.40 [M+H]+; ¹H-NMR (400 MHz, CD₃OD) δ = 8.51 (S, 1H), 8.26 (S, 1H), 7.67-7.62 (m, 2H), 7.57 (dd, J = 8.6, 6.7 Hz, 2H), 7.45 (d, J = 7.4 Hz, 2H), 7.38-7.30 (m, 6H), 7.27-7.22 (m, 2H), 5.02 (dd, J = 11.3, 2.7 Hz, 1H), 4.97 (dd, J = 11.0, 2.7 Hz, 1H), 4.77-4.69 (m, 2H), 4.37 (d, J = 11.3 Hz, 1H), 4.17 (d, J = 11.0 Hz, 1H), 4.13-4.06 (m, 6H), 4.03-3.94 (m, 2H), 3.82-3.70 (m, 6H), 2.35-2.31 (m, 1H), 2.28-2.23 (m, 1H), 2.09-1.99 (m, 2H), 1.95-1.85 (m, 1H), 1.51 (S, 3H), 1.49 (S, 3H)

### Example 124: Synthesis of Compound A138

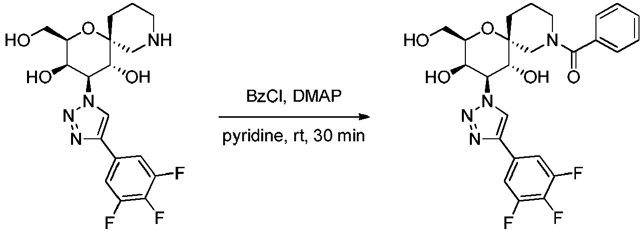

### Synthesis of ((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(phenyl)methanone

(2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) was dissolved in 0.6 mL of pyridine, and then benzoyl chloride (3.3 µL, 0.029 mmol) and 4-dimethylaminopyridine (1.0 mg, excessive) were added thereto. This mixture was stirred at room temperature for 30 minutes. The reaction mixture was concentrated under reduced pressure, and was azeotroped with n-heptane (10 mL x 3 times). The remaining concentrate was subjected to silica gel column chromatography with 0% to 10% methanol/dichloromethane to obtain the title compound as a white solid (6.2 mg, yield: 50%).

ESIMS: m/z 519.32 [M+H]+; ¹H-NMR (400 MHz, CD₃OD) δ = 8.51 (S, 1H), 7.66-7.62 (m, 2H), 7.48-7.42 (m, 5H), 5.18 (d, J = 14.1 Hz, 1H), 5.05 (dd, J = 11.3, 2.0 Hz, 1H), 4.29 (d, J = 11.3 Hz, 1H), 4.19-4.13 (m, 2H), 3.72-3.65 (m, 3H), 3.16-3.05 (m, 2H), 2.24-2.16 (m, 1H), 2.09-2.01 (m, 2H), 1.83 (d, J = 12.9 Hz, 1H)

### Example 125: Synthesis of Compound A139

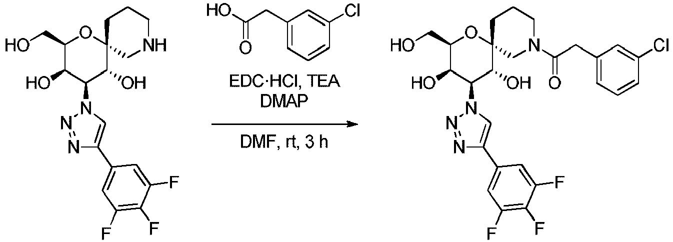

### Synthesis of 2-(3-chlorophenyl)-1-((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)ethanone

(2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) was dissolved in 1.0 mL of *N*,*N*-dimethylformamide, and then 2-(3-chlorophenyl)acetic acid (4.9 mg, 0.029 mmol), *N*-(3-dimethylaminopropyl)-*N*'-ethylcarbodiimide hydrochloride (15.0 mg, 0.078 mmol), triethylamine (15 µL, 0.063 mmol), and 4-dimethylaminopyridine (1 mg, 0.008 mmol) were added dropwise at room temperature, followed by stirring for 3 hours. The reaction solution was diluted with dichloromethane (5 mL), and then washed with a saturated aqueous sodium hydrogen carbonate solution (3 mL x 2). The organic layer was dried over anhydrous sodium sulfate, filtered, and then concentrated under reduced pressure. The concentrate was subjected to silica gel column chromatography with 0% to 10% methanol/dichloromethane to synthesize the title compound as a white solid (6.5 mg, yield: 48%).

ESIMS: m/z 567.27 [M+H]+; ¹H-NMR (400 MHz, CD₃OD) δ = 8.47 (S, 1H), 7.65-7.61 (m, 2H), 7.35-7.24 (m, 4H), 5.07 (d, J = 14.5 Hz, 1H), 4.97 (dd, J = 11.5, 2.5 Hz, 1H), 4.22 (d, J = 11.7 Hz, 1H), 4.15 (d, J = 2.3 Hz, 1H), 4.06 (t, J = 6.3 Hz, 1H), 4.00 (d, J = 13.7 Hz, 1H), 3.88 (d, J = 15.3 Hz, 1H), 3.79 (d, J = 15.7 Hz, 1H), 3.66-3.56 (m, 2H), 3.05 (td, J = 13.1, 2.1 Hz, 1H), 2.95 (d, J = 14.1 Hz, 1H), 2.15-2.01 (m, 2H), 1.84-1.72 (m, 2H)

### Example 126: Synthesis of Compound A140

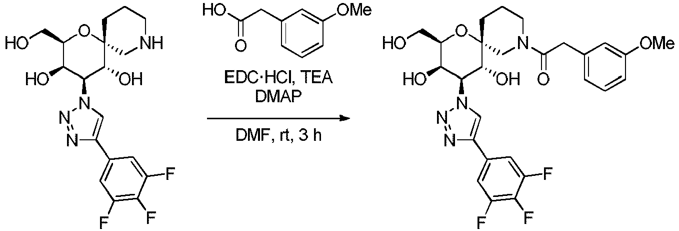

### Synthesis of 1-((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)-2-(3-methoxyphenyl)ethanone

The title compound (3.5 mg, yield: 26%) as a white solid was synthesized from (2*R*,3*R*,4*S*,5*R,*6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 2-(3-chlorophenyl)-1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)ethanone.

ESIMS: m/z 563.30 [M+H]+; ¹H-NMR (400 MHz, CD₃OD) δ = 8.47 (S, 1H), 7.65-7.61 (m, 2H), 7.24 (t, J = 8.0 Hz, 1H), 6.89 (d, J = 6.3 Hz, 2H), 6.82 (dd, J = 8.0, 1.8 Hz, 1H), 5.07 (d, J = 14.1 Hz, 1H), 4.97 (dd, J = 11.3, 2.7 Hz, 1H), 4.21 (d, J = 11.3 Hz, 1H), 4.16 (d, J = 1.6 Hz, 1H), 4.11-4.00 (m, 2H), 3.84 (d, J = 15.3 Hz, 1H), 3.79 (S, 3H), 3.76 (d, J = 15.3 Hz, 1H), 3.68-3.56 (m, 2H), 3.02 (td, J = 13.3, 2.2 Hz, 1H), 2.94 (d, J = 14.1 Hz, 1H), 2.15-2.01 (m, 2H), 1.83-1.70 (m, 2H)

### Example 127: Synthesis of Compound A141

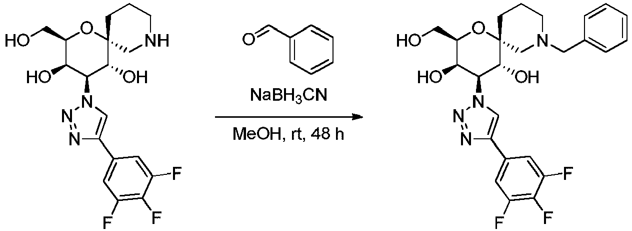

### Synthesis of (2R,3R,4S,5R,6R)-8-benzyl-2-(hydroxymethyl)-4-(4-(3,4,5-trifluorophenyl)-1H-1,2,3-triazol-1-yl)-1-oxa-8-azaspiro[5.5]undecane-3,5-diol

(2*R*,3*R*,4*S*,5*R*,6*R*)-2-(hydroxymethyl)-4-(4-(3,4,5-trifluorophenyl)-1*H*-1,2,3 -triazol-1-yl)-1-oxa-8-azaspiro[5.5]undecane-3,5-diol (15.0 mg, 0.036 mmol) and benzaldehyde (50 µL, excessive) were dissolved in 0.7 mL of methanol, and sodium cyanoborohydride (10.0 mg, excessive) was added thereto. The reaction mixture was stirred at room temperature for about 48 hours. The reaction solution was diluted with dichloromethane (5 mL), and then washed with a saturated aqueous sodium hydrogen carbonate solution (3 mL x 2). The organic layer was dried over anhydrous sodium sulfate, filtered, and then concentrated under reduced pressure. The concentrate was subjected to silica gel column chromatography with 0% to 7% methanol/dichloromethane (containing a 25% aqueous ammonium hydroxide solution at a concentration of 1%) to synthesize the title compound as a white solid (2.8 mg, yield: 23%).

ESIMS: m/z 505.34 [M+H]+; ¹H-NMR (400 MHz, CD₃OD) δ = 8.44 (S, 1H), 7.63-7.60 (m, 2H), 7.43-7.33 (m, 5H), 4.75 (dd, J = 11.3, 2.7 Hz, 1H), 4.22 (d, J = 11.3 Hz, 1H), 3.99 (d, J = 2.0 Hz, 1H), 3.87-3.64 (m, 5H), 3.16 (d, J = 11.0 Hz, 1H), 2.88 (d, J = 9.0 Hz, 1H), 2.72 (d, J = 11.7 Hz, 1H), 2.39 (t, J = 10.4 Hz, 1H), 2.09-1.93 (m, 2H), 1.78-1.66 (m, 2H)

### Example 128: Synthesis of Compound A142

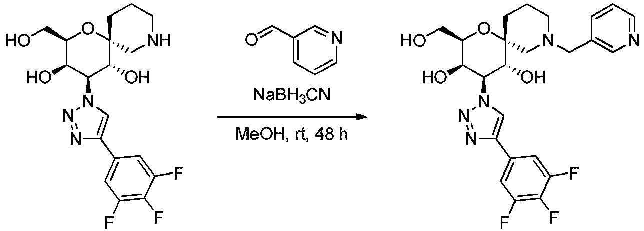

### Synthesis of (2R,3R,4S,5R,6R)-2-(hydroxymethyl)-8-(pyridin-3-ylmethyl)-4-(4-(3,4,5-trifluorophenyl)-1H-1,2,3-triazol-1-yl)-1-oxa-8-azaspiro[5.5]undecane-3,5-diol

The title compound (6.4 mg, yield: 53%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (15.0 mg, 0.033 mmol) in a similar manner to the preparation method of (2*R*,3*R*,4*S*,5*R*,6*R*)-8-benzyl-2-(hydroxymethyl)-4-(4-(3,4,5-trifluorophenyl)-1*H*-1,2,3-triazol-1-yl)-1-oxa-8-azaspiro[5.5]undecane-3,5-diol.

ESIMS: m/z 506.35 [M+H]+; ¹H-NMR (400 MHz, CD₃OD) δ = 8.57 (S, 1H), 8.47 (d, J = 4.3 Hz, 1H), 8.44 (S, 1H), 7.93 (d, J = 7.8 Hz, 1H), 7.61 (t, J = 7.6 Hz, 2H), 7.45 (dd, J = 7.8, 5.1 Hz, 1H), 4.81 (dd, J = 11.7, 2.7 Hz, 1H), 4.19 (d, J = 11.7 Hz, 1H), 4.02 (d, J = 2.3 Hz, 1H), 3.81-3.63 (m, 5H), 3.17 (d, J = 12.5 Hz, 1H), 2.83 (d, J = 10.6 Hz, 1H), 2.54 (d, J = 12.5 Hz, 1H), 2.21 (t, J = 10.4 Hz, 1H), 2.09-2.01 (m, 1H), 1.97-1.90 (m, 1H), 1.70-1.62 (m, 2H)

### Example 129: Synthesis of Compound A143

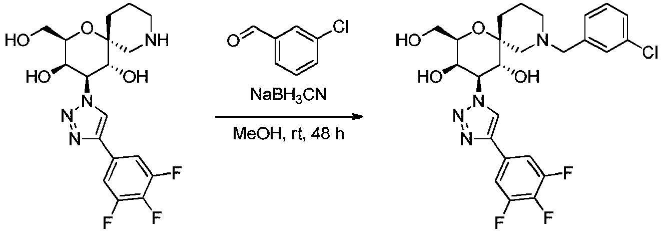

### Synthesis of (2R,3R,4S,5R,6R)-8-(3-chlorobenzyl)-2-(hydroxymethyl)-4-(4-(3,4,5-trifluorophenyl)-1H-1,2,3-triazol-1-yl)-1-oxa-8-azaspiro[5.5]undecane-3,5-diol

The title compound (9.0 mg, yield: 70%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (15.0 mg, 0.033 mmol) in a similar manner to the preparation method of (2*R*,3*R*,4*S*,5*R*,6*R*)-8-benzyl-2-(hydroxymethyl)-4-(4-(3,4,5-trifluorophenyl)-1*H*-1,2,3-triazol-1-yl)-1-oxa-8-azaspiro[5.5]undecan-3,5-diol.

ESIMS: m/z 539.30 [M+H]+; ¹H-NMR (400 MHz, CD₃OD) δ = 8.44 (S, 1H), 7.63-7.59 (m, 2H), 7.43 (S, 1H), 7.35-7.28 (m, 3H), 4.80 (dd, J = 11.3, 2.3 Hz, 1H), 4.21 (d, J = 11.3 Hz, 1H), 4.03 (d, J = 2.3 Hz, 1H), 3.82-3.57 (m, 5H), 3.10 (d, J = 12.5 Hz, 1H), 2.77 (d, J = 10.6 Hz, 1H), 2.59 (d, J = 12.5 Hz, 1H), 2.22 (t, J = 10.0 Hz, 1H), 2.10-2.01 (m, 1H), 1.97-1.90 (m, 1H), 1.69-1.67 (m, 2H)

### Example 130: Synthesis of Compound A144

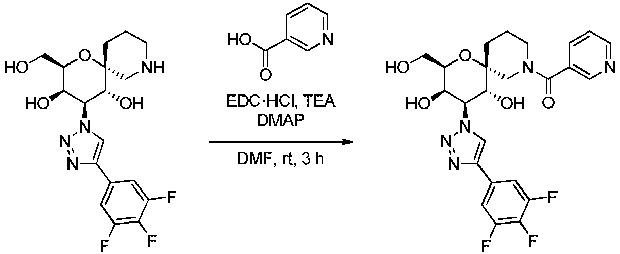

### Synthesis of ((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(pyridin-3-yl)methanone

The title compound (2.1 mg, yield: 17%) as a white solid was synthesized from (2*R*,3*R*,4*S*,5*R*,*6R*)-2-(hydroxymethyl)-4-(4-(3,4,5-trifluorophenyl)-1*H*-1,2,3-triazol-1-yl)-1-oxa-8-azaspiro[5.5]undecane-3,5-diol (15.0 mg, 0.036 mmol) in a similar manner to the preparation method of 2-(3-chlorophenyl)-1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)ethanone.

ESIMS: m/z 520.27 [M+H]+; ¹H-NMR (400 MHz, CD₃OD) δ = 8.64 (S, 2H), 8.50 (S, 1H), 7.93 (d, J = 7.8 Hz, 1H), 7.66-7.53 (m, 3H), 5.19 (d, J = 14.1 Hz, 1H), 5.05 (dd, J = 11.5, 2.5 Hz, 1H), 4.30 (d, J = 11.3 Hz, 1H), 4.18-4.15 (m, 2H), 3.71-3.70 (m, 2H), 3.63-3.59 (m, 2H), 3.18 (d, J = 14.5 Hz, 1H), 2.24-2.01 (m, 3H), 1.85 (d, J = 12.1 Hz, 1H)

### Example 131: Synthesis of Compound A145

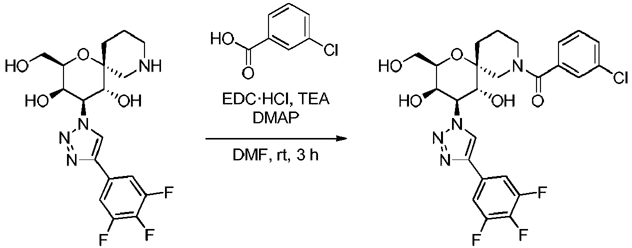

### Synthesis of (3-chlorophenyl)((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)methanone

The title compound (3.5 mg, yield: 26%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (15.0 mg, 0.036 mmol) in a similar manner to the preparation method of 2-(3-chlorophenyl)-1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)ethanone.

ESIMS: m/z 553.29 [M+H]+; ¹H-NMR (400 MHz, CD₃OD) δ 8.50 (s, 1H), 7.66-7.62 (m, 2H), 7.48-7.44 (m, 4H), 7.37 (d, J = 6.7 Hz, 1H), 5.15 (d, J = 14.1 Hz, 1H), 5.04 (dd, J = 11.2, 2.2 Hz, 1H), 4.29 (d, J = 11.3 Hz, 1H), 4.18-4.15 (m, 2H), 3.71 (d, J = 5.9 Hz, 2H), 3.63-3.59 (m, 1H), 3.16-3.08 (m, 2H), 2.21-2.15 (m, 1H), 2.08-2.01 (m, 2H), 1.85-1.82 (m, 1H)

### Example 132: Synthesis of Compound A146

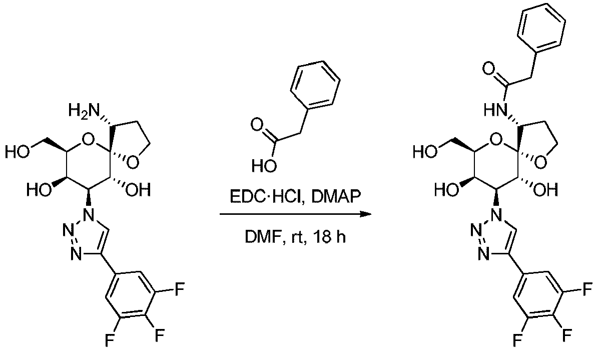

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-phenylacetamide

The title compound (10.8 mg, yield: 84%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 535.27 [M+H]+; ¹H-NMR (400 MHz, CD₃OD) δ = 8.42 (S, 1H), 7.62-7.58 (m, 2H), 7.38-7.31 (m, 4H), 7.25 (t, J = 7.0 Hz, 1H), 5.01 (dd, J = 11.3, 2.7 Hz, 1H), 4.75 (t, J = 9.2 Hz, 1H), 4.32 (d, J= 11.0 Hz, 1H), 4.13-4.10 (m, 3H), 4.00 (q, J = 8.1 Hz, 1H), 3.81-3.73 (m, 2H), 3.61 (q, J = 13.6 Hz, 2H), 2.37-2.29 (m, 1H), 2.08-1.98 (m, 1H)

### Example 133: Synthesis of Compound A147

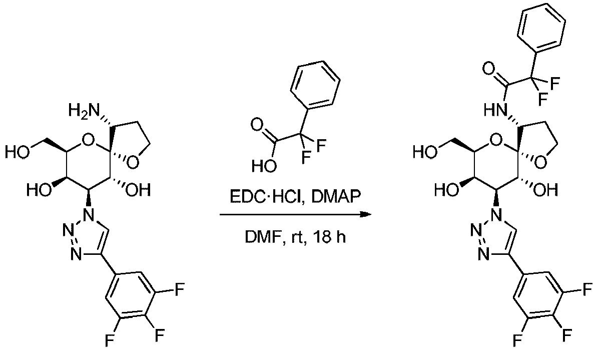

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2,2-difluoro-2-phenylacetamide

The title compound (4.2 mg, yield: 31%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 571.24 [M+H]+; ¹H-NMR (400 MHz, CD₃OD) δ = 8.38 (S, 1H), 7.73 (d, J = 7.0 Hz, 2H), 7.61-7.57 (m, 2H), 7.54-7.49 (m, 3H), 5.03 (dd, J = 11.3, 2.7 Hz, 1H), 4.32 (d, J = 11.3 Hz, 1H), 4.18-4.10 (m, 3H), 4.03 (q, J = 7.8 Hz, 1H), 3.82 (dd, J = 11.3, 7.8 Hz, 1H), 3.74 (dd, J = 11.3, 4.3 Hz, 1H), 2.38-2.32 (m, 1H), 2.23-2.13 (m, 1H)

### Example 134: Synthesis of Compound A148

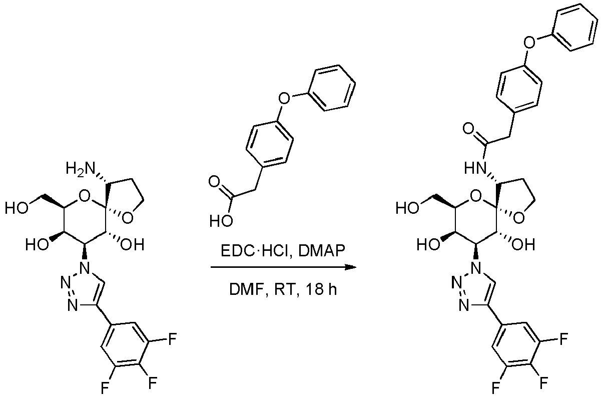

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(4-phenoxyphenyl)acetamide

The title compound (6.7 mg, yield: 45%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 627.32 [M+H]+; ¹H-NMR (400 MHz, CD₃OD) δ = 8.42 (S, 1H), 7.61-7.58 (m, 2H), 7.37 (d, J = 8.2 Hz, 2H), 7.30 (t, J = 7.8 Hz, 2H), 7.06 (t, J = 7.4 Hz, 1H), 6.97-6.95 (m, 4H), 5.01 (dd, J = 11.3, 2.7 Hz, 1H), 4.76 (t, J = 9.4 Hz, 1H), 4.31 (d, J = 11.3 Hz, 1H), 4.13-4.10 (m, 3H), 4.01 (q, J = 8.2 Hz, 1H), 3.81-3.73 (m, 2H), 3.60 (q, J = 15.8 Hz, 2H), 2.37-2.30 (m, 1H), 2.09-1.99 (m, 1H)

### Example 135: Synthesis of Compound A149

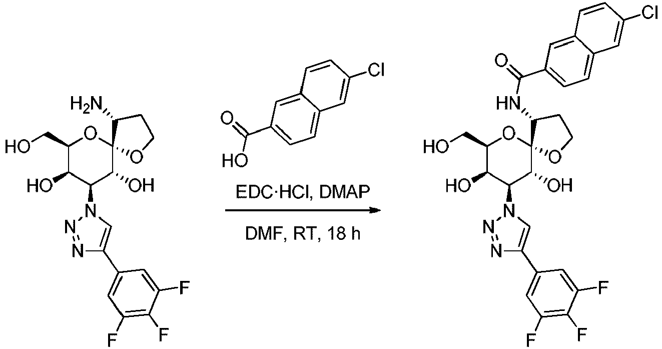

### Synthesis of 6-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

The title compound (8.1 mg, yield: 57%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 589.26 [M+H]+; ¹H-NMR (400 MHz, CD₃OD) δ = 8.53 (S, 1H), 8.48 (S, 1H), 8.07 (dd, J = 9.0, 5.5 Hz, 1H), 7.97 (q, J = 9.1 Hz, 2H), 7.62-7.59 (m, 3H), 7.40 (td, J = 8.7, 2.5 Hz, 1H), 5.11-5.05 (m, 2H), 4.47 (d, J = 11.3 Hz, 1H), 4.20-4.19 (m, 2H), 4.13 (d, J = 2.0 Hz, 1H), 4.09 (q, J = 8.0 Hz, 1H), 3.86 (dd, J = 11.3, 7.4 Hz, 1H), 3.77 (dd, J = 11.3, 4.7 Hz, 1H), 2.52-2.45 (m, 1H), 2.29-2.19 (m, 1H)

### Example 136: Synthesis of Compound A150

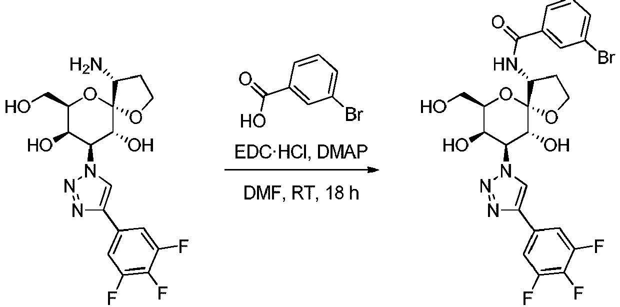

### Synthesis of 3-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzamide

The title compound (9.9 mg, yield: 69%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 599.22 [M+H]+; ¹H-NMR (400 MHz, CD₃OD) δ = 8.52 (S, 1H), 8.07 (S, 1H), 7.87 (d, J = 7.8 Hz, 1H), 7.72 (d, J = 7.8 Hz, 1H), 7.63-7.60 (m, 2H), 7.42 (t, J = 8.0 Hz, 1H), 5.07 (dd, J = 11.3, 2.7 Hz, 1H), 5.00 (t, J = 9.4 Hz, 1H), 4.41 (d, J = 11.3 Hz, 1H), 4.20-4.15 (m, 2H), 4.11 (d, J = 1.6 Hz, 1H), 4.06 (q, J = 8.2 Hz, 1H), 3.83 (dd, J = 11.2, 7.6 Hz, 1H), 3.74 (dd, J = 11.3, 4.7 Hz, 1H), 2.47-2.40 (m, 1H), 2.24-2.14 (m, 1H)

### Example 137: Synthesis of Compound A151

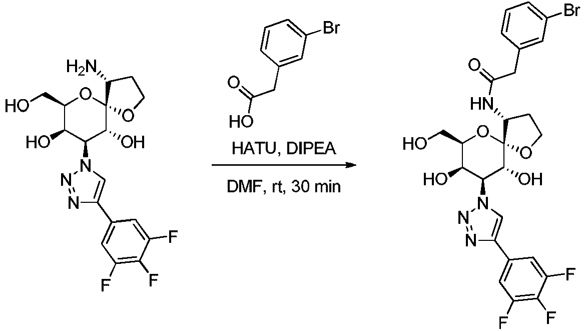

### Synthesis of 2-(3-bromophenyl)-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)acetamide

The title compound (9.6 mg, yield: 65%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R,*5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 613.14 [M+H]+; ¹H-NMR(400 MHz, CD₃OD) δ 8.43 (s, 1H), 7.62-7.59 (m, 2H), 7.55 (s, 1H), 7.43 (d, J = 8.2 Hz, 1H), 7.36 (d, J = 7.4 Hz, 1H), 7.26 (t, J = 7.8 Hz, 1H), 5.01 (dd, J = 11.0, 2.7 Hz, 1H), 4.74 (t, J = 9.4 Hz, 1H), 4.31 (d, J = 11.3 Hz, 1H), 4.13-4.11 (m, 3H), 4.01 (q, J = 8.1 Hz, 1H), 3.83-3.74 (m, 2H), 3.61 (q, J = 13.7 Hz, 2H), 2.37-2.30 (m, 1H), 2.09-1.99 (m, 1H)

### Example 138: Synthesis of Compound A152

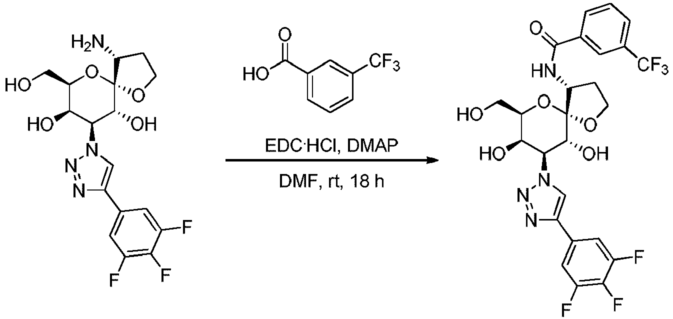

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3-(trifluoromethoxy)benzamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 3-(trifluoromethoxy)benzoic acid (7.5 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (17.3 mg, yield: 84%).

ESIMS: m/z 589.26 [M+H]+; ¹H-NMR (400 MHz, CD₃OD) δ 8.52 (s, 1H), 8.21 (s, 1H), 8.16 (d, J = 8.0 Hz, 1H), 7.87 (d, J = 7.6 Hz, 1H), 7.71 (t, J = 7.8 Hz, 1H), 7.61 (dd, J = 8.6, 6.7 Hz, 2H), 5.10-5.00 (m, 2H), 4.43 (d, J = 11.2 Hz, 1H), 4.22-4.16 (m, 2H), 4.11 (d, J = 2.0 Hz, 1H), 4.09-4.04 (m, 1H), 3.82 (dd, J = 11.4, 7.6 Hz, 1H), 3.75 (dd, J = 11.4, 4.8 Hz, 1H), 2.48-2.39 (m, 1H), 2.28-2.14 (m, 1H).

### Example 139: Synthesis of Compound A153

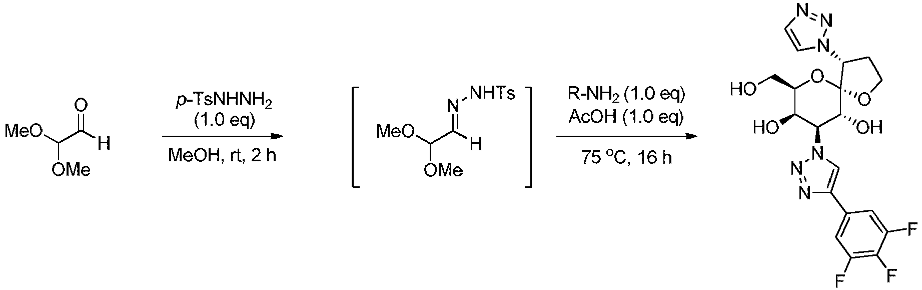

### Synthesis of (4R,5S,7R,8R,9S,10R)-7-(hydroxymethyl)-4-(1H-1,2,3-triazol-1-yl)-9-(4-(3,4,5-trifluorophenyl)-1H-1,2,3-triazol-1-yl)-1,6-dioxaspiro[4.5]decane-8,10-diol

60% glyoxal dimethyl acetal in purified water (6.2 mg, 0.036 mmol) and p-TsNHNH₂ (6.7 mg, 0.14 mmol) were dissolved in 1.0 mL of methanol and stirred at room temperature for 2 hours, then (4*R*,5*S*,7*R*,8*R*,9*S*,10*R*)-4-amino-7-(hydroxymethyl)-9-(4-(3,4,5-trifluorophenyl)-1*H*-1,2,3-triazol-1-yl)-1,6-dioxaspiro[4.5]decane-8,10-diol (15 mg, 0.036 mmol) and AcOH (1 drop) were added and reacted at 75 °C for 16 hours, and then extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (9.0 mg, yield: 54%).

ESIMS: m/z 469.36 [M+H]+; ¹H-NMR (400 MHz, CD₃OD) δ 8.43 (s, 1H), 8.27 (s, 1H), 7.75 (s, 1H), 7.58 (dd, J = 8.5, 6.7 Hz, 2H), 5.64 (dd, J = 10.6, 9.2 Hz, 1H), 5.08 (dd, J = 11.2, 2.8 Hz, 1H), 4.38-4.26 (m, 2H), 4.20 (dd, J = 16.1, 8.1 Hz, 1H), 4.14 (t, J = 5.8 Hz, 1H), 4.05 (d, J = 1.9 Hz, 1H), 3.64 (ddd, J = 16.6, 11.7, 6.1 Hz, 2H), 2.80 (ddd, J = 20.3, 11.4, 9.0 Hz, 1H), 2.70-2.60 (m, 1H).

### Example 140: Synthesis of Compound A154

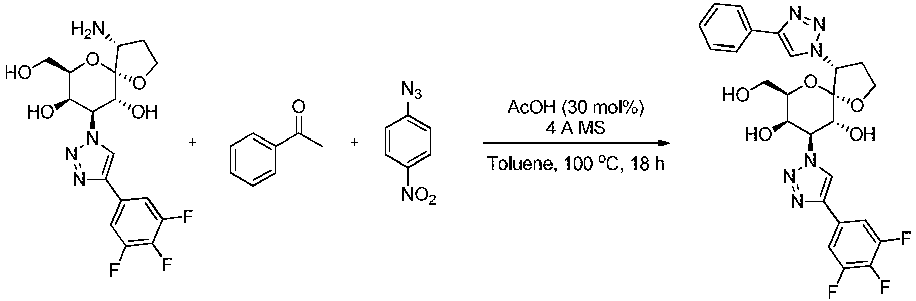

### Synthesis of (4R,5S,7R,8R,9S,10R)-7-(hydroxymethyl)-4-(4-phenyl-1H-1,2,3-triazol-1-yl)-9-(4-(3,4,5-trifluorophenyl)-1H-1,2,3-triazol-1-yl)-1,6-dioxaspiro[4.5]decane-8,10-diol

(4*R*,5*S*,7*R,*8*R,*9*S,*10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol), acetophenone (5.6 mg, 0.047 mmol), azidonitrophenyl (7.7 mg, 0.047 mmol), and 4 Å molecular sieves were dissolved in 2 mL of toluene, then stirred at 100 °C for 18 hours. The resulting mixture was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (7.3 mg, yield: 43%).

ESIMS: m/z 545.28 [M+H]+; ¹H-NMR (400 MHz, CD₃OD) δ 8.08 (s, 1H), 7.75 (s, 1H), 7.67-7.53 (m, 7H), 5.29 (dd, J = 9.1, 4.7 Hz, 1H), 4.95 (dd, J = 11.3, 2.8 Hz, 1H), 4.49 (dd, J = 15.2, 8.0 Hz, 1H), 4.26-4.16 (m, 1H), 4.07 (d, J = 2.2 Hz, 1H), 4.02 (t, J = 6.4 Hz, 1H), 3.93 (d, J = 11.3 Hz, 1H), 3.51 (dd, J = 11.4, 5.9 Hz, 1H), 3.44 (dd, J = 11.0, 6.1 Hz, 1H), 2.98-2.91 (m, 1H), 2.64-2.53 (m, 1H).

### Example 141: Synthesis of Compound A155

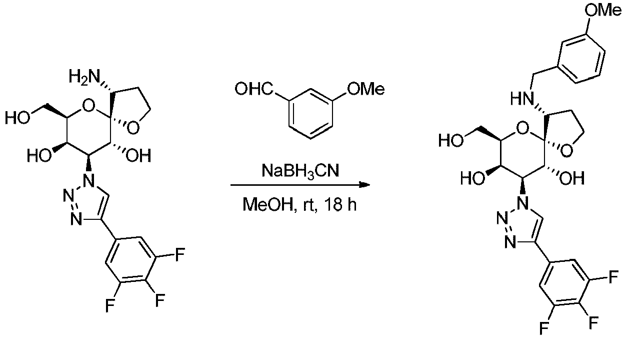

### Synthesis of (4R,5S,7R,8R,9S,10R)-7-(hydroxymethyl)-4-((3-methoxylbenzyl)amino)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of methanol, and then 3-methoxybenzaldehyde (7.3 mg, 0.054 mmol) and sodium cyanoborohydride (4.5 mg, 0.054 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (2.0 mg, yield: 10%).

ESIMS: m/z 537.28 [M+H]+; ¹H-NMR (400 MHz, CD₃OD) δ 8.53 (s, 1H), 7.64 (dd, J = 8.4, 6.7 Hz, 2H), 6.93 (dd, J = 7.7, 1.2 Hz, 1H), 6.87-6.79 (m, 2H), 5.03 (dd, J = 11.2, 2.7 Hz, 1H), 4.49 (d, J = 11.3 Hz, 1H), 4.18-3.93 (m, 6H), 3.85 (s, 3H), 3.77-3.67 (m, 3H), 2.38 (s, 1H), 2.02 (d, J = 6.5 Hz, 2H).

### Example 142: Synthesis of Compound A156

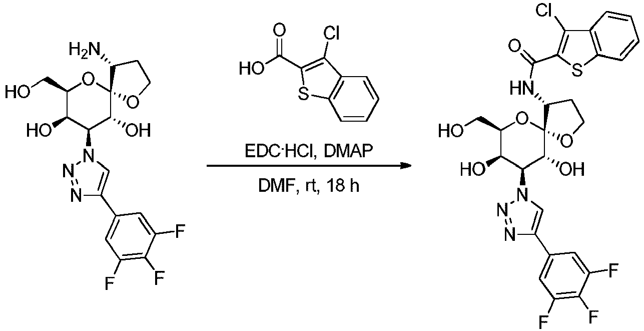

### Synthesis of 3-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-2-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 3-chlorobenzo[*b*]thiophene-2-carboxylic acid (8.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (10.9 mg, yield: 50%).

ESIMS: m/z 611.25 [M+H]+; ¹H-NMR (400 MHz, CD₃OD) δ 8.56 (s, 1H), 8.00-7.89 (m, 2H), 7.65-7.51 (m, 4H), 5.10 (dd, J = 11.2, 2.7 Hz, 1H), 4.94 (t, J = 9.1 Hz, 1H), 4.51 (d, J = 11.2 Hz, 1H), 4.26-4.15 (m, 3H), 4.10 (dd, J = 16.0, 8.2 Hz, 1H), 3.85 (dd, J = 11.3, 6.7 Hz, 1H), 3.79 (dd, J = 11.3, 5.2 Hz, 1H), 2.59-2.49 (m, 1H), 2.24-2.13 (m, 1H).

### Example 143: Synthesis of Compound A157

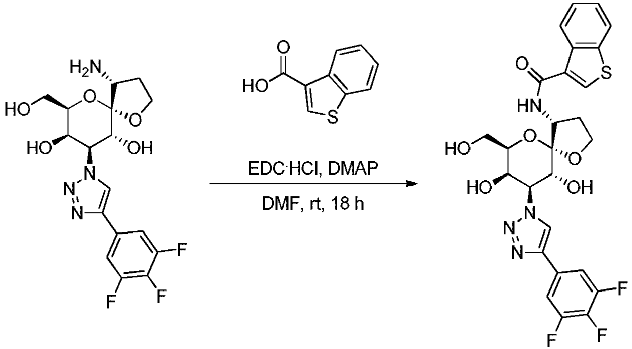

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-3-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N-*dimethylformamide, and then benzo[*b*]thiophene-3-carboxylic acid (8.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (20.5 mg, yield: 99%).

ESIMS: m/z 577.23 [M+H]+; ¹H-NMR (400 MHz, CD₃OD) δ 8.54 (s, 1H), 8.43 (d, J = 7.6 Hz, 1H), 8.27 (s, 1H), 7.94 (d, J = 7.5 Hz, 1H), 7.60 (dd, J = 8.7, 6.8 Hz, 2H), 7.49-7.38 (m, 2H), 5.14-5.01 (m, 2H), 4.51 (d, J = 11.2 Hz, 1H), 4.23-4.13 (m, 3H), 4.08 (dd, J = 16.1, 8.3 Hz, 1H), 3.86 (dd, J = 11.4, 7.4 Hz, 1H), 3.77 (dd, J = 11.4, 4.8 Hz, 1H), 2.53-2.42 (m, 1H), 2.26-2.13 (m, 1H).

### Example 144: Synthesis of Compound A158

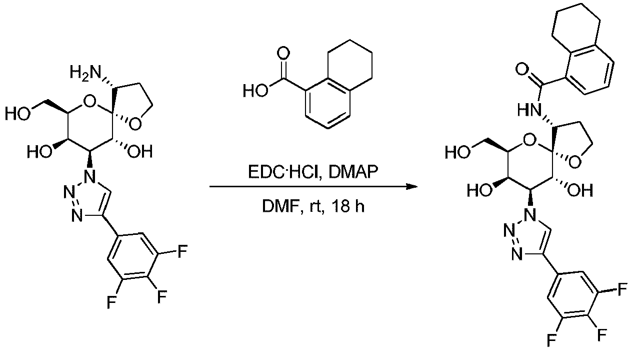

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-5,6,7,8-tetrahydronaphthalene-1-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 5,6,7,8-tetrahydronaphthalene-1-carboxylic acid (7.0 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (18.2 mg, yield: 88%).

ESIMS: m/z 575.27 [M+H]+; ¹H-NMR (400 MHz, CD₃OD) δ 8.51 (s, 1H), 7.64 (dd, J = 8.6, 6.7 Hz, 2H), 7.21 (dd, J = 8.4, 4.9 Hz, 1H), 7.17-7.13 (m, 2H), 5.05 (dd, J = 11.2, 2.7 Hz, 1H), 4.95 (t, J = 9.3 Hz, 1H), 4.51 (d, J = 11.2 Hz, 1H), 4.19-4.01 (m, 4H), 3.74 (ddd, J = 16.3, 11.4, 6.0 Hz, 2H), 3.31 (d, J = 1.5 Hz, 2H), 2.84-2.78 (m, 2H), 2.49-2.39 (m, 1H), 2.14-2.04 (m, 1H), 1.88-1.77 (m, 4H).

### Example 145: Synthesis of Compound A159

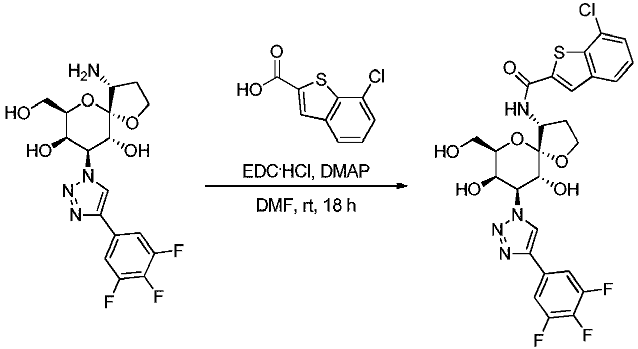

### Synthesis of 7-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-2-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 7-chlorobenzo[*b*]thiophene-2-carboxylic acid (8.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (14.9 mg, yield: 68%).

ESIMS: m/z 611.19 [M+H]+; ¹H-NMR (400 MHz, CD₃OD) δ 8.55 (s, 1H), 8.13 (s, 1H), 7.87 (d, J = 7.8 Hz, 1H), 7.59 (dd, J = 8.5, 6.8 Hz, 2H), 7.49 (d, J = 7.5 Hz, 1H), 7.43 (t, J = 7.8 Hz, 1H), 5.08 (dd, J = 11.2, 2.8 Hz, 1H), 5.01 (t, J = 9.4 Hz, 1H), 4.47 (d, J = 11.2 Hz, 1H), 4.24-4.15 (m, 2H), 4.13 (d, J = 2.3 Hz, 1H), 4.08 (dd, J = 16.2, 8.2 Hz, 1H), 3.86 (dd, J = 11.4, 7.6 Hz, 1H), 3.78 (dd, J = 11.4, 4.8 Hz, 1H), 2.51-2.41 (m, 1H), 2.30-2.17 (m, 1H).

### Example 146: Synthesis of Compound A160

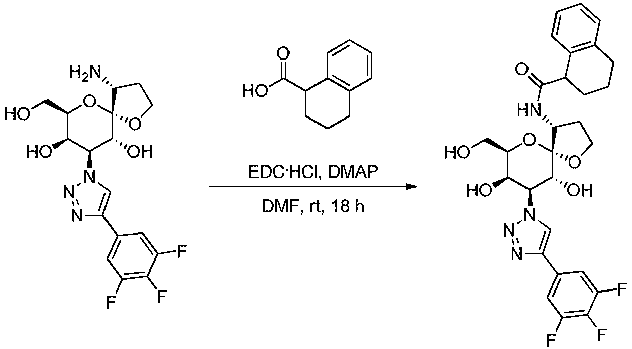

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1,2,3,4-tetrahydronaphthalene-1-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 1,2,3,4-tetrahydronaphthalene-1-carboxylic acid (7.0 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (12.3 mg, yield: 60%).

ESIMS: m/z 575.34 [M+H]+; ¹H-NMR (400 MHz, CD₃OD) δ 8.53 (s, 1H), 7.70-7.61 (m, 2H), 7.19-7.06 (m, 4H), 5.02 (dd, J = 11.2, 2.4 Hz, 1H), 4.77 (t, J = 10.4 Hz, 1H), 4.40 (dd, J = 11.2, 6.3 Hz, 1H), 4.17-3.96 (m, 4H), 3.85-3.76 (m, 2H), 3.70 (d, J = 6.0 Hz, 1H), 2.90-2.72 (m, 2H), 2.45-2.27 (m, 1H), 2.19-1.93 (m, 4H), 1.84-1.69 (m, 1H).

### Example 147: Synthesis of Compound A161

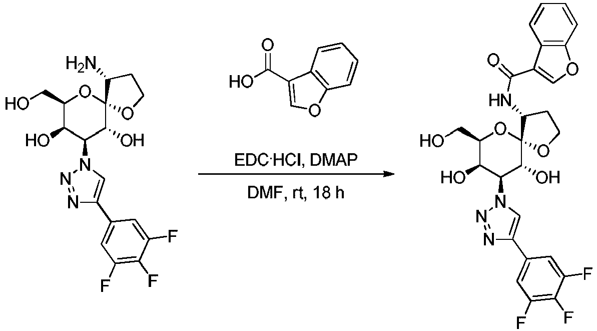

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzofuran-3-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then benzofuran-3-carboxylic acid (6.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (8.7 mg, yield: 43%).

ESIMS: m/z 561.22 [M+H]+; ¹H-NMR (400 MHz, CD₃OD) δ 8.54 (s, 1H), 8.42 (s, 1H), 8.10 (d, J = 7.2 Hz, 1H), 7.59 (dd, J = 16.1, 7.5 Hz, 3H), 7.43-7.33 (m, 2H), 5.09 (dd, J = 11.2, 2.8 Hz, 1H), 5.02 (t, J = 9.3 Hz, 1H), 4.48 (d, J = 11.2 Hz, 1H), 4.23-4.13 (m, 3H), 4.08 (dd, J = 16.1, 8.1 Hz, 1H), 3.86 (dd, J = 11.4, 7.3 Hz, 1H), 3.78 (dd, J = 11.4, 4.9 Hz, 1H), 2.53-2.42 (m, 1H), 2.24-2.13 (m, 1H).

### Example 148: Synthesis of Compound A162

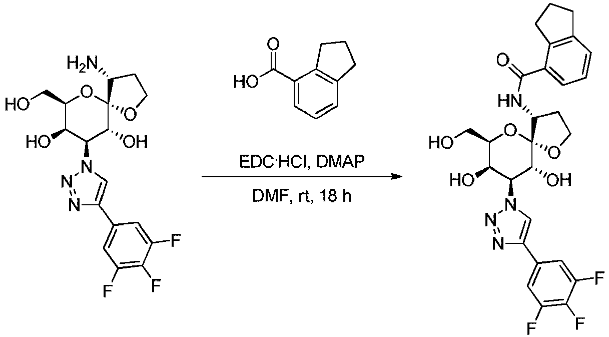

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2,3-dihydro-1H-indene-4-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then 2,3-dihydro-1*H*-indene-4-carboxylic acid (6.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (14.6 mg, yield: 72%).

ESIMS: m/z 561.29 [M+H]+; ¹H-NMR (400 MHz, CD₃OD) δ 8.53 (s, 1H), 7.62 (dd, J = 8.7, 6.7 Hz, 2H), 7.46 (d, J = 7.5 Hz, 1H), 7.37 (d, J = 7.3 Hz, 1H), 7.22 (t, J = 7.6 Hz, 1H), 5.07 (dd, J = 11.2, 2.7 Hz, 1H), 4.96 (t, J = 9.3 Hz, 1H), 4.46 (d, J = 11.2 Hz, 1H), 4.22-4.11 (m, 3H), 4.06 (dd, J = 16.1, 8.3 Hz, 1H), 3.81 (dd, J = 11.4, 7.1 Hz, 1H), 3.74 (dd, J = 11.4, 4.9 Hz, 1H), 3.24-3.16 (m, 2H), 2.95 (t, J = 7.4 Hz, 2H), 2.51-2.40 (m, 1H), 2.20-2.04 (m, 3H).

### Example 149: Synthesis of Compound A163

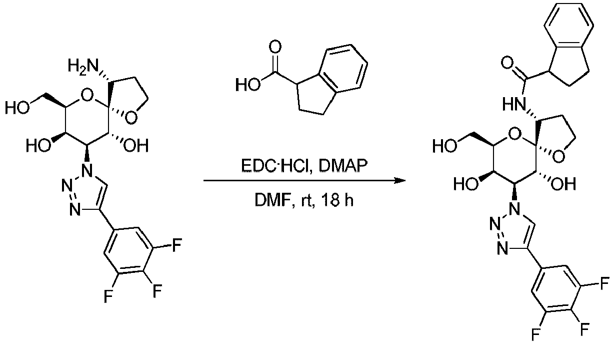

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2,3-dihydro-1H-indene-1-carboxamide

(4*R*,5*S*,7*R*,8*R*,9*S*,10*R*)-4-amino-7-(hydroxymethyl)-9-(4-(3,4,5-trifluorophenyl)-1H-1,2,3-triazol-1-yl)-1,6-dioxaspiro[4.5]decane-8,10-diol (15.0 mg, 0.036 mmol) was dissolved in 1.0 mL of *N,N*-dimethylformamide, and then 2,3-dihydro-1*H*-indene-1-carboxylic acid (6.4 mg, 0.0396 mmol), *N*-(3-dimethylaminopropyl)-*N*'-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (4.5 mg, yield: 22%).

ESIMS: m/z 561.29 [M+H]+; ¹H-NMR (400 MHz, CD₃OD) δ 8.55 (s, 1H), 7.67-7.62 (m, 2H), 7.32 (d, J = 6.4 Hz, 1H), 7.26 (d, J = 6.7 Hz, 1H), 7.22-7.16 (m, 2H), 5.04 (dd, J = 11.1, 2.7 Hz, 1H), 4.83-4.76 (m, 1H), 4.41 (d, J = 11.1 Hz, 1H), 3.16-3.05 (m, 3H), 2.97-2.89 (m, 2H), 3.77 (t, J = 5.8 Hz, 2H), 3.16-3.05 (m, 1H), 2.97-2.89 (m, 1H), 2.45-2.33 (m, 3H), 2.11-2.04 (m, 1H).

### Example 150: Synthesis of Compound A164

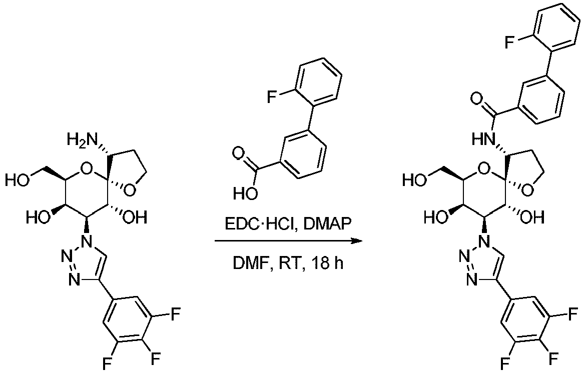

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl) -2'-fluoro-[1,1'-biphenyl]-3-carboxamide

The title compound (9.4 mg, yield: 64%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 615.28 [M+H]+; ¹H-NMR (400 MHz, CD₃OD) δ = 8.53 (S, 1H), 8.06 (S, 1H), 7.91 (d, J = 7.8 Hz, 1H), 7.75 (d, J = 7.4 Hz, 1H), 7.63-7.54 (m, 4H), 7.41 (q, J = 6.4 Hz, 1H), 7.29 (t, J = 7.0 Hz, 1H), 7.22 (dd, J = 10.6, 8.6 Hz, 1H), 5.09-5.01 (m, 2H), 4.43 (d, J = 11.3 Hz, 1H), 4.20-4.16 (m, 2H), 4.10 (d, J = 2.0 Hz, 1H), 4.08 (q, J = 7.8 Hz, 1H), 3.82 (dd, J = 11.3, 7.4 Hz, 1H), 3.73 (dd, J = 11.3, 4.7 Hz, 1H), 2.49-2.42 (m, 1H), 2.26-2.16 (m, 1H)

### Example 151: Synthesis of Compound A165

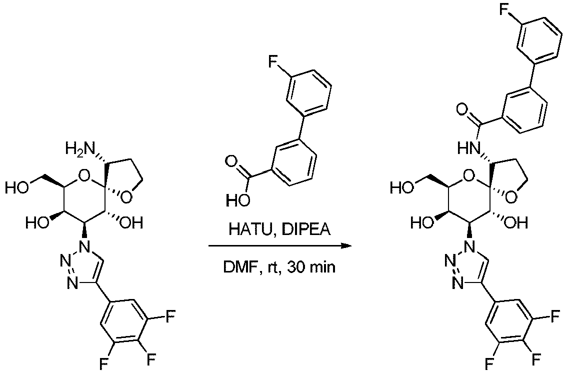

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl) -3'-fluoro-[1,1'-biphenyl]-3-carboxamide

(4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol), 3'-fluoro-[1,1'-biphenyl]-3-carboxylic acid (7.78 mg, 0.036 mmol), 1-[bis(dimethylamino)methylene]-1*H-*1,2,3-triazolo[4,5-*b*]pyridinium 3-oxide hexafluorophosphate (10 mg, 0.26 mmol) was dissolved in 0.6 mL of *N,N-*dimethylformamide, and then diisopropylethylamine (15 µL, 0.057 mmol) was added thereto at room temperature. The reaction mixture was stirred at room temperature for 30 minutes. Methanol (1 mL) was added to the reaction mixture to terminate the reaction, and then the reaction solution was concentrated under reduced pressure. The remaining concentrate was subjected to silica gel column chromatography with 0% to 7% methanol/dichloromethane to synthesize the title compound as a white solid (12.3 mg, yield: 83%).

ESIMS: m/z 615.28 [M+H]+; ¹H-NMR (400 MHz, CD₃OD) δ = 8.52 (S, 1H), 8.15 (S, 1H), 7.90 (d, J = 7.4 Hz, 1H), 7.84 (d, J = 7.8 Hz, 1H), 7.62-7.45 (m, 6H), 7.12 (t, J = 8.4 Hz, 1H), 5.09-5.00 (m, 2H), 4.45 (d, J = 11.3 Hz, 1H), 4.22-4.17 (m, 2H), 4.11 (d, J = 2.0 Hz, 1H), 4.08 (q, J = 7.8 Hz, 1H), 3.84 (dd, J = 11.3, 7.8 Hz, 1H), 3.75 (dd, J = 11.3, 4.7 Hz, 1H), 2.49-2.41 (m, 1H), 2.27-2.18 (m, 1H)

### Example 152: Synthesis of Compound A166

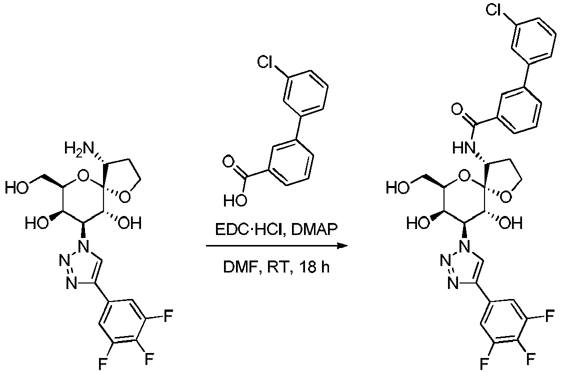

### Synthesis of 3'-chloro-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-[1.1'-biphenyl]-3-carboxamide

The title compound (6.4, yield: 42%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 631.29 [M+H]+; ¹H-NMR (400 MHz, CD₃OD) δ = 8.52 (S, 1H), 8.14 (S, 1H), 7.91 (d, J = 7.8 Hz, 1H), 7.84 (d, J = 7.8 Hz, 1H), 7.74 (S, 1H), 7.66-7.58 (m, 4H), 7.47 (t, J = 7.8 Hz, 1H), 7.39 (d, J = 7.8 Hz, 1H), 5.13-5.03 (m, 2H), 4.44 (d, J = 11.0 Hz, 1H), 4.21-4.17 (m, 2H), 4.11 (d, J = 2.0 Hz, 1H), 4.09 (q, J = 8.2 Hz, 1H), 3.84 (dd, J = 11.2, 7.6 Hz, 1H), 3.75 (dd, J = 11.5, 4.5 Hz, 1H), 2.49-2.42 (m, 1H), 2.28-2.18 (m, 1H)

### Example 153: Synthesis of Compounds A167 and A168

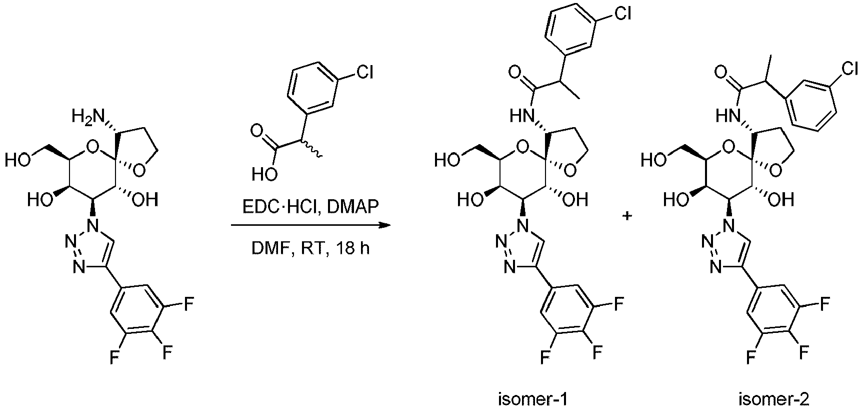

### Synthesis of 2-(3-chlorophenyl)-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)propanamide

The title compound was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-N-((4*R*,5*S*,7*R*,8*R*,9*S*,10*R*)-8,10-dihydroxy-7-(hydroxymethyl)-9-(4-(3,4,5-trifluorophenyl)-1*H-*1,2,3-triazol-1-yl)-1,6-dioxaspiro[4.5]decan-4-yl)acetamide, and subjected to preparative thin layer chromatography using methanol: dichloromethane = 1:30 (x 4 times) to isolate stereoisomers. Isomer-1 (A167) (5.9 mg, yield: 42%) as a white solid and isomer-2 (A168) (6.3 mg, yield: 45%) as a white solid were synthesized.

A167: ESIMS: m/z 583.28 [M+H]+; ¹H-NMR (400 MHz, CD₃OD) δ = 8.52 (S, 1H), 7.66-7.62 (m, 2H), 7.41 (S, 1H), 7.32-7.29 (m, 2H), 7.28-7.24 (m, 1H), 5.02 (dd, J = 11.3, 2.7 Hz, 1H), 4.73 (t, J = 9.4 Hz, 1H), 4.37 (d, J = 11.3 Hz, 1H), 4.13-4.06 (m, 4H), 3.98 (q, J = 8.1 Hz, 1H), 3.81-3.71 (m, 3H), 2.32-2.24 (m, 1H), 1.97-1.87 (m, 1H), 1.50 (d, J = 7.0 Hz, 3H)

A168 ESIMS: m/z 583.28 [M+H]+; ¹H-NMR (400 MHz, CD₃OD) δ = 8.28 (S, 1H), 7.59-7.55 (m, 2H), 7.46 (S, 1H), 7.38 (d, J = 7.4 Hz, 1H), 7.33 (t, J = 7.8 Hz, 1H), 7.26 (d, J = 7.8 Hz, 1H), 4.96 (dd, J = 11.2, 2.5 Hz, 1H), 4.71 (t, J = 9.4 Hz, 1H), 4.15-4.05 (m, 4H), 4.00 (q, J = 8.0 Hz, 1H), 3.81-3.69 (m, 3H), 2.38-2.31 (m, 1H), 2.10-2.00 (m, 1H), 1.49 (d, J = 7.0 Hz, 3H)

### Example 154: Synthesis of Compound A169

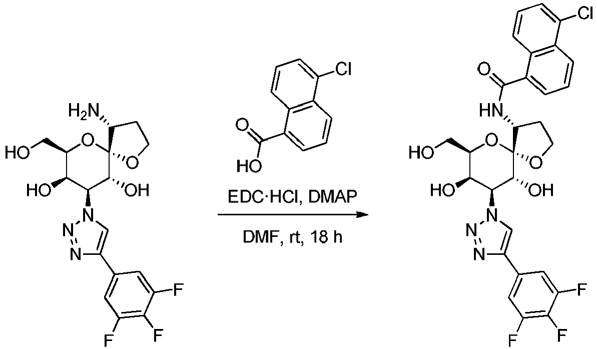

### Synthesis of 5-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

The title compound (2.8 mg, yield: 19%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 605.20 [M+H]+; ¹H-NMR (400 MHz, CD₃OD) δ = 8.58 (S, 1H), 8.42 (d, J = 8.6 Hz, 1H), 8.37 (d, J = 8.6 Hz, 1H), 7.80 (d, J = 7.0 Hz, 1H), 7.72-7.62 (m, 4H), 7.54 (t, J = 8.0 Hz, 1H), 5.15-5.08 (m, 2H), 4.65 (d, J = 11.0 Hz, 1H), 4.20-4.14 (m, 3H), 4.09 (q, J = 8.0 Hz, 1H), 3.82-3.71 (m, 2H), 2.53-2.47 (m, 1H), 2.23-2.13 (m, 1H)

### Example 155: Synthesis of Compound A170

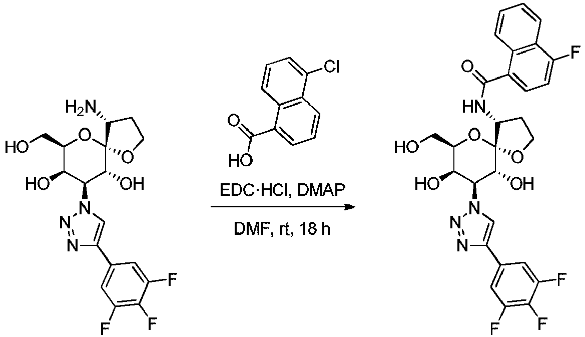

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-4-difluoro-1-naphthamide

The title compound (6.0 mg, yield: 42%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide.

ESIMS: m/z 589.26 [M+H]+; ¹H-NMR (400 MHz, CD₃OD) δ = 8.57 (S, 1H), 8.46 (d, J = 8.2 Hz, 1H), 8.15 (d, J = 7.8 Hz, 1H), 7.74 (dd, J = 7.8, 5.5 Hz, 1H), 7.70-7.62 (m, 4H), 7.27 (dd, J = 10.4, 8.0 Hz, 1H), 5.14-5.08 (m, 2H), 4.63 (d, J = 11.3 Hz, 1H), 4.21-4.14 (m, 3H), 4.09 (q, J = 8.0 Hz, 1H), 3.81 (dd, J = 11.3, 7.4 Hz, 1H), 3.74 (dd, J = 11.3, 4.7 Hz, 1H), 2.52-2.45 (m, 1H), 2.23-2.13 (m, 1H)

### Example 156: Synthesis of Compound A171

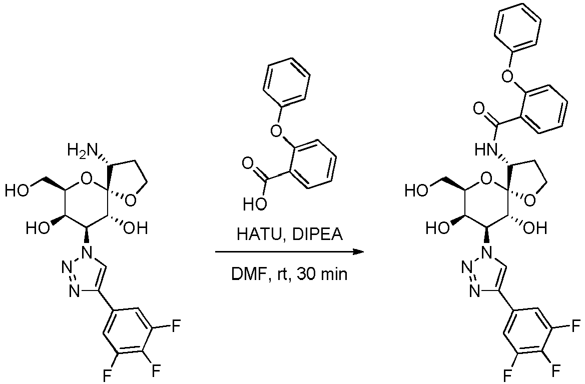

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-phenoxybenzamide

The title compound (13.4 mg, yield: 91%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 613.33 [M+H]+; ¹H-NMR (400 MHz, CD₃OD) δ = 8.42 (S, 1H), 8.01 (dd, J = 7.8, 1.6 Hz, 1H), 7.59-7.55 (m, 2H), 7.48-7.41 (m, 3H), 7.25-7.20 (m, 2H), 7.17 (S, 1H), 7.15 (S, 1H), 6.88 (d, J = 8.2 Hz, 1H), 5.03 (dd, J = 11.3, 2.7 Hz, 1H), 4.90 (t, J = 9.4 Hz, 1H), 4.39 (d, J = 11.3 Hz, 1H), 4.16-4.00 (m, 4H), 3.51 (dd, J = 11.3, 5.9 Hz, 1H), 3.45 (dd, J = 11.2, 6.5 Hz, 1H), 2.49-2.40 (m, 1H), 2.05-1.89 (m, 1H)

### Example 157: Synthesis of Compound A172

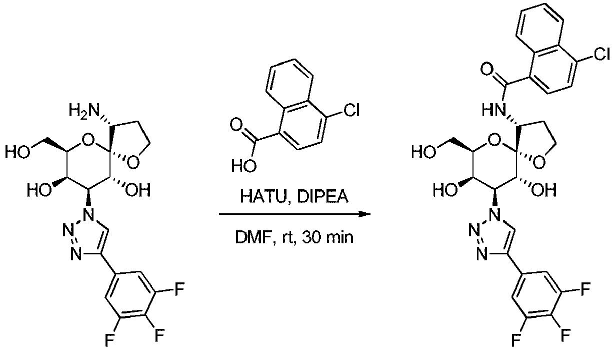

### Synthesis of 4-chloro-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

The title compound (12.4 mg, yield: 85%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 605.27 [M+H]+; ¹H-NMR (400 MHz, CD₃OD) δ = 8.58 (S, 1H), 8.46-8.44 (m, 1H), 8.35-8.32 (m, 1H), 7.72-7.67 (m, 4H), 7.66-7.61 (m, 2H), 5.15-5.08 (m, 2H), 4.65 (d, J = 11.3 Hz, 1H), 4.20-4.14 (m, 3H), 4.09 (q, J = 8.1 Hz, 1H), 3.80 (dd, J = 11.3, 7.0 Hz, 1H), 3.74 (dd, J = 11.3, 5.1 Hz, 1H), 2.52-2.44 (m, 1H), 2.23-2.13 (m, 1H)

### Example 158: Synthesis of Compound A173

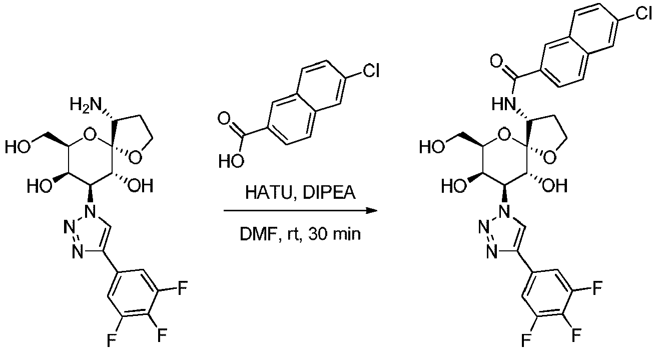

### Synthesis of 6-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

The title compound (12.0 mg, yield: 83%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 605.20 [M+H]+; ¹H-NMR (400 MHz, CD₃OD) δ = 8.54 (S, 1H), 8.45 (S, 1H), 8.00-7.90 (m, 4H), 7.64-7.57 (m, 2H), 7.54 (dd, J = 8.8, 1.8 Hz, 1H), 5.11-5.05 (m, 2H), 4.47 (d, J = 11.0 Hz, 1H), 4.23-4.18 (m, 2H), 4.13 (d, J = 2.0 Hz, 1H), 4.09 (q, J = 8.2 Hz, 1H), 3.86 (dd, J = 11.3, 7.8 Hz, 1H), 3.77 (dd, J = 11.7, 4.7 Hz, 1H), 2.52-2.44 (m, 1H), 2.29-2.19 (m, 1H)

### Example 159: Synthesis of Compound A174

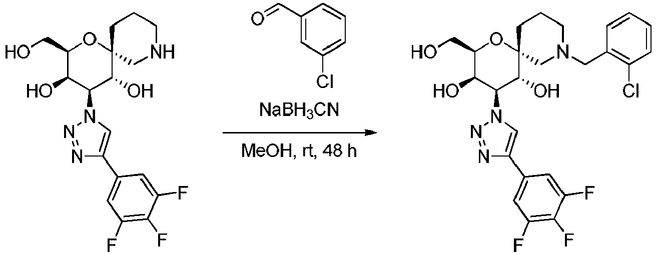

### Synthesis of (2R,3R,4S,5R,6R)-8-(3-chlorobenzyl)-2-(hydroxymethyl)-4-(4-(3,4,5-trifluorophenyl)-1H-1,2,3-triazol-1-yl)-1-oxa-8-azaspiro[5.5]undecane-3,5-diol

The title compound (7.8 mg, yield: 60%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of (2*R*,3*R*,4*S*,5*R*,6*R*)-8-benzyl-2-(hydroxymethyl)-4-(4-(3,4,5-trifluorophenyl)-1*H*-1,2,3-triazol-1-yl)-1-oxa-8-azaspiro[5.5]undecane-3,5-diol.

ESIMS: m/z 539.23 [M+H]+; ¹H-NMR (400 MHz, CD₃OD) δ = 8.45 (S, 1H), 7.65-7.60 (m, 2H), 7.57-7.55 (m, 1H), 7.40 (dd, J = 7.6, 1.0 Hz, 1H), 7.34-7.25 (m, 2H), 4.80 (dd, J = 11.3, 2.7 Hz, 1H), 4.24 (d, J = 11.3 Hz, 1H), 4.03 (d, J = 2.3 Hz, 1H), 3.82-3.65 (m, 5H), 3.03 (d, J = 11.7 Hz, 1H), 2.86 (d, J= 12.1 Hz, 1H), 2.72-2.70 (m, 1H), 2.38-2.34 (m, 1H), 2.03-1.94 (m, 2H), 1.75-1.67 (m, 2H)

### Example 160: Synthesis of Compound A175

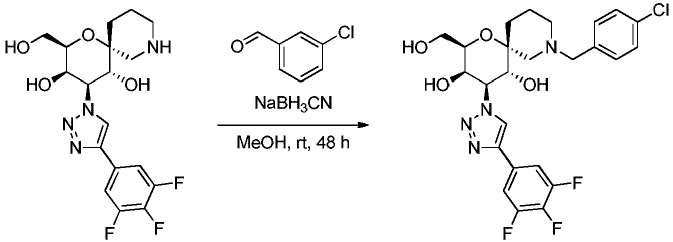

### Synthesis of (2R,3R,4S,5R,6R)-8-(3-chlorobenzyl)-2-(hydroxymethyl)-4-(4-(3,4,5-trifluorophenyl)-1H-1,2,3-triazol-1-yl)-1-oxa-8-azaspiro[5.5]undecane-3,5-diol

The title compound (7.4 mg, yield: 57%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of (2*R*,3*R*,4*S*,5*R*,6*R*)-8-benzyl-2-(hydroxymethyl)-4-(4-(3,4,5-trifluorophenyl)-1*H*-1,2,3-triazol-1-yl)-1-oxa-8-azaspiro[5.5]undecane-3,5-diol.

ESIMS: m/z 539.23 [M+H]+; ¹H-NMR (400 MHz, CD₃OD) δ = 8.45 (S, 1H), 7.65-7.60 (m, 2H), 7.56 (d, J = 7.0 Hz, 1H), 7.40 (d, J = 7.8 Hz, 1H), 7.34-7.26 (m, 2H), 4.80 (dd, J = 11.3, 2.7 Hz, 1H), 4.24 (d, J = 11.3 Hz, 1H), 4.03 (S, 1H), 3.82-3.65 (m, 5H), 3.03 (d, J = 12.1 Hz, 1H), 2.86 (d, J = 12.1 Hz, 1H), 2.72-2.68 (m, 1H), 2.38-2.34 (m, 1H), 2.03-1.94 (m, 2H), 1.73-1.67 (m, 2H)

### Example 161: Synthesis of Compound A176

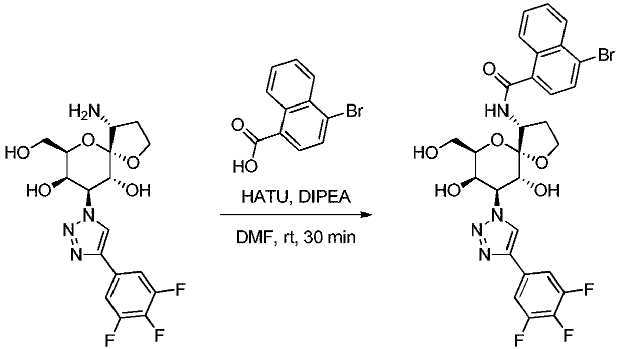

### Synthesis of 4-bromo-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

The title compound (14.1 mg, yield: 90%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 649.12 [M+H]+; ¹H-NMR (400 MHz, CD₃OD) δ = 8.58 (S, 1H), 8.43-8.41 (m, 1H), 8.32-8.29 (m, 1H), 7.90 (d, J = 7.8 Hz, 1H), 7.71-7.58 (m, 5H), 5.14-5.08 (m, 2H), 4.66 (d, J = 11.3 Hz, 1H), 4.25-4.06 (m, 4H), 3.82-3.72 (m, 2H), 2.52-2.44 (m, 1H), 2.23-2.13 (m, 1H)

### Example 162: Synthesis of Compound A177

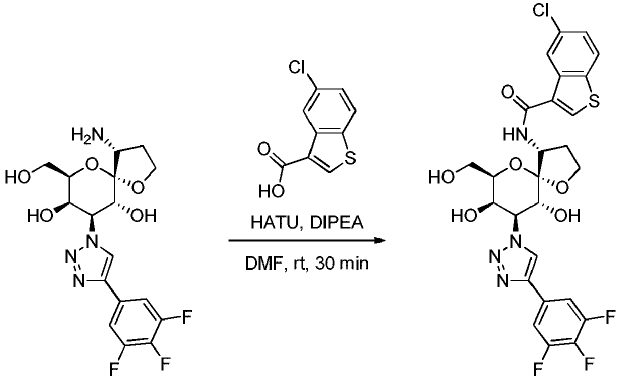

### Synthesis of 5-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-3-carboxamide

The title compound (13.4 mg, yield: 91%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 611.19 [M+H]+; ¹H-NMR (400 MHz, CD₃OD) δ = 8.54 (S, 1H), 8.44 (d, J = 1.6 Hz, 1H), 8.40 (S, 1H), 7.93 (d, J = 8.6 Hz, 1H), 7.60 (t, J = 7.6 Hz, 2H), 7.41 (dd, J = 8.6, 1.6 Hz, 1H), 5.10-5.02 (m, 2H), 4.48 (d, J = 11.0 Hz, 1H), 4.19-4.05 (m, 4H), 3.87 (dd, J = 11.3, 7.4 Hz, 1H), 3.77 (dd, J = 11.3, 4.7 Hz, 1H), 2.51-2.43 (m, 1H), 2.24-2.14 (m, 1H)

### Example 163: Synthesis of Compound A178

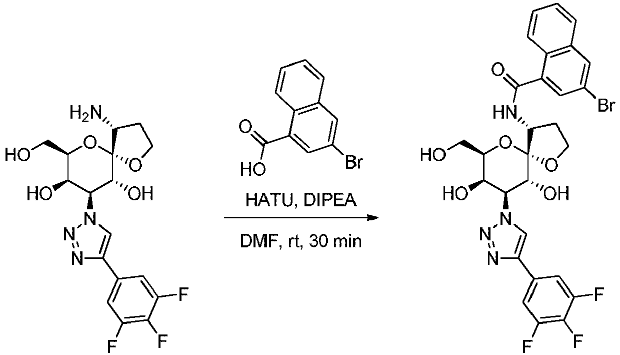

### Synthesis of 3-bromo-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

The title compound (13.1 mg, yield: 84%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 649.18 [M+H]+; ¹H-NMR (400 MHz, CD₃OD) δ = 8.58 (S, 1H), 8.34 (d, J = 8.2 Hz, 1H), 8.20 (S, 1H), 7.89 (d, J = 7.4 Hz, 1H), 7.83 (d, J = 2.0 Hz, 1H), 7.65-7.57 (m, 4H), 5.14-5.08 (m, 2H), 4.63 (d, J = 11.0 Hz, 1H), 4.20-4.06 (m, 4H), 3.83 (dd, J = 11.3, 7.4 Hz, 1H), 3.75 (dd, J = 11.3, 4.7 Hz, 1H), 2.52-2.44 (m, 1H), 2.24-2.14 (m, 1H)

### Example 164: Synthesis of Compound A179

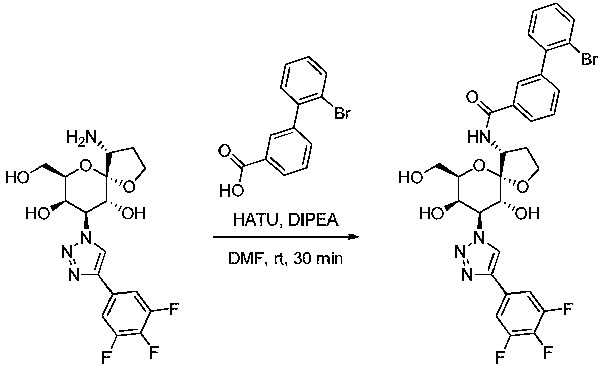

### Synthesis of 2'-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-[1,1'-biphenyl]-3-carboxamide

The title compound (11.1 mg, yield: 68%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 675.20 [M+H]+; ¹H-NMR (400 MHz, CD₃OD) δ = 8.52 (S, 1H), 7.98-7.92 (m, 2H), 7.71 (d, J = 8.2 Hz, 1H), 7.63-7.55 (m, 4H), 7.46-7.40 (m, 2H), 7.31-7.27 (m, 1H), 5.09-5.01 (m, 2H), 4.43 (d, J = 11.0 Hz, 1H), 4.21-4.15 (m, 2H), 4.10-4.05 (m, 2H), 3.81 (dd, J = 11.5, 7.6 Hz, 1H), 3.72 (dd, J = 11.3, 4.7 Hz, 1H), 2.49-2.41 (m, 1H), 2.26-2.15 (m, 1H)

### Example 165: Synthesis of Compound A180

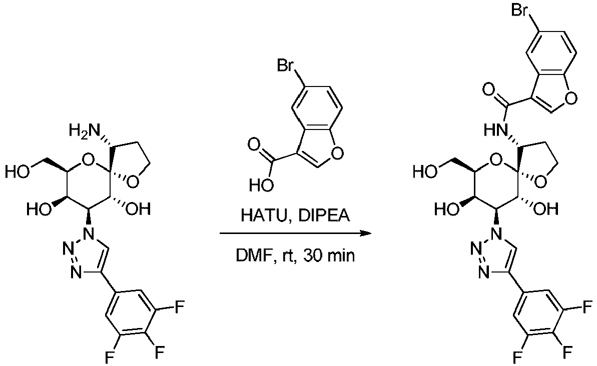

### Synthesis of 5-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzofuran-3-carboxamide

The title compound (12.4, yield: 81%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 639.16 [M+H]+; ¹H-NMR (400 MHz, CD₃OD) δ = 8.55 (S, 1H), 8.46 (S, 1H), 8.25 (S, 1H), 7.62-7.59 (m, 2H), 7.52 (S, 2H), 5.08 (dd, J = 11.3, 2.7 Hz, 1H), 5.02 (t, J = 9.4 Hz, 1H), 4.46 (d, J = 11.0 Hz, 1H), 4.19 (dd, J = 7.8, 5.5 Hz, 2H), 4.14 (d, J = 2.0 Hz, 1H), 4.08 (q, J = 8.1 Hz, 1H), 3.90 (dd, J = 11.3, 7.4 Hz, 1H), 3.80 (dd, J = 11.5, 4.9 Hz, 1H), 2.49-2.41 (m, 1H), 2.23-2.13 (m, 1H)

### Example 166: Synthesis of Compound A181

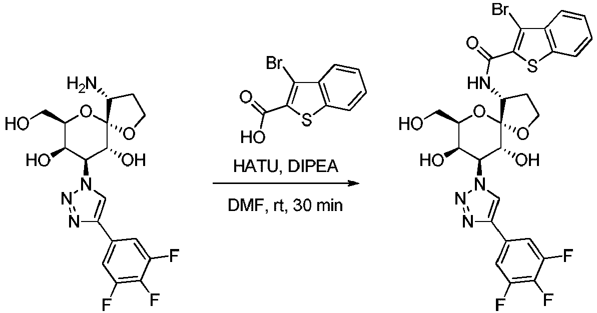

### Synthesis of 3-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-2-carboxamide

The title compound (9.8 mg, yield: 62%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 655.04 [M+H]+; ¹H-NMR (400 MHz, CD₃OD) δ = 8.55 (S, 1H), 7.97-7.91 (m, 2H), 7.63-7.54 (m, 4H), 5.10 (dd, J = 11.0, 2.7 Hz, 1H), 4.94 (t, J = 9.0 Hz, 1H), 4.53 (d, J = 11.3 Hz, 1H), 4.25-4.17 (m, 3H), 4.10 (q, J = 8.1 Hz, 1H), 3.86 (dd, J = 11.3, 7.0 Hz, 1H), 3.78 (dd, J = 11.2, 4.9 Hz, 1H), 2.58-2.50 (m, 1H), 2.23-2.14 (m, 1H)

### Example 167: Synthesis of Compound A182

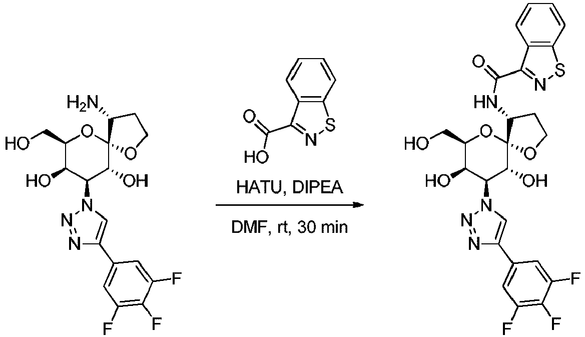

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[d]isothiazole-3-carboxamide

The title compound (11.0 mg, yield: 79%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 578.11 [M+H]+; ¹H-NMR (400 MHz, CD₃OD) δ = 8.85 (d, J = 8.2 Hz, 1H), 8.53 (S, 1H), 8.12 (d, J = 8.2 Hz, 1H), 7.65-7.55 (m, 4H), 5.10 (dd, J = 11.3, 2.7 Hz, 1H), 5.04 (t, J = 9.4 Hz, 1H), 4.47 (d, J = 11.0 Hz, 1H), 4.24-4.18 (m, 2H), 4.13-4.07 (m, 2H), 3.85 (dd, J = 11.5, 7.2 Hz, 1H), 3.76 (dd, J = 11.5, 4.9 Hz, 1H), 2.54-2.46 (m, 1H), 2.28-2.18 (m, 1H)

### Example 168: Synthesis of Compound A183

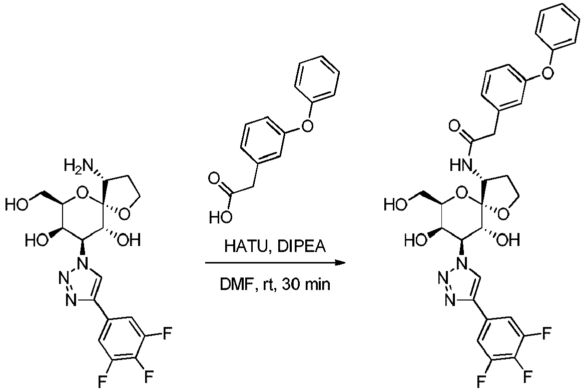

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-(3-phenoxyphenyl)acetamide

The title compound (10.4 mg, yield: 69%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 627.25 [M+H]+; ¹H-NMR (400 MHz, CD₃OD) δ = 8.44 (S, 1H), 7.59 (t, J = 7.6 Hz, 2H), 7.35-7.29 (m, 3H), 7.14-7.07 (m, 2H), 7.01-6.98 (m, 3H), 6.87 (d, J = 8.2 Hz, 1H), 5.01 (dd, J = 11.0, 2.7 Hz, 1H), 4.73 (t, J = 9.2 Hz, 1H), 4.31 (d, J = 11.3 Hz, 1H), 4.12-4.09 (m, 3H), 4.00 (q, J = 8.1 Hz, 1H), 3.80-3.72 (m, 2H), 3.63-3.55 (m, 2H), 2.36-2.28 (m, 1H), 2.05-1.95 (m, 1H)

### Example 169: Synthesis of Compound A184

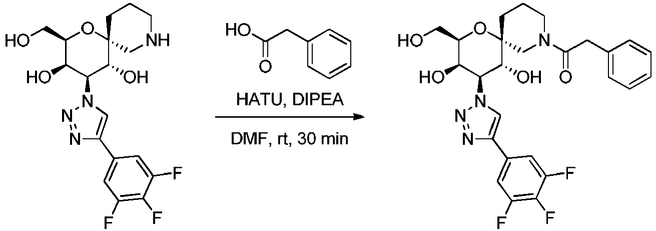

### Synthesis of 1-((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)-2-phenylethanone

(2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol), 2-phenylacetic acid (4.9 mg, 0.036 mmol), and 1-[bis(dimethylamino)methylene]-1*H*-1,2,3-triazolo[4,5-*b*]pyridinium 3-oxide hexafluorophosphate (10.0 mg, 0.232 mmol) were dissolved in 0.6 mL of *N,N-*dimethylformamide, and then diisopropylethylamine (15 µL, 0.057 mmol) was added thereto at room temperature. The reaction mixture was stirred at room temperature for 30 minutes. Methanol (1 mL) was added to the reaction mixture to terminate the reaction, and then the reaction solution was concentrated under reduced pressure. The remaining concentrate was subjected to silica gel column chromatography with 0% to 5% methanol/dichloromethane to synthesize the title compound as a white solid (7.8 mg, yield: 61%).

ESIMS: m/z 533.19 [M+H]+; ¹H-NMR (400 MHz, CD₃OD) δ = 8.47 (S, 1H), 7.62 (t, J = 7.6 Hz, 2H), 7.35-7.24 (m, 5H), 5.08 (d, J = 14.1 Hz, 1H), 4.97 (dd, J = 11.3, 2.7 Hz, 1H), 4.21 (d, J = 11.7 Hz, 1H), 4.16 (d, J = 1.6 Hz, 1H), 4.08-4.00 (m, 2H), 3.89-3.75 (m, 2H), 3.66-3.54 (m, 2H), 3.01 (t, J = 13.1 Hz, 1H), 2.94 (d, J = 14.1 Hz, 1H), 2.12-2.05 (m, 1H), 1.80-1.70 (m, 2H), 1.46 (d, J = 12.5 Hz, 1H)

### Example 170: Synthesis of Compound A185

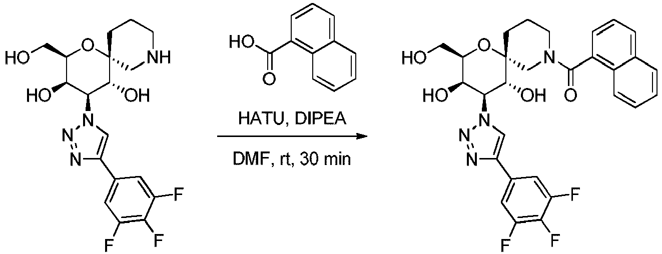

### Synthesis of ((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(naphthalen-1-yl)methanone

The title compound (10.8 mg, yield: 79%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 569.23 [M+H]+; ¹H-NMR (400 MHz, CD₃OD) δ = 8.54-8.53 (m, 1H), 8.19-8.17 (m, 0.5H), 7.97-7.93 (m, 2H), 7.80-7.77 (m, 0.5H), 7.67-7.53 (m, 5.5H), 7.42-7.41 (m, 0.5H), 5.40-5.33 (m, 1H), 5.15-5.09 (m, 1H), 4.38-4.21 (m, 3H), 3.98-3.88 (m, 1H), 3.80-3.78 (m, 1H), 3.34-3.22 (m, 2H), 3.06-2.98 (m, 1H), 2.24-2.14 (m, 1H), 2.07-1.80 (m, 2H), 1.41-1.33 (m, 1H)

### Example 171: Synthesis of Compound A186

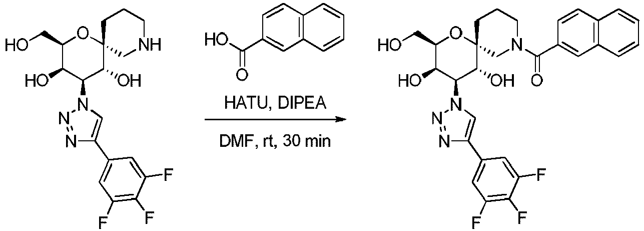

### Synthesis of ((2R,3R,4S,5R,6R)-3,5-dihydroxy-2-(hydroxymethyl)-4-(4-(3,4,5-trifluorophenyl)-1H-1,2,3 -triazol-1-yl)-1-oxa-8-azaspiro[5.5]undecan-8-yl)(naphthalen-2-yl)methanone

The title compound (11.2 mg, yield: 82%) as a white solid was synthesized from (2R,3R,4S, SR,6R)-2-(hydroxymethyl)-4-(4-(3,4,5-trifluorophenyl)- 1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 569.23 [M+H]+; ¹H-NMR (400 MHz, CD₃OD) δ = 8.52 (S, 1H), 7.97-7.93 (m, 4H), 7.66-7.52 (m, 5H), 5.24 (d, J = 14.1 Hz, 1H), 5.09 (d, J = 11.0 Hz, 1H), 4.32 (d, J = 11.7 Hz, 1H), 4.24 (t, J = 6.1 Hz, 1H), 4.20 (S, bR, 1H), 3.77-3.71 (m, 3H), 3.19 (d, J = 14.1 Hz, 1H), 3.12 (t, J = 12.5 Hz, 1H), 2.23-2.01 (m, 2H), 1.91-1.84 (m, 1H), 1.48 (d, J = 12.1 Hz, 1H)

### Example 172: Synthesis of Compound A187

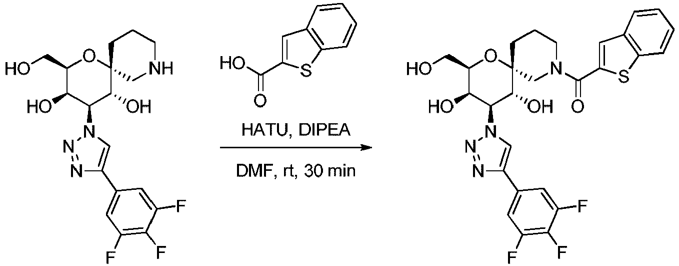

### Synthesis of benzo[b]thiophen-2-yl((2R,3R,4R,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)methanone

The title compound (12.4 mg, yield: 90%) as a white solid was synthesized from (2*R*,3*R*,4*R*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 575.15 [M+H]+; ¹H-NMR (400 MHz, CD₃OD) δ = 8.50-8.50 (m, 1H), 7.93-7.91 (m, 2H), 7.64-7.61 (m, 3H), 7.44-7.42 (m, 2H), 5.18-5.04 (m, 2H), 4.32-4.29 (m, 1H), 4.18-4.18 (m, 3H), 3.69-3.67 (m, 2H), 3.20-3.19 (m, 2H), 2.26-2.10 (m, 2H), 1.87-1.84 (m, 1H), 1.62-1.59 (m, 1H)

### Example 173: Synthesis of Compound A188

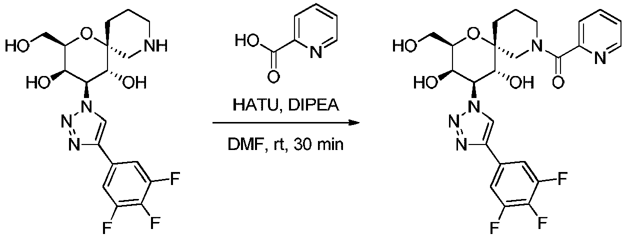

### Synthesis of ((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(pyridin-2-yl)methanone

The title compound (10.5 mg, yield: 84%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 520.27 [M+H]+; ¹H-NMR (400 MHz, CD₃OD) δ = 8.68 (d, J = 4.7 Hz, 0.2H), 8.60 (d, J = 4.7 Hz, 0.8H), 8.51 (S, 0.8H), 8.43 (S, 0.2H), 8.03-7.94 (m, 1H), 7.76 (d, J = 7.8 Hz, 0.2H), 7.66-7.55 (m, 3H), 7.50 (dd, J = 7.0, 5.5 Hz, 0.8H), 5.17 (d, J = 13.7 Hz, 0.8H), 5.06 (dd, J = 11.5, 2.5 Hz, 0.8H), 4.60-4.50 (m, 0.4H), 4.30 (d, J = 11.7 Hz, 0.8H), 4.23-4.17 (m, 2H), 3.84 (S, bR, 0.2H), 3.76-3.59 (m, 3.2H), 3.18 (d, J = 14.1 Hz, 0.8H), 3.13-3.07 (m, 0.8H), 3.03-2.93 (m, 0.4H), 2.23-2.02 (m, 2H), 1.83 (d, J = 12.9 Hz, 1H), 1.70 (d, J = 12.5 Hz, 0.2H), 1.47 (d, J = 13.3 Hz, 0.8H)

### Example 174: Synthesis of Compound A189

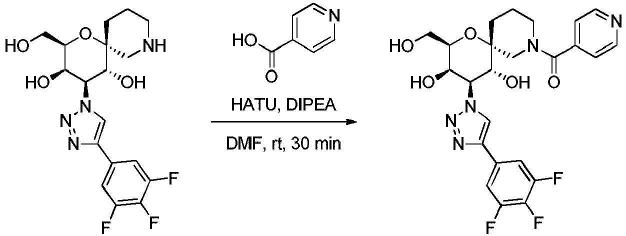

### Synthesis of ((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(pyridin-4-yl)methanone

The title compound (9.7 mg, yield: 78%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 520.21 [M+H]+; ¹H-NMR (400 MHz, CD₃OD) δ = 8.70 (d, J = 5.5 Hz, 0.2H), 8.66 (d, J = 5.5 Hz, 1.8H), 8.50 (S, 0.9H), 8.39 (S, 0.1H), 7.66-7.56 (m, 2.2H), 7.48 (d, J = 5.5 Hz, 1.8H), 5.16 (d, J = 13.7 Hz, 0.9H), 5.05 (dd, J = 11.3, 2.3 Hz, 0.9H), 4.65-4.48 (m, 0.2H), 4.30 (d, J = 11.3 Hz, 0.9H), 4.21-4.15 (m, 2H), 3.93 (d, J = 14.5 Hz, 0.1H), 3.76-3.63 (m, 2.1H), 3.52 (d, J = 12.1 Hz, 0.9H), 3.18-3.09 (m, 1.8H), 3.01 (t, J = 6.1 Hz, 0.1H), 2.87 (t, J = 12.1 Hz, 0.1H), 2.23-2.00 (m, 2H), 1.84 (d, J = 12.5 Hz, 1H), 1.67 (d, J = 12.5 Hz, 0.1H), 1.50

### (d, J = 12.9 Hz, 0.9H)

### Example 175: Synthesis of Compound A190

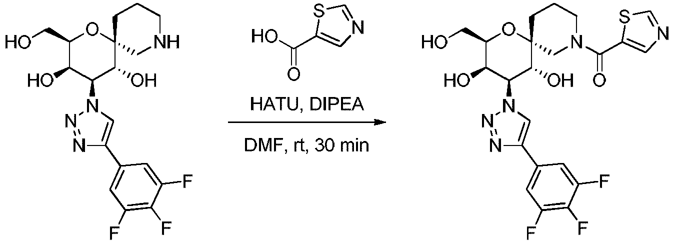

### Synthesis of ((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(thiazol-5-yl)methanone

The title compound (10.8 mg, yield: 86%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 526.13 [M+H]+; ¹H-NMR (400 MHz, CD₃OD) δ = 8.46 (d, J = 5.1 Hz, 1H), 8.05 (d, J = 2.7 Hz, 0.3H), 7.94 (d, J = 3.1 Hz, 0.7H), 7.84-7.79 (m, 1H), 7.63-7.59 (m, 2H), 6.08 (d, J = 14.9 Hz, 0.3H), 5.32 (d, J = 13.3 Hz, 0.7H), 5.13 (d, J = 13.7 Hz, 0.7H), 5.02 (dd, J = 11.3, 2.3 Hz, 0.7H), 4.80 (dd, J = 11.9, 2.2 Hz, 0.7H), 4.61 (d, J = 13.3 Hz, 0.3H), 4.28-4.13 (m, 1H), 4.18 (S, bR, 0.7H), 4.15 (t, J = 6.3 Hz, 0.7H), 4.02 (S, bR, 0.3H), 3.67-3.40 (m, 2.6H), 3.20 (d, J = 14.1 Hz, 0.7H), 3.13 (t, J = 11.9 Hz, 0.7H), 2.87 (t, J = 11.7 Hz, 0.3H), 2.24-2.01 (m, 2H), 1.83 (d, J = 12.1 Hz, 1H), 1.68 (d, J = 14.5 Hz, 0.3H), 1.61 (d, J = 12.9 Hz, 0.7H)

### Example 176: Synthesis of Compound A191

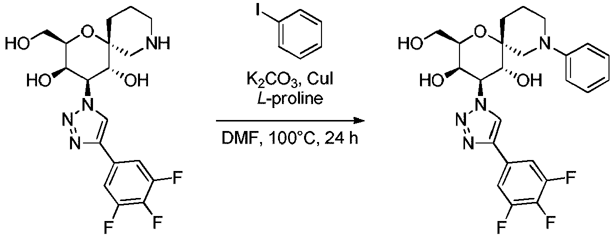

### Synthesis of (2R,3R,4S,5R,6R)-2-(hydroxymethyl)-8-phenyl-4-(4-(3,4,5-trifluorophenyl)-1H-1,2,3 -triazol-1-yl)-1-oxa-8-azaspiro[5.5]undecane-3,5-diol

(2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (25 mg, 0.060 mmol) and iodobenzene (20 µL, 0.180 mmol) were dissolved in 1 mL of N,N-dimethylformamide, and then patassium carbonate (24.9 mg, 0.180 mmol), copper(I) iodide (5.7 mg, 0.03 mmol), and L-proline (6.9 mg, 0.06 mmol) were added thereto. The reaction mixture was stirred at 100 °C for 24 hours. The reaction mixture was diluted with 10 mL of ethyl acetate, and then washed with a saturated aqueous ammonium chloride solution (3 mL x 3 times). The organic layer was dried over anhydrous sodium sulfate, filtered, and then concentrated under reduced pressure. The remaining concentrate was subjected to silica gel column chromatography with 0% to 4% methanol/dichloromethane to synthesize the title compound as a brown solid (11.1 mg, yield: 38%).

ESIMS: m/z 491.22 [M+H]+; ¹H-NMR (400 MHz, CD₃OD) δ = 8.48 (S, 1H), 7.64-7.61 (m, 2H), 7.25 (t, J = 7.8 Hz, 2H), 7.07 (d, J = 7.8 Hz, 2H), 6.83 (t, J = 7.2 Hz, 1H), 5.07 (dd, J = 11.5, 2.5 Hz, 1H), 4.22 (d, J = 11.7 Hz, 1H), 4.15-4.12 (m, 2H), 3.89 (t, J = 5.9 Hz, 1H), 3.62-3.55 (m, 2H), 3.47 (dd, J = 10.8, 5.7 Hz, 1H), 3.04 (d, J = 13.7 Hz, 1H), 2.75 (t, J = 11.0 Hz, 1H), 2.27-2.15 (m, 1H), 2.11-2.01 (m, 1H), 1.78 (d, J = 12.5 Hz, 1H), 1.64 (d, J = 12.1 Hz, 1H)

### Example 177: Synthesis of Compound A192

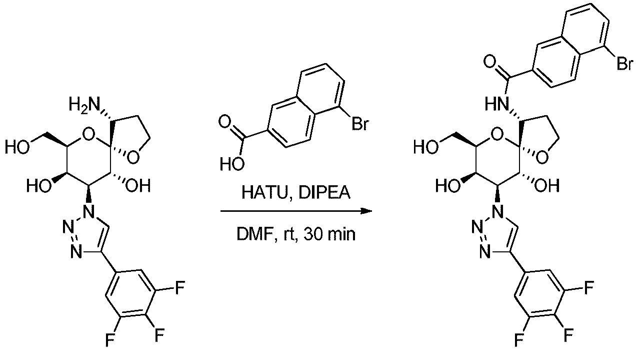

### Synthesis of 5-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

The title compound (15.7 mg, yield: 99%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 649.18 [M+H]+; ¹H-NMR (400 MHz, CD₃OD) δ = 8.56 (S, 1H), 8.47 (S, 1H), 8.27 (d, J = 9.0 Hz, 1H), 8.06 (dd, J = 9.0, 1.6 Hz, 1H), 8.01 (d, J = 8.2 Hz, 1H), 7.94-7.90 (m, 1H), 7.61-7.57 (m, 2H), 7.45 (t, J = 7.8 Hz, 1H), 5.11-5.07 (m, 2H), 4.50 (d, J = 11.0 Hz, 1H), 4.23-4.19 (m, 2H), 4.14-4.06 (m, 2H), 3.88 (dd, J = 11.3, 7.8 Hz, 1H), 3.78 (dd, J = 11.3, 4.7 Hz, 1H), 2.52-2.44 (m, 1H), 2.30-2.20 (m, 1H)

### Example 178: Synthesis of Compound A193

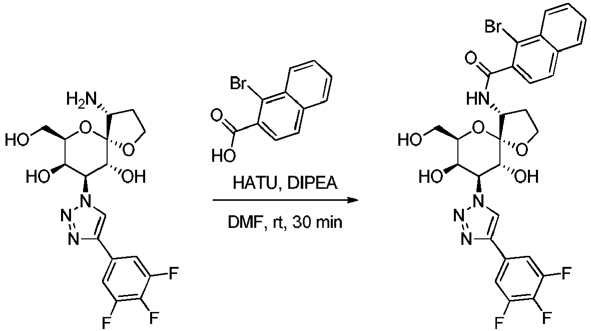

### Synthesis of 1-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

The title compound (14.0 mg, yield: 90%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 649.12 [M+H]+; ¹H-NMR (400 MHz, CD₃OD) δ = 8.53 (S, 1H), 8.37 (d, J = 8.6 Hz, 1H), 7.99-7.94 (m, 2H), 7.71-7.55 (m, 5H), 5.10-5.01 (m, 2H), 4.61 (d, J = 11.3 Hz, 1H), 4.20-4.06 (m, 4H), 3.79 (dd, J = 11.5, 6.8 Hz, 1H), 3.71 (dd, J = 11.3, 5.1 Hz, 1H), 2.55-2.47 (m, 1H), 2.26-2.16 (m, 1H)

### Example 179: Synthesis of Compound A194

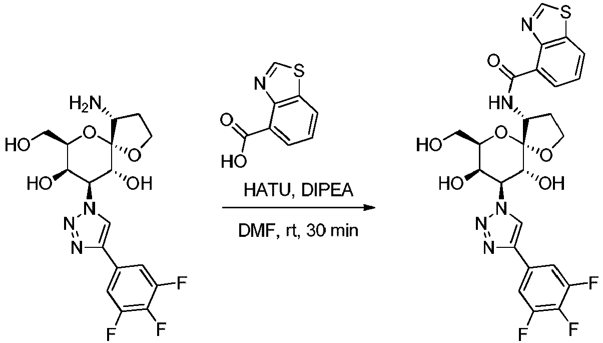

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[d]thiazole-4-carboxamide

The title compound (12.9 mg, yield: 93%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 578.17 [M+H]+; ¹H-NMR (400 MHz, CD₃OD) δ = 9.33 (S, 1H), 8.52 (S, 1H), 8.24 (d, J = 7.8 Hz, 1H), 8.16 (d, J = 7.8 Hz, 1H), 7.70 (t, J = 7.8 Hz, 1H), 7.59-7.55 (m, 2H), 5.12-5.08 (m, 2H), 4.45 (d, J = 11.3 Hz, 1H), 4.21 (dd, J = 7.8, 5.1 Hz, 2H), 4.09 (dd, J = 16.2, 8.0 Hz, 2H), 3.86 (dd, J = 11.3, 7.8 Hz, 1H), 3.77 (dd, J = 11.3, 4.7 Hz, 1H), 2.52-2.45 (m, 1H), 2.29-2.19 (m, 1H)

### Example 180: Synthesis of Compound A195

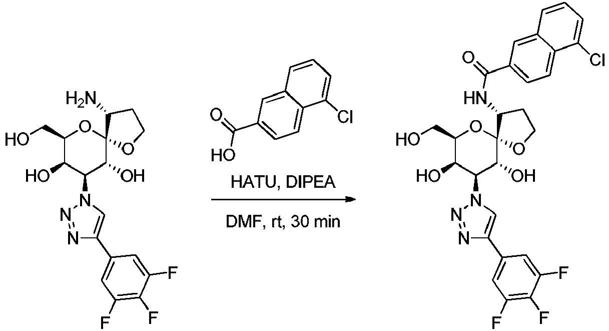

### Synthesis of 5-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

The title compound (14.5 mg, yield: 99%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 605.20 [M+H]+; ¹H-NMR (400 MHz, CD₃OD) δ = 8.57 (S, 1H), 8.48 (S, 1H), 8.30 (d, J = 9.0 Hz, 1H), 8.07 (dd, J = 8.8, 1.4 Hz, 1H), 7.97 (d, J = 8.2 Hz, 1H), 7.70 (d, J = 7.4 Hz, 1H), 7.60 (t, J = 7.6 Hz, 2H), 7.52 (t, J = 8.0 Hz, 1H), 5.12-5.07 (m, 2H), 4.50 (d, J = 11.3 Hz, 1H), 4.23-4.19 (m, 2H), 4.14-4.05 (m, 2H), 3.88 (dd, J = 11.5, 7.6 Hz, 1H), 3.78 (dd, J = 11.3, 4.7 Hz, 1H), 2.52-2.43 (m, 1H), 2.31-2.19 (m, 1H)

### Example 181: Synthesis of Compound A196

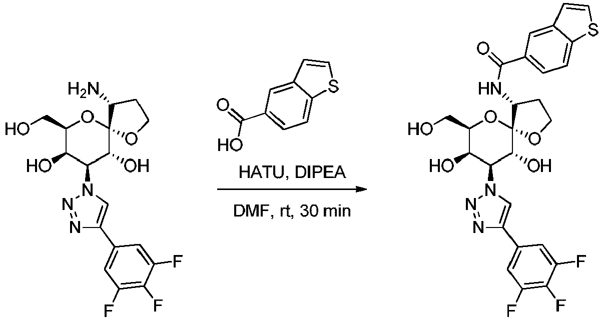

### Synthesis of N-((4R,5S,7R,8R,9S,10R)-8,10-dihydroxy-7-(hydroxymethyl)-9-(4-(3,4,5-trifhiorophenyl)-1H-1,2,3-triazol-1-yl)-1,6-dioxaspiro[4.5]decan-4-yl)benzo[b]thiophene-5-carboxamide

The title compound (12.4 mg, yield: 90%) as a white solid was synthesized from (4*R*,5*S*,7*R*,8*R*,9*S*,10*R*)-4-amino-9-(4-(3,4, 5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 577.16 [M+H]+; ¹H-NMR (400 MHz, CD₃OD) δ = 8.54 (S, 1H), 8.41 (S, 1H), 8.01 (d, J = 8.6 Hz, 1H), 7.86 (d, J = 8.2 Hz, 1H), 7.69 (d, J = 5.5 Hz, 1H), 7.62-7.58 (m, 2H), 7.49 (d, J = 5.5 Hz, 1H), 5.10-5.03 (m, 2H), 4.46 (d, J = 11.3 Hz, 1H), 4.21-4.18 (m, 2H), 4.12-4.05 (m, 2H), 3.86 (dd, J = 11.3, 7.8 Hz, 1H), 3.76 (dd, J = 11.3, 4.7 Hz, 1H), 2.51-2.43 (m, 1H), 2.27-2.17 (m, 1H)

### Example 182: Synthesis of Compound A197

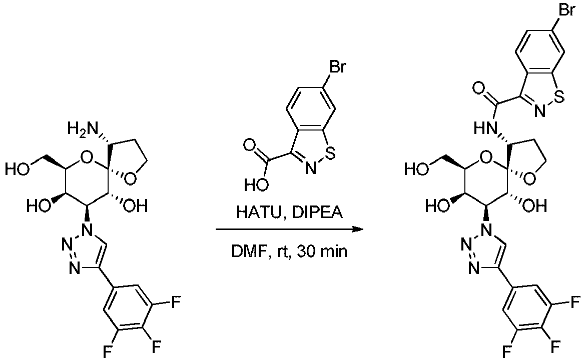

### Synthesis of 6-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[d]isothiazole-3-carboxamide

The title compound (14.2 mg, yield: 90%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 656.05 [M+H]+; ¹H-NMR (400 MHz, CD₃OD) δ = 8.73 (d, J = 8.6 Hz, 1H), 8.59 (S, 1H), 8.34 (S, 1H), 7.66 (dd, J = 9.0, 1.6 Hz, 1H), 7.59-7.55 (m, 2H), 5.09 (dd, J = 11.3, 2.7 Hz, 1H), 5.03 (t, J = 9.4 Hz, 1H), 4.51 (d, J = 11.3 Hz, 1H), 4.22-4.17 (m, 2H), 4.12 (d, J = 1.6 Hz, 1H), 4.06 (q, J = 8.1 Hz, 1H), 3.86 (dd, J = 11.3, 7.4 Hz, 1H), 3.77 (dd, J = 11.3, 5.1 Hz, 1H), 2.47-2.41 (m, 1H), 2.28-2.18 (m, 1H)

### Example 183: Synthesis of Compound A198

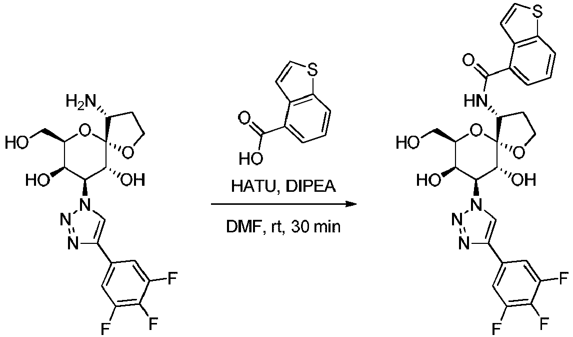

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-4-carboxamide

The title compound (13.5 mg, yield: 98%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 577.16 [M+H]+; ¹H-NMR (400 MHz, CD₃OD) δ = 8.56 (S, 1H), 8.08 (d, J = 8.2 Hz, 1H), 8.00 (d, J = 5.5 Hz, 1H), 7.74-7.69 (m, 2H), 7.63-7.60 (m, 2H), 7.44 (t, J = 7.6 Hz, 1H), 5.12-5.08 (m, 2H), 4.54 (d, J = 11.3 Hz, 1H), 4.21-4.16 (m, 3H), 4.09 (q, J = 8.1 Hz, 1H), 3.86 (dd, J = 11.3, 7.4 Hz, 1H), 3.76 (dd, J = 11.3, 4.7 Hz, 1H), 2.51-2.43 (m, 1H), 2.23-2.13 (m, 1H)

### Example 184: Synthesis of Compound A199

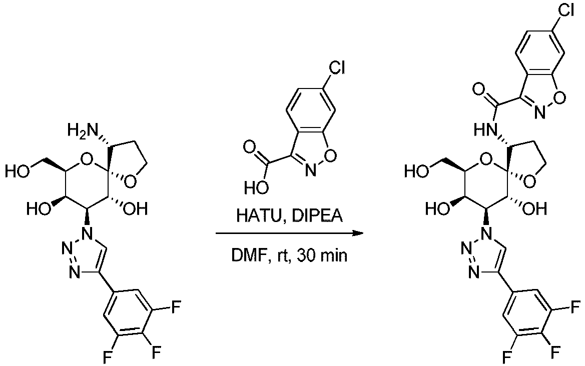

### Synthesis of 6-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[d]isoxazole-3-carboxamide

The title compound (13.5 mg, yield: 94%) as a white solid was synthesized from (*4R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 596.19 [M+H]+; ¹H-NMR (400 MHz, CDCl₃) δ = 7.93 (S, 1H), 7.87-7.81 (m, 4H), 7.50-7.45 (m, 5H), 6.15 (d, J = 9.0 Hz, 1H), 5.10 (dd, J = 11.3, 2.3 Hz, 1H), 4.85 (q, J = 9.3 Hz, 1H), 4.37-4.31 (m, 2H), 4.06-3.95 (m, 3H), 3.86-3.76 (m, 4H), 2.35-2.17 (m, 3H), 1.88-1.77 (m, 1H), 1.33 (S, 3H), 1.29 (S, 3H)

### Example 185: Synthesis of Compound A200

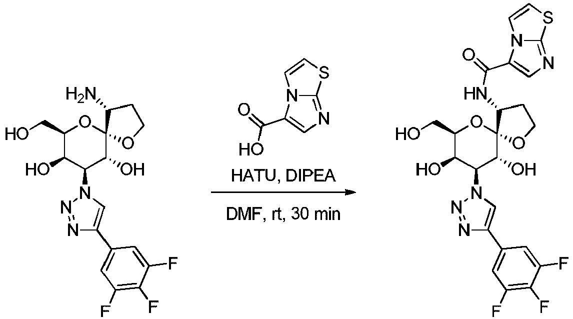

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)imidazo[2,1-b]thiazole-5-carboxamide

The title compound (12.9 mg, yield: 95%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 567.15 [M+H]+; ¹H-NMR (400 MHz, MeOD) δ = 8.70 (S, 1H), 8.42 (d, J = 4.6 Hz, 1H), 7.96 (d, J = 0.7 Hz, 1H), 7.64-7.56 (m, 2H), 7.32 (dd, J = 4.6, 1.0 Hz, 1H), 5.11-5.02 (m, 2H), 4.56 (d, J = 11.2 Hz, 1H), 4.21-4.13 (m, 3H), 3.99 (q, J = 8.2 Hz, 1H), 3.88 (dd, J = 11.4, 7.5 Hz, 1H), 3.80 (dd, J = 11.5, 4.9 Hz, 1H), 2.40-2.33 (m, 1H), 2.28-2.18 (m, 1H)

### Example 186: Synthesis of Compound A201

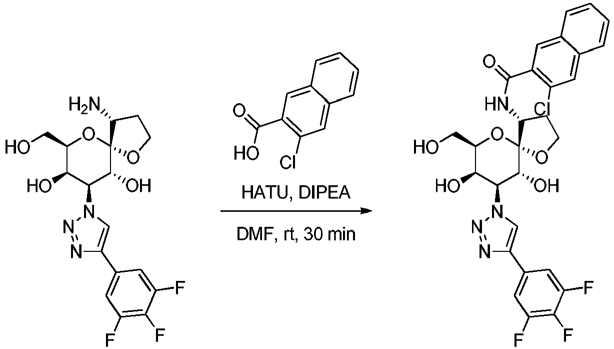

### Synthesis of 3-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-naphthamide

The title compound (12.8 mg, yield: 88%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 605.14 [M+H]+; ¹H-NMR (400 MHz, MeOD) δ = 8.56 (S, 1H), 8.18 (S, 1H), 8.04 (S, 1H), 7.98 (d, J = 7.6 Hz, 1H), 7.90 (d, J = 7.6 Hz, 1H), 7.69-7.58 (m, 4H), 5.11 (dd, J = 11.1, 2.8 Hz, 1H), 5.06-5.02 (m, 1H), 4.58 (d, J = 11.2 Hz, 1H), 4.24-4.09 (m, 4H), 3.84 (dd, J = 11.4, 7.0 Hz, 1H), 3.77 (dd, J = 11.5, 4.9 Hz, 1H), 2.57-2.50 (m, 1H), 2.27-2.17 (m, 1H)

### Example 187: Synthesis of Compound A202

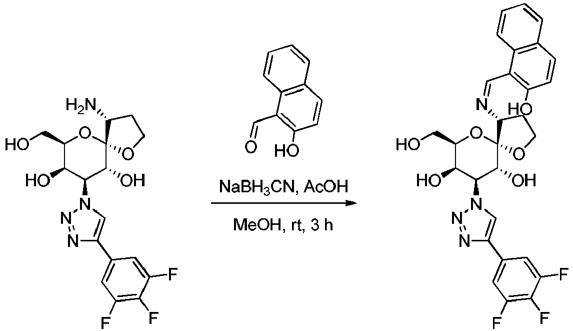

### Synthesis of (4R,5S,7R,8R,9S,10R)-7-(hydroxymethyl)-4-((Z)-((2-hydroxynaphthalen-1-yl)methylene)amino)-9-(4-(3,4,5-trifluorophenyl)-1H-1,2,3-triazol-1-yl)-1,6-dioxaspiro[4.5]decane-8,10-diol

(4*R*,5*S*,7*R,*8*R*,9*S*,10*R*)*-*4-amino-7-(hydroxymethyl)-9-(4-(3,4,5-trifluorophenyl)-1*H-*1,2,3-triazol-1-yl)-1,6-dioxaspiro[4.5]decane-8,10-diol (10 mg, 0.024 mmol), 2-hydroxy-1-naphthaldehyde (12.4 mg, 0.048 mmol) and acetic acid (1 drop, excessive) were dissolved in 0.6 mL of methanol, and then sodium cyanoborohydride (4.52 mg, 0.072 mmol) was added thereto. The reaction mixture was stirred at room temperature for about 3 hours. The reaction solution was diluted with dichloromethane (5 mL), and then washed with a saturated aqueous sodium hydrogen carbonate solution (3 mL x 2). The organic layer was dried over anhydrous sodium sulfate, filtered, and then concentrated under reduced pressure. The concentrate was subjected to silica gel column chromatography with 0% to 7% methanol/dichloromethane to synthesize the title compound as a white solid (11.4 mg, yield: 83%).

ESIMS: m/z 571.14 [M+H]+; ¹H-NMR (400 MHz, MeOD) δ = 9.08 (S, 1H), 8.54 (S, 1H), 8.03 (d, J = 8.3 Hz, 1H), 7.78 (d, J = 9.3 Hz, 1H), 7.63 (d, J = 7.0 Hz, 1H), 7.60-7.53 (m, 2H), 7.49-7.44 (m, 1H), 7.27-7.23 (m, 1H), 6.79 (d, J = 9.3 Hz, 1H), 5.13 (dd, J = 11.3, 3.0 Hz, 1H), 4.66-4.62 (m, 1H), 4.46 (d, J = 11.3 Hz, 1H), 4.28 (td, J = 8.8, 3.1 Hz, 1H), 4.23-4.20 (m, 1H), 4.17-4.11 (m, 2H), 3.93 (dd, J = 11.5, 6.5 Hz, 1H), 3.84 (dd, J = 11.6, 5.4 Hz, 1H), 2.65-2.57 (m, 1H), 2.49-2.39 (m, 1H)

### Example 188: Synthesis of Compound A203

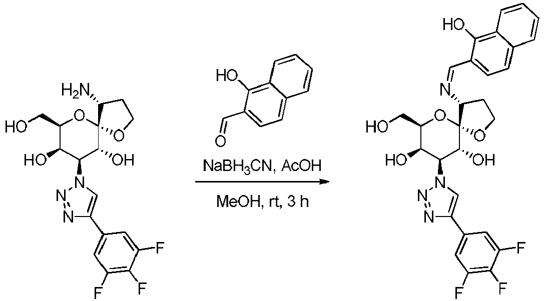

### Synthesis of (4R,5S,7R,8R,9S,10R)-7-(hydroxymethyl)-4-((Z)-((1-hydroxynaphthalen-2-yl)methylene)amino)-9-(4-(3,4,5-trifluorophenyl)-1H-1,2,3-triazol-1-yl)-1,6-dioxaspiro[4.5]decane-8,10-diol

The title compound (8.6 mg, yield: 62%) as a white solid was synthesized from (4*R*,5*S*,7*R*,8*R*,9*S*,10*R*)-4-amino-7-(hydroxymethyl)9-(4-(3,4,5-trifluorophenyl)-1*H*-1,2,3-triazol-1-yl)-1,6-dioxaspiro[4.5]decane-8,10-diol (10 mg, 0.024 mmol) in a similar manner to the preparation method of (4*R*,5*S*,7*R*,8*R*,9*S*,10*R*)-7-(hydroxymethyl)-4-((Z)-((2-hydroxynaphthalen-1-yl)methylene)amino)-9-(4-(3,4,5-trifluorophenyl)-1*H*-1,2,3-triazol-1-yl)-1,6-dioxaspiro[4.5]decane-8, 10-diol.

ESIMS: m/z 571.24 [M+H]+; ¹H-NMR (400 MHz, MeOD) δ = 8.56 (S, 1H), 8.39 (d, J = 7.8 Hz, 1H), 8.21 (S, 1H), 7.64-7.55 (m, 4H), 7.45-7.41 (m, 1H), 7.07 (d, J = 8.8 Hz, 1H), 6.82 (d, J = 9.0 Hz, 1H), 5.13 (dd, J = 11.1, 2.9 Hz, 1H), 4.57-4.53 (m, 1H), 4.46 (d, J = 11.3 Hz, 1H), 4.28 (td, J = 8.9, 3.2 Hz, 1H), 4.24-4.21 (m, 1H), 4.16-4.11 (m, 2H), 3.97 (dd, J = 11.8, 6.8 Hz, 1H), 3.85 (dd, J = 11.6, 5.1 Hz, 1H), 2.67-2.55 (m, 1H), 2.48-2.37 (m, 1H)

### Example 189: Synthesis of Compound A204

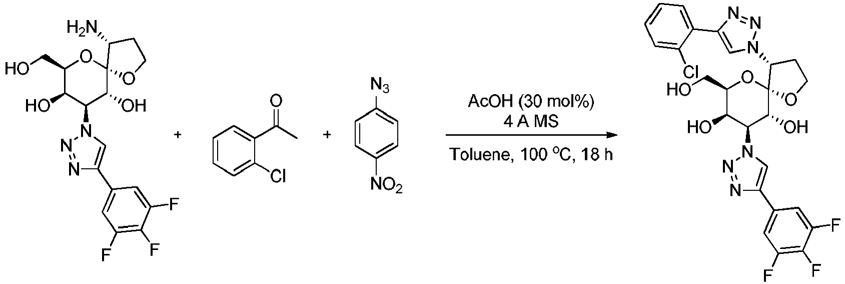

### Synthesis of (4R,5S,7R,8R,9S,10R)-4-(4-(2-chlorophenyl)-1H-1,2,3-triazol-1-yl)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol), 1-(2-chlorophenyl)ethanone (7.7 mg, 0.047 mmol), azidonitrophenyl (7.7 mg, 0.047 mmol), and 4 Å molecular sieves were dissolved in 2 mL of toluene, followed by stirring at 100 °C for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (4.0 mg, yield: 19%).

ESIMS: m/z 579.18 [M+H]+; ¹H-NMR (400 MHz, CD₃OD) δ 8.12 (s, 1H), 7.80-7.77 (m, 1H), 7.73-7.68 (m, 1H), 7.63-7.52 (m, 5H), 5.10 (dd, J = 8.1, 5.7 Hz, 1H), 4.97-4.90 (m, 1H), 4.45 (dd, J = 15.6, 8.2 Hz, 1H), 4.27-4.17 (m, 1H), 4.10-4.00 (m, 2H), 3.83 (d, J = 11.0 Hz, 1H), 3.51 (t, J = 6.6 Hz, 2H), 3.06-2.96 (m, 1H), 2.65-2.55 (m, 1H), 2.02 (d, J = 10.0 Hz, 1H).

### Example 190: Synthesis of Compound A205

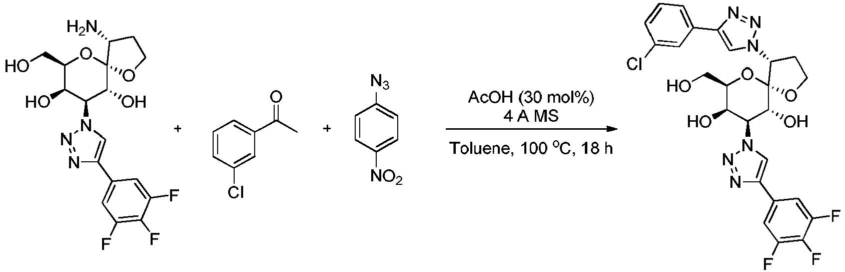

### Synthesis of (4R,5S,7R,8R,9S,10R)-4-(4-(3-chlorophenyl)-1H-1,2,3-triazol-1-yl)-7-(hydroxymethyl)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol), 1-(3-chlorophenyl)ethanone (7.7 mg, 0.047 mmol), azidonitrophenyl (7.7 mg, 0.047 mmol), and 4 Å molecular sieves were dissolved in 2 mL of toluene, followed by stirring at 100 °C for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (4.0 mg, yield: 22%).

ESIMS: m/z 579.18 [M+H]+; ¹H-NMR (400 MHz, CD₃OD) δ 8.14 (s, 1H), 7.79 (s, 1H), 7.72 (s, 1H), 7.64-7.51 (m, 5H), 5.23 (dd, J = 8.6, 4.6 Hz, 1H), 4.96 (dd, J = 11.1, 2.8 Hz, 1H), 4.49 (dd, J = 15.1, 7.3 Hz, 1H), 4.24 (dd, J = 10.5, 3.2 Hz, 1H), 4.07-3.99 (m, 2H), 3.88 (d, J = 11.0 Hz, 1H), 3.50 (dd, J = 11.2, 6.6 Hz, 1H), 3.41 (dd, J = 10.8, 6.0 Hz, 1H), 3.03-2.92 (m, 1H), 2.64-2.53 (m, 1H), 2.05-1.99 (m, 1H).

### Example 191: Synthesis of Compound A206

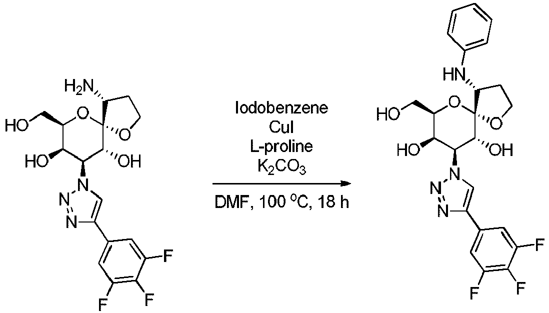

### Synthesis of (4R,5S,7R,8R,9S,10R)-7-(hydroxymethyl)-4-(phenylamino)-9-(4-(3,4,5-trifluorophenyl)-1H-1,2,3-triazol-1-yl)-1,6-dioxaspiro[4.5]decane-8,10-diol

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (20.0 mg, 0.048 mmol), iodobenzene (14.7 mg, 0.072 mmol), CuI (4.6 mg, 0.024 mmol), L-proline (5.5 mg, 0.048 mmol), and potassium carbonate (20 mg, 0.144 mmol) were dissolved in 20 mL of *N*,*N*-dimethylformamide, followed by stirring at 100 °C for 18 hours. The reaction solution was filtered through Celite, and then the filtrate was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (7.9 mg, yield: 34%).

ESIMS: m/z 493.18 [M+H]+; ¹H-NMR (400 MHz, MeOD) δ 8.47 (s, 1H), 7.61 (dd, J = 8.9, 6.6 Hz, 2H), 7.16-7.10 (m, 2H), 6.77-6.73 (m, 2H), 6.64 (t, J = 7.3 Hz, 1H), 5.08 (dd, J = 11.3, 2.9 Hz, 1H), 4.44 (d, J = 11.3 Hz, 1H), 4.35 (dd, J = 9.8, 8.1 Hz, 1H), 4.20-4.02 (m, 5H), 3.86-3.75 (m, 2H), 2.55-2.46 (m, 1H), 1.93 (td, J = 9.9, 2.1 Hz, 1H).

### Example 192: Synthesis of Compound A207

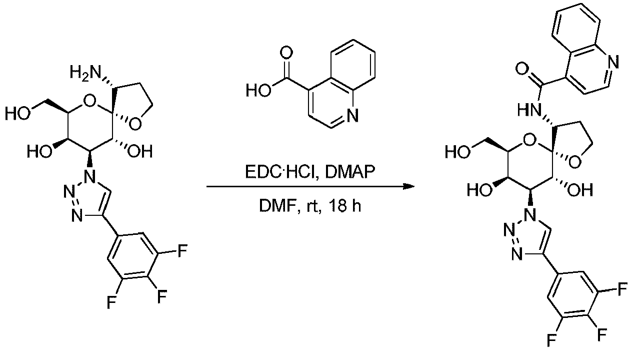

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-4-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of*N*,*N*-dimethylformamide, and then quinoline-4-carboxylic acid (7.0 mg, 0.0396 mmol), *N*-(3-dimethylaminopropyl)-*N'*-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (10.5 mg, yield: 49%).

ESIMS: m/z 572.25 [M+H]+; ¹H-NMR (400 MHz, MeOD) δ 8.95 (d, J = 4.4 Hz, 1H), 8.57 (s, 1H), 8.42 (d, J = 7.9 Hz, 1H), 8.10 (d, J = 8.4 Hz, 1H), 7.84 (ddd, J = 8.4, 6.9, 1.3 Hz, 1H), 7.70 (ddd, J = 8.2, 7.0, 1.1 Hz, 1H), 7.66 (d, J = 4.4 Hz, 1H), 7.63 (dd, J = 8.9, 6.6 Hz, 2H), 5.18-5.06 (m, 2H), 4.67 (d, J = 11.2 Hz, 1H), 4.21-4.14 (m, 3H), 4.09 (dd, J = 15.9, 8.2 Hz, 1H), 3.77 (qd, J = 11.3, 6.1 Hz, 2H), 2.48 (ddd, J = 8.2, 4.7, 2.5 Hz, 1H), 2.23-2.13 (m, 1H).

### Example 193: Synthesis of Compound A208

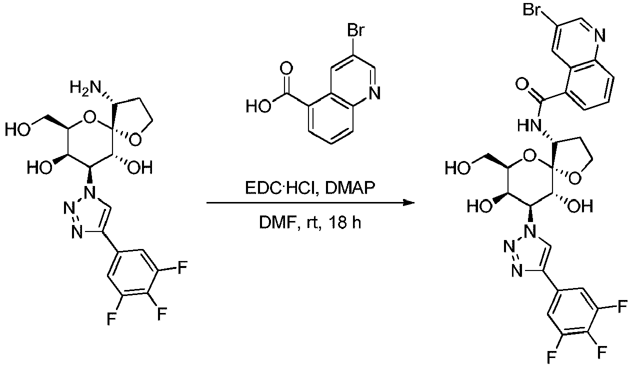

### Synthesis of 3-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-5-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of N,N-dimethylformamide, and then 3-bromoqunoline-5-carboxylic acid (10.0 mg, 0.0396 mmol), N-(3-dimethylaminopropyl)-N-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (15 mg, yield: 64%).

ESIMS: m/z 650.19 [M+H]+; ¹H-NMR (400 MHz, MeOD) δ 9.00 (dd, J = 2.2, 0.7 Hz, 1H), 8.94 (d, J = 2.3 Hz, 1H), 8.54 (s, 1H), 8.15 (d, J = 8.4 Hz, 1H), 7.94 (dd, J = 7.1, 1.1 Hz, 1H), 7.84 (dd, J = 8.4, 7.2 Hz, 1H), 7.60 (dd, J = 8.9, 6.6 Hz, 2H), 5.10-5.04 (m, 2H), 4.60 (d, J = 11.2 Hz, 1H), 4.21-4.05 (m, 4H), 3.76 (qd, J = 11.4, 6.1 Hz, 2H), 2.53-2.44 (m, 1H), 2.24-2.16 (m, 1H).

### Example 194: Synthesis of Compound A209

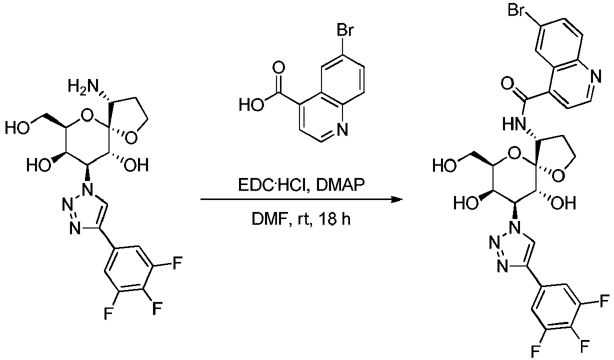

### Synthesis of 6-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-4-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then 6-bromoqunoline-4-carboxylic acid (10.0 mg, 0.0396 mmol), *N*-(3-dimethylaminopropyl)-N-ethylcarbodiimide hydrochloride (13.8 mg, 0.072 mmol), and 4-dimethylaminopyridine (0.22 mg, 0.002 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subj ected to amine silica gel column chromatography with 0% to 5% methanol/dichloromethane to obtain the title compound (9.3 mg, yield: 40%).

ESIMS: m/z 650.13 [M+H]+; ¹H-NMR (400 MHz, MeOD) δ 8.98 (d, J = 4.4 Hz, 1H), 8.55 (s, 1H), 8.49 (d, J = 2.1 Hz, 1H), 8.01 (d, J = 9.0 Hz, 1H), 7.93 (dd, J = 9.0, 2.2 Hz, 1H), 7.72 (d, J = 4.4 Hz, 1H), 7.62 (dd, J = 8.9, 6.6 Hz, 2H), 5.11-5.05 (m, 2H), 4.60 (d, J = 11.2 Hz, 1H), 4.21-4.06 (m, 4H), 3.75 (qd, J = 11.4, 6.1 Hz, 2H), 2.57-2.47 (m, 1H), 2.25-2.13 (m, 1H).

### Example 195: Synthesis of Compound A210

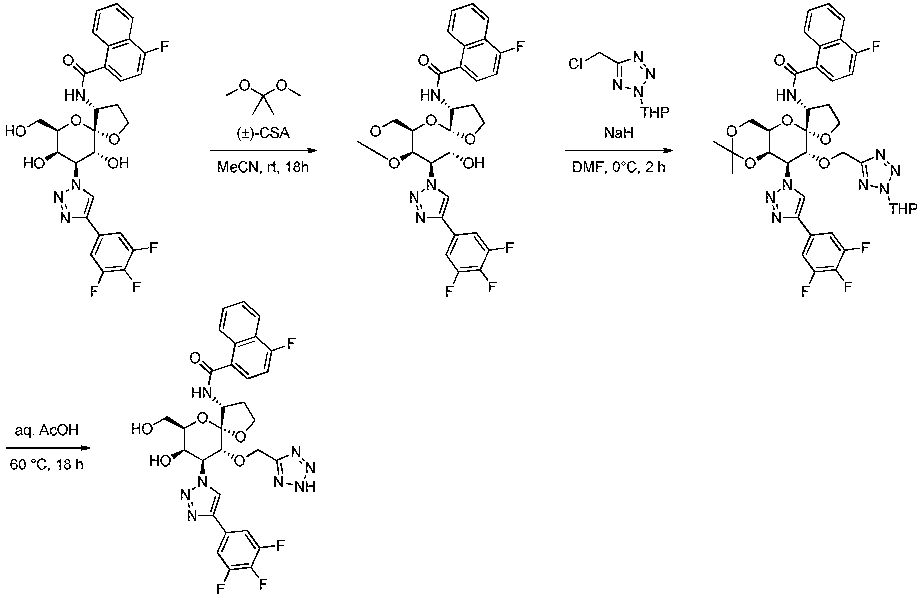

### Synthesis of 4-fluoro-N-((2S,3R,4a'R,7'R,8'R,8a'R) -7'-hydroxy-2',2'-dimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-3-yl)-1-naphthamide

*N*-(4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1-naphthamide (31.2 mg, 0.055 mmol) and 2,2-dimethoxypropane (10 µL, 0.083 mmol) were dissolved in 1 mL of acetonitrile, and then (±)-camphorsulfonic acid (2.6 mg, 0.011 mmol) was added. The reaction mixture was stirred at room temperature for about 18 hours. The reaction mixture was diluted with 10 mL of ethyl acetate, and then washed with a saturated aqueous sodium hydrogen carbonate solution (3 mL x 2 times). The organic layer was dried over anhydrous sodium sulfate, filtered, and then concentrated under reduced pressure. The remaining concentrate was subjected to silica gel column chromatography with 0% to 100% ethyl acetate/n-hexane to obtain the title compound as a white solid (24.9 mg, yield: 72%).

¹H-NMR (400 MHz, CDCl₃) δ = 8.51 (d, J = 8.2 Hz, 1H), 8.16 (d, J = 7.8 Hz, 1H), 7.98 (S, 1H), 7.80 (dd, J = 7.6, 5.3 Hz, 1H), 7.68-7.59 (m, 2H), 7.42 (t, J = 7.4 Hz, 2H), 7.15-7.11 (m, 1H), 6.56 (d, J = 9.0 Hz, 1H), 5.21 (dd, J = 11.2, 2.9 Hz, 1H), 5.14 (q, J = 9.4 Hz, 1H), 4.58 (d, J = 11.0 Hz, 1H), 4.44 (d, J = 2.0 Hz, 1H), 4.19-4.08 (m, 3H), 3.94 (d, J = 13.3 Hz, 1H), 3.90 (S, 1H), 2.53 (t, J = 3.7 Hz, 1H), 2.16-2.06 (m, 1H), 1.71 (S, 1H), 1.29 (S, 3H), 1.28 (S, 3H)

### Synthesis of N-((2S,3R,4a'R,7'R,8'S,8a'R)-2',2'-dimethyl-7'-((2-tetrahydro-2H-pyran-2-yl)-2H-tetrazol-5-yl)methoxy)-8'-(4-(3,4,5-trifluorophenyl)-1H-1,2,3-triazol-1-yl)hexahydro-3H,4'H-spiro[furan-2,6'-pyrano[3,2-d][1,3]dioxin]-3-yl)-4-fluoro-1-naphthamide

The title compound (16.9 mg, yield: 54%) as a white solid was synthesized from 4-fluoro-*N*-((2*S*,3*R*,4a'*R*,7'*R*,8'*R*,8a'*R*)-7'-hydroxy-2',2'-dimethyl-8'-(4-(3,4,5-trifluorophenyl)-1*H-*1,2,3-triazol-1-yl)hexahydro-3*H*,4'*H*-spiro[furan-2,6'-pyrano[3,2-*d*][1,3-*d*]dioxin]-3-yl)-1-naphthamide (24.9 mg, 0.040 mmol) in a similar manner to the preparation method of 5-(((2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl)oxy)methyl)-2-(tetrahydro-2*H*-pyran-2-yl)-2H-tetrazole.

¹H-NMR (400 MHz, CDCl₃) δ = 8.54-8.52 (m, 1H), 8.23-8.12 (m, 1H), 8.05-7.99 (m, 1H), 7.89-7.83 (m, 1H), 7.70-7.60 (m, 2H), 7.50-7.41 (m, 2H), 7.22-7.16 (m, 1H), 6.50-6.48 (m, 1H), 5.80-5.76 (m, 1H), 5.43-5.40 (m, 1H), 5.19-5.15 (m, 1H), 4.85-4.78 (m, 1H), 4.71-4.61 (m, 2H), 4.39-4.37 (m, 1H), 4.21-4.13 (m, 2H), 3.97-3.93 (m, 2H), 3.86-3.81 (m, 1H), 3.69-3.61 (m, 1H), 2.57-2.56 (m, 1H), 2.28-2.14 (m, 1H), 2.08-1.88 (m, 2H), 1.79-1.61 (m, J = 18.0 Hz, 4H), 1.42-1.42 (m, 3H), 1.38-1.34 (m, 3H)

### Synthesis of N-((4R,5S,7R,8R,9S,10R)-10-((2H-tetrazol-5-yl)methoxy)-8-hydroxy-7-(hydroxymethyl)-9-(4-(3,4,5-trifluorophenyl)-1H-1,2,3-triazol-1-yl)-1,6-dioxaspiro[4.5]decan-4-yl)-4-fluoro-1 -naphthamide

The title compound as a white solid (10.3 mg, yield: 72%) was synthesized from *N-*(2*S*,3*R*,4a'*R*,7'*R*,8'*R*,8a'*R*)-2',2'-dimethyl-7'-((2-(tetrahydro-2*H*-pyran-2-yl)-2*H*-tetrazol-5-yl)methoxy)-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-3-yl)-4-fluoro-1-naphthamide (16.9 mg, 0.022 mmol) in a similar manner to the preparation method of (5*S*,7*R*,8*R*,9*S*,10*R*)-10-((2H-tetrazol-5-yl)methoxy)-7-(hydroxymethyl)-9-(4-(3,4,5-trifluorophenyl)-1*H*-1,2,3-triazol-1-yl)-1,6-dioxaspiro[4.5]decan-8-ol.

ESIMS: m/z 671.33 [M+H]+; ¹H-NMR (400 MHz, CD₃OD) δ = 8.61 (S, 1H), 8.52 (d, J = 8.6 Hz, 1H), 8.16 (d, J = 7.8 Hz, 1H), 7.78-7.64 (m, 3H), 7.59 (t, J = 7.6 Hz, 2H), 7.28 (dd, J = 10.2, 8.2 Hz, 1H), 5.31-5.27 (m, 2H), 5.15 (t, J = 9.4 Hz, 1H), 4.83 (d, J = 11.0 Hz, 1H), 4.75 (d, J = 13.3 Hz, 1H), 4.16-4.14 (m, 3H), 4.09 (q, J = 7.3 Hz, 1H), 3.86-3.75 (m, 2H), 2.49-2.42 (m, 1H), 2.30-2.20 (m, 1H)

### Example 196: Synthesis of Compound A211

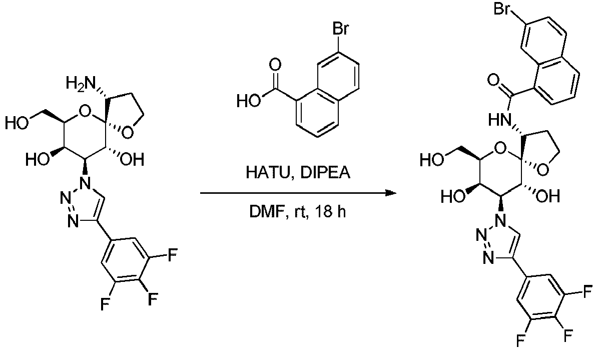

### Synthesis of 7-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

(4*R*,5*S*,7*R*,8*R*,9*S,*10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then 7-bromo-1-naphtho acid (10.0 mg, 0.0396 mmol), HATU (20.5 mg, 0.054 mmol), and *N*,*N-*diisopropylethylamine (19 µL, 0.108 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (21.0 mg, yield: 90%).

ESIMS: m/z 649.33 [M+H]+; ¹H-NMR (400 MHz, CD₃OD) δ 8.57 (s, 1H), 8.51 (s, 1H), 8.02 (d, J = 8.2 Hz, 1H), 7.88 (d, J = 8.8 Hz, 1H), 7.82 (d, J = 6.6 Hz, 1H), 7.68-7.57 (m, 4H), 5.08 (t, J = 9.3 Hz, 2H), 4.58 (d, J = 11.2 Hz, 1H), 4.23-4.07 (m, 4H), 3.80 (dd, J = 11.4, 7.2 Hz, 1H), 3.72 (dd, J = 11.4, 4.9 Hz, 1H), 2.59-2.49 (m, 1H), 2.26-2.15 (m, 1H).

### Example 197: Synthesis of Compound A212

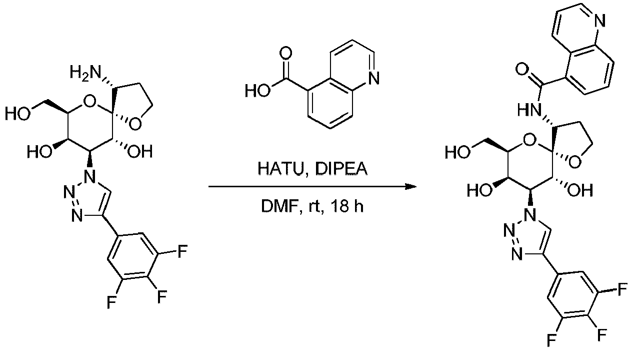

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-5-carboxamide

(4*R*,5*S*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N,N*-dimethylformamide, and then quinoline-5-carboxylic acid (7.0 mg, 0.0396 mmol), HATU (20.5 mg, 0.054 mmol), and *N,N-*diisopropylethylamine (19 µL, 0.108 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (16.8 mg, yield: 82%).

ESIMS: m/z 572.32 [M+H]+; ¹H-NMR (400 MHz, CD₃OD) δ 8.96-8.89 (m, 2H), 8.59 (s, 1H), 8.16 (d, J = 7.8 Hz, 1H), 7.90-7.82 (m, 2H), 7.67-7.59 (m, 3H), 5.19-5.07 (m, 2H), 4.66 (d, J = 11.2 Hz, 1H), 4.22-4.05 (m, 4H), 3.83-3.71 (m, 2H), 2.53-2.43 (m, 1H), 2.25-2.16 (m, 1H).

### Example 198: Synthesis of Compound A213

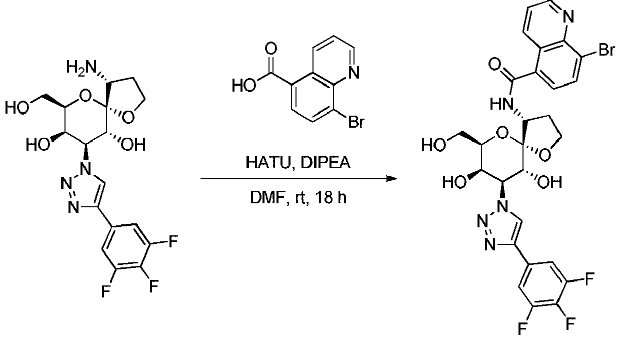

### Synthesis of 8-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-5-carboxamide

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15.0 mg, 0.036 mmol) was dissolved in 1.0 mL of *N*,*N*-dimethylformamide, and then 8-bromoquinoline-5-carboxylic acid (10.0 mg, 0.0396 mmol), HATU (20.5 mg, 0.054 mmol), and *N*,*N*-diisopropylethylamine (19 µL, 0.108 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (13.0 mg, yield: 56%).

ESIMS: m/z 650.28 [M+H]+; ¹H-NMR (400 MHz, CD₃OD) δ 8.99 (dd, J = 4.1, 1.3 Hz, 1H), 8.93 (dd, J = 8.6, 1.4 Hz, 1H), 8.59 (s, 1H), 8.20 (d, J = 7.7 Hz, 1H), 7.73 (d, J = 7.7 Hz, 1H), 7.70-7.58 (m, 3H), 5.17-5.06 (m, 2H), 4.66 (d, J = 11.2 Hz, 1H), 4.17 (d, J = 6.0 Hz, 3H), 4.09 (dd, J = 16.0, 8.1 Hz, 1H), 3.81-3.72 (m, 2H), 2.51-2.41 (m, 1H), 2.23-2.13 (m, 1H).

### Example 199: Synthesis of Compound A214

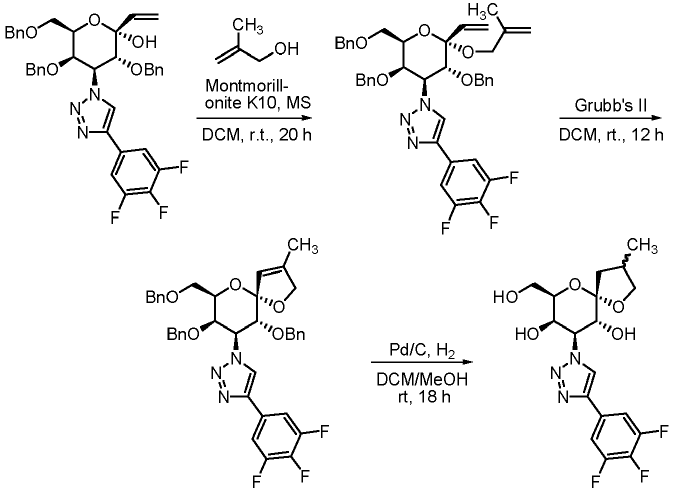

### Synthesis of 1-((2S,3R,4S,5R,6R)-3,5-bis(benzyloxy)-6-((benzyloxy)methyl)-2-((2-methylallyl)oxy)-2-vinyltetrahydro-2H-pyran-4-yl)-4-(3,4,5-trifluorophenyl)-1H-1,2,3-triazole

(2*S*,3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)-2-vinyltetrahydro-2*H*-pyran-2-ol (200 mg, 0.304 mmol), 4 Å molecular sieves (240 wt%, 480 mg), and montmorillonite K-10 (240 wt%, 480 mg) were dissolved in 5 mL of dichloromethane, and then 2-methyl-2-propen-1-ol (154 µL, 1.825 mmol) was added dropwise thereto, followed by stirring at room temperature for 20 hours. The reaction mixture was filtered through Celite and then the filtrate was concentrated and subjected to silica gel column chromatography with 0% to 30% ethyl acetate/n-hexane to obtain the title compound (67 mg, yield: 31%).

¹H-NMR (400 MHz, CDCl₃) δ 7.49 (s, 1H), 7.31 (d, J = 3.4 Hz, 7H), 7.10 (dd, J = 14.8, 7.5 Hz, 8H), 6.90 (d, J = 7.1 Hz, 2H), 6.06 (dd, J = 17.4, 10.9 Hz, 1H), 5.70 (d, J = 15.9 Hz, 1H), 5.50 (t, J = 11.3 Hz, 2H), 5.08 (s, 1H), 4.90 (s, 1H), 4.60-4.48 (m, 3H), 4.45 (d, J = 11.1 Hz, 1H), 4.35 (d, J = 11.6 Hz, 1H), 4.22-4.15 (m, 2H), 4.12 (d, J = 2.1 Hz, 1H), 3.93 (d, J = 13.1 Hz, 1H), 3.86-3.75 (m, 3H), 3.71-3.65 (m, 1H), 1.78 (s, 3H).

### Synthesis of 1-((5S,7R,9S,10R)-8,10-bis(benzyloxy)-7-((benzyloxy)methyl)-3-methyl-1,6-dioxaspiro[4.5]dec-3-en-9-yl)-4-(3,4,5-trifluorophenyl)-1H-1,2,3-triazole

1-((2*S*,3*R*,4*S*,5*R*,6*R*)-3,5-bis(benzyloxy)-6-((benzyloxy)methyl)-2-((2-methylallyl)oxy)-2-vinyltetrahydro-2*H*-pyran-4-yl)-4-(3,4,5-trifluorophenyl)-1*H*-1,2,3-triazole (65 mg, 0.091 mmol) was dissolved in 5.0 mL of dichloromethane, and then Grubbs catalyst II (4.7 mg, 0.006 mmol) was added dropwise thereto, and the mixture was stirred at room temperature for 12 hours. The reaction mixture was filtered through Celite and then the filtrate was concentrated and subjected to silica gel column chromatography with 0% to 30% ethyl acetate/n-hexane to obtain the title compound (12 mg, yield: 20%).

¹H-NMR (400 MHz, CD₃OD) δ 8.21 (s, 1H), 7.42-7.37 (m, 2H), 7.32 (d, J = 6.7 Hz, 3H), 7.30-7.25 (m, 2H), 7.21-7.16 (m, 3H), 7.11-7.04 (m, 5H), 6.89-6.85 (m, 2H), 5.58 (s, 1H), 5.21 (dd, J = 11.2, 3.2 Hz, 1H), 4.66 (d, J = 13.3 Hz, 1H), 4.61-4.54 (m, 2H), 4.51 (d, J = 11.4 Hz, 3H), 4.44-4.39 (m, 1H), 4.35 (d, J = 11.9 Hz, 2H), 4.12 (d, J = 3.0 Hz, 1H), 3.81 (d, J = 10.7 Hz, 1H), 3.68 (d, J = 7.9 Hz, 1H), 3.62-3.57 (m, 1H), 1.85 (s, 3H).

### Synthesis of (5S,7R,8R,9S,10R)-7-(hydroxymethyl)-3-methyl-9-(4-(3,4,5-trifluorophenyl)-1H-1,2,3-triazol-1-yl)-1,6-dioxaspiro[4.5]decane-8,10-diol

1-((5*S*,7*R*,8*R*,9*S*,10*R*)-8,10-bis(benzyloxy)-7-((benzyloxy)methyl)-3-methyl-1,6-dioxaspiro[4.5]dec-3-en-9-yl)-4-(3,4,5-trifluorophenyl)-1*H*-1,2,3-triazole (10 mg, 0.015 mmol) was dissolved in 1.0 mL of a solution of dichloromethane/methanol (3:1), and the mixture was added dropwise to a round-bottom flask, in which 10% Pd/C (20 mg, 20 wt%) was dissolved in 1.0 mL of a solution of dichloromethane/methanol (3:1), and degassed with a hydrogen balloon for 10 minutes, followed by stirring in a hydrogen atmosphere for 18 hours. The reaction mixture was filtered through Celite and then the filtrate was concentrated and subjected to silica gel column chromatography with 0% to 5% methanol/n-hexane to obtain the title compound (4.5 mg, yield: 74%).

ESIMS: m/z 416.28 [M+H]+; ¹H-NMR (400 MHz, CD₃OD) δ 8.49 (s, 1H), 7.64 (dd, J = 8.3, 7.0 Hz, 2H), 5.05-4.97 (m, 1H), 4.37 (d, J = 11.3 Hz, 1H), 4.16-4.02 (m, 3H), 3.68 (t, J = 6.3 Hz, 2H), 3.55 (dd, J = 16.5, 8.9 Hz, 1H), 2.43-2.32 (m, 1H), 1.93 (dd, J = 23.4, 11.3 Hz, 1H), 1.69 (dd, J = 12.9, 7.3 Hz, 1H), 1.09 (dd, J = 14.6, 6.7 Hz, 3H).

### Example 200: Synthesis of Compound A215

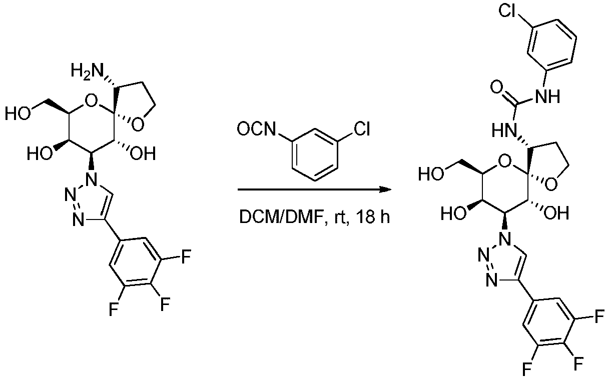

### Synthesis of 1-(3-chlorophenyl)-3-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)urea

(4*R*,5*S*,7*R*,8*R*,9*S*,10*R*)-4-amino-7-(hydroxymethyl)-9-(4-(3,4,5-trifluorophenyl)-1*H-*1,2,3-triazol-1-yl)-1,6-dioxaspiro[4.5]decane-8,10-diol (15 mg, 0.036 mmol) was dissolved in 5.0 mL of dichloromethane, and then was completely dissolved by dropping *N,N-*dimethylformamide. 3-chlorophenylisocyanate (4.8 µl, 0.0396 mmol) was added dropwise thereto at 0 °C, followed by stirring at room temperature for 12 hours. The reaction mixture was subjected to silica gel column chromatography with 0% to 5% methanol/dichloromethane to obtain the title compound (18.6 mg, yield: 91%).

ESIMS: m/z 570.31 [M+H]+; ¹H-NMR (400 MHz, CD₃OD) δ 8.55 (s, 1H), 7.65-7.51 (m, 3H), 7.25-7.16 (m, 2H), 7.00-6.93 (m, 1H), 5.05 (dd, J = 11.2, 2.7 Hz, 1H), 4.67 (dd, J= 10.0, 8.6 Hz, 1H), 4.42 (d, J = 11.2 Hz, 1H), 4.13 (dd, J = 11.1, 5.4 Hz, 3H), 4.02 (dd, J= 16.3, 8.4 Hz, 1H), 3.78 (d, J = 6.1 Hz, 2H), 2.43-2.33 (m, 1H), 2.09-1.94 (m, 1H).

### Example 201: Synthesis of Compound A216

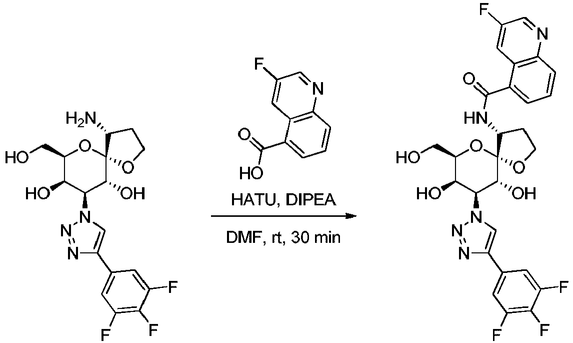

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3-fluoroquinoline-5-carboxamide

The title compound (10.4 mg, yield: 74%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 590.35 [M+H]+; ¹H-NMR (400 MHz, CD₃OD) δ = 8.90 (d, J = 2.7 Hz, 1H), 8.61 (dd, J = 10.0, 2.2 Hz, 1H), 8.57 (S, 1H), 8.21 (d, J = 8.2 Hz, 1H), 7.95 (d, J = 7.0 Hz, 1H), 7.82 (t, J = 7.8 Hz, 1H), 7.62 (t, J = 7.6 Hz, 2H), 5.14-5.07 (m, 2H), 4.63 (d, J = 11.3 Hz, 1H), 4.21-4.07 (m, 4H), 3.84-3.74 (m, 2H), 2.52-2.44 (m, 1H), 2.25-2.15 (m, 1H)

### Example 202: Synthesis of Compound A217

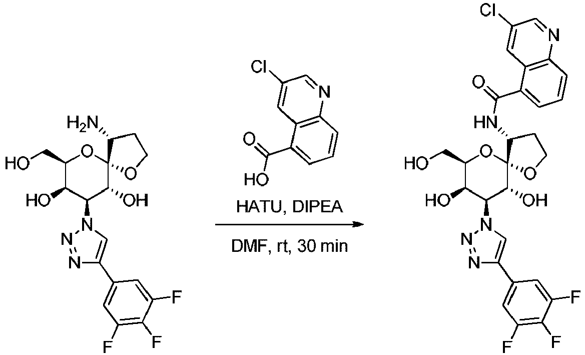

### Synthesis of 3-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-5-carboxamide

The title compound (8.7 mg, yield: 60%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 606.35 [M+H]+; ¹H-NMR (400 MHz, CD₃OD) δ = 8.89-8.86 (m, 2H), 8.57 (S, 1H), 8.18 (d, J = 8.2 Hz, 1H), 7.96 (d, J = 6.3 Hz, 1H), 7.85 (t, J = 7.8 Hz, 1H), 7.64-7.60 (m, 2H), 5.12-5.07 (m, 2H), 4.62 (d, J = 11.3 Hz, 1H), 4.21-4.07 (m, 4H), 3.82-3.72 (m, 2H), 2.54-2.46 (m, 1H), 2.25-2.15 (m, 1H)

### Example 203: Synthesis of Compound A218

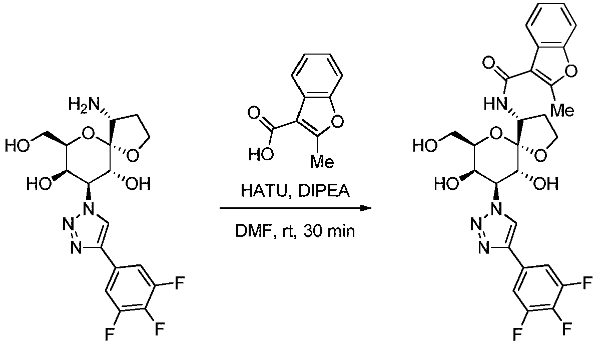

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-methylbenzofuran-3-carboxamide

The title compound (11.4 mg, yield: 83%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 575.35 [M+H]+; ¹H-NMR (400 MHz, CD₃OD) δ = 8.56 (S, 1H), 7.97-7.95 (m, 1H), 7.62-7.58 (m, 2H), 7.48-7.46 (m, 1H), 7.32-7.27 (m, 2H), 5.10 (dd, J = 11.3, 2.7 Hz, 1H), 5.02 (t, J = 9.2 Hz, 1H), 4.52 (d, J = 11.3 Hz, 1H), 4.23-4.16 (m, 3H), 4.09 (q, J = 8.1 Hz, 1H), 3.89 (dd, J = 11.5, 7.2 Hz, 1H), 3.80 (dd, J = 11.3, 4.7 Hz, 1H), 2.71 (S, 3H), 2.51-2.43 (m, 1H), 2.21-2.11 (m, 1H)

### Example 204: Synthesis of Compound A219

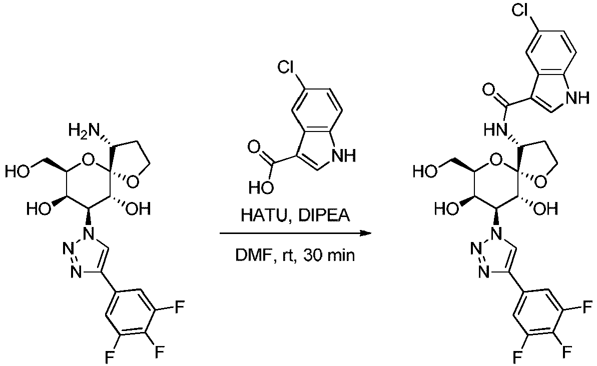

### Synthesis of 5-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1H-indole-3-carboxamide

The title compound (4.9 mg, yield: 34%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 594.32 [M+H]+; ¹H-NMR (400 MHz, CD₃OD) δ = 8.55 (S, 1H), 8.13 (d, J = 1.6 Hz, 1H), 8.03 (S, 1H), 7.62-7.58 (m, 2H), 7.42 (d, J = 8.6 Hz, 1H), 7.18 (dd, J = 8.6, 2.0 Hz, 1H), 5.09 (dd, J = 11.3, 2.7 Hz, 1H), 5.01 (t, J = 9.4 Hz, 1H), 4.47 (d, J = 11.0 Hz, 1H), 4.21-4.20 (m, 2H), 4.14 (d, J = 1.6 Hz, 1H), 4.08 (q, J = 8.2 Hz, 1H), 3.94 (dd, J = 11.7, 7.4 Hz, 1H), 3.81 (dd, J = 11.5, 4.9 Hz, 1H), 2.50-2.44 (m, 1H), 2.22-2.12 (m, 1H)

### Example 205: Synthesis of Compound A220

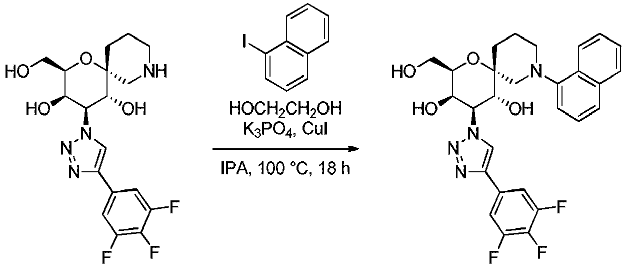

### Synthesis of (2R,3R,4S,5R,6R)-2-(hydroxymethyl)-8-(naphthalen-1-yl)-4-(4-(3,4,5-trifluorophenyl)-1H-1,2,3-triazol-1-yl)-1-oxa-8-azaspiro[5.5]undecane-3,5-diol

(2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol), 1-iodonaphthalene (17 µL, 0.096 mmol), and ethylene glycol (8 µL, 0.144 mmol) were dissolved in 1 mL of isopropyl alcohol, and then tripotassium phosphate (20 mg, 0.096 mmol) and copper(I) iodide (4.6 mg, 0.024 mmol) were added thereto. The reaction mixture was degassed by using an argon balloon, and then stirred at 100 °C for 18 hours. The reaction mixture was diluted with 10 mL of a solution of dichloromethane:methanol (10:1 v/v), and then washed with a 25% ammonia solution (3 mL x 3 times). The organic layer was dried over anhydrous sodium sulfate, filtered, and then concentrated under reduced pressure. The remaining concentrate was subjected to silica gel column chromatography with 0% to 5% methanol/dichloromethane to synthesize the title compound as a brown solid (8.3 mg, yield: 32%).

ESIMS: m/z 541.38 [M+H]+; ¹H-NMR (400 MHz, CD₃OD) δ = 8.20-8.18 (m, 1H), 8.07-8.05 (m, 1H), 7.76-7.39 (m, 6H), 7.04-6.99 (m, 2H), 4.68-4.58 (m, 1H), 4.18 (dd, J = 11.0, 2.7 Hz, 0.5H), 4.03 (dd, J = 11.2, 2.5 Hz, 0.5H), 3.82 (S, 0.5), 3.71-3.55 (m, 2.5H), 3.43 (dd, J= 11.3, 4.7 Hz, 0.5H), 3.28-3.26 (m, 0.5H), 2.92-2.79 (m, 1.5H), 2.75-2.71 (m, 0.5H), 2.65-2.61 (m, 0.5H), 2.55-2.52 (m, 0.5H), 2.49-2.36 (m, 1H), 2.07-1.87 (m, 2H), 1.75-1.70 (m, 1H), 1.46-1.46 (m, 1H)

### Example 206: Synthesis of Compound A221

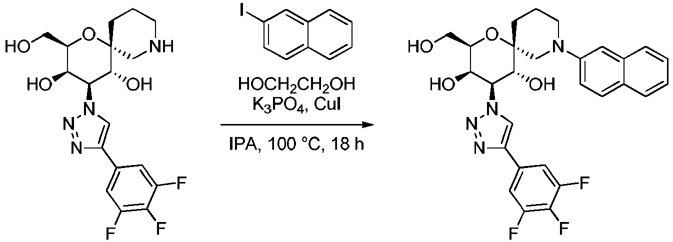

### Synthesis of (2R,3R,4S,5R,6R)-2-(hydroxymethyl)-8-(naphthalen-1-yl)-4-(4-(3,4,5-trifluorophenyl)-1H-1,2,3-triazol-1-yl)-1-oxa-8-azaspiro[5.5]undecane-3,5-diol

The title compound (3.6 mg, yield: 14%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol) in a similar manner to the preparation method of (2*R*,3*R*,4*S*,5*R*,6*R*)-2-(hydroxymethyl)-(naphthalen-1-yl)-4-(4-(3,4,5-trifluorophenyl)-1*H*-1,2,3-triazol-1-yl)-1-oxa-8-azaspiro[5.5]undecane-3,5-diol.

ESIMS: m/z 541.44 [M+H]+; ¹H-NMR (400 MHz, CD₃OD) δ = 8.12 (d, J = 8.2 Hz, 1H), 8.05 (S, 1H), 8.02 (d, J = 8.2 Hz, 1H), 7.97 (d, J = 7.8 Hz, 1H), 7.68-7.61 (m, 2H), 7.49 (d, J = 8.6 Hz, 1H), 7.19-7.15 (m, 2H), 4.59 (d, J = 11.3 Hz, 1H), 4.38 (dd, J = 11.2, 2.5 Hz, 1H), 3.86 (S, 1H), 3.71 (dd, J = 11.3, 6.7 Hz, 1H), 3.58 (dd, J = 10.4, 5.3 Hz, 1H), 3.47 (t, J = 5.7 Hz, 1H), 3.00 (d, J = 14.5 Hz, 1H), 2.92 (d, J = 13.3 Hz, 1H), 2.77 (d, J = 14.5 Hz, 1H), 2.50-2.44 (m, 1H), 2.09-1.88 (m, 2H), 1.74 (d, J = 12.9 Hz, 1H), 1.48 (d, J = 12.5 Hz, 1H)

### Example 207: Synthesis of Compound A222

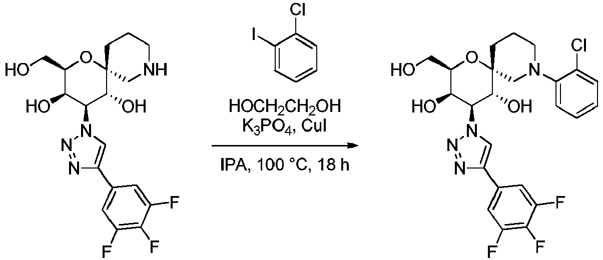

### Synthesis of (2R,3R,4S,5R,6R)-8-(2-chlorophenyl)-2-(hydroxymethyl)-4-(4-(3,4,5-trifluorophenyl)-1H-1,2,3-triazol-1-yl)-1-oxa-8-azaspiro[5.5]undecane-3,5-diol

The title compound (0.6 mg, yield: 2.4%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol) in a similar manner to the preparation method of (2R,3R,4S,5R,6R)-2-(hydroxymethyl)-(naphthalen-1-yl)-4-(4-(3,4,5-trifluorophenyl)-1*H*-1,2,3-triazol-1-yl)-1-oxa-8-azaspiro[5.5]undecane-3,5-diol.

ESIMS: m/z 525.37 [M+H]+; ¹H-NMR (400 MHz, CD₃OD) δ = 8.49 (S, 1H), 7.66-7.62 (m, 2H), 7.36 (d, J = 7.8 Hz, 1H), 7.28 (t, J = 7.6 Hz, 1H), 7.11 (d, J = 8.2 Hz, 1H), 6.93 (t, J = 7.8 Hz, 1H), 5.02 (dd, J = 11.3, 2.7 Hz, 1H), 4.34-4.20 (m, 4H), 3.67-3.57 (m, 2H), 3.02 (d, J = 12.9 Hz, 1H), 2.91 (d, J = 13.7 Hz, 1H), 2.60 (t, J = 11.5 Hz, 1H), 2.17-1.96 (m, 2H), 1.76 (d, J = 12.5 Hz, 1H), 1.56 (d, J = 12.9 Hz, 1H)

### Example 208: Synthesis of Compound A223

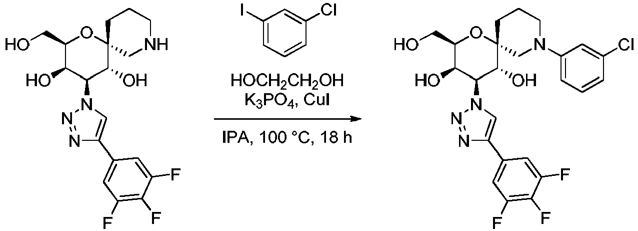

### Synthesis of (2R,3R,4S,5R,6R)-8-(3-chlorophenyl)-2-(hydroxymethyl)-4-(4-(3,4,5-trifluorophenyl)-1H-1,2,3-triazol-1-yl)-1-oxa-8-azaspiro[5.5]undecane-3,5-diol

The title compound (9.7 mg, yield: 42%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol) in a similar manner to the preparation method of (2*R*,3*R*,4*S*,5*R*,6*R*)-2-(hydroxymethyl)-(naphthalen-1-yl)-4-(4-(3,4,5-trifluorophenyl)-1*H*-1,2,3-triazol-1-yl)-1-oxa-8-azaspiro[5.5]undecane-3,5-diol.

ESIMS: m/z 525.37 [M+H]+; ¹H-NMR (400 MHz, CD₃OD) δ = 8.48 (S, 1H), 7.65-7.61 (m, 2H), 7.18 (t, J = 8.2 Hz, 1H), 7.01-6.97 (m, 2H), 6.75 (d, J = 7.8 Hz, 1H), 5.10 (dd, J = 11.5, 2.5 Hz, 1H), 4.23-4.15 (m, 3H), 3.82 (t, J = 5.9 Hz, 1H), 3.66 (d, J = 13.3 Hz, 1H), 3.55 (dd, J = 10.8, 6.8 Hz, 1H), 3.38 (dd, J = 10.8, 5.3 Hz, 1H), 3.09 (d, J = 13.7 Hz, 1H), 2.81-2.75 (m, 1H), 2.22-2.05 (m, 2H), 1.78 (d, J = 12.9 Hz, 1H), 1.61 (d, J = 11.7 Hz, 1H)

### Example 209: Synthesis of Compound A224

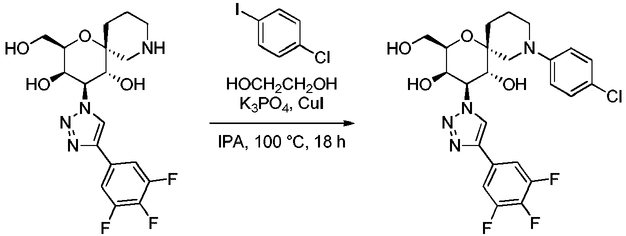

### Synthesis of (2R,3R,4S,5R,6R)-8-(4-chlorophenyl)-2-(hydroxymethyl)-4-(4-(3,4,5-trifluorophenyl)-1H-1,2,3-triazol-1-yl)-1-oxa-8-azaspiro[5.5]undecane-3,5-diol

The title compound (4.4 mg, yield: 17%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol) in a similar manner to the preparation method of (2*R*,3*R*,4*S*,5*R*,6*R*)-2-(hydroxymethyl)-(naphthalen-1-yl)-4-(4-(3,4,5-trifluorophenyl)-1*H*-1,2,3-triazol-1-yl)-1-oxa-8-azaspiro[5.5]undecane-3,5-diol.

ESIMS: m/z 525.31 [M+H]+; ¹H-NMR (400 MHz, CD₃OD) δ = 8.47 (S, 1H), 7.65-7.61 (m, 2H), 7.19 (d, J = 9.0 Hz, 2H), 7.02 (d, J = 9.0 Hz, 2H), 5.08 (dd, J = 11.3, 2.7 Hz, 1H), 4.22-4.14 (m, 3H), 3.84 (t, J = 6.3 Hz, 1H), 3.63-3.55 (m, 2H), 3.43 (dd, J = 11.0, 5.5 Hz, 1H), 3.05 (d, J = 13.7 Hz, 1H), 2.75 (t, J = 11.2 Hz, 1H), 2.25-2.01 (m, 2H), 1.77 (d, J = 12.5 Hz, 1H), 1.62 (d, J = 12.1 Hz, 1H)

### Example 210: Synthesis of Compound A225

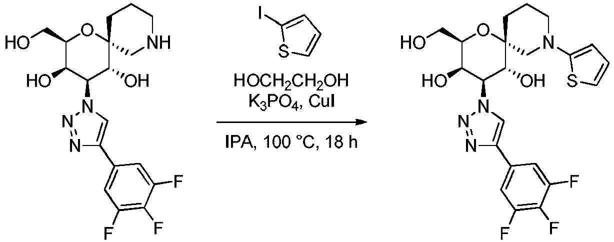

### Synthesis of (2R,3R,4S,5R,6R)-2-(hydroxymethyl)-8-(thiophen-2-yl)-4-(4-(3,4,5-trifluorophenyl)-1H-1,2,3-triazol-1-yl)-1-oxa-8-azaspiro[5.5]undecane-3,5-diol

The title compound (3.4 mg, yield: 14%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol) in a similar manner to the preparation method of (2*R*,3*R*,4*S*,5*R*,6*R*)-2-(hydroxymethyl)-(naphthalen-1-yl)-4-(4-(3,4,5-trifluorophenyl)-1*H*-1,2,3-triazol-1-yl)-1-oxa-8-azaspiro[5.5]undecane-3,5-diol.

ESIMS: m/z 497.39 [M+H]+; ¹H-NMR (400 MHz, CD₃OD) δ = 8.48 (S, 1H), 7.65-7.61 (m, 2H), 6.76 (dd, J = 5.5, 3.9 Hz, 1H), 6.64 (dd, J = 5.5, 1.2 Hz, 1H), 6.31 (d, J = 2.7 Hz, 1H), 5.02 (dd, J = 11.5, 2.5 Hz, 1H), 4.23 (d, J = 11.7 Hz, 1H), 4.16 (S, 1H), 4.00 (d, J = 13.3 Hz, 1H), 3.92 (t, J = 6.3 Hz, 1H), 3.70-3.57 (m, 2H), 3.51 (d, J = 11.0 Hz, 1H), 3.04 (d, J = 13.3 Hz, 1H), 2.76-2.70 (m, 1H), 2.29-2.19 (m, 1H), 2.03 (td, J = 13.1, 4.0 Hz, 1H), 1.76 (d, J = 13.7 Hz, 1H), 1.62 (d, J = 11.7 Hz, 1H)

### Example 211: Synthesis of Compound A226

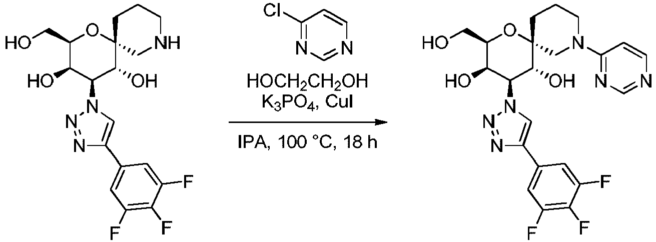

### Synthesis of (2R,3R,4S,5R,6R)-2-(hydroxymethyl)-8-(pyrimidin-4-yl)-4-(4-(3,4,5-trifluorophenyl)-1H-1,2,3-triazol-1-yl)-1-oxa-8-azaspiro[5.5]undecane-3,5-diol

The title compound (4.5 mg, yield: 19%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (20 mg, 0.048 mmol) in a similar manner to the preparation method of (2*R*,3*R*,4*S*,5*R*,6*R*)-2-(hydroxymethyl)-(naphthalen-1-yl)-4-(4-(3,4,5-trifluorophenyl)-1*H*-1,2,3-triazol-1-yl)-1-oxa-8-azaspiro[5.5]undecane-3,5-diol.

ESIMS: m/z 493.36 [M+H]+; ¹H-NMR (400 MHz, CD₃OD) δ = 8.49 (S, 1H), 8.46 (S, 1H), 8.07 (d, J = 6.3 Hz, 1H), 7.66-7.62 (m, 2H), 6.82 (d, J = 6.3 Hz, 1H), 5.29 (S, 1H), 5.06 (dd, J = 11.3, 2.0 Hz, 1H), 4.26-4.16 (m, 4H), 3.50 (dd, J = 10.8, 7.2 Hz, 1H), 3.27-3.22 (m, 2H), 2.99 (t, J = 13.3 Hz, 1H), 2.22 (td, J = 13.1, 4.6 Hz, 1H), 2.05-1.96 (m, 1H), 1.82 (d, J = 12.1 Hz, 1H), 1.62 (d, J = 12.5 Hz, 1H)

### Example 212: Synthesis of Compound A227

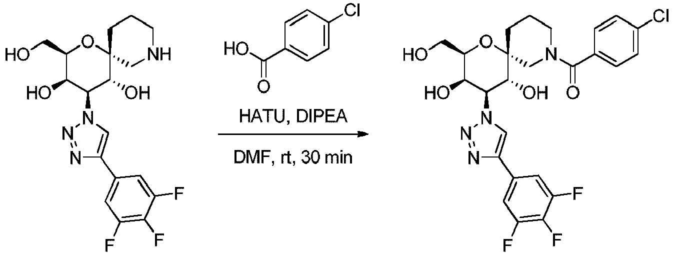

### Synthesis of (4-chlorophenyl)((2R,3R,4S,5R,6R)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)methanone

The title compound (10.1 mg, yield: 76%) as a white solid was synthesized from (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol (10.0 mg, 0.024 mmol) in a similar manner to the preparation method of 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone.

ESIMS: m/z 553.29 [M+H]+; ¹H-NMR (400 MHz, CD₃OD) δ = 8.50 (S, 0.9H), 8.41 (S, 0.1H), 7.65-7.42 (m, 6H), 5.16 (d, J = 14.1 Hz, 0.9H), 5.04 (d, J = 11.0 Hz, 0.9H), 4.62 (d, J = 11.0 z, 0.1H), 4.44 (d, J = 11.3 Hz, 0.1H), 4.29 (d, J = 11.3 Hz, 0.9H), 4.16-4.05 (m, 1.9H), 3.86 (S, 0.1H), 3.71-3.62 (m, 3H), 3.15-3.06 (m, 1.8H), 2.97-2.87 (m, 0.2H), 2.18 (td, J = 13.1, 4.0 Hz, 1H), 2.09-1.99 (m, 1H), 1.84-1.81 (m, 1H), 1.51-1.48 (m, 1H)

### Example 213: Synthesis of Compound A228

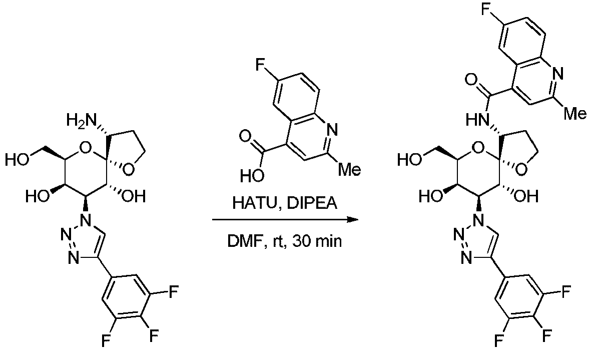

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-6-fluoro-2-methylquinoline-4-carboxamide

The title compound (10.0 mg, yield: 69%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z **604.46** [M+H]+; ¹H-NMR (400 MHz, CD₃OD) δ = 8.56 (S, 1H), 8.05 (dd, J = 9.4, 5.5 Hz, 1H), 8.00 (dd, J = 9.8, 2.7 Hz, 1H), 7.64-7.61 (m, 4H), 5.13-5.07 (m, 2H), 4.62 (d, J = 11.3 Hz, 1H), 4.21-4.07 (m, 4H), 3.84-3.74 (m, 2H), 2.77 (S, 3H), 2.52-2.44 (m, 1H), 2.24-2.15 (m, 1H)

### Example 214: Synthesis of Compound A229

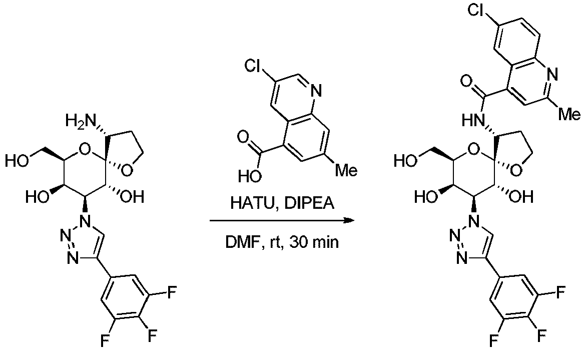

### Synthesis of 6-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-methylquinoline-4-carboxamide

The title compound (8.6 mg, yield: 58%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-3'-fluoro-[1,1'-biphenyl]-3-carboxamide.

ESIMS: m/z 620.41 [M+H]+; ¹H-NMR (400 MHz, CD₃OD) δ = 8.56 (S, 1H), 8.27 (d, J = 2.0 Hz, 1H), 7.99 (d, J = 9.0 Hz, 1H), 7.76 (dd, J = 9.0, 2.3 Hz, 1H), 7.66 (S, 1H), 7.64-7.61 (m, 2H), 5.11-5.07 (m, 2H), 4.61 (d, J = 11.0 Hz, 1H), 4.21-4.07 (m, 4H), 3.82-3.73 (m, 2H), 2.77 (S, 3H), 2.54-2.46 (m, 1H), 2.25-2.15 (m, 1H)

### Example 215: Synthesis of Compound A230

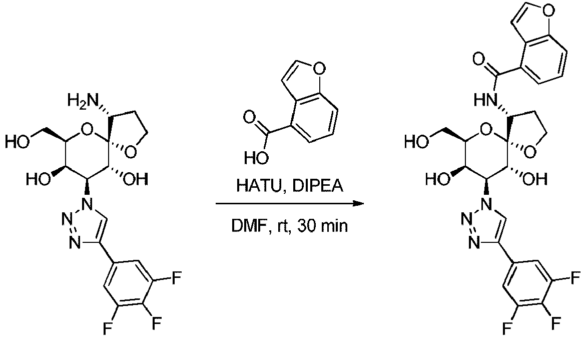

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzofuran-4-carboxamide

The title compound (11.7 mg, yield: 87%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 561.36 [M+H]+; ¹H-NMR (400 MHz, CD₃OD) δ = 8.55 (S, 1H), 7.88 (d, J = 2.0 Hz, 1H), 7.72-7.69 (m, 2H), 7.62-7.59 (m, 2H), 7.43 (S, 1H), 7.40 (d, J = 8.2 Hz, 1H), 5.11-5.04 (m, 2H), 4.51 (d, J = 11.3 Hz, 1H), 4.20-4.17 (m, 2H), 4.15 (d, J = 1.2 Hz, 1H), 4.09 (q, J = 8.1 Hz, 1H), 3.87 (dd, J = 11.3, 7.4 Hz, 1H), 3.77 (dd, J = 11.3, 4.7 Hz, 1H), 2.51-2.45 (m, 1H), 2.23-2.14 (m, 1H)

### Example 216: Synthesis of Compound A231

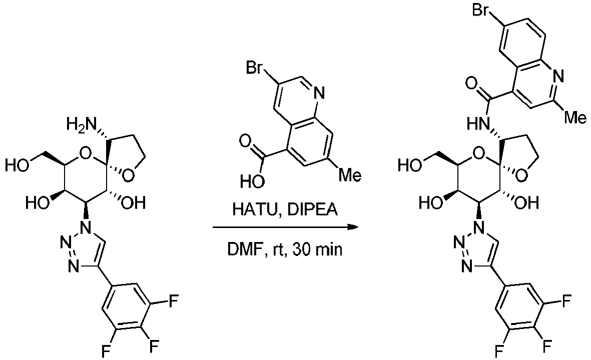

### Synthesis of 6-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-methylquinoline-4-carboxamide

The title compound (14.9 mg, yield: 93%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 664.39 [M+H]+; ¹H-NMR (400 MHz, CD₃OD) δ = 8.56 (S, 1H), 8.42 (S, 1H), 7.93-7.87 (m, 2H), 7.65 (S, 1H), 7.64-7.60 (m, 2H), 5.10-5.07 (m, 2H), 4.60 (d, J = 11.3 Hz, 1H), 4.22-4.07 (m, 4H), 3.82-3.73 (m, 2H), 2.76 (S, 3H), 2.55-2.47 (m, 1H), 2.25-2.15 (m, 1H)

### Example 217: Synthesis of Compound A232

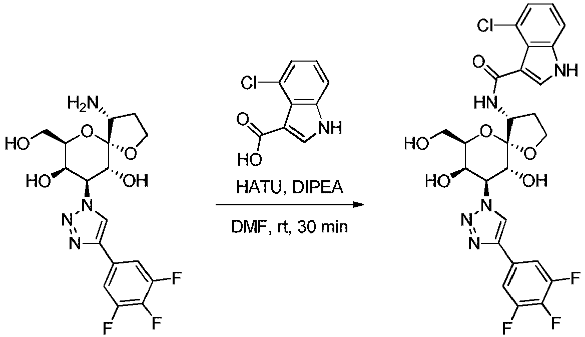

### Synthesis of 4-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1H-indole-3-carboxamide

The title compound (8.1 mg, yield: 57%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*S*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 594.38 [M+H]+; ¹H-NMR (400 MHz, CD₃OD) δ = 8.50 (S, 1H), 7.86 (S, 1H), 7.63-7.59 (m, 2H), 7.41 (dd, J = 6.1, 2.9 Hz, 1H), 7.17-7.15 (m, 2H), 5.07 (dd, J = 11.0, 2.7 Hz, 1H), 4.97 (t, J = 9.2 Hz, 1H), 4.49 (d, J = 11.3 Hz, 1H), 4.20-4.05 (m, 4H), 3.81 (dd, J = 11.3, 6.7 Hz, 1H), 3.74 (dd, J = 11.3, 5.5 Hz, 1H), 2.54-2.48 (m, 1H), 2.23-2.13 (m, 1H)

### Example 218: Synthesis of Compound A233

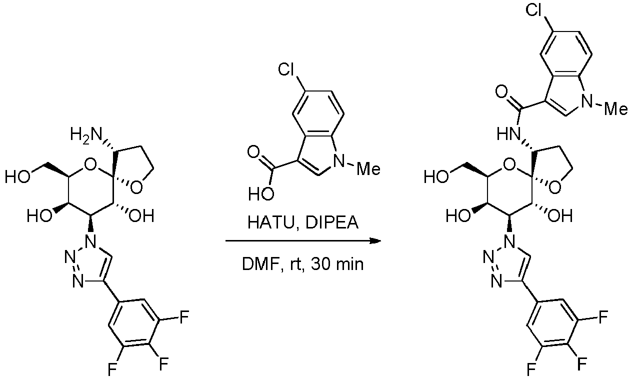

### Synthesis of 5-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-methyl-1H-indole-3-carboxamide

The title compound (12.6 mg, yield: 86%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 608.43 [M+H]+; ¹H-NMR (400 MHz, CD₃OD) δ = 8.54 (S, 1H), 8.12 (d, J = 2.0 Hz, 1H), 7.96 (S, 1H), 7.60-7.56 (m, 2H), 7.44 (d, J = 8.6 Hz, 1H), 7.24 (dd, J = 8.8, 1.8 Hz, 1H), 5.09 (dd, J = 11.3, 2.7 Hz, 1H), 5.00 (t, J = 9.4 Hz, 1H), 4.48 (d, J = 11.0 Hz, 1H), 4.21-4.16 (m, 2H), 4.14 (d, J = 2.0 Hz, 1H), 4.07 (q, J = 8.1 Hz, 1H), 3.94 (dd, J = 11.5, 7.2 Hz, 1H), 3.82 (dd, J = 11.5, 4.9 Hz, 1H), 2.50-2.42 (m, 1H), 2.21-2.11 (m, 1H)

### Example 219: Synthesis of Compound A234

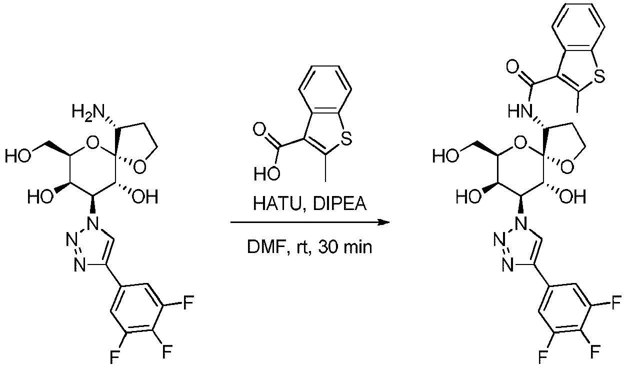

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-methylbenzo[b]thiophene-3-carboxamide

The title compound (10.1 mg, yield: 71%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 591.29 [M+H]+; ¹H-NMR (400 MHz, CD₃OD) δ = 8.56 (S, 1H), 8.09 (d, J = 7.8 Hz, 1H), 7.79 (d, J = 7.8 Hz, 1H), 7.63 (t, J = 7.6 Hz, 2H), 7.39 (t, J = 7.2 Hz, 1H), 7.32 (t, J = 7.4 Hz, 1H), 5.14-5.08 (m, 2H), 4.59 (d, J = 11.3 Hz, 1H), 4.20-4.13 (m, 3H), 4.08 (q, J = 8.1 Hz, 1H), 3.83 (dd, J = 11.2, 7.2 Hz, 1H), 3.75 (dd, J = 11.3, 4.7 Hz, 1H), 2.74 (S, 3H), 2.49-2.41 (m, 1H), 2.22-2.12 (m, 1H)

### Example 220: Synthesis of Compound A235

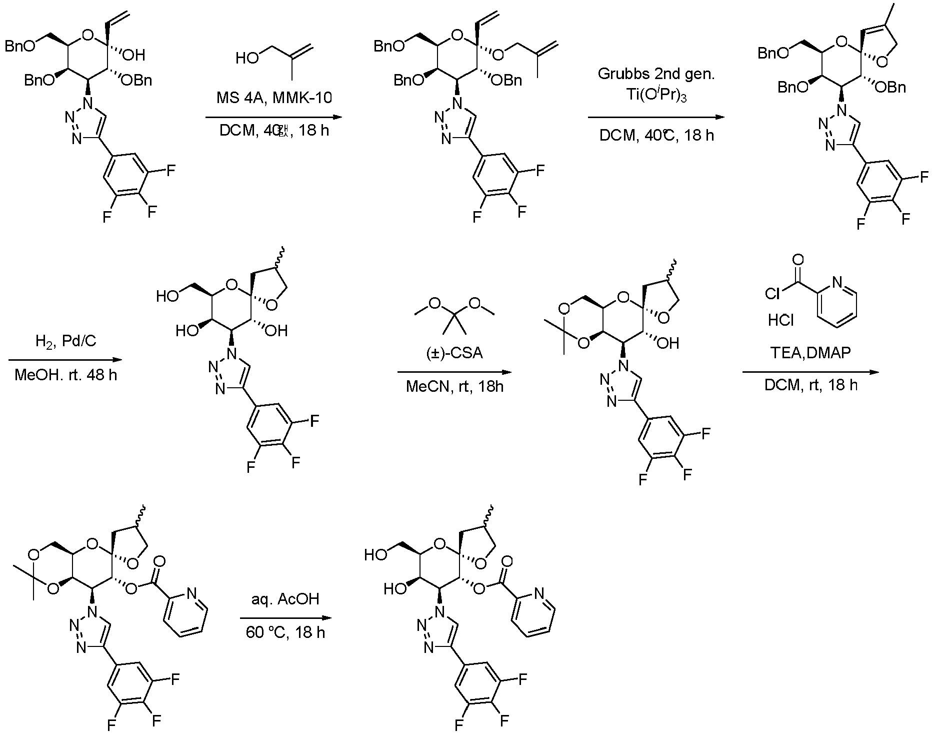

### Synthesis of 1-((2S,3R,4S,5R,6R)-3,5-bis(benzyloxy)-6-((benzyloxy)methyl)-2-((2-methylallyl)oxy)-2-vinyltetrahydro-2H-pyran-4-yl)-4-(3,4,5-trifluorophenyl)-1H-1,2,3-triazole

(2*S*,3*R*,4*S*,5*R*,6*R*)-3,5-bis(benzyloxy)-6-((benzyloxy)methyl)-4-(4-(3,4,5-trifluorophenyl)-1*H*-1,2,3-triazol-1-yl)-2-vinyltetrahydro-2*H*-pyran-2-ol (320 mg, 0.487 mmol) was dissolved in 4.9 mL of dichloromethane, and then 4 Å molecular sieves (800 mg, 250 wt%), and 2-methylprop-2-ene-1-ol (80 µL, 0.974 mmol) were added dropwise thereto, followed by stirring at room temperature for 30 hours. Next, montmorillonite K-10 (800 mg, 250 wt%) was added thereto, and the mixture was stirred at 40 °C for 18 hours. The reaction mixture was filtered through a pad of Celite 545 and the filtrate was concentrated under reduced pressure. The remaining concentrate was subjected to gel column chromatography with 0% to 15% ethyl acetate/n-hexane to obtain the title compound as a white solid (156.0 mg, yield: 45%).

¹H-NMR (400 MHz, CDCl₃) δ = 7.51 (S, 1H), 7.35-7.26 (m, 8H), 7.13-7.05 (m, 7H), 6.91-6.89 (m, 2H), 6.06 (dd, J = 17.2, 11.0 Hz, 1H), 5.70 (dd, J = 17.4, 1.4 Hz, 1H), 5.54-5.47 (m, 2H), 5.08 (S, 1H), 4.90 (S, 1H), 4.59-4.50 (m, 3H), 4.44 (d, J = 11.3 Hz, 1H), 4.36 (d, J = 11.3 Hz, 1H), 4.21-4.18 (m, 2H), 4.12 (d, J = 2.3 Hz, 1H), 3.93 (d, J = 12.9 Hz, 1H), 3.85-3.76 (m, 3H), 3.68 (dd, J = 9.2, 5.7 Hz, 1H), 1.78 (S, 3H)

### Synthesis of 1-((5S,7R,8R,9S,10R)-8,10-bis(benzyloxy)-7-((benzyloxy)methyl)-3-methyl-1,6-dioxaspiro[4.5]dec-3-en-9-yl)-4-(3,4,5-trifluorophenyl)-1H-1,2,3-triazole

1-((2*S*,3*R*,4*S*,5*R*,6*R*)-3,5-bis(benzyloxy)-6-((benzyloxy)methyl)-2-((2-methylallyl)oxy)-2-vinyltetrahydro-2*H*-pyran-4-yl)-4-(3,4, 5-trifluorophenyl)-1*H*-1,2,3-triazole (156 mg, 0.219 mmol) and titanium isopropoxide (50 µL, 0.175 mmol) were dissolved in 4.2 mL of dichloromethane, and then Grubbs 2 generation catalyst (29 mg, 0.033 mmol) was added thereto. The reaction mixture was degassed by using an argon balloon for 5 minutes, and then stirred at 40 °C for 18 hours. The reaction mixture was concentrated under reduced pressure, and the remaining concentrate was subjected to column chromatography with 0% to 25% ethyl acetate/n-hexane to synthesize the title compound as a white solid (108.0 mg, yield: 72%).

¹H-NMR (400 MHz, CDCl₃) δ = 7.41 (S, 1H), 7.33-7.24 (m, 8H), 7.15-7.07 (m, 7H), 6.87 (t, J = 3.7 Hz, 2H), 5.50 (S, 1H), 5.30 (dd, J = 11.2, 2.9 Hz, 1H), 4.68 (d, J = 13.3 Hz, 1H), 4.59-4.50 (m, 3H), 4.47-4.41 (m, 3H), 4.33 (d, J = 11.3 Hz, 1H), 4.26 (d, J = 11.3 Hz, 1H), 4.12 (d, J = 2.3 Hz, 1H), 3.82 (d, J = 11.0 Hz, 1H), 3.73-3.68 (m, 1H), 3.63 (dd, J = 8.8, 5.3 Hz, 1H), 1.85 (S, 3H)

### Synthesis of (5S,7R,8R,9S,10R)-7-(hydroxymethyl)-3-methyl-9-(4-(3,4,5-trifluorophenyl)-1H-1,2,3-triazol-1-yl)-1,6-dioxaspiro[4.5]decane-8,10-diol

The title compound as a white solid (61.0 mg, yield: 67%) (a mixture of stereoisomers 1:1.2) was synthesized from 1-((5*S*,7*R*,8*R*,9*S*,10*R*)-8,10-bis(benzyloxy)-7-((benzyloxy)methyl)-3-methyl-1,6-dioxaspiro[4.5]dec-3-en-9-yl)-4-(3,4,5-trifluorophenyl)-1*H*-1,2,3-triazole (150.0 mg, 0.219 mmol) in a similar manner to the preparation method of (5*R*,7*R*,8*R*,9*S*,10*R*)-7-(hydroxymethyl)-9-(4-(3,4,5-trifluorophenyl)-1*H*-1,2,3-triazol-1-yl)-1,6-dioxaspiro[4.5]decane-8,10-diol.

¹H-NMR (400 MHz, CD₃OD) δ = 8.49 (S, 1H), 7.65-7.61 (m, 2H), 5.03-4.99 (m, 1H), 4.37 (d, J = 11.3 Hz, 1H), 4.19-4.05 (m, 3H), 3.70-3.67 (m, 2H), 3.60-3.52 (m, 1H), 2.68-2.59 (m, 0.55H), 2.52 (dd, J = 12.9, 9.4 Hz, 0.45H), 2.42-2.33 (m, 0.45H), 2.12 (dd, J = 12.7, 7.6 Hz, 0.55H), 1.94 (dd, J = 12.5, 9.4 Hz, 0.55H), 1.69 (dd, J = 12.9, 7.4 Hz, 0.45H), 1.11 (d, J = 6.7 Hz, 1.35H), 1.07 (d, J = 6.7 Hz, 1.65H)

### Synthesis of (2S,4a'R,7'R,8'R,8a'R)-2',2',4-trimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-ol

The title compound as a white solid (56.3 mg, yield: 84%) (a mixture of stereoisomers 1:1.2) was synthesized from (5*S*,7*R*,8*R*,9*S*,10*R*)-7-(hydroxymethyl)-3-methyl-9-(4-(3,4,5-trifluorophenyl)-1*H*-1,2,3-triazol-1-yl)-1,6-dioxaspiro[4.5]decane-8,10-diol (61.0 mg, 0.147 mmol) in a similar manner to the preparation method of 4-fluoro-*N*-((2*S*,3*R*,4a'*R*,7'*R*,8'*R*,8a'*R*)-7'-hydroxy-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-3-yl)-1-naphthamide.

¹H-NMR (400 MHz, CDCl₃) δ = 7.97 (S, 1H), 7.48-7.44 (m, 2H), 5.09-5.05 (m, 1H), 4.37 (d, J = 2.7 Hz, 1H), 4.27 (d, J = 11.0 Hz, 1H), 4.18 (t, J = 8.0 Hz, 1H), 4.13-4.07 (m, 2H), 3.91 (dd, J = 12.5, 7.8 Hz, 1H), 3.81 (d, J = 14.9 Hz, 1H), 3.59-3.50 (m, 1H), 2.74-2.64 (m, 0.55H), 2.57 (dd, J = 13.1, 9.6 Hz, 0.45H), 2.41-2.34 (m, 0.45H), 2.27 (dd, J = 12.7, 7.2 Hz, 0.55H), 2.04-1.95 (m, 0.55H), 1.88-1.82 (m, 0.45H), 1.46 (S, 3H), 1.32 (S, 3H), 1.12 (d, J = 6.7 Hz, 1.35H), 1.07 (d, J = 6.7 Hz, 1.65H)

### Synthesis of (2S,4a'R,7'R,8'S,8a'R)-2',2',4-trimethyl-8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl picolinate

The title compound as a white solid (16.8 mg, yield: 97%) (a mixture of stereoisomers 1:1.2) was synthesized from (2*S*,4a'*R*,7'*R*,8'*R*,8a'*R*)-2',2',4-trimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol in a similar manner to the preparation method of (2*S*,4a'*R*,7'*R*,8'*S*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-yl picolinate.

¹H-NMR (400 MHz, CDCl₃) δ = 8.69 (d, J = 3.9 Hz, 1H), 8.04 (S, 1H), 7.89 (t, J = 7.8 Hz, 1H), 7.77 (t, J = 7.8 Hz, 1H), 7.44-7.41 (m, 1H), 7.37-7.33 (m, 2H), 6.11-6.07 (m, 1H), 5.68-5.65 (m, 1H), 4.42 (d, J = 2.0 Hz, 1H), 4.18-4.11 (m, 2H), 4.00-3.93 (m, 2H), 3.63-3.54 (m, 1H), 2.66-2.56 (m, 0.55H), 2.38-2.30 (m, 1H), 2.26-2.17 (m, 0.45H), 1.92-1.86 (m, 0.45H), 1.55 (m, 3.55H), 1.33 (S, 3H), 1.03 (d, J = 6.7 Hz, 1.35H), 0.92 (d, J = 6.7 Hz, 1.65H)

### Synthesis of (5S,7R,8R,9S,10R)-8-hydroxy-7-(hydroxymethyl) -3-methyl-9-(4-(3,4,5-trifluorophenyl)-1H-1,2,3-triazol-1-yl)-1,6-dioxaspiro[4.5]decan-10-yl picolinate

The title compound as a white solid (8.0 mg, yield: 51%) (a mixture of stereoisomers 1:1.2) was synthesized from (2*S*,4a'*R*,7'*R*,8'*S*,8a'*R*)-2',2',4-trimethyl-8'-(4-(3,4,5-trifluorophenyl)-1*H*-1,2,3-triazol-1-yl)hexahydro-3*H*,4'*H*-spiro[furan-2,6'-pyrano[3,2-d][1,3]dioxin]-7'-yl picolinate (16.8 mg, 0.030 mmol) in a similar manner to the preparation method of (5*S*,7*R*,8*R*,9*S*,10*R*)-8-hydroxy-7-(hydroxymethyl)-9-(4-(3,4,5-trifluorophenyl)-1H-1,2,3-triazol-1-yl)-1,6-dioxaspiro[4.5]decan-10-yl 2-fluorobenzoate.

ESIMS: m/z 521.40 [M+H]+; ¹H-NMR (400 MHz, CD₃OD) δ = 8.60-8.59 (m, 1H), 8.57 (S, 1H), 7.98-7.92 (m, 2H), 7.59-7.50 (m, 3H), 6.18 (d, J = 11.3 Hz, 1H), 5.50-5.45 (m, 1H), 4.25-4.15 (m, 3H), 3.76 (t, J = 6.1 Hz, 2H), 3.65-3.56 (m, 1H), 2.66-2.57 (m, 0.55H), 2.36-2.18 (m, 1.45H), 1.80 (dd, J = 13.3, 7.4 Hz, 0.45H), 1.59 (dd, J = 13.1, 8.4 Hz, 0.55H), 1.06 (d, J = 6.7 Hz, 1.35H), 0.91 (d, J = 6.7 Hz, 1.65H)

### Example 221: Synthesis of Compound A236

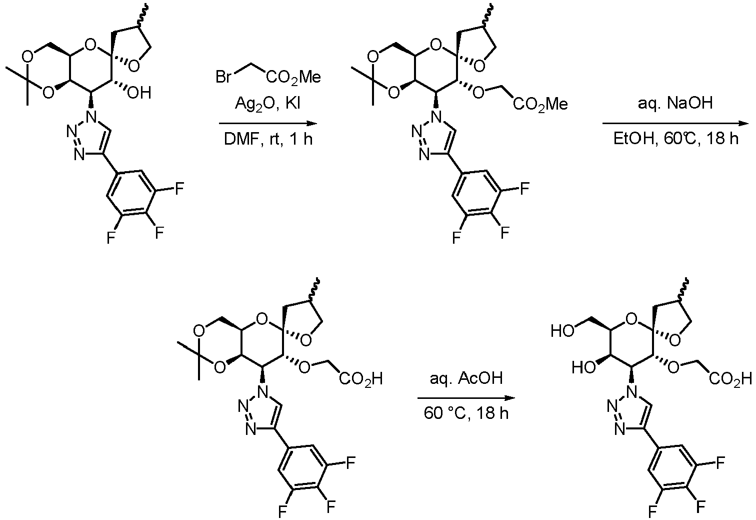

### Synthesis of 2-(((2S,4a'R,7'R,8'S,8a'R)-2',2',4-trimethyl -8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)acetate

(2*S*,4a'*R*,7'*R*,8'*R*,8a'*R*)-2',2',4-trimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (14.1 mg, 0.030 mmol) was dissolved in 0.6 mL of *N,N-*dimethylformamide, and then silver(I) oxide (35.9 mg, 0.150 mmol), potassium iodide (10.3 mg, 0.060 mmol), and methyl 2-bromoacetate (4 µL, 0.045 mmol) were added thereto. The reaction mixture was stirred at room temperature for about 1 hours. The reaction mixture was filtered through a pad of Celite 545 and then the filtrate was concentrated under reduced pressure. The remaining concentrate was subjected to column chromatography with 0% to 30% ethyl acetate/n-hexane to synthesize the title compound as a white solid (17.0 mg, yield: 104%).

¹H-NMR (400 MHz, CDCl₃) δ = 8.04-8.03 (m, 1H), 7.46 (t, J = 7.2 Hz, 2H), 5.43 (dd, J = 11.0, 2.7 Hz, 1H), 4.36-4.30 (m, 2H), 4.18-4.07 (m, 2H), 3.95-3.84 (m, 3H), 3.65-3.51 (m, 5H), 2.72-2.60 (m, 1H), 2.51-2.41 (m, 0.45H), 2.25 (dd, J = 12.7, 7.2 Hz, 0.55H), 2.17-2.04 (m, 0.45H), 1.83 (dd, J = 13.1, 7.2 Hz, 0.55H), 1.50 (S, 3H), 1.33 (S, 3H), 1.11 (d, J = 6.7 Hz, 1.35H), 1.08 (d, J = 6.7 Hz, 1.65H)

### Synthesis of 2-(((2S,4a'R,7'R,8'S,8a'R)-2',2',4-trimethyl -8'-(4-(3,4,5-trifluorophenyl)-1H-1,2,3-triazol-1-yl)hexahydro-3H,4'H-spiro[furan-2,6'-pyrano[3,2-d][1,3]dioxin]-7'-yl)oxy)acetic acid

Methyl 2-(((2*S*,4a*R*,7'*R*,8'*S*,8a'*R*)-2',2',4-trimethyl-8'-(4-(3,4,5-trifluorophenyl)-1*H-*1,2,3-triazol-1-yl)hexahydro-3*H*,4'*H*-spiro[furan-2,6'-pyrano[3,2-*d*][1,3]dioxin]-7'-yl)oxy) acetate (17.0 mg, 0.032 mmol) was dissolved in 1 mL of a 2.5N aqueous sodium hydroxide solution and 1 mL of ethanol, followed by stirring at 60 °C for 18 hours. The reaction mixture was concentrated under reduced pressure, and stirred with toluene (10 mL x 3 times). The remaining concentrate was subjected to column chromatography with 0% to 5% methanol/dichloromethane (including 1% acetic acid) to synthesize the title compound as a white solid (13.0 mg, yield: 79%).

### Synthesis of 2-(((5S,7R,8R,9S,10R)-8-hydroxy-7-(hydroxymethyl)-3-methyl-9-(4-(3,4,5-trifluorophenyl)-1H-1,2,3-triazol-1-yl)-1,6-dioxaspiro[4.5]decan-10-yl)oxy)acetic acid

The title compound as a white solid (8.4 mg, yield: 71%) (a mixture of stereoisomers 1:1.2) was synthesized from 2-(((2*S*,4a'*R*,7'*R*,8'*S*,8a'*R*)-2',2',4-trimethyl-8'-(4-(3,4,5-trifluorophenyl)-1*H*-1,2,3-triazol-1-yl)hexahydro-3*H*,4'*H*-spiro[furan-2,6'-pyrano[3,2-d][1,3]dioxin]-7'-yl)oxy)acetic acid (13.0 mg, 0.025 mmol) in a similar manner to the preparation method of 4-((((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)methyl)tetrahydro-2*H*-thiopyran 1,1-dioxide.

ESIMS: m/z 474.45 [M+H]+; ¹H-NMR (400 MHz, CD₃OD) δ = 8.62 (S, 1H), 7.66-7.62 (m, 2H), 5.22-5.17 (m, 1H), 4.45 (d, J = 11.0 Hz, 1H), 4.16-4.05 (m, 3H), 3.94-3.85 (m, 2H), 3.69 (t, J = 6.3 Hz, 2H), 3.59-3.54 (m, 1H), 2.68-2.58 (m, 1H), 2.50-2.41 (m, 0.45H), 2.14 (dd, J = 12.7, 7.2 Hz, 0.55H), 2.05-2.00 (m, 0.55H), 1.77-1.69 (m, 0.45H), 1.11 (d, J = 6.7 Hz, 1.35H), 1.08 (d, J = 6.7 Hz, 1.65H)

### Example 222: Synthesis of Compound A237

### Synthesis of 1-((3aR,5R,6S,6aR)-5-((R)-2,2-dimethyl-1,3-dioxolan-4-yl)-2,2-dimethyltetrahydrofuro[2,3-d][1,3]dioxol-6-yl)-4-iodo-1H-pyrazole

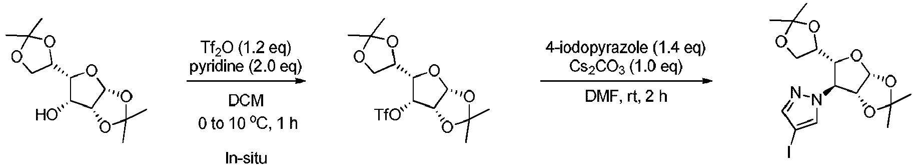

(3a*R*,5*R*,6*R*,6a*R*)-5-((*R*)-2,2-dimethyl-1,3-dioxolan-4-yl)-2,2-dimethyltetrahydrofuro[2,3-*d*][1,3]dioxol-6-ol (CAS No. 14686-89-6, 10.0 g, 38.42 mmol) was dissolved in 150 mL of dichloromethane, and then pyridine (6.2 mL, 76.84 mmol) was added dropwise thereto. The reactant was cooled to 0 °C and then Tf2O (7.74 mL, 46.10 mmol) was slowly added dropwise. The reaction mixture was stirred at 10 °C for 1 hour and then put into ice. The reaction mixture was neutralized with 1 M Hcl, then extracted to obtain an organic layer. The organic layer was sequentially washed with a saturated aqueous sodium hydrogen carbonate solution and a saturated aqueous sodium chloride solution. The organic layer was dried over anhydrous magnesium sulfate, filtered, and then concentrated. Cesium carbonate (12.5 g, 38.42 mmol) was added thereto, dissolved in 150 mL of N,N-dimethylformamide, then 4-iodopyrazole (10.43 g, 53.79 mmol) was added thereto, stirred at room temperature for 2 hours, ice was then added thereto, and the mixture was stirred for 30 minutes. The resulting solid was filtered, washed with 100 mL of a 33% aqueous methanol solution, and then dried to obtain the title compound (16.2 g, yield: 97%).

### Synthesis of (3R,4S,5R,6R)-6-(acetoxymethyl)-4-(4-iodo-1H-pyrazol-1-yl)tetrahydro-2H-pyran-2,3,5-triyl triacetate

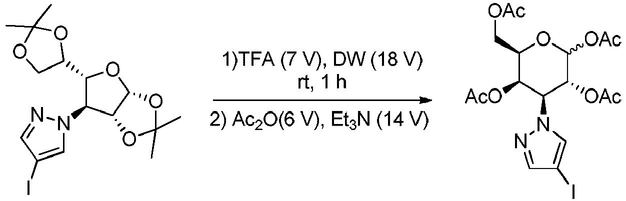

1-((3a*R*,5*R*,6*S*,6a*R*)-5-((*R*)-2,2-dimethyl-1,3-dioxolan-4-yl)-2,2-dimethyltetrahydrofuro[2,3-*d*][1,3]dioxol-6-yl)-4-iodo-1*H*-pyrazole (16.2 g, 37.14 mmol) was dissolved in 200 mL of purified water, and then trifluoroacetic acid (80 mL) was added dropwise thereto.

The resulting mixture was stirred at room temperature for 1 hour and then concentrated. The mixture was dissolved in 210 mL of triethylamine, 106 mL of Ac₂O was added thereto, stirred at room temperature for 18 hours, and then 2 M HCl was added dropwise thereto at 0 °C to neutralize the mixture, followed by extraction. The organic layer was washed sequentially with a saturated aqueous sodium hydrogen carbonate solution and a saturated aqueous sodium chloride solution. The organic layer was dried over anhydrous magnesium sulfate, filtered, and then concentrated. The concentrate was dissolved in 100 mL of ethyl acetate, and then 260 mL of petroleum ether was slowly added dropwise thereto, and the resulting solid was filtered to obtain the title compound (11.5 g, yield: 60%).

### Synthesis of (3R,4S,5R,6R)-6-(acetoxymethyl)-4-(4-(3,4,5-trifluorophenyl)-1H-pyrazol-1-yl)tetrahydro-2H-pyran-2,3,5-triyl triacetate

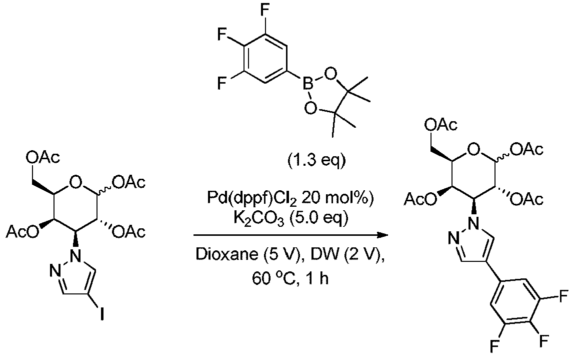

(3*R*,4*S*,5*R*,6*R*)-6-(acetoxymethyl)-4-(4-iodo-1*H*-pyrazol-1-yl)tetrahydro-2*H*-pyran-2,3,5-triyl triacetate (5.1 g, 9.73 mmol), Pd(dppf)Cl₂ (1.42 g, 1.95 mmol), and potassium carbonate (6.7 g, 48.65 mmol) were dissolved in 100 mL of 1,4-dioxane and 100 mL of purified water, and then 4,4,5,5-tetramethyl-2-(3,4,5-trifluorophenyl)-1,3,2-dioxaborolane (3.27 g, 12.65 mmol) was added dropwise thereto. The mixture was stirred at 70 °C for 1 hour, was then filtered through Celite, and the filtrate was washed sequentially with a saturated aqueous ammonium chloride solution and a saturated aqueous sodium chloride solution. The organic layer was dried over anhydrous magnesium sulfate and filtered, and then the resulting mixture was concentrated. The resulting mixture was subjected to silica gel column chromatography with 30% ethyl acetate/n-hexane to obtain the title compound (3.7 g, yield: 72%).

### Synthesis of (2R,3R,4S,5R,6S)-2-(acetoxymethyl)-6-(phenylthio)-4-(4-(3,4,5-trifluorophenyl)-1H-pyrazol-1-yl)tetrahydro-2H-pyran-3,5-diyl diacetate

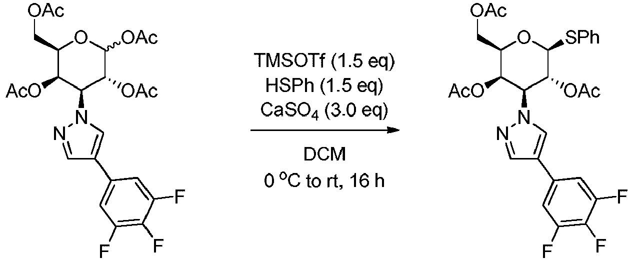

(3*R*,4*S*,5*R*,6*R*)-6-(acetoxymethyl)-4-(4-(3,4,5-trifluorophenyl)-1*H*-pyrazol-1-yl)tetrahydro-2*H*-pyran-2,3,5-triyl triacetate (3.7 g, 7.0 mmol) and CaSO₄ (6.4 g, 200 wt%) were dissolved in 40 mL of acetonitrile, and then thiophenol (2.17 mL, 21.0 mmol) and TMSOTf (1.9 mL, 21.0 mmol) were added dropwise thereto at 0 °C. The resulting mixture was stirred at room temperature for 18 hours to obtain an organic layer. The organic layer was washed sequentially with a saturated aqueous sodium hydrogen carbonate solution and a saturated aqueous sodium chloride solution. The organic layer was dried over anhydrous magnesium sulfate and filtered, and then the resulting mixture was concentrated. The resulting mixture was subjected to silica gel column chromatography with 0% to 50% ethyl acetate/n-hexane to obtain the title compound (762 mg, yield: 19%).

¹H-NMR (400 MHz, CDCl₃) δ 7.69 (s, 1H), 7.61 (s, 1H), 7.52-7.47 (m, 2H), 7.33 (d, J = 6.8 Hz, 3H), 7.07-6.98 (m, 2H), 6.08 (d, J = 5.6 Hz, 1H), 6.00 (dd, J = 11.6, 5.6 Hz, 1H), 5.57 (s, 1H), 4.92 (dd, J = 11.6, 3.0 Hz, 1H), 4.87 (t, J = 6.2 Hz, 1H), 4.09 (ddd, J = 18.7, 11.5, 6.5 Hz, 2H), 2.01 (s, 3H), 1.97 (d, J = 3.9 Hz, 6H).

### Synthesis of (2R,3R,4S,5R,6S)-2-(hydroxymethyl)-6-(phenylthio)-4-(4-(3,4,5-trifluorophenyl)-1H-pyrazol-1-yl)tetrahydro-2H-pyran-3,5-diol

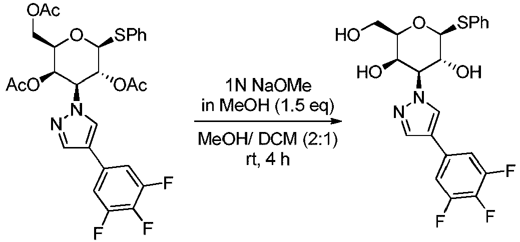

(2*R*,3*R*,4*S*,5*R*,6*S*)-2-(acetoxymethyl)-6-(phenylthio)-4-(4-(3,4,5-trifluorophenyl)-1*H-*pyrazol-1-yl)tetrahydro-2*H*-pyran-3,5-diyl diacetate (762 mg, 1.32 mmol) was dissolved in 20 mL of dichloromethane/10 mL of methanol, then 25% NaOMe (854 µL, 3.95 mmol) in methanol was added dropwise thereto, and the mixture was stirred at room temperature for 4 hours. Amberlyst^{®} 15 (762 mg, 100 wt%) was then stirred for 10 minutes, and then filtered and concentrated to obtain a compound. The compound was subjected to silica gel column chromatography with 0% to 10% methanol/dichloromethane to obtain the title compound (571 mg, yield: 95.6%).

¹H-NMR (400 MHz, CD₃OD) δ 8.13 (s, 1H), 7.88 (s, 1H), 7.59 (d, J = 7.3 Hz, 2H), 7.39-7.23 (m, 5H), 4.80 (d, J = 9.5 Hz, 1H), 4.41 (dd, J = 10.5, 2.6 Hz, 1H), 4.23 (t, J = 9.9 Hz, 1H), 4.13 (d, J = 2.2 Hz, 1H), 3.81-3.69 (m, 3H).

### Synthesis of 1-((2R,3R,4S,5R,6S)-3,5-bis(benzyloxy)-2-((benzyloxy)methyl)-6-(phenylthio)tetrahydro-2H-pyran-4-yl)-4-(3,4,5-trifluorophenyl)-1H-pyrazole

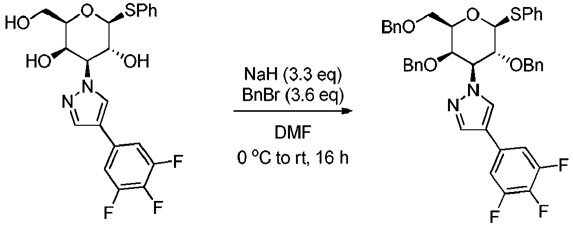

(2*R*,3*R*,4*S*,5*S*,6*S*)-2-(hydroxymethyl)-6-(phenylthio)-4-(4-(3,4,5-trifluorophenyl)-1*H-*pyrazol-1-yl)tetrahydro-2*H*-pyran-3,5-diol (571 mg, 1.26 mmol) was dissolved in 20 mL of *N,N-*dimethylformamide, then 60% sodium hydride (60% dispersion in mineral oil) (176.4 mg, 4.41 mmol) was added dropwise thereto at 0 °C, stirred for 30 minutes, benzyl bromide (539.6 mg, 4.54 mmol) was added dropwise thereto, and stirred at room temperature for 16 hours to obtain an organic layer. The organic layer was washed sequentially with a saturated aqueous ammonium chloride solution and a saturated aqueous sodium chloride solution. The organic layer was dried over anhydrous magnesium sulfate and filtered, and then the resulting mixture was concentrated. The resulting mixture was subjected to silica gel column chromatography with 0% to 50% ethyl acetate/n-hexane to obtain the title compound (800 mg, yield: 88%).

### Synthesis of (3R,4S,5R,6R)-3,5-bis(benzyloxy)-6-((benzyloxy)methyl)-4-(4-(3,4,5-trifluorophenyl)-1H-pyrazol-1-yl)tetrahydro-2H-pyran-2-one

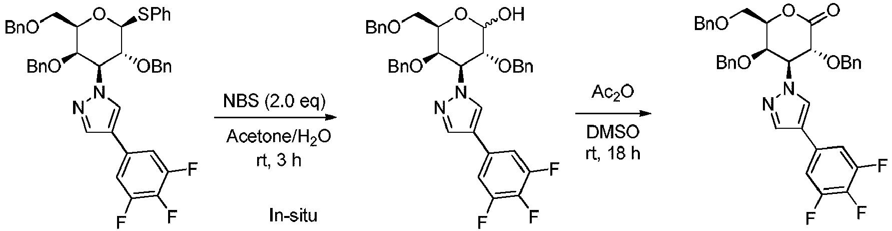

1-((2*R*,3*R*,4*S*,5*R*,6*S*)-3,5-bis(benzyloxy)-2-((benzyloxy)methyl)-6-(phenylthio)tetrahydro-2*H*-pyran-4-yl)-4-(3,4,5-trifluorophenyl)-1*H*-pyrazol (800 mg, 1.11 mmol) was dissolved in 18 mL of acetone/2 mL of purified water, NBS (295.5 mg, 1.66 mmol) was added dropwise thereto, stirred for 30 minutes, then NBS (295.5 mg, 1.66 mmol) was added thereto, and the resulting mixture was stirred at room temperature for 4 hours to obtain an organic layer. The organic layer was washed sequentially with a saturated aqueous ammonium chloride solution and a saturated aqueous sodium chloride solution. The organic layer was dried over anhydrous magnesium sulfate, filtered, and concentrated to obtain a mixture. The obtained mixture was subjected to silica gel column chromatography with 0% to 50% ethyl acetate/n-hexane. The resulting mixture was dissolved in 50 mL of DMSO, Ac₂O (15 mL) was added dropwise thereto, and stirred at room temperature for 18 hours to obtain an organic layer. The obtained organic layer was washed three times with purified water, and then washed with a saturated aqueous sodium chloride solution. The organic layer was dried over anhydrous magnesium sulfate and filtered, and then the resulting mixture was concentrated. The resulting mixture was subjected to silica gel column chromatography with 0% to 50% ethyl acetate/n-hexane to obtain the title compound (514 mg, yield: 64.4%).

¹H-NMR (400 MHz, CDCl₃) δ 7.71 (s, 1H), 7.35-7.23 (m, 9H), 7.19 (d, J = 4.8 Hz, 3H), 7.14 (d, J = 5.4 Hz, 3H), 7.02 (d, J = 6.1 Hz, 2H), 6.76 (dd, J = 7.8, 7.0 Hz, 2H), 5.03 (d, J = 11.4 Hz, 1H), 4.86-4.77 (m, 3H), 4.64 (t, J = 7.1 Hz, 1H), 4.55 (d, J = 11.8 Hz, 1H), 4.49 (d, J = 11.7 Hz, 1H), 4.38-4.33 (m, 2H), 3.97 (d, J = 10.8 Hz, 1H), 3.75 (d, J = 7.1 Hz, 2H).

### Synthesis of (2S,3R,4S,5R,6R)-3,5-bis(benzyloxy)-6-((benzyloxy)methyl)-4-(4-(3,4,5-trifluorophenyl)-1H-pyrazol-1-yl)-2-vinyltetrahydro-2H-pyran-2-ol

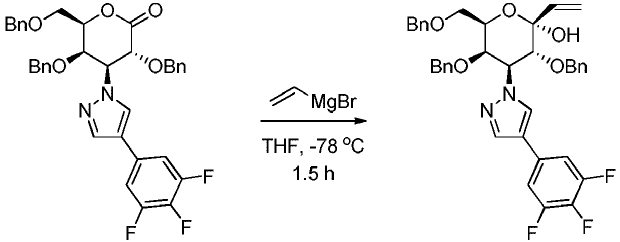

(3*R,*4*S*,5*R*,6*R*)-3,5-bis(benzyloxy)-6-((benzyloxy)methyl)-4-(4-(3,4,5-trifluorophenyl)-1*H*-pyrazol-1-yl)tetrahydro-2*H*-pyran-2-one (400 mg, 0.636 mmol) was dissolved in 10 mL of tetrahydrofuran, then vinylmagnesium bromide (1M tetrahydrofuran solution) (890 µL, 0.890 mmol) was added dropwise thereto at -78 °C, and then the mixture was stirred at -78 °C for 4 hours. Then, a saturated aqueous sodium hydrogen carbonate solution was added thereto at 0 °C to terminate the reaction, followed by stirring from 0 °C to room temperature for 30 minutes. The reaction mixture was extracted with ethyl acetate, organic layer was then dried over anhydrous magnesium sulfate and filtered, and then concentrated. The concentrated reaction mixture was subjected to silica gel column chromatography with 0% to 30% ethyl acetate/n-hexane to obtain the title compound (196 mg, yield: 47%).

¹H-NMR (400 MHz, CDCl₃) δ 7.72 (s, 1H), 7.59 (s, 1H), 7.33-7.27 (m, 9H), 7.15-7.11 (m, 4H), 6.94-6.88 (m, 2H), 6.80 (dd, J = 8.4, 6.9 Hz, 2H), 6.14-6.08 (m, 1H), 5.70 (d, J = 18.2 Hz, 1H), 5.42 (d, J = 10.6 Hz, 1H), 4.86 (d, J = 13.9 Hz, 1H), 4.55-4.44 (m, 5H), 4.29-4.22 (m, 2H), 4.07 (d, J = 2.5 Hz, 1H), 3.91 (d, J = 10.9 Hz, 1H), 3.72 (t, J = 8.8 Hz, 1H), 3.68-3.62 (m, 1H).

### Synthesis of 1-((2S,3R,4S,5R,6R)-2-(allyloxy)-3,5-bis(benzyloxy)-6-((benzyloxy)methyl)-2-vinyltetrahydro-2H-pyran-4-yl)-4-(3,4,5-trifluorophenyl)-1H-pyrazole

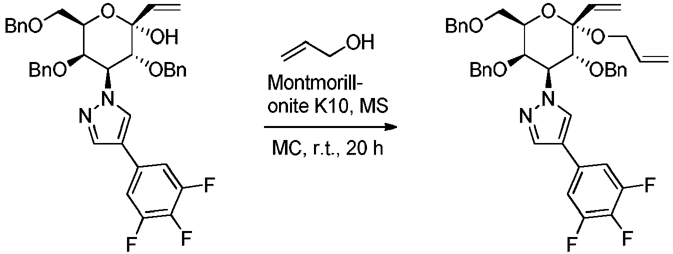

(2*S*,3*R*,4*S*,5*R*,6*R*)-3,5-bis(benzyloxy)-6-((benzyloxy)methyl)-4-(4-(3,4,5-trifluorophenyl)-1*H*-pyrazol-1-yl)-2-vinyltetrahydro-2*H*-pyran-2-ol (196 mg, 0.298 mmol), 4 Å molecular sieves (470 mg, 240 wt%), and montmorillonite K-10 (470 mg, 240 wt%) were dissolved in 20 mL of dichloromethane, and then 3-buten-1-ol (105 mg, 1.79 mmol) was added dropwise thereto, followed by stirring at room temperature for 20 hours. The reaction mixture was filtered through Celite and then the filtrate was concentrated and subjected to silica gel column chromatography with 0% to 30% ethyl acetate/n-hexane to obtain the title compound (144 mg, yield: 70%).

¹H-NMR (400 MHz, CDCl₃) δ 7.71 (s, 1H), 7.60 (s, 1H), 7.31-7.24 (m, 8H), 7.12 (t, J = 6.4 Hz, 5H), 6.95 (dd, J = 7.6, 1.5 Hz, 2H), 6.78 (dd, J = 8.6, 6.5 Hz, 2H), 6.10-5.91 (m, 2H), 5.67 (d, J = 17.4 Hz, 1H), 5.45 (dd, J = 11.0, 1.5 Hz, 1H), 5.32 (dd, J = 17.3, 1.5 Hz, 1H), 5.20-5.13 (m, 2H), 4.56-4.42 (m, 4H), 4.26 (dd, J = 11.0, 6.0 Hz, 2H), 4.18-4.12 (m, 1H), 4.10-4.01 (m, 2H), 3.94 (dd, J = 13.0, 6.0 Hz, 1H), 3.81 (d, J = 11.1 Hz, 1H), 3.76-3.70 (m, 1H), 3.67-3.61 (m, 1H).

### Synthesis of 1-((5S,7R,8R,9S,10R)-8,10-bis(benzyloxy)-7-((benzyloxy)methyl)-1,6-dioxaspiro[4.5]dec-3-en-9-yl)-4-(3,4,5-trifluorophenyl)-1H-pyrazole

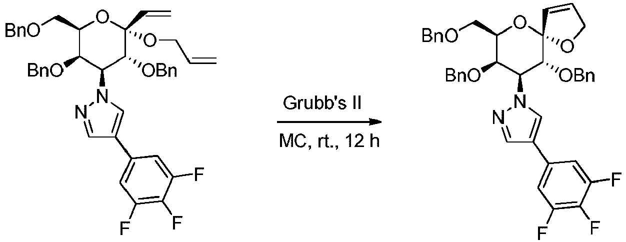

1-((2*S*,3*R*,4*S*,5*R*,6*R*)-2-(allyloxy)-3,5-bis(benzyloxy)-6-((benzyloxy)methyl)-2-vinyltetrahydro-2*H*-pyran-4-yl)-4-(3,4,5-trifluorophenyl)-1*H*-pyrazole (140 mg, 0.201 mmol) was dissolved in 5.0 mL of dichloromethane, and then Grubbs catalyst II (10.0 mg, 0.012 mmol) was added dropwise thereto, and the mixture was stirred at room temperature for 4 hours. The reaction mixture was filtered through Celite and then the filtrate was concentrated and subjected to silica gel column chromatography with 0% to 30% ethyl acetate/n-hexane to obtain the title compound (138 mg, yield: 99%).

¹H-NMR (400 MHz, CDCl₃) δ 7.73 (s, 1H), 7.52 (s, 1H), 7.30-7.25 (m, 9H), 7.13-7.09 (m, 4H), 6.94-6.89 (m, 2H), 6.79 (dd, J = 8.5, 6.4 Hz, 2H), 6.34 (d, J = 5.9 Hz, 1H), 5.89 (d, J = 6.0 Hz, 1H), 5.03 (dd, J = 11.3, 3.0 Hz, 1H), 4.81 (d, J = 14.6 Hz, 1H), 4.73 (d, J = 14.5 Hz, 1H), 4.51-4.41 (m, 5H), 4.35-4.28 (m, 2H), 4.12 (s, 1H), 3.82 (d, J = 10.9 Hz, 1H), 3.67 (d, J = 8.8 Hz, 1H), 3.62-3.57 (m, 1H).

### Synthesis of (5S,7R,8R,9S,10R)-7-(hydroxymethyl)-9-(4-(3,4,5-trifluorophenyl)-1H-pyrazol-1-yl)-1,6-dioxaspiro[4.5]decane-8,10-diol

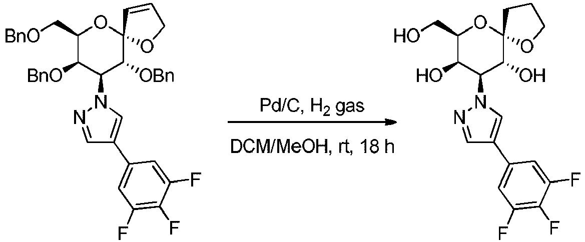

1-((5*S*,7*R*,8*R*,9*S*,10*R*)-8,10-bis(benzyloxy)-7-((benzyloxy)methyl)-1,6-dioxaspiro[4.5]dec-3-en-9-yl)-4-(3,4,5-trifluorophenyl)-1*H*-pyrazole (25 mg, 0.037 mmol) was dissolved in 2.0 mL of a solution of dichloromethane:methanol (1:2), and the mixture was added dropwise to a round-bottom flask, in which 10% palladium/carbon (25 mg) was suspended in 2.0 mL of a solution of dichloromethane:methanol (1:2), and degassed with a hydrogen balloon for 10 minutes, followed by stirring in a hydrogen atmosphere at room temperature for 18 hours. The reaction solution was filtered through Celite and then the filtrate was concentrated and subjected to silica gel column chromatography with 0% to 5% methanol/dichloromethane to obtain the title compound (14.4 mg, yield: 99%).

ESIMS: m/z 401.29 [M+H]+; ¹H-NMR (400 MHz, CD₃OD) δ 8.12 (s, 1H), 7.88 (s, 1H), 7.35 (dd, J = 9.0, 6.7 Hz, 2H), 4.61 (dd, J = 11.1, 2.7 Hz, 1H), 4.36 (d, J = 11.1 Hz, 1H), 4.09 (s, 1H), 4.02 (t, J = 6.5 Hz, 3H), 3.67 (d, J = 6.3 Hz, 2H), 2.36-2.24 (m, 1H), 2.15-2.05 (m, 1H), 2.03-1.86 (m, 2H).

### Example 223: Synthesis of Compound A238

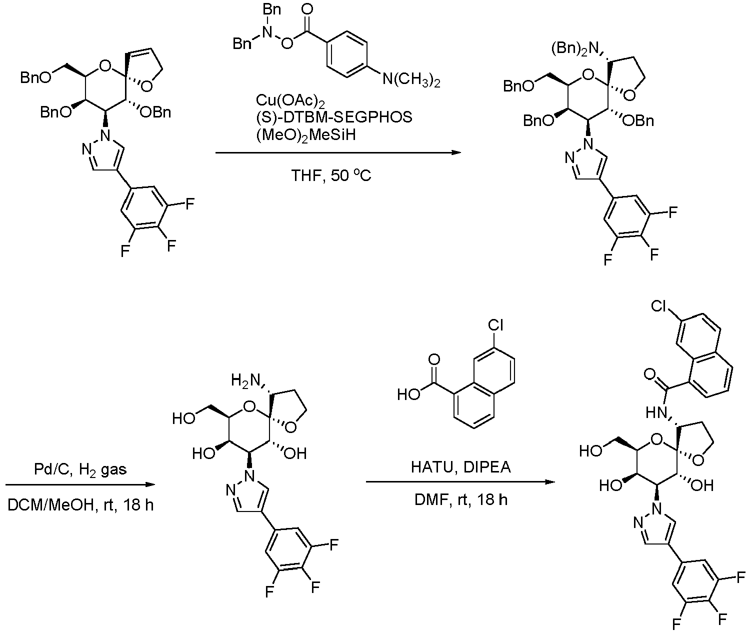

### Synthesis of (4R,5S,7R,8R,9S,10R)-N,N-dibenzyl-8,10- bis(benzyloxy)-7-((benzyloxy)methyl)-9-(4-(3,4,5-trifluorophenyl)-1H-pyrazol-1-yl)-1,6-dioxaspiro[4.5]decan-4-amine

Cu(OAc)₂ (2.0 mg, 0.011 mmol) and (S)-DTBM-SEGPHOS^{®} (14 mg, 0.012 mmol) were dissolved in 1.0 mL of tetrahydrofuran, stirred for 5 minutes, and then dimethoxymethylsilane (84 µL, 0.627 mmol) was added thereto, followed by stirring at room temperature for 15 minutes. This solution was slowly added dropwise to a mixture in which 1-((5*S*,7*R*,8*R*,9*S*,10*R*)-8,10-bis(benzyloxy)-7-((benzyloxy)methyl)-1,6-dioxaspiro[4.5]dec-3-en-9-yl)-4-(3,4,5-trifluorophenyl)-1*H*-pyrazole (140 mg, 0.209 mmol) and hydroxylamine ester (90 mg, 0.230 mmol) were dissolved in 1.0 mL of tetrahydrofuran, followed by stirring at 45 °C for 3 days. The reaction solution was filtered through Celite and then the filtrate was concentrated and subjected to silica gel column chromatography with 0% to 5% methanol/dichloromethane to obtain the title compound (51 mg, yield: 28%).

¹H-NMR (400 MHz, CD₃OD) δ 7.86 (s, 1H), 7.76 (s, 1H), 7.43 (d, J = 4.1 Hz, 4H), 7.34 (s, 5H), 7.19 (d, J = 4.5 Hz, 5H), 7.12 (s, 6H), 7.03 (s, 3H), 6.95-6.86 (m, 2H), 6.62 (s, 2H), 4.81 (d, J = 2.4 Hz, 1H), 4.38 (dd, J = 18.7, 10.5 Hz, 4H), 4.26 (t, J = 6.2 Hz, 1H), 4.16-4.05 (m, 3H), 4.00 (s, 1H), 3.87 (d, J = 10.9 Hz, 2H), 3.72-3.66 (m, 1H), 3.59 (dt, J = 15.3, 9.0 Hz, 2H), 3.47 (d, J = 11.1 Hz, 1H), 3.38 (d, J = 13.2 Hz, 2H), 3.18 (d, J = 11.1 Hz, 1H), 2.28-2.15 (m, 1H), 2.03 (d, J = 19.8 Hz, 1H).

### Synthesis of (4R,5S,7R,8R,9S,10R)-4-amino-7-(hydroxymethyl)-9-(4-(3,4,5-trifluorophenyl)-1H-pyrazol-1-yl)-1,6-dioxaspiro[4.5]decane-8,10-diol

(4*R*,5*S*,7*R*,8*R*,9*S*,10*R*)-*N*,*N-*dibenzyl-8,10-bis(benzyloxy)-7-((benzyloxy)methyl)-9-(4-(3,4,5-trifluorophenyl)-1*H*-pyrazol-1-yl)-1,6-dioxaspiro[4.5]decan-4-amine (50 mg, 0.058 mmol) was dissolved in 10 mL of a solution of dichloromethane:methanol (1:2), and the mixture was added dropwise to a round-bottom flask, in which 10% palldium/carbon (50 mg) was dissolved in 10 mL of a solution of dichloromethane:methanol (1:2), and then degassed with a hydrogen balloon for 10 minutes, followed by stirring in a hydrogen atmosphere at room temperature for 18 hours. The reaction solution was filtered through Celite and then the filtrate was concentrated to obtain the title compound (25 mg).

### Synthesis of 7-chloro-N-((4R,5S,7R,8R,9S,10R)-8,10-dihydroxy-7-(hydroxymethyl)-9-(4-(3,4,5-trifluorophenyl)-1H-pyrazol-1-yl)-1,6-dioxaspiro[4.5]decan-4-yl)-1-naphthamide

(4*R*,5*S*,7*R*,8*R*,9*S*,10*R*)-4-amino-7-(hydroxymethyl)-9-(4-(3,4,5-trifluorophenyl)-1*H-*pyrazol-1-yl)-1,6-dioxaspiro[4.5]decane-8,10-diol (10.0 mg, 0.024 mmol) was dissolved in 0.6 mL of *N*,*N-*dimethylformamide, and then 8-bromoquinoline-5-carboxylic acid (7.5 mg, 0.036 mmol), HATU (10 mg, 0.026 mmol), and *N,N*-diisopropylethylamine (15 µL, 0.096 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (5.3 mg, yield: 37%).

ESIMS: m/z 604.46 [M+H]+; ESIMS: m/z [M+H]+; ¹H-NMR(400 MHz, CD₃OD) δ 8.33 (s, 1H), 8.19 (s, 1H), 8.02 (d, J = 8.5 Hz, 1H), 7.94 (d, J = 8.7 Hz, 1H), 7.90 (s, 1H), 7.82 (d, J = 7.1 Hz, 1H), 7.61-7.55 (m, 1H), 7.53 (dd, J = 8.7, 1.9 Hz, 1H), 7.35 (dd, J = 8.9, 6.9 Hz, 2H), 5.06 (t, J = 9.3 Hz, 1H), 4.66 (dd, J = 11.2, 2.4 Hz, 1H), 4.52 (d, J = 11.2 Hz, 1H), 4.18 (td, J = 8.8, 3.4 Hz, 1H), 4.14-4.02 (m, 3H), 3.78 (dd, J = 11.4, 7.1 Hz, 1H), 3.70 (dd, J = 11.5, 4.9 Hz, 1H), 2.58-2.50 (m, 1H), 2.25-2.14 (m, 1H).

### Example 224: Synthesis of Compound A239

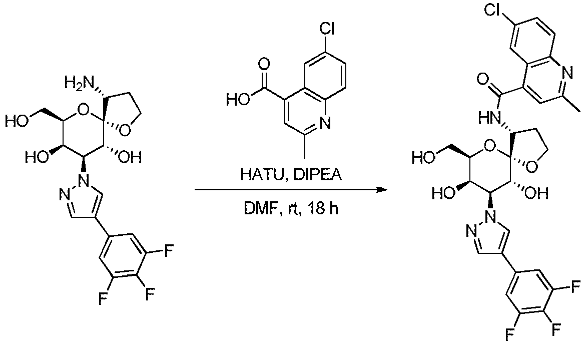

### Synthesis of 6-chloro-N-((4R,5S,7R,8R,9S,10R)-8, 10-dihydroxy-7-(hydroxymethyl)-9-(4-(3,4,5-trifluorophenyl)-1H-pyrazol-1-yl)-1,6-dioxaspiro[4.5]decan-4-yl)-2-methylquinoline-4-carboxamide

(4*R*,5*S*,7*R*,8*R*,9*S*,10*R*)-4-amino-7-(hydroxymethyl)-9-(4-(3,4,5-trifluorophenyl)-1*H-*pyrazol-1-yl)-1,6-dioxaspiro[4.5]decane-8,10-diol (10.0 mg, 0.024 mmol) was dissolved in 10.6 mL of *N,N*-dimethylformamide, and then 8-bromoquinoline-5-carboxylic acid (8.0 mg, 0.036 mmol), HATU (10.0 mg, 0.026 mmol), and *N,N*-diisopropylethylamine (15 µL, 0.096 mmol) were slowly added dropwise thereto at room temperature, followed by stirring at room temperature for 18 hours. The reaction solution was extracted with ethyl acetate, dried over anhydrous magnesium sulfate, filtered, and then concentrated. The concentrated reaction mixture was subjected to amine silica gel column chromatography with 0% to 5% methanol/dichloromethane to obtain the title compound (2.7 mg, yield: 18%).

ESIMS: m/z 619.46 [M+H]+; ESIMS: m/z [M+H]+; ¹H-NMR(400 MHz, CD₃OD) δ 8.25 (d, J = 2.0 Hz, 1H), 8.19 (s, 1H), 7.99 (d, J = 9.0 Hz, 1H), 7.90 (s, 1H), 7.76 (dd, J = 9.2, 2.2 Hz, 1H), 7.66 (s, 1H), 7.35 (dd, J = 9.1, 6.6 Hz, 2H), 5.07 (t, J = 9.2 Hz, 1H), 4.65 (dd, J = 11.2, 2.4 Hz, 1H), 4.52 (d, J = 11.1 Hz, 1H), 4.21-4.06 (m, 4H), 3.75 (ddd, J = 16.5, 11.4, 6.0 Hz, 2H), 2.77 (s, 3H), 2.56-2.47 (m, 1H), 2.25-2.14 (m, 1H).

### Example 225: Synthesis of Compound A240

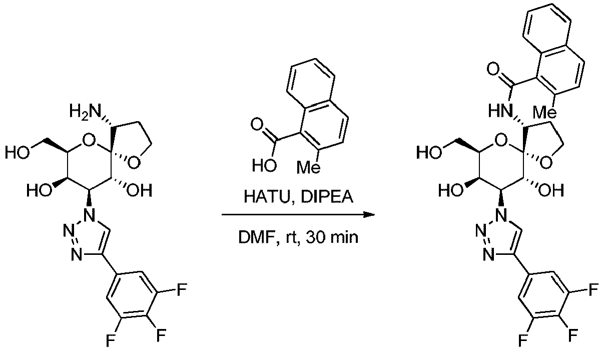

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-methyl-1-naphthamide

The title compound (10.9 mg, yield: 78%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 585.49 [M+H]+; ¹H-NMR(400 MHz, CD₃OD) δ 8.55 (s, 1H), 8.07 (s, br, 1H), 7.84 (d, J = 8.6 Hz, 2H), 7.67-7.63 (m, 2H), 7.54 (dd, J = 7.2, 7.2 Hz, 1H), 7.45 (dd, J = 7.4, 7.4 Hz, 1H), 7.40 (d, J = 8.2 Hz, 1H), 5.17 (t, J = 9.2 Hz, 1H), 5.10 (dd, J = 11.0, 2.7 Hz, 1H), 4.74 (d, J = 11.3 Hz, 1H), 4.18-4.06 (m, 4H), 3.76 (dd, J = 11.3, 7.0 Hz, 1H), 3.69 (dd, J = 11.3, 4.7 Hz, 1H), 2.57 (s, 3H), 2.53-2.49 (m, 1H), 2.22-2.12 (m, 1H)

### Example 226: Synthesis of Compound A241

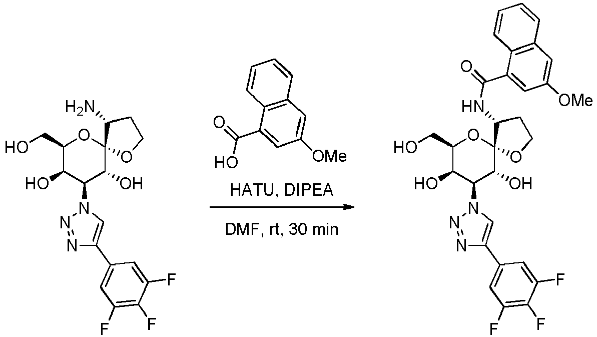

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-3- methoxy-1-naphthamide

The title compound (13.0 mg, yield: 90%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 601.5 [M+H]+; ¹H-NMR(400 MHz, CD₃OD) δ 8.57 (s, 1H), 8.26 (d, J = 8.2 Hz, 1H), 7.83 (d, J = 8.2 Hz, 1H), 7.65-7.61 (m, 2H), 7.48 (dd, J = 7.2, 7.2 Hz, 1H), 7.43-7.38 (m, 3H), 5.11 (t, J = 9.4 Hz, 2H), 4.62 (d, J = 11.0 Hz, 1H), 4.19-4.06 (m, 4H), 3.95 (s, 3H), 3.82 (dd, J = 11.3, 7.4 Hz, 1H), 3.73 (dd, J = 11.3, 4.7 Hz, 1H), 2.53-2.48 (m, 1H), 2.23-2.13 (m, 1H)

### Example 227: Synthesis of Compound A242

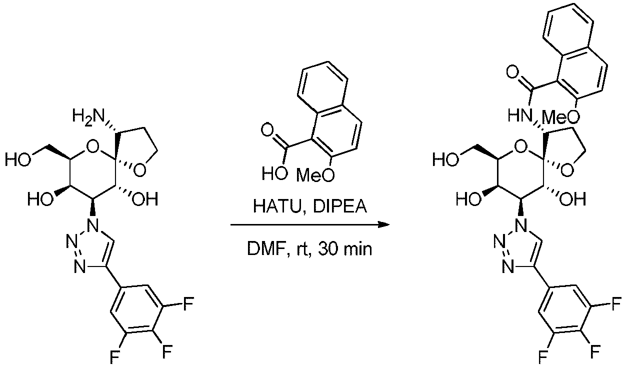

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2- methoxy-1-naphthamide

The title compound (9.5 mg, yield: 66%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 601.5 [M+H]+; ¹H-NMR(400 MHz, CD₃OD) δ 8.55 (s, 1H), 8.05 (d, J = 8.6 Hz, 1H), 7.97 (d, J = 9.0 Hz, 1H), 7.83 (d, J = 8.2 Hz, 1H), 7.66-7.63 (m, 2H), 7.52 (dd, J = 7.6, 7.6 Hz, 1H), 7.46 (d, J = 9.0 Hz, 1H), 7.37 (dd, J = 7.6, 7.6 Hz, 1H), 5.15-5.08 (m, 2H), 4.72 (d, J = 11.3 Hz, 1H), 4.16-4.06 (m, 4H), 4.02 (s, 3H), 3.77 (dd, J = 11.3, 7.0 Hz, 1H), 3.70 (dd, J = 11.3, 5.1 Hz, 1H), 2.54-2.46 (m, 1H), 2.21-2.11 (m, 1H)

### Example 228: Synthesis of Compound A243

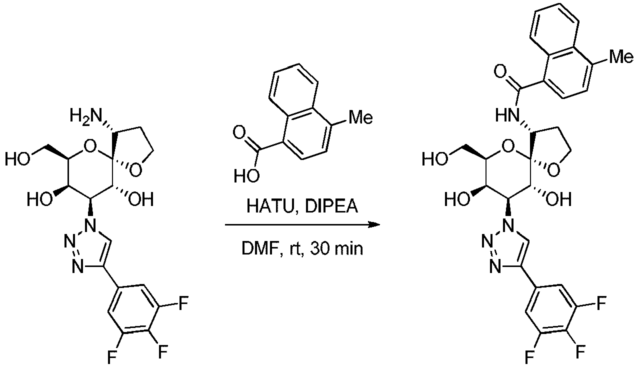

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-4-methyl-1-naphthamide

The title compound (13.7 mg, yield: 98%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 585.49 [M+H]+; ¹H-NMR(400 MHz, CD₃OD) δ 8.57 (s, 1H), 8.40 (dd, J = 6.8, 2.5 Hz, 1H), 8.10 (dd, J = 6.7, 2.7 Hz, 1H), 7.65-7.58 (m, 5H), 7.40 (d, J = 7.4 Hz, 1H), 5.14-5.08 (m, 2H), 4.63 (d, J = 11.3 Hz, 1H), 4.20-4.06 (m, 4H), 3.81 (dd, J = 11.3, 7.4 Hz, 1H), 3.73 (dd, J = 11.3, 4.7 Hz, 1H), 2.73 (s, 3H), 2.53-2.45 (m, 1H), 2.23-2.13 (m, 1H)

### Example 229: Synthesis of Compound A244

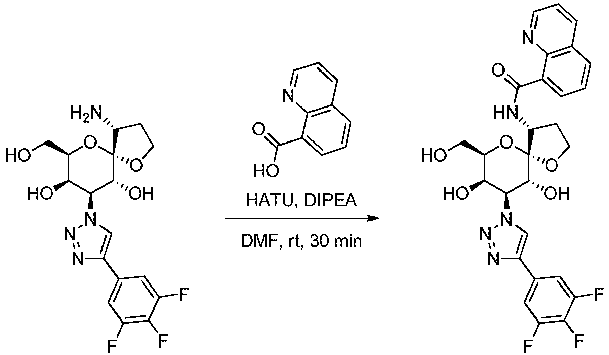

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-8-carboxamide

The title compound (11.7 mg, yield: 85%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 572.45 [M+H]+; ¹H-NMR(400 MHz, CD₃OD) δ 9.05 (d, J = 2.7 Hz, 1H), 8.70 (d, J = 7.0 Hz, 1H), 8.51 (s, 1H), 8.46 (d, J = 7.4 Hz, 1H), 8.13 (d, J = 8.2 Hz, 1H), 7.72 (dd, J = 7.8, 7.8 Hz, 1H), 7.61 (dd, J = 8.2, 4.3 Hz, 1H), 7.57-7.53 (m, 2H), 5.13 (dd, J = 11.2, 2.5 Hz, 1H), 5.00 (t, J = 9.2 Hz, 1H), 4.53 (d, J = 11.3 Hz, 1H), 4.28-4.20 (m, 3H), 4.13 (q, J = 8.1 Hz, 1H), 3.95 (dd, J = 11.0, 6.3 Hz, 1H), 3.89 (dd, J = 11.2, 5.7 Hz, 1H), 2.64-2.60 (m, 1H), 2.27-2.17 (m, 1H)

### Example 230: Synthesis of Compound A245

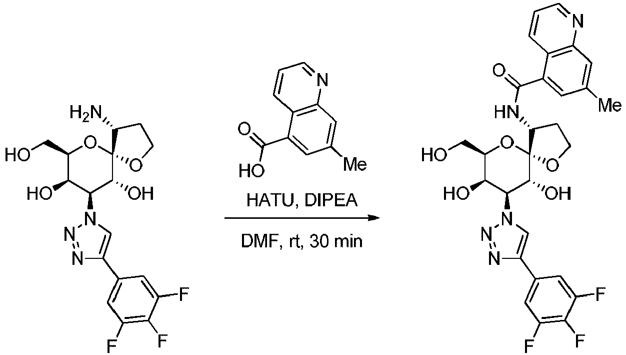

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-7-methylquinoline-5-carboxamide

The title compound (10.7 mg, yield: 76%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 586.44 [M+H]+; ¹H-NMR(400 MHz, CD₃OD) δ 8.87-8.84 (m, 2H), 8.57 (s, 1H), 7.93 (s, 1H), 7.75 (s, 1H), 7.64-7.61 (m, 2H), 7.55 (dd, J = 8.4, 4.5 Hz, 1H), 5.16-5.08 (m, 2H), 4.64 (d, J = 11.3 Hz, 1H), 4.21-4.15 (m, 3H), 4.09 (dd, J = 15.8, 8.0 Hz, 1H), 3.81 (dd, J = 11.2, 7.2 Hz, 1H), 3.76 (dd, J = 11.2, 5.3 Hz, 1H), 2.62 (s, 3H), 2.50-2.43 (m, 1H), 2.24-2.14 (m, 1H)

### Example 231: Synthesis of Compound A246

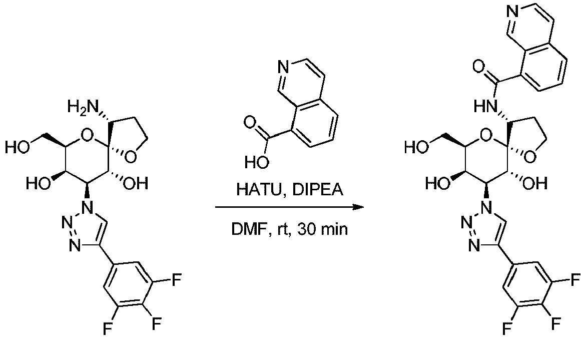

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)isoquinoline-8-carboxamide

The title compound (8.2 mg, yield: 76%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 572.51 [M+H]+; ¹H-NMR(400 MHz, CD₃OD) δ 9.71 (s, 1H), 8.59 (s, 1H), 8.52 (d, J = 5.5 Hz, 1H), 8.09 (d, J = 7.8 Hz, 1H), 7.94-7.84 (m, 3H), 7.67-7.63 (m, 2H), 5.15-5.08 (m, 2H), 4.63 (d, J = 11.3 Hz, 1H), 4.22-4.08 (m, 4H), 3.80 (dd, J = 11.5, 7.2 Hz, 1H), 3.74 (dd, J = 11.3, 5.1 Hz, 1H), 2.53-2.49 (m, 1H), 2.26-2.16 (m, 1H)

### Example 232: Synthesis of Compound A247

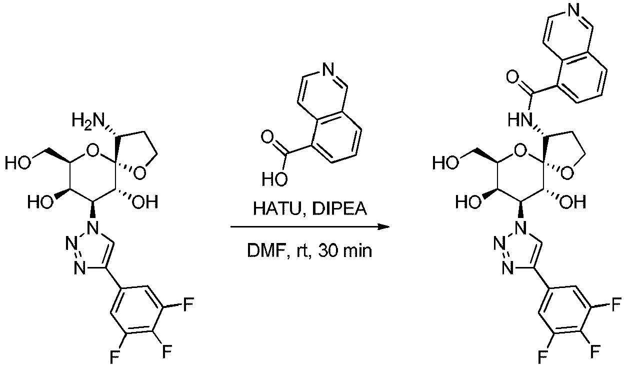

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)isoquinoline-5-carboxamide

The title compound (10.4 mg, yield: 76%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 572.45 [M+H]+; ¹H-NMR(400 MHz, dmso) δ 9.39 (s, 1H), 8.71 (s, 1H), 8.56 (d, J = 5.9 Hz, 1H), 8.33-8.30 (m, 2H), 8.27 (d, J = 7.8 Hz, 1H), 7.96 (d, J = 7.0 Hz, 1H), 7.89-7.85 (m, 2H), 7.78 (d, J = 7.0 Hz, 1H), 5.42 (d, J = 6.3 Hz, 1H), 5.22 (d, J = 9.4 Hz, 1H), 5.03 (d, J = 9.4 Hz, 1H), 4.93 (dd, J = 11.2, 2.2 Hz, 1H), 4.48-4.43 (m, 2H), 4.10-3.94 (m, 4H), 3.63-3.55 (m, 2H), 2.37-2.31 (m, 1H), 2.09-2.01 (m, 1H)

### Example 233: Synthesis of Compound A248

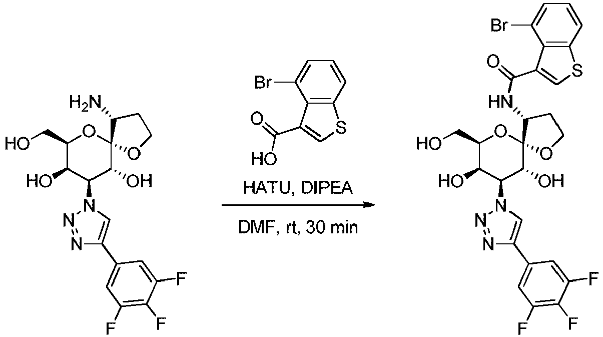

### Synthesis of 4-bromo-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-3-carboxamide

The title compound (14.7 mg, yield: 93%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 655.32 [M+H]+; ¹H-NMR(400 MHz, CD₃OD) δ 8.49 (s, 1H), 7.98-7.95 (m, 2H), 7.68-7.61 (m, 3H), 7.32-7.28 (m, 1H), 5.06 (dd, J = 11.3, 2.7 Hz, 1H), 4.97 (t, J = 9.4 Hz, 1H), 4.59 (d, J = 11.3 Hz, 1H), 4.20-4.05 (m, 4H), 3.77 (dd, J = 11.5, 6.8 Hz, 1H), 3.70 (dd, J = 11.3, 5.1 Hz, 1H), 2.57-2.49 (m, 1H), 2.26-2.16 (m, 1H)

### Example 234: Synthesis of Compound A249

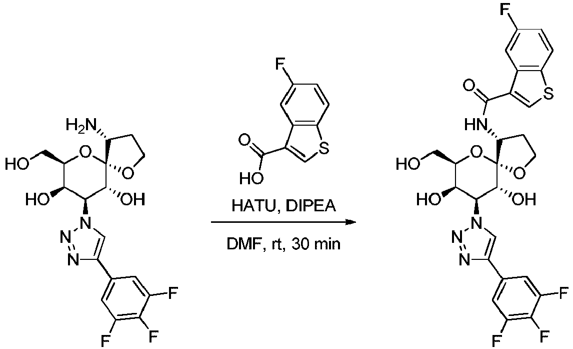

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-5-fluorobenzo[b]thiophene-3-carboxamide

The title compound (11.4 mg, yield: 80%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 595.45 [M+H]+; ¹H-NMR(400 MHz, CD₃OD) δ 8.54 (s, 1H), 8.41 (s, 1H), 8.16 (dd, J = 10.2, 2.3 Hz, 1H), 7.94 (dd, J = 8.8, 4.9 Hz, 1H), 7.62-7.58 (m, 2H), 7.23 (td, J = 8.7, 2.2 Hz, 1H), 5.10-5.02 (m, 2H), 4.48 (d, J = 11.0 Hz, 1H), 4.19-4.05 (m, 4H), 3.87 (dd, J = 11.2, 7.6 Hz, 1H), 3.78 (dd, J = 11.3, 4.7 Hz, 1H), 2.50-2.44 (m, 1H), 2.24-2.14 (m, 1H)

### Example 235: Synthesis of Compound A250

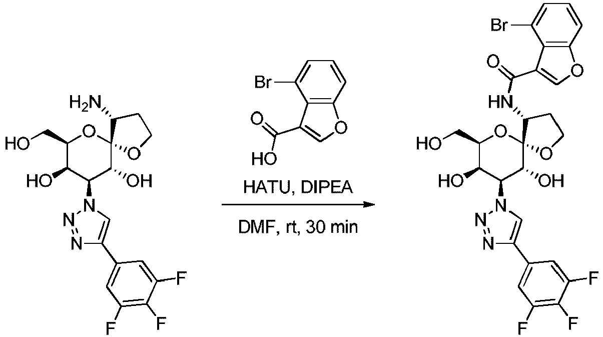

### Synthesis of 4-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzofuran-3-carboxamide

The title compound (12.9 mg, yield: 84%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 639.38 [M+H]+; ¹H-NMR(400 MHz, CD₃OD) δ 8.49 (s, 1H), 8.22 (s, 1H), 7.64-7.52 (m, 4H), 7.30 (dd, J = 8.0, 8.0 Hz, 1H), 5.06 (dd, J= 11.2, 2.5 Hz, 1H), 4.98 (t, J = 9.2 Hz, 1H), 4.53 (d, J = 11.0 Hz, 1H), 4.19-4.05 (m, 4H), 3.80 (dd, J = 11.5, 6.8 Hz, 1H), 3.72 (dd, J = 11.3, 5.1 Hz, 1H), 2.54-2.47 (m, 1H), 2.25-2.15 (m, 1H)

### Example 236: Synthesis of Compound A251

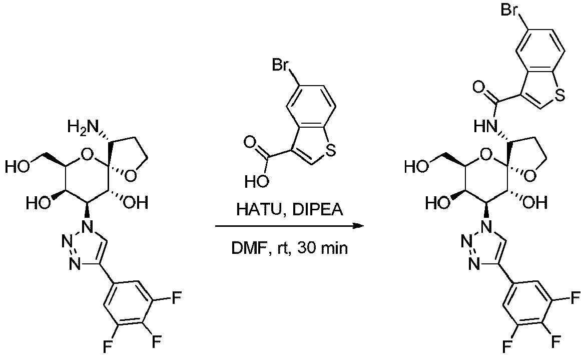

### Synthesis of 5-bromo-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[b]thiophene-3-carboxamide

The title compound (13.8 mg, yield: 88%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 655.38 [M+H]+; ¹H-NMR(400 MHz, CD₃OD) δ 8.60 (d, J = 1.6 Hz, 1H), 8.54 (s, 1H), 8.38 (s, 1H), 7.87 (d, J = 8.6 Hz, 1H), 7.62-7.58 (m, 2H), 7.54 (dd, J = 8.6, 1.6 Hz, 1H), 5.10-5.01 (m, 2H), 4.48 (d, J = 11.0 Hz, 1H), 4.19-4.05 (m, 4H), 3.87 (dd, J = 11.3, 7.4 Hz, 1H), 3.77 (dd, J = 11.5, 4.9 Hz, 1H), 2.51-2.45 (m, 1H), 2.24-2.14 (m, 1H)

### Example 237: Synthesis of Compound A252

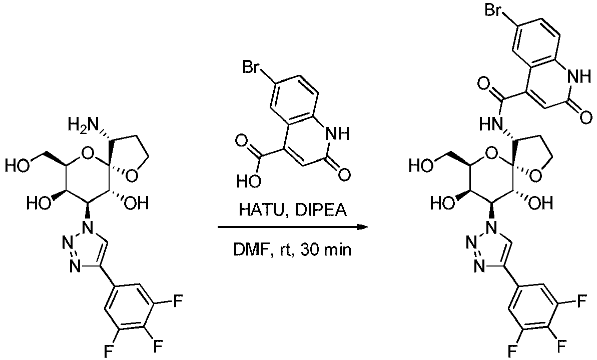

### Synthesis of 6-bromo-N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-oxo-1,2-dihydroquinoline-4-carboxamide

The title compound (6.4 mg, yield: 40%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 666.22 [M+H]+; ¹H-NMR(400 MHz, CD₃OD) δ 8.57 (s, 1H), 8.07 (d, J = 2.0 Hz, 1H), 7.71 (dd, J = 9.0, 2.0 Hz, 1H), 7.65-7.61 (m, 2H), 7.32 (d, J = 8.6 Hz, 1H), 6.84 (s, 1H), 5.07 (dd, J = 11.3, 2.7 Hz, 1H), 5.02 (t, J = 9.2 Hz, 1H), 4.56 (d, J = 11.3 Hz, 1H), 4.21-4.06 (m, 4H), 3.79 (dd, J = 11.3, 7.4 Hz, 1H), 3.74 (dd, J = 11.3, 5.1 Hz, 1H), 2.53-2.45 (m, 1H), 2.23-2.13 (m, 1H)

### Example 238: Synthesis of Compound A253

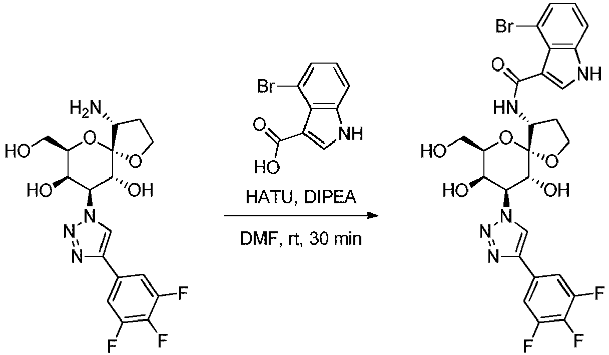

### Synthesis of 4-bromo-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1H-indole-3-carboxamide

The title compound (10.3 mg, yield: 67%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9S*,*10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 638.37 [M+H]+; ¹H-NMR(400 MHz, CD₃OD) δ 8.49 (s, 1H), 7.79 (s, 1H), 7.64-7.60 (m, 2H), 7.44 (d, J = 8.2 Hz, 1H), 7.35 (d, J = 7.4 Hz, 1H), 7.08 (dd, J = 8.2, 8.2 Hz, 1H), 5.07 (dd, J = 11.2, 2.5 Hz, 1H), 4.98 (t, J = 9.4 Hz, 1H), 4.52 (d, J = 11.3 Hz, 1H), 4.19-4.05 (m, 4H), 3.81 (dd, J = 11.3, 7.0 Hz, 1H), 3.73 (dd, J = 11.3, 5.1 Hz, 1H), 2.55-2.48 (m, 1H), 2.26-2.16 (m, 1H)

### Example 239: Synthesis of Compound A254

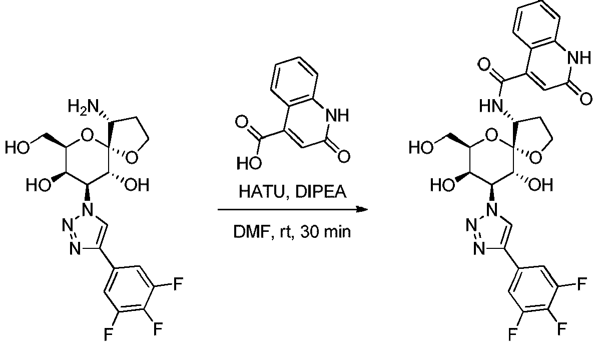

### Synthesis of N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-2-oxo-1,2-dihydroquinoline-4-carboxamide

The title compound (6.9 mg, yield: 49%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: 588.52 m/z [M+H]+; ¹H-NMR(400 MHz, CD₃OD) δ 8.58 (s, 1H), 8.03 (d, J = 8.2 Hz, 1H), 7.66-7.58 (m, 3H), 7.39 (d, J = 8.2 Hz, 1H), 7.31 (dd, J = 5.7, 5.7 Hz, 1H), 6.76 (s, 1H), 5.10-5.04 (m, 2H), 4.62 (d, J = 11.0 Hz, 1H), 4.20-4.05 (m, 4H), 3.78 (dd, J = 11.2, 7.2 Hz, 1H), 3.73 (dd, J = 11.3, 5.5 Hz, 1H), 2.48-2.43 (m, 1H), 2.22-2.12 (m, 1H)

### Example 240: Synthesis of Compound A255

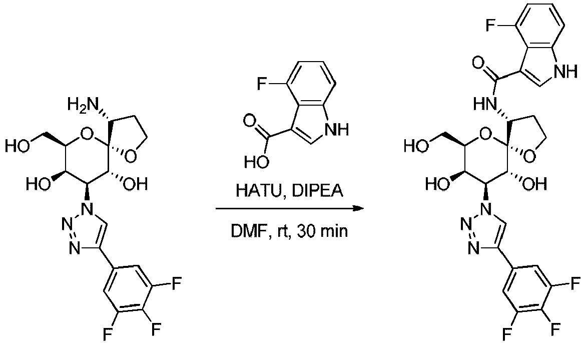

### Synthesis of N-((4R,5S,7R,8R,9S,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-4-fluoro-1H-indole-3-carboxamide

The title compound (2.2 mg, yield: 16%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 578.5 [M+H]+; ¹H-NMR(400 MHz, CD₃OD) δ 8.54 (s, 1H), 8.00 (s, 1H), 7.62-7.58 (m, 2H), 7.32 (d, J = 8.2 Hz, 1H), 7.20 (ddd, J = 12.4 Hz, 1H), 6.92 (dd, J = 12.7, 8.0 Hz, 1H), 5.09 (dd, J = 11.2, 2.5 Hz, 1H), 4.97 (t, J = 9.4 Hz, 1H), 4.45 (d, J = 11.0 Hz, 1H), 4.22-4.05 (m, 4H), 3.86 (dd, J = 11.3, 6.3 Hz, 1H), 3.81 (dd, J = 11.0, 5.9 Hz, 1H), 2.56-2.51 (m, 1H), 2.17-2.09 (m, 1H)

### Example 241: Synthesis of Compound A256

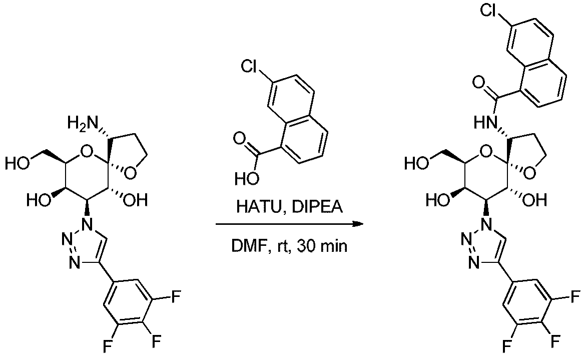

### Synthesis of 7-chloro-N-((4R,5S,7R,8R,9S,10R)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-naphthamide

The title compound (9.3 mg, yield: 64%) as a white solid was synthesized from (4*R*,5*S*,7*R*,8*R*,9*S*,10*R*)-4-amino-9-(4-(3,4,5-trifluorophenyl)-1*H*-1,2,3-triazol-1-yl)-7-(hydroxymethyl)-1,6-dioxaspiro[4.5]decane-8,10-diol (10.0 mg, 0.024 mmol) in a similar manner to the preparation method of *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide.

ESIMS: m/z 605.47 [M+H]+; ¹H-NMR(400 MHz, CD₃OD) δ 8.56 (s, 1H), 8.35 (s, 1H), 8.03 (d, J = 8.2 Hz, 1H), 7.95 (d, J = 8.6 Hz, 1H), 7.82 (d, J = 7.0 Hz, 1H), 7.64-7.56 (m, 3H), 7.53 (dd, J = 8.6, 1.6 Hz, 1H), 5.10-5.06 (m, 2H), 4.59 (d, J = 11.3 Hz, 1H), 4.21-4.08 (m, 4H), 3.80 (dd, J = 11.5, 7.2 Hz, 1H), 3.73 (dd, J = 11.5, 4.9 Hz, 1H), 2.57-2.49 (m, 1H), 2.25-2.16 (m, 1H)

### Example 242: Synthesis of Compound A257

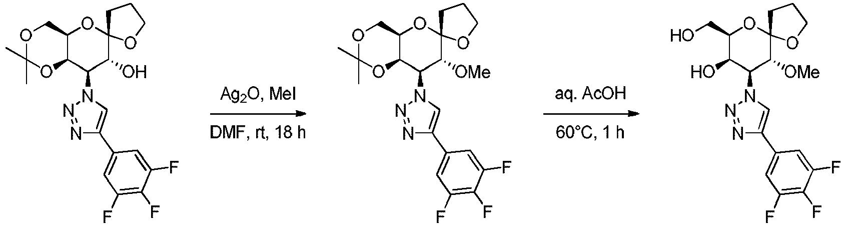

### Synthesis of 1-((2S,4a'R,7'R,8'S,8a'R)-7'-methoxy-2',2'-dimethylhexahydro-3H,4'H-spiro[furan-2,6'-pyrano[3,2-d][1,3]dioxin]-8'-yl)-4-(3,4,5-trifluorophenyl)-1H-1,2,3-triazole

(2*S*,4a'*R*,7'*R*,8'*R*,8a'*R*)-2',2'-dimethyl-8'-(4-(3,4,5-trifluorophenyl)-1*H*-1,2,3-triazol-1-yl)hexahydro-3*H*,4'*H*-spiro[furan-2,6'-pyrano[3,2-*d*][1,3]dioxin]-7'-ol (15.0 mg, 0.034 mmol) was dissolved in 0.7 mL of *N,N*-dimethylformamide, and then silver(I) oxide (15.7 mg, 0.068 mmol), and methyl iodide (7.0 µL, 0.068 mmol) were added thereto. The reaction mixture was stirred at room temperature for about 18 hours. The reaction mixture was filtered through a pad of Celite 545 and then the filtrate was concentrated under reduced pressure. The remaining concentrate was subjected to column chromatography with 0% to 35% ethyl acetate/n-hexane to synthesize the title compound as a white solid (13.8 mg, yield: 89%).

ESIMS: m/z [M+H]+; ¹H-NMR(400 MHz, CDCl₃) δ 8.05 (s, 1H), 7.49-7.45 (m, 2H), 5.36 (dd, J = 11.0, 3.1 Hz, 1H), 4.34 (d, J = 2.0 Hz, 1H), 4.11-3.98 (m, 4H), 3.90 (d, J = 12.9 Hz, 1H), 3.82 (s, 1H), 3.08 (s, 3H), 2.22-2.06 (m, 3H), 2.02-1.92 (m, 1H), 1.52 (s, 3H), 1.34 (s, 3H)

### Synthesis of (5S,7R,8R,9S,10R)-7-(hydroxymethyl)-10-methoxy-9-(4-(3,4,5-trifluorophenyl)-1H-1,2,3-triazol-1-yl)-1,6-dioxaspiro[4.5]decan-8-ol

The title compound as a white solid (9.6 mg, yield: 77%) was synthesized from 1-((2S,4a'R,7'R,8'S,8a'R)-7'-methoxy-2',2'-dimethylhexahydro-3H,4'H-spiro[furan-2,6'-pyrano[3,2-d][1,3]dioxin]-8'-yl)-4-(3,4,5-trifluorophenyl)-1H-1,2,3-triazole (13.8 mg, 0.030 mmol) in a similar manner to the preparation method of 4-((((5S,7R,8R,9S,10R)-8-hydroxy-7-(hydroxymethyl)-9-(4-(3,4,5-trifluorophenyl)-1H-1,2,3-triazol-1-yl)-1,6-dioxaspiro[4.5]decan-10-yl)oxy)methyl)tetrahydro-2H-thiopyran 1,1-dioxide.

ESIMS: m/z 416.41 [M+H]+; 1H-NMR(400 MHz, CD3OD) δ 8.67 (s, 1H), 7.68-7.64 (m, 2H), 5.09 (dd, J = 11.3, 2.7 Hz, 1H), 4.24 (d, J = 11.0 Hz, 1H), 4.09-3.96 (m, 4H), 3.68 (d, J = 5.9 Hz, 2H), 3.18 (s, 3H), 2.21-1.93 (m, 4H)

### TR-FRET Assay (in vitro Galectin-3 Binding Inhibitory Activity Test)

TR-FRET assay was performed in 1/2 Area Plate-96 (PerkinElmer). Final concentrations of components in the assay were 60 nM his-galectin-3 (Abeam), 15 nM biotin-ASF (Axxora), 2 nM Mab anti-6xHis Tb (Cisbio) and 20 nM SA-d2 (Cisbio). The reaction was performed in a buffer containing 25 mM HEPES, 100 mM NaCl, 0.005% Tween 20 and 0.05% BSA. 20 µL his-galectin-3 and 1 µL compound were mixed first, and then 20 µL biotin-ASF, 5 µL anti-His Tb and 5 µL SA-d2 were added in order. After incubation for at least 3.5 hours at RT, TR-FRET signals were measured at 615/665 nm excitation/emission wavelengths using a VICTOR Nivo (PerkinElmer). The positive control used DMSO instead of the compound, and the negative control excluded ASF-biotin. N-acetyl-D-lactosamine (LacNAc), an endogenous ligand unit of galectin-3, was evaluated together as a control substance to compare galectin-3 binding inhibitory activity.

The inhibition rates of the compounds of Examples are shown in Tables 1 and 2 below. At the 20 µM concentration, the compounds were classified as D when the inhibition rate was less than 25%, C when the inhibition rate was 25% to 50% (exclusive of 50%), B when the inhibition rate was 50% to 75% (exclusive of 75%), and A when the inhibition rate was at least 75%.

**[Table 1]**

| Compound | % Inhibitory activity (at 20 µM) | Compound | % Inhibitory activity (at 20 µM) | Compound | % Inhibitory activity (at 20 µM) | Compound | % Inhibitory activity (at 20 µM) |
|---|---|---|---|---|---|---|---|
| LacNAc | D | A45 | B | A82 | D | A118 | A |
| A08 | D | A46 | C | A83 | C | A119 | A |
| A09 | D | A47 | C | A84 | D | A120 | C |
| A10 | D | A48 | C | A85 | A | A121 | C |
| A11 | C | A49 | C | A86 | A | A122 | B |
| A12 | C | A50 | C | A87 | C | A123 | B |
| A13 | C | A51 | C | A88 | D | A124 | B |
| A16 | C | A52 | D | A89 | D | A125 | D |
| A17 | C | A53 | C | A90 | D | A126 | C |
| A18 | C | A54 | C | A91 | D | A127 | B |
| A19 | C | A55 | B | A92 | C | A128 | B |
| A20 | C | A56 | D | A93 | D | A129 | B |
| A21 | B | A57 | B | A94 | D | A130 | B |
| A22 | C | A58 | C | A95 | D | A131 | D |
| A23 | C | A59 | C | A96 | D | A132 | C |
| A24 | C | A60 | D | A97 | D | A133 | A |
| A25 | C | A61 | C | A98 | D | A134 | A |
| A26 | C | A62 | B | A100 | C | A135 | D |
| A27 | C | A63 | D | A101 | C | A136 | A |
| A28 | C | A64 | D | A102 | C | A137 | B |
| A29 | D | A65 | D | A103 | D | A138 | D |
| A30 | D | A66 | C | A104 | D | A139 | D |
| A31 | C | A69 | C | A105 | D | A140 | D |
| A32 | C | A70 | D | A106 | B | A141 | D |
| A33 | C | A71 | C | A107 | C | A142 | D |
| A34 | C | A72 | B | A108 | C | A143 | D |
| A35 | C | A73 | D | A109 | D | A144 | D |
| A36 | B | A74 | C | A110 | D | A145 | D |
| A37 | C | A75 | C | A111 | C | A146 | C |
| A38 | C | A76 | B | A112 | B | A147 | D |
| A39 | B | A77 | C | A113 | B | A148 | C |
| A40 | C | A78 | D | A114 | D | A149 | B |
| A41 | D | A79 | D | A115 | B | A150 | A |
| A42 | D | A80 | C | A116 | B | A151 | A |
| A44 | C | A81 | C | A117 | B | A152 | B |

**[Table 2]**

| Compound | % Inhibitory activity (at 20 µM) | Compound | % Inhibitory activity (at 20 µM) | Compound | % Inhibitory activity (at 20 µM) |
|---|---|---|---|---|---|
| A153 | D | A188 | D | A223 | D |
| A154 | C | A189 | D | A224 | D |
| A155 | D | A190 | D | A225 | D |
| A156 | B | A191 | D | A226 | D |
| A157 | A | A192 | C | A227 | D |
| A158 | B | A193 | B | A228 | A |
| A159 | B | A194 | B | A229 | A |
| A160 | B | A195 | C | A230 | B |
| A161 | A | A196 | B | A231 | A |
| A162 | B | A197 | B | A232 | A |
| A163 | B | A198 | A | A233 | B |
| A164 | A | A199 | C | A234 | A |
| A165 | B | A200 | B | A235 | B |
| A166 | B | A201 | A | A236 | B |
| A167 | C | A202 | D | A237 | C |
| A168 | B | A203 | C | A238 | A |
| A169 | A | A204 | D | A239 | A |
| A170 | A | A205 | D | A240 | B |
| A171 | C | A206 | A | A241 | A |
| A172 | A | A207 | A | A242 | B |
| A173 | C | A208 | A | A243 | A |
| A174 | D | A209 | A | A244 | B |
| A175 | D | A210 | A | A245 | A |
| A176 | A | A211 | A | A246 | A |
| A177 | A | A212 | A | A247 | A |
| A178 | A | A213 | B | A248 | D |
| A179 | B | A214 | B | A249 | A |
| A180 | A | A215 | A | A250 | A |
| A181 | A | A216 | A | A251 | A |
| A182 | B | A217 | A | A252 | A |
| A183 | D | A218 | A | A253 | A |
| A184 | D | A219 | B | A254 | B |
| A185 | D | A220 | D | A255 | A |
| A186 | D | A221 | D | A256 | A |
| A187 | D | A222 | D | A257 | C |

The found values for some compounds of Examples are listed in Table 3.

**[Table 3]**

| Compound | % Inhibitory activity (at 20 µM) |
|---|---|
| A08 | 21.8 |
| A22 | 28.07 |
| A32 | 36.68 |
| A36 | 68.16 |
| A48 | 43.74 |
| A55 | 60.02 |
| A65 | 17.3 |
| A75 | 32.03 |
| A86 | 80.35 |
| A100 | 48.1 |
| A112 | 53.77 |
| A119 | 93.65 |
| A130 | 56.64 |
| A146 | 40.71 |
| A162 | 69.93 |
| A170 | 85.28 |
| A182 | 72.99 |
| A191 | 24.01 |
| A200 | 63.29 |
| A210 | 90.56 |
| A224 | 24.19 |
| A230 | 66.52 |
| A240 | 72.09 |
| A249 | 89.86 |
| A257 | 39.23 |

## Claims

1. A compound represented by Formula (I) below, a stereoisomer, or a pharmaceutically acceptable salt thereof: 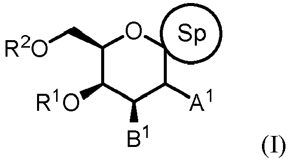
wherein Sp is an unsaturated or saturated C₃₋₁₅ monocyclic or polycyclic carbocycle or 3- to 15-membered heterocycle, and Sp has or does not have 1 to 3 substituents selected from among halogen, CN, NO₂, OH, oxo, Rx and Ry;
A¹ is H, halogen, CN, NO₂, OH, Rx, or Ry;
B¹ is halogen, CN, NO₂, Rx, or Ry;
R¹ and R² are each independently H or a metabolizable group in the body;
Rx is H, C₁₋₆ alkyl, C₂₋₆ alkenyl, C₂₋₆ alkynyl, C₃₋₁₄ cycloalkyl, 4- to 14-membered heterocycloalkyl, C₆₋₁₀ aryl, 5- to 14-membered heteroaryl, C₃₋₁₄ cycloalkyl-C₁₋₆ alkyl-, (4- to 14-membered heterocycloalkyl)-C₁₋₆ alkyl-, C₆₋₁₀ aryl-C₁₋₆ alkyl-, C₆₋₁₀ aryl-C₃₋₆ cycloalkyl-, (5- to 14-membered heteroaryl)-C₁₋₆ alkyl-, C₆₋₁₀ aryl-C₆₋₁₀ aryl-, C₆₋₁₀ aryl-C₆₋₁₀ aryl-C₁₋₆ alkyl, C₆₋₁₀ aryloxy-C₆₋₁₀ aryl-, C₆₋₁₀ aryl-(5- to 14-membered heteroaryl)-, or (5- to 14-membered heteroaryl)-C₆₋₁₀ aryl-C₁₋₆ alkyl-;
Ry's are each independently -ORx, -SRx, -NHORx, -N(OH)Rx, -C(O)Rx, -C(O)NRxRx, -C(O)ORx, -OC(O)Rx, -OC(RxRx)-C(O)Rx, -OC(RxRx)-C(O)ORx, -OC(O)NRxRx, - OC(RxRx)-C(O)NRxRx, -NRxRx, -NRxC(O)Rx, -NRxC(O)ORx, -NRxC(O)NRxRx, - C(=NRx)Rx, -C(=NRx)NRxRx, -NRxC(=NRx)NRxRx, -N=CRxRx, -NRxS(O)Rx, - NRxS(O)₂Rx, -NRxS(O)₂NRxRx, -S(O)Rx, -S(O)NRxRx, -S(O)₂Rx, -S(O)₂NRxRx, -OS(O)₂Rx, -PRxRx, -P(O)RxRx, -OP(O)RxRx, -OP(O)(ORx)₂, or -P(O)₂RxRx;
the C₁₋₆ alkyl moiety has or does not have 1 to 3 substituents selected from halogen; the C₃₋₁₄ cycloalkyl moiety, the 4- to 14-membered heterocycloalkyl moiety, the C₆₋₁₀ aryl moiety and the 5- to 14-membered heteroaryl moiety each independently include a monocyclic or polycyclic ring, and have or do not have 1 to 3 substituents selected from the group consisting of halogen, OH, CN, NO₂, oxo, C₁₋₆ alkylamido, C₁₋₆ alkoxycarbonyl, C₁₋₆ alkyl, haloC₁₋₆ alkyl, C₁₋₆ alkoxy, and haloC₁₋₆ alkoxy; and
the C₃₋₁₄ cycloalkyl moiety and the 4- to 14-membered heterocycloalkyl moiety are each independently saturated or unsaturated within a range having no aromaticity.

2. The compound according to claim 1, a stereoisomer, or a pharmaceutically acceptable salt thereof, wherein
Sp is 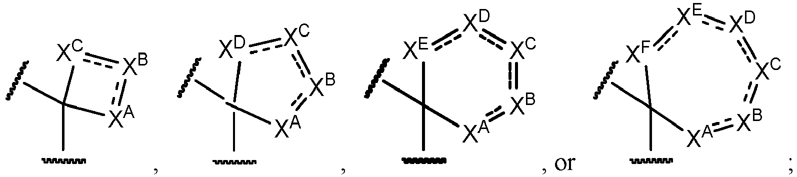
X^{A} to X^{F} are each independently an atom selected from the group consisting of C, O, N, and S, wherein the C atom has or does not have a substituent selected from halogen, CN, NO₂, oxo, Rx, Ry, and Y¹, the N atom has or does not have a substituent selected from Rx, Ry, and Y¹, and the S atom has or does not have an oxo substituent;
Y¹'s are each independently H or OH, or two adjacent Y¹'s may be linked to form an unsaturated or saturated C₃₋₇ carbocycle or a 3- to 7-membered heterocycle, wherein the C₃₋₇ carbocycle and the 3- to 7-membered heterocycle each have or do not have 1 to 3 substituents selected from among halogen, CN, NO₂, OH, oxo, Rx and Ry;
-̅ -̅ -̅ -̅ -̅ is a single bond or a double bond which meets an electrovalence of atoms each independently linked thereto; and
Rx and Ry are each the same as defined in claim 1.

3. The compound according to claim 1, a stereoisomer, or a pharmaceutically acceptable salt thereof, wherein
Sp is selected from the group consisting of 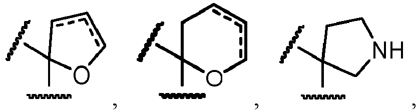 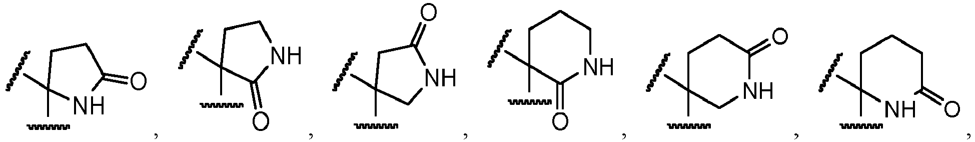 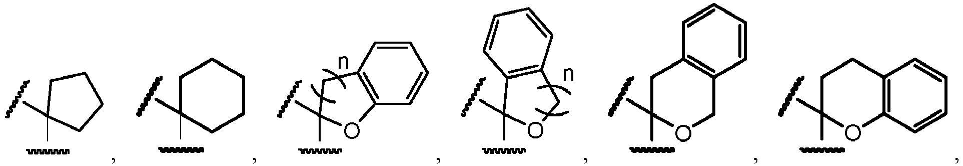 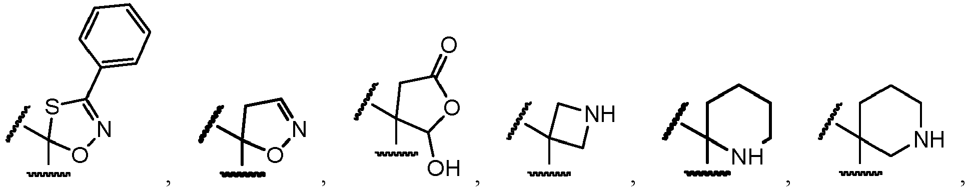 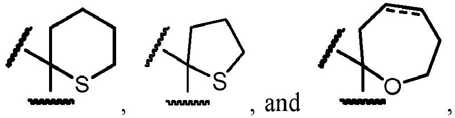 Sp has or does not have one to three substituents selected from among halogen, CN, NO₂, OH, Rx, and Ry;
-̅ -̅ -̅ -̅ -̅ is a single bond or a double bond which meets an electrovalence of atoms each independently linked thereto;
n is 1 or 2; and
Rx and Ry are each the same as defined in claim 1.

4. The compound according to claim 3, a stereoisomer, or a pharmaceutically acceptable salt thereof, wherein
Rx's substituted at Sp are each independently C₁₋₆ alkyl, C₆₋₁₀ aryl, 5- to 14-membered heteroaryl, (4- to 14-membered heterocycloalkyl)-C₁₋₆ alkyl-, C₆₋₁₀ aryl-C₁₋₆ alkyl-, C₆₋₁₀ aryl-(5-to 14-membered heteroaryl)-, or (5- to 14-membered heteroaryl)-C₁₋₆ alkyl-;
Ry's substituted at Sp are each independently -ORx, -C(O)Rx, -NRxRx, -N=CRxRx, - NRxC(O)NRxRx, or -NRxC(O)Rx, wherein Rx's contained in Ry's are each independently H, C₁₋₆ alkyl, C₃₋₁₄ cycloalkyl, 4- to 14-membered heterocycloalkyl, C₆₋₁₀ aryl, 5- to 14-membered heteroaryl, C₃₋₁₄ cycloalkyl-C₁₋₆ alkyl-, (4- to 14-membered heterocycloalkyl)-C₁₋₆ alkyl-, C₆₋₁₀ aryl-C₁₋₆ alkyl-, C₆₋₁₀ aryl-C₃₋₆ cycloalkyl-, (5- to 14-membered heteroaryl)-C₁₋₆ alkyl-, C₆₋₁₀ aryl-C₆₋₁₀ aryl-, C₆₋₁₀ aryl-C₆₋₁₀ aryl-C₁₋₆ alkyl-, or C₆₋₁₀ aryloxy-C₆₋₁₀ aryl-; and
the C₁₋₆ alkyl moiety has or does not have 1 to 3 substituents selected from halogen; the C₃₋₁₄ cycloalkyl moiety, the 4- to 14-membered heterocycloalkyl moiety, the C₆₋₁₀ aryl moiety, and the 5- to 14-membered heteroaryl moiety each independently include a monocyclic or polycyclic ring, and have or do not have 1 to 3 substituents selected from the group consisting of halogen, OH, oxo, C₁₋₆ alkyl, haloC₁₋₆ alkyl, C₁₋₆ alkoxy, and haloC₁₋₆ alkoxy; and
the C₃₋₁₄ cycloalkyl moiety and the 4- to 14-membered heterocycloalkyl moiety are each independently saturated or unsaturated within a range having no aromaticity.

5. The compound according to claim 1, a stereoisomer, or a pharmaceutically acceptable salt thereof, wherein
A¹ is -ORx, -OC(O)Rx, -OC(RxRx)-C(O)Rx, -OC(RxRx)-C(O)ORx, -OC(O)NRxRx, - OC(RxRx)-C(O)NRxRx, -OS(O)₂Rx, or -OP(O)(ORx)₂; and
Rx's are each independently the same as defined in claim 1.

6. The compound according to claim 5, a stereoisomer, or a pharmaceutically acceptable salt thereof, wherein
Rx's included in A¹ are each independently H, C₁₋₆ alkyl, halo C₁₋₆ alkyl, 4- to 14-membered heterocycloalkyl, C₆₋₁₀ aryl, 5- to 14-membered heteroaryl, (4- to 14-membered heterocycloalkyl)-C₁₋₆ alkyl-, C₆₋₁₀ aryl-C₁₋₆ alkyl-, (5- to 14-membered heteroaryl)-C₁₋₆ alkyl-, or (5- to 14-membered heteroaryl)-C₆₋₁₀ aryl-C₁₋₆ alkyl-;
the C₁₋₆ alkyl moiety has or does not have 1 to 3 substituents selected from halogen; the C₃₋₁₄ cycloalkyl moiety, the 4- to 14-membered heterocycloalkyl moiety, the C₆₋₁₀ aryl moiety, and the 5- to 14-membered heteroaryl moiety each independently include a monocyclic or polycyclic ring, and have or do not have 1 to 3 substituents selected from the group consisting of halogen, OH, oxo, C₁₋₆ alkyl, haloC₁₋₆ alkyl, C₁₋₆ alkoxy, and haloC₁₋₆ alkoxy; and
the C₃₋₁₄ cycloalkyl moiety and the 4- to 14-membered heterocycloalkyl moiety are each independently saturated or unsaturated within a range having no aromaticity.

7. The compound according to claim 1, a stereoisomer, or a pharmaceutically acceptable salt thereof, wherein
B¹ is 5- to 14-membered heteroaryl containing one or more N atoms, 4- to 14-membered heterocycloalkyl containing one or more N atoms, C₆₋₁₀ aryl-(5- to 14-membered heteroaryl)-, C₆₋₁₀ aryl, (5- to 14-membered heteroaryl)-C₁₋₆ alkyloxy-, (4- to 14-membered heterocycloalkyl)-C₁₋₆ alkyloxy-, C₆₋₁₀ aryl-C₁₋₆ alkyloxy-, (5- to 14-membered heteroaryl)-amido-, (4- to 14-membered heterocycloalkyl)-amido-, C₆₋₁₀ aryl-C₁₋₆ amido-, (5- to 14-membered heteroaryl)-C₁₋₆ alkylamido-, (4- to 14-membered heterocycloalkyl)-C₁₋₆ alkylamido-, or C₆₋₁₀ aryl-C₁₋₆ alkylamido-, wherein with regard to the 5- to 14-membered heteroaryl and the 4- to 14-membered heterocycloalkyl containing one or more N atoms, any one among the N atoms is linked to the galactoside ring;
the 5- to 14-membered heteroaryl moiety, the 4- to 14-membered heterocycloalkyl moiety, and the C₆₋₁₀ aryl moiety in B¹ each independently include a monocyclic or polycyclic ring, and have or do not have 1 to 5 substituents selected from among halogen, OH, CF₃, CN, NO₂, oxo, and C₁₋₆ alkoxy; and
the 4- to 14-membered heterocycloalkyl moieties are each independently saturated or unsaturated within a range having no aromaticity.

8. The compound according to claim 7, a stereoisomer, or a pharmaceutically acceptable salt thereof, wherein
the 5- to 14-membered heteroaryl moiety in B¹ is imidazolyl, pyrazolyl, triazolyl, or chromenonyl.

9. The compound according to claim 1, a stereoisomer, or a pharmaceutically acceptable salt thereof, wherein
R¹ and R² are each independently H, carbamate, ether, phosphate, sulfate, oxyalkylphosphate, oxyalkyl sulfate, carbonate, amide, ester, *N*-acylsulfonamide, sulfonamide, imine, acyloxyalkylamine, phosphate, phsphoroimidate, carbonyloxymethyl, acetylthioethanol, dithioethanol, alkoxyalkylmonoester, or acetyl.

10. The compound according to claim 1, a stereoisomer, or a pharmaceutically acceptable salt thereof, wherein the compound represented by Formula (I) is selected from the group consisting of:
1) 3-((((2*R*,3'R,4'S,5'S,6'R)-3',5'-dihydroxy-6'-(hydroxymethyl)-6-methoxy-3',4',5',6'-tetrahydrospiro[chromane-2,2'-pyran]-4'-yl)oxy)methyl)-5,6-difluoro-2*H*-chromen-2-one;
2) 3-((((5*S*,7*R*,8*S*,9*S*,10*R*)-8,10-dihydroxy-7-(hydroxymethyl)-1,6-dioxaspiro[4.5]decan-9-yl)oxy)methyl)-5,6-difluoro-2*H*-chromen-2-one;
3) (3'*R*,4'*S*,5'*R*,6'*R*)-6'-(hydroxymethyl)-6-methoxy-4'-(4-(3,4,5-trifluorophenyl)-1*H-*1,2,3-triazol-1-yl)-3',4',5',6'-tetrahydrospiro[chromane-2,2'-pyran]-3',5'-diol;
4) (5*S*,7*R*,8*R*,9*S*,10*R*)-7-(hydroxymethyl)-9-(4-(3,4,5-trifluorophenyl)-1*H*-1,2,3-triazol-1-yl)-1,6-dioxaspiro[4.5]decane-8,10-diol;
5) (5*S*,7*R*,8*R*,9*S*,10*R*)-10-(benzyloxy)-7-(hydroxymethyl)-9-(4-(3,4,5-trifluorophenyl)-1*H*-1,2,3-triazol-1-yl)-1,6-dioxaspiro[4.5]decan-8-ol;
6) (5*S*,7*R*,8*R*,9*S*,10*R*)-7-(hydroxymethyl)-10-(pyridin-3-ylmethoxy)-9-(4-(3,4,5-trifluorophenyl)-1*H*-1,2,3-triazol-1-yl)-1,6-dioxaspiro[4.5]decan-8-ol;
7) (5*S*,7*R*,8*R*,9*S*,10*R*)-7-(hydroxymethyl)-10-(pyridin-2-ylmethoxy)-9-(4-(3,4,5-trifluorophenyl)-1*H*-1,2,3-triazol-1-yl)-1,6-dioxaspiro[4.5]decan-8-ol;
8) (5*S*,7*R*,8*R*,9*S*,10*R*)-7-(hydroxymethyl)-10-(pyridin-4-ylmethoxy)-9-(4-(3,4,5-trifluorophenyl)-1*H*-1,2,3-triazol-1-yl)-1,6-dioxaspiro[4.5]decan-8-ol;
9) (5*S*,7*R*,8*R*,9*S*,10*R*)-7-(hydroxymethyl)-10-((5-(trifluoromethyl)furan-2-yl)methoxy)-9-(4-(3,4,5-trifluorophenyl)-1*H*-1,2,3-triazol-1-yl)-1,6-dioxaspiro[4.5]decane-8-ol;
10) 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-1-phenylethanone;
11) 2-(((5*S*,7*R*,8*R*,9*S*,10*S*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)acetic acid;
12) 2-(((5*S*,7*R*,8*R*,9*S*,10*S*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-1-(4-hydroxypiperidin1-yl)ethanone;
13) 2-(((5*S*,7*R*,85,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-*N-*phenylacetamide;
14) (2*R*,3*R*,4*S*,5*R*,6*S*)-2-(hydroxymethyl)-4-(4-(3,4,5-trifluorophenyl)-1*H*-1,2,3-triazol-1-yl)-1,7-dioxaspiro[5.5]undecane-3,5-diol;
15) (2*R*,3*R*,4*S*,5*R*,6*S*)-5-(benzyloxy)-2-(hydroxymethyl)-4-(4-(3,4,5-trifluorophenyl)-1*H*-1,2,3-triazol-1-yl)-1,7-dioxaspiro[5.5]undecan-3-ol;
16) 4-((((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)methyl)tetrahydro-2H-thiopyran 1,1-dioxide;
17) (5*S*,7*R*,8*R*,9*S*,10*R*)-10-(2,2-difluoroethoxy)-7-(hydroxymethyl)-9-(4-(3,4,5-trifluorophenyl)-1*H*-1,2,3-triazol-1-yl)-1,6-dioxaspiro[4.5]decan-8-ol;
18) (5*S*,7*R*,8*R*,9*S*,10*R*)-7-(hydroxymethyl)-10-((4-(2-methyl-2*H*-tetrazol-5-yl)benzyl)oxy)-9-(4-(3,4,5-trifluorophenyl)-1*H-*1,2,3-triazol-1-yl)-1,6-dioxaspiro[4.5]decan-8-ol;
19) *N*-(6-chloropyridazin-3-yl)-2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)acetamide;
20) (4*S*,5*S*,7*R*,8*R*,9*S*,10*R*)-7-(hydroxymethyl)-4-phenyl-9-(4-(3,4,5-trifluorophenyl)-1*H*-1,2,3-triazol-1-yl)-1,6-dioxaspiro[4.5]decane-8,10-diol;
21) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl benzoate;
22) methyl-2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)acetate;
23) 1-(3,3-difluoroazetidin-1-yl)-2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)ethanone;
24) 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)-1-(3-hydroxyazetidin-1-yl)ethanone;
25) (5*S*,7*R*,8*R*,9*S*,10*R*)-10-(benzo[*d*][1,3]dioxol-5-ylmethoxy)-7-(hydroxymethyl)-9-(4-(3,4,5-trifluorophenyl)-1*H*-1,2,3-triazol-1-yl)-1,6-dioxaspiro[4.5]decan-8-ol;
26) (5*S*,7*R*,8*R*,9*S*,10*R*)-10-((2*H*-tetrazol-5-yl)methoxy)-7-(hydroxymethyl)-9-(4-(3,4,5-trifluorophenyl)-1*H*-1,2,3-triazol-1-yl)-1,6-dioxaspiro[4.5]decan-8-ol;
27) (5*S*,7*R*,8*R*,9*S*,10*R*)-10-((2-hydroxybenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
28) (5*S*,7*R*,8*R*,9*S*,10*R*)-10-((3-hydroxybenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
29) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3-fluorobenzoate;
30) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3-methoxybenzoate;
31) (5*S*,7*R*,8*R*,9*S*,10*S*)-7-(hydroxymethyl)-9-(4-(3,4,5-trifluorophenyl)-1*H*-1,2,3-triazol-1-yl)-1,6-dioxaspiro[4.5]decane-8,10-diol;
32) (5*S*,7*R*,8*R*,9*S*,10*S*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl benzoate;
33) (2*R*,3*R*,4*S*,5*R*,6*S*)-5-(benzo[*d*][1,3]dioxol-5-ylmethoxy)-2-(hydroxymethyl)-4-(4-(3,4,5-trifluorophenyl)-1*H*-1,2,3-triazol-1-yl)-1,7-dioxaspiro[5.5]undecan-3-ol;
34) (2*R*,3*R*,4*S*,5*R*,6*S*)-5-((2H-tetrazol-5-yl)methoxy)-2-(hydroxymethyl)-4-(4-(3,4,5-trifluorophenyl)-1*H*-1,2,3-triazol-1-yl)-1,7-dioxaspiro[5.5]undecan-3-ol;
35) (2*R*,3*R*,4*S*,5*R*,6*S*)-5-(2,2-difluoroethoxy)-2-(hydroxymethyl)-4-(4-(3,4,5-trifluorophenyl)-1*H*-1,2,3-triazol-1-yl)-1,7-dioxaspiro[5.5]undecan-3-ol;
36) (2*R*,3*R*,4*S*,5*R*,6*S*)-5-((2-hydroxybenzyl)oxy)-2-(hydroxymethyl)-4-(4-(3,4,5-trifluorophenyl)-1*H*-1,2,3-triazol-1-yl)-1,7-dioxaspiro[5.5]undecan-3-ol;
37) (2*R*,3*R*,4*S*,5*R*,6*S*)-5-((3-fluorobenzyl)oxy)-2-(hydroxymethyl)-4-(4-(3,4,5-trifluorophenyl)-1*H*-1,2,3-triazol-1-yl)-1,7-dioxaspiro[5.5]undecan-3-ol;
38) (2*R*,3*R*,4*S*,5*R*,6*S*)-2-(hydroxymethyl)-5-((3-(trifluoromethyl)benzyl)oxy)-4-(4-(3,4,5-trifluorophenyl)-1*H*-1,2,3-triazol-1-yl)-1,7-dioxaspiro[5.5]undecan-3-ol;
39) (2*R*,3*R*,4*S*,5*R*,6*S*)-2-(hydroxymethyl)-5-(pyridin-2-ylmethoxy)-4-(4-(3,4,5-trifluorophenyl)-1*H*-1,2,3-triazol-1-yl)-1,7-dioxaspiro[5.5]undecan-3-ol;
40) (2*R*,3*R*,4*S*,5*R*,6*S*)-3-hydroxy-2-(hydroxymethyl)-4-(4-(3,4,5-trifluorophenyl)-1H-1,2,3-triazol-1-yl)-1,7-dioxaspiro[5.5]undecan-5-yl 3-fluorobenzoate;
41) (2*R*,3*R*,4*S*,5*R*,6*S*)-3-hydroxy-2-(hydroxymethyl)-4-(4-(3,4,5-trifluorophenyl)-1*H-*1,2,3-triazol-1-yl)-1,7-dioxaspiro[5.5]undecan-5-yl 3-(trifluoromethyl)benzoate;
42) (2*R*,3*R*,4*S*,5*R*,6*S*)-3-hydroxy-2-(hydroxymethyl)-4-(4-(3,4,5-trifluorophenyl)-1*H-*1,2,3-triazol-1-yl)-1,7-dioxaspiro[5.5]undecan-5-yl 3-methoxybenzoate;
43) 2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)acetic acid;
44) 2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)propanoic acid;
45) (4*R*,5*S*,7*R*,8*R*,9*S*,10*R*)-4-amino-7-(hydroxymethyl)-9-(4-(3,4,5-trifluorophenyl)-1*H*-1,2,3-triazol-1-yl)-1,6-dioxaspiro[4.5]decane-8,10-diol;
46) (2*R*,3*R*,4*S*,5*R*,6*S*)-3-hydroxy-2-(hydroxymethyl)-4-(4-(3,4,5-trifluorophenyl)-1*H-*1,2,3-triazol-1-yl)-1,7-dioxaspiro[5.5]undecan-5-yl picolinate;
47) (2*R*,3*R*,4*S*,5*R*,6*S*)-2-(hydroxymethyl)-5-(pyridin-3-ylmethoxy)-4-(4-(3,4,5-trifluorophenyl)-1*H*-1,2,3-triazol-1-yl)-1,7-dioxaspiro[5.5]undecan-3-ol;
48) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 4-methylbenzenesulfonate;
49) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-fluorobenzamide;
50) (2*R*,3*R*,4*S*,5*R*,6*S*)-3-hydroxy-2-(hydroxymethyl)-4-(4-(3,4,5-trifluorophenyl)-1*H-*1,2,3-triazol-1-yl)-1,7-dioxaspiro[5.5]undecan-5-yl nicotinate;
51) (4*R*,5*S*,7*R*,8*R*,9*S*,10*R*)-4-((3-fluorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
52) (4*R*,5*S*,7*R*,8*R*,9*S*,10*R*)-4-((2,3-difluoro-6-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
53) (5*S*,7*R*,8*R*,9*S*,10*R*)-10-((3-chlorobenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
54) (5*S*,7*R*,8*R*,9*S*,10*R*)-10-((2,3-difluorobenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
55) 2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)propanoic acid;
56) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 2-fluorobenzoate;
57) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 2-chlorobenzoate;
58) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 2,3-difluorobenzoate;
59) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl picolinate;
60) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3-(trifluoromethyl)benzoate;
61) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3-(trifluoromethyl)benzoate;
62) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl nicotinate;
63) (2*R*,3*R*,4*S*,5*R*,6*S*)-3-hydroxy-2-(hydroxymethyl)-4-(4-(3,4,5-trifluorophenyl)-1*H-*1,2,3-triazol-1-yl)-1,7-dioxaspiro[5.5]undecan-5-yl dihydrogen phosphate;
64) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3-fluoropicolinate;
65) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 2,4-difluorobenzoate;
66) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 4-fluorobenzoate;
67) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl isonicotinate;
68) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3-fluoropicolinate;
69) (*R*)-2-(((5*S*,7*R*,8*R*,9*S*,10*R*)-8-hydroxy-7-(hydroxymethyl)-9-(4-(3,4,5-trifluorophenyl)-1*H*-1,2,3-triazol-1-yl)-1,6-dioxaspiro[4.5]decan-10-yl)oxy)propanoic acid;
70) (*R*)-2-(((2*R*,3*R*,4*S*,5*R*,6*S*)-3-hydroxy-2-(hydroxymethyl)-4-(4-(3,4,5-trifluorophenyl)-1*H*-1,2,3-triazol-1-yl)-1,7-dioxaspiro[5.5]undecan-5-yl)oxy)propanoic acid;
71) (5*S*,7*R*,8*R*,9*S*,10*R*)-10-((5-fluoropyridin-3-yl)methoxy)-7-(hydroxymethyl)-9-(4-(3,4,5-trifluorophenyl)-1*H*-1,2,3-triazol-1-yl)-1,6-dioxaspiro[4.5]decan-8-ol;
72) (4*R*,5*S*,7*R*,8*R*,9*S*,10*R*)-7-(hydroxymethyl)-4-((pyridin-3-ylmethyl)amino)-9-(4-(3,4,5-trifluorophenyl)-1*H*-1,2,3-triazol-1-yl)-1,6-dioxaspiro[4.5]decane-8,10-diol;
73) (4*R*,5*S*,7*R*,8*R*,9*S*,10*R*)-4-((3-chlorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
74) (4*R*,5*S*,7*R*,8*R*,9*S*,10*R*)-4-((2-fluorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
75) (2*R*,3*R*,4*S*,5*R*)-3,5-dihydroxy-2-(hydroxymethyl)-4-(4-(3,4,5-trifluorophenyl)-1*H-*1,2,3-triazol-1-yl)-1-oxa-7-azaspiro[5.5]undecan-8-one;
76) (5*S*,7*R*,8*R*,9*S*,10*R*)-7-(hydroxymethyl)-10-((3-(trifluoromethyl)benzyl)oxy)-9-(4-(3,4,5-trifluorophenyl)-1*H*-1,2,3-triazol-1-yl)-1,6-dioxaspiro[4.5]decan-8-ol;
77) (5*S*,7*R*,8*R*,9*S*,10*R*)-10-((3-fluorobenzyl)oxy)-7-(hydroxymethyl)-9-(4-(3,4,5-trifluorophenyl)-1*H*-1,2,3-triazol-1-yl)-1,6-dioxaspiro[4.5]decan-8-ol;
78) (5*S*,7*R*,8*R*,9*S*,10*R*)-8-hydroxy-7-(hydroxymethyl)-9-(4-(3,4,5-trifluorophenyl)-1*H-*1,2,3-triazol-1-yl)-1,6-dioxaspiro[4.5]decan-10-yl 3,5-difluorobenzoate;
79) (2*R*,3*R*,4*S*,5*R*)-5-(benzyloxy)-3-hydroxy-2-(hydroxymethyl)-4-(4-(3,4,5-trifluorophenyl)-1*H*-1,2,3-triazol-1-yl)-1-oxa-7-azaspiro[5.5]undecan-8-one;
80) (2*R*,3*R*,4*S*,S*R*)-7-(2,2-difluoroethyl)-3,5-dihydroxy-2-(hydroxymethyl)-4-(4-(3,4,5-trifluorophenyl)-1*H*-1,2,3-triazol-1-yl)-1-oxa-7-azaspiro[5.5]undecan-8-one;
81) (2*R*,3*R*,4*S*,5*R*)-7-((1,1-dioxidotetrahydro-2*H*-thiopyran-4-yl)methyl)-3,5-dihydroxy-2-(hydroxymethyl)-4-(4-(3,4,5-trifluorophenyl)-1*H*-1,2,3-triazol-1-yl)-1-oxa-7-azaspiro[5.5]undecan-8-one;
82) (4*R*,5*S*,7*R*,8*R*,9*S*,10*R*)-7-(hydroxymethyl)-4-((3-(trifluoromethyl)benzyl)amino)-9-(4-(3,4,5-trifluorophenyl)-1*H*-1,2,3-triazol-1-yl)-1,6-dioxaspiro[4.5]decane-8,10-diol;
83) (4*R*,5*S*,7*R*,8*R*,9*S*,10*R*)-4-((4-chlorobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
84) (4*R*,5*S*,7*R*,8*R*,9*S*,10*R*)-7-(hydroxymethyl)-4-((pyridin-2-ylmethyl)amino)-9-(4-(3,4,5-trifluorophenyl)-1*H*-1,2,3-triazol-1-yl)-1,6-dioxaspiro[4.5]decane-8,10-diol;
85) (4*R*,5*S*,7*R*,8*R*,9*S*,10*R*)-7-(hydroxymethyl)-4-((pyridin-4-ylmethyl)amino)-9-(4-(3,4,5-trifluorophenyl)-1*H*-1,2,3-triazol-1-yl)-1,6-dioxaspiro[4.5]decane-8,10-diol;
86) (5*R*,7*R*,8*R*,9*S*,10*R*)-7-(hydroxymethyl)-9-(4-(3,4,5-trifluorophenyl)-1*H*-1,2,3-triazol-1-yl)-1,6-dioxaspiro[4.5]decane-8,10-diol;
87) (7*R*,8*R*,9*S*,10*R*)-7-(hydroxymethyl)-9-(4-(3,4,5-trifluorophenyl)-1*H*-1,2,3-triazol-1-yl)-6-oxaspiro[4.5]decane-8,10-diol;
88) (4*R*,5*S*,7*R*,8*R*,9*S*,10*R*)-4-((3-bromobenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
89) (4*R*,5*S*,7*R*,8*R*,9*S*,10*R*)-4-((3-chlorobenzyl)(methyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
90) (4*R*,5*S*,7*R*,8*R*,9*S*,10*R*)-4-((cyclopentylmethyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
91) (4*R*,5*S*,7*R*,8*R*,9*S*,10*R*)-4-(benzylamino)-7-(hydroxymethyl)-9-(4-(3,4,5-trifluorophenyl)-1*H*-1,2,3-triazol-1-yl)-1,6-dioxaspiro[4.5]decane-8,10-diol;
92) 2-(3-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
93) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)cyclopentanecarboxamide;
94) 2-cyclopentyl-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
95) (2*R*,3*R*,4*S*,5*R*,6*R*)-2-(hydroxymethyl)-4-(4-(3,4,5-trifluorophenyl)-1*H*-1,2,3-tiiazol-1-yl)-1-oxa-7-thiaspiro[5.5]undecane-3,5-diol;
96) (2*R*,3*R*,4*S*,5*R*,6*R*)-2-(hydroxymethyl)-4-(4-(3,4,5-trifluorophenyl)-1*H*-1,2,3-triazol-1-yl)-1-oxa-8-azaspiro[5.5]undecane-3,5-diol;
97) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(naphthalen-1-yl)acetamide;
98) 2-(2-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
99) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-fluorophenyl)acetamide;
100) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-phenylcyclopropanecarboxamide;
101) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(naphthalen-2-yl)acetamide;
102) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-methoxyphenyl)acetamide;
103) 2-(4-chlorophenyl)-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
104) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-methoxybenzamide;
105) 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzamide;
106) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-phenoxybenzamide;
107) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-2-carboxamide;
108) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-(trifluoromethoxy)benzamide;
109) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-2-carboxamide;
110) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-2-carboxamide;
111) (4*R*,5*S*,7*R*,8*R*,9*S*,10*R*)-4-((2-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
112) (4*R*,5*S*,7*R*,8*R*,9*S*,10*R*)-4-((5-chloro-2-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
113) (4*R*,5*S*,7*R*,8*R*,9*S*,10*R*)-4-((3-chloro-2-hydroxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
114) (4*R*,5*S*,7*R*,8*R*,9*S*,10*R*)-4-(((5-chlorofuran-2-yl)methyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
115) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
116) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-phenoxybenzamide;
117) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-4-carboxamide;
118) (4*R*,5*S*,7*R*,8*R*,9*S*,10*R*)-4-((2-hydroxy-3-methoxybenzyl)amino)-7-(hydroxymethyl)-9-(4-(3,4,5-trifluorophenyl)-1*H*-1,2,3-triazol-1-yl)-1,6-dioxaspiro[4.5]decane-8,10-diol;
119) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
120) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-3-carboxamide;
121) 2-([1,1'-biphenyl]-4-yl)-N-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
122) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-(trifluoromethyl)phenyl)acetamide;
123) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-phenylpropanamide;
124) ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(phenyl)methanone;
125) 2-(3-chlorophenyl)-1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)ethanone;
126) 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-(3-methoxyphenyl)ethanone;
127) (2*R*,3*R*,4*S*,S*R*,6*R*)-8-benzyl-2-(hydroxymethyl)-4-(4-(3,4,5-trifluorophenyl)-1H-1,2,3-triazol-1-yl)-1-oxa-8-azaspiro[5.5]undecane-3,5-diol;
128) (2*R*,3*R*,4*S*,5*R*,6*R*)-2-(hydroxymethyl)-8-(pyridin-3-ylmethyl)-4-(4-(3,4,5-trifluorophenyl)-1*H*-1,2,3-triazol-1-yl)-1-oxa-8-azaspiro[5.5]undecane-3,5-diol;
129) (2*R*,3*R*,4*S*,5*R*,6*R*)-8-(3-chlorobenzyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
130) ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(pyridin-3-yl)methanone;
131) (3-chlorophenyl)((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)methanone;
132) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-phenylacetamide;
133) *N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2,2-difluoro-2-phenylacetamide;
134) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(4-phenoxyphenyl)acetamide;
135) 6-chloro-*N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
136) 3-bromo-N-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzamide;
137) 2-(3-bromophenyl)-*N*-((4-R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)acetamide;
138) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-(trifluoromethyl)benzamide;
139) (4*R*,5*S*,7*R*,8*R*,9*S*,10*R*)-7-(hydroxymethyl)-4-(1*H*-1,2,3-triazol-1-yl)-9-(4-(3,4,5-trifluorophenyl)-1*H*-1,2,3-triazol-1-yl)-1,6-dioxaspiro[4.5]decane-8,10-diol;
140) (4*R*,5*S*,7*R*,8*R*,9*S*,10*R*)-7-(hydroxymethyl)-4-(4-phenyl-1H-1,2,3-triazol-1-yl)-9-(4-(3,4,5-trifluorophenyl)-1*H*-1,2,3-triazol-1-yl)-1,6-dioxaspiro[4.5]decane-8,10-diol;
141) (4*R*,5*S*,7*R*,8*R*,9*S*,10*R*)-7-(hydroxymethyl)-4-((3-methoxybenzyl)amino)-9-(4-(3,4,5-trifluorophenyl)-1*H*-1,2,3-triazol-1-yl)-1,6-dioxaspiro[4.5]decane-8,10-diol;
142) 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-2-carboxamide;
143) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-3-carboxamide;
144) *N*-((4R,5S,7R,8R,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-5,6,7,8-tetrahydronaphthalene-1-carboxamide;
145) 7-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-2-carboxamide;
146) *N*-((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1,2,3,4-tetrahydronaphthalene-1-carboxamide;
147) *N*-((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-3-carboxamide;
148) *N*-((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2,3-dihydro-1H-indene-4-carboxamide;
149) *N*-((4*R*,5*S*,7*R*,8*R*,9*S*,10*R*)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)-2,3-dihydro-1*H*-indene-1-carboxamide;
150) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2'-fluoro-[1,1'-biphenyl]-3-carboxamide;
151) *N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3'-fluoro-[1,1'-biphenyl]-3-carboxamide;
152) 3'-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-3-carboxamide;
153) 2-(3-chlorophenyl)-*N*-((4*R*,5*S*,7*R*,8*R*,9*S*,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)propanamide;
154) 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
155) *N*-((4R,5S,7R,8R,9S, 10R)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1-naphthamide;
156) *N-*((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-phenoxybenzamide;
157) 4-chloro-*N*-((4R,5S,7R,8R,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
158) 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
159) (2*R*,3*R*,4*S*,5*R*,6*R*)-8-(3-chlorobenzyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
160) (2*R*,3*R*,4*S*,5*R*,6*R*)-8-(3-chlorobenzyl)-2-(hydroxymethyl)-4-(4-(3,4, 5-trifluorophenyl)-1*H*-1,2,3-triazol-1-yl)-1-oxa-8-azaspiro[5.5]undecane-3,5-diol;
161) 4-bromo-*N*-(*(4R,5S,*7*R,8R,9S,10R)*-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
162) 5-chloro-N-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-3-carboxamide;
163) 3-bromo-*N*-((4R,5S,7R,8R,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
164) 2'-bromo-*N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-[1,1'-biphenyl]-3-carboxamide;
165) 5-bromo-*N*-((4R,5S,7R,8R,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-3-carboxamide;
166) 3-bromo-*N-*((4A,55,7A,8A,95,10A)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-2-carboxamide;
167) *N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*d*]isothiazole-3-carboxamide;
168) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-(3-phenoxyphenyl)acetamide;
169) 1-((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)-2-phenylethanone;
170) ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(naphthalen-1-yl)methanone;
171) ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(naphthalen-2-yl)methanone;
172) benzo[b]thiophen-2-yl((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)methanone;
173) ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(pyridin-2-yl)methanone;
174) ((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)(pyridin-4-yl)methanone;
175) ((2*R*,3*R*,4*S*,5*R*,6*R*)-3,5-dihydroxy-2-(hydroxymethyl)-4-(4-(3,4,5-trifluorophenyl)-1H-1,2,3-triazol-1-yl)-1-oxa-8-azaspiro[5.5]undecan-8-yl)(thiazol-5-yl)methanone;
176) (2*R*,3*R*,4*S*,5*R*,6*R*)-2-(hydroxymethyl)-8-phenyl-4-(4-(3,4,5-trifluorophenyl)-1H-1,2,3-triazol-1-yl)-1-oxa-8-azaspiro[5.5]undecane-3,5-diol;
177) 5-bromo-*N*-((4R,5*S*,7*R*,8*R*,9S,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
178) 1-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
179) *N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[d]thiazole-4-carboxamide;
180) 5-chloro-N-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
181) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-5-carboxamide;
182) 6-bromo-*N*-((4*R*,5*S*,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*d*]isothiazole-3-carboxamide;
183) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[b]thiophene-4-carboxamide;
184) 6-chloro-*N*-((4R,5S,7R,8R,9S,10*R*)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)benzo[*d*]isoxazole-3-carboxamide;
185) N-((4*R*,5*S*,7*R*,8*R*,9*S*,10R)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)imidazo[2,1-b]thiazole-5-carboxamide;
186) 3-chloro-*N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-naphthamide;
187) (4*R*,5*S*,7*R*,8*R*,9*S*,10*R*)-7-(hydroxymethyl)-4-((Z)-((2-hydroxynaphthalen-1-yl)methylene)amino)-9-(4-(3,4, 5-trifluorophenyl)-1*H*-1,2,3-triazol-1-yl)-1, 6-dioxaspiro[4.5]decane-8,10-diol;
188) (4*R*,5*S*,7*R*,8*R*,9*S*,10*R*)-7-(hydroxymethyl)-4-((Z)-((1-hydroxynaphthalen-yl)methylene)amino)-9-(4-(3,4,5-trifluorophenyl)-1*H*-1,2,3-triazol-1-yl)-1,6-dioxaspiro[4.5]decane-8,10-diol;
189) (4*R*,5*S*,7*R*,8*R*,9*S*,10*R*)-4-(4-(2-chlorophenyl)-1H-1,2,3-triazol-1-yl)-7-(hydroxymethyl)-9-(4-(3,4,5-trifluorophenyl)-1*H*-1,2,3-triazol-1-yl)-1,6-dioxaspiro[4.5]decane-8,10-diol;
190) (4*R*,5*S*,7*R*,8*R*,9*S*,10*R*)-4-(4-(3-chlorophenyl)-1H-1,2,3-triazol-1-yl)-7-(hydroxymethyl)-9-(4-(3,4,5-trifluorophenyl)-1H-1,2,3-triazol-1-yl)-1,6-dioxaspiro[4.5]decane-8,10-diol;
191) (4R,5S,7R,8R,9S,10R)-7-(hydroxymethyl)-4-(phenylamino)-9-(4-(3,4,5-trifluorophenyl)-1*H*-1,2,3-triazol-1-yl)-1,6-dioxaspiro[4.5]decane-8,10-diol;
192) N-((4*R*,5*S*,7*R*,8*R*,9*S*,10R)-8, 10-dihydroxy-7-(hydroxymethyl)-9-(4-(3,4,5-trifluorophenyl)-1H-1,2,3-triazol-1-yl)-1,6-dioxaspiro[4.5]decan-4-yl)quinoline-4-carboxamide;
193) 3-bromo-N-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
194) 6-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-4-carboxamide;
195) *N*-((4*R*,5*S*,7*R*,8*R*,9*S*,10*R*)-10-((2H-tetrazol-5-yl)methoxy)-8-hydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1-naphthamide;
196) 7-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide;
197) *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
198) 8-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
199) (5*S*,7*R*,8*R*,9*S*,10*R*)-7-(hydroxymethyl)-3-methyl-9-(4-(3,4,5-trifluorophenyl)-1H-1,2,3-triazol-1-yl)-1,6-dioxaspiro[4.5]decane-8,10-diol;
200) 1-(3-chlorophenyl)-3-((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)urea;
201) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-fluoroquinoline-5-carboxamide;
202) 3-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-5-carboxamide;
203) *N*-((4R,5S,7R,8R,9S,10R)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylbenzofuran-3-carboxamide;
204) 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1*H*-indole-3-carboxamide;
205) (2*R*,3*R*,4*S*,5*R*,6*R*)-2-(hydroxymethyl)-8-(naphthalen-1-yl)-4-(4-(3,4,5-trifluorophenyl)-1*H*-1,2,3-triazol-1-yl)-1-oxa-8-azaspiro[5.5]undecane-3,5-diol;
206) (2*R*,3*R*,4*S*,5*R*,6*R*)-2-(hydroxymethyl)-8-(naphthalen-2-yl)-4-(4-(3,4,5-trifluorophenyl)-1*H*-1,2,3-triazol-1-yl)-1-oxa-8-azaspiro[5.5]undecane-3,5-diol;
207) (2*R*,3*R*,4*S*,5*R*,6*R*)-8-(2-chlorophenyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
208) (2*R*,3*R*,4*S*,S*R*,6*R*)-8-(3-chlorophenyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
209) (2*R*,3*R*,4*S*,5*R*,6*R*)-8-(4-chlorophenyl)-2-(hydroxymethyl)-4-(4-(3,4,5-trifluorophenyl)-1*H*-1,2,3-triazol-1-yl)-1-oxa-8-azaspiro[5.5]undecane-3,5-diol;
210) (2*R*,3*R*,4*S*,5*R*,6*R*)-2-(hydroxymethyl)-8-(thiophen-2-yl)-4-(4-(3,4,5-trifluorophenyl)-1*H*-1,2,3-triazol-1-yl)-1-oxa-8-azaspiro[5.5]undecane-3,5-diol;
211) (2*R*,3*R*,4*S*,5*R*,6*R*)-2-(hydroxymethyl)-8-(pyrimidin-4-yl)-4-(4-(3,4, 5-trifluorophenyl)-1*H*-1,2,3-triazol-1-yl)-1-oxa-8-azaspiro[5.5]undecane-3,5-diol;
212) (4-chlorophenyl)((2*R*,3*R*,4*S*,5*R*,6*R*)-3,5-dihydroxy-2-(hydroxymethyl)-4-(4-(3,4,5-trifluorophenyl)-1*H*-1,2,3-triazol-1-yl)-1-oxa-8-azaspiro[5.5]undecan-8-yl)methanone;
213) *N*-((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-6-fluoro-2-methylquinoline-4-carboxamide;
214) 6-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylquinoline-4-carboxamide;
215) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-4-carboxamide;
216) 6-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylquinoline-4-carboxamide;
217) 4-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1*H*-indole-3-carboxamide;
218) 5-chloro-*N*-((4*R*,5*S*,7*R*,8*R*,9*S*,10*R*)-8,10-dihydroxy-7-(hydroxymethyl)-9-(4-(3,4,5-trifluorophenyl)-1H-1,2,3-triazol-1-yl)-1,6-dioxaspiro[4.5]decan-4-yl)-1-methyl-1*H-*indole-3-carboxamide;
219) *N*-((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methylbenzo[*b*]thiophene-3-carboxamide;
220) (5*S*,7*R*,8*R*,9*S*,10*R*)-8-hydroxy-7-(hydroxymethyl)-3-methyl-9-(4-(3,4,5-trifluorophenyl)-1*H*-1,2,3-triazol-1-yl)-1,6-dioxaspiro[4.5]decan-10-yl picolinate;
221) 2-(((5*S*,7*R*,8*R*,9*S*,10*R*)-8-hydroxy-7-(hydroxymethyl)-3-methyl-9-(4-(3,4,5-trifluorophenyl)-1*H*-1,2,3-triazol-1-yl)-1,6-dioxaspiro[4.5]decan-10-yl)oxy)acetic acid;
222) (5*S*,7*R*,8*R*,9*S*,10*R*)-7-(hydroxymethyl)-9-(4-(3,4,5-trifluorophenyl)-1H-pyrazol-1-yl)-1,6-dioxaspiro[4.5]decane-8,10-diol;
223) 7-chloro-*N*-((4*R*,5*S*,7*R*,8*R*,9*S*,10*R*)-8,10-dihydroxy-7-(hydroxymethyl)-9-(4-(3,4,5-trifluorophenyl)-1*H*-pyrazol-1-yl)-1,6-dioxaspiro[4.5]decan-4-yl)-1-naphthamide;
224) 6-chloro-N-((4*R*,5*S*,7*R*,8*R*,9*S*,10*R*)-8,10-dihydroxy-7-(hydroxymethyl)-9-(4-(3,4,5-trifluorophenyl)-1*H*-pyrazol-1-yl)-1,6-dioxaspiro[4.5]decan-4-yl)-2-methylquinoline-4-carboxamide;
225) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methyl-1-naphthamide;
226) *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-3-methoxy-1-naphthamide;
227) *N-*((4*R*,5*S*,7*R*,8*R*,9*S*,10*R*10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-methoxy-1-naphthamide;
228) *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-methyl-1-naphthamide;
229) *N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)quinoline-8-carboxamide;
230) *N-*((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-7-methylquinoline-5-carboxamide;
231) *N-*((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)isoquinoline-8-carboxamide;
232) *N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)isoquinoline-5-carboxamide;
233) 4-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-3-carboxamide;
234) *N-*((4*R*,5*S*,7*R*,8*R*,9*S*,10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-5-fluorobenzo[*b*]thiophene-3-carboxamide;
235) 4-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzofuran-3-carboxamide;
236) 5-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)benzo[*b*]thiophene-3-carboxamide;
237) 6-bromo-*N*-((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-oxo-1,2-dihydroquinoline-4-carboxamide;
238) 4-bromo-*N-*((4*R*,5*S*,7*R*,8*R*,9*S*,10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1*H*-indole-3-carboxamide;
239) *N-*((4*R*,5*S*,7*R*,8*R*,9*S*, 10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-2-oxo-1,2-dihydroquinoline-4-carboxamide;
240) *N-*((4*R*,5*S*,7*R*,8*R*,9*S*, 10*R*)-8, 10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-4-fluoro-1*H*-indole-3-carboxamide;
241) 7-chloro-*N*((4*R*,5*S*,7*R*,8*R*,9*S*, 10*R*)-8,10-dihydroxy-7-(hydroxymethyl)-9-(4-(3,4,5-trifluorophenyl)-1*H*-1,2,3-triazol-1-yl)-1,6-dioxaspiro[4.5]decan-4-yl)-1-naphthamide; and
242) (4*R*,5*S*,7*R*,8*R*,9*S*,10*R*)-7-(hydroxymethyl)-10-methoxy-9-(4-(3,4,5-trifluorophenyl)-1*H*-1,2,3-triazol-1-yl)-1,6-dioxaspiro[4.5]decan-8-ol.

11. A pharmaceutical composition for preventing or treating galectin-3 related diseases or disorders, the composition including the compound represented by Formula (I) according to any one of claims 1 to 10, a stereoisomer, or a pharmaceutically acceptable salt thereof as an active ingredient.

12. The pharmaceutical composition according to claim 11, wherin the galectin-3 related diseases or disorders are selected from the group consisting of inflammation; inflammation-induced thrombosis; atopic dermatitis; acute coronary syndrome; fibrosis; topical fibrosis; fibrotic complications; scleroderma; scars; keloid formation; covid-19; acute lung injury; acute respiratory distress syndrome; viral pneumonia; surgical adhesions; septic shock; cancer; transplant rejection; metastatic cancer; aging; bone disease; pulmonary hypertension; autoimmune diseases; viral infections; metabolic disorders; heart disease; heart failure; ocular diseases; atherosclerosis; metabolic diseases; diabetes; insulin resistance; obesity; Marfans syndrome; Loeys-Dietz syndrome; kidney diseases; fibrotic lung complications; interstitial lung diseases; liver diseases; and uterine diseases.

13. A pharmaceutical composition containing the compound represented by Formula (I) according to any one of claims 1 to 10, a stereisomer, or a pharmaceutically acceptable salt thereof, and a pharmaceutically acceptable carrier.

14. Use of the compound represented by Formula (I) according to any one of claims 1 to 10, a stereoisomer, or a pharmaceutically acceptable salt thereof for preventing or treating galectin-3 related diseases or disorders.

15. Use of the compound represented by Formula (I) according to any one of claims 1 to 10, the stereoisomer, or a pharmaceutically acceptable salt thereof for preparing a medicament for preventing or treating galectin-3 related diseases or disorders.

16. A method for preventing or treating galectin-3 related diseases or disorders, the method including administering, to a subject in need thereof, the compound represented by Formula (I) according to any one of claims 1 to 10, a stereoisomer, or a pharmaceutically acceptable salt thereof.

17. A method for inhibiting the activity of galectin-3, the method including a step of treating a specimen or cells with the compound of Formula (I) according to any one of claims 1 to 10, a stereoisomer, or a pharmaceutically acceptable salt thereof.

18. A galectin-3 inhibitor including the compound of Formula (I) according to any one of claims 1 to 10, a stereoisomer, or a pharmaceutically acceptable salt thereof as an active ingredient.
